(19) Europäisches Patentamt / European Patent Office / Office européen des brevets

(11) EP 2 851 426 B1

(12) **EUROPEAN PATENT SPECIFICATION**

(45) Date of publication and mention of the grant of the patent: **22.08.2018 Bulletin 2018/34**

(51) Int Cl.: ***C12N 15/113*** $^{(2010.01)}$ ***A61P 35/00*** $^{(2006.01)}$ ***C12N 9/02*** $^{(2006.01)}$

(21) Application number: **14197970.8**

(22) Date of filing: **27.09.2011**

# (54) Compositions and methods for inhibiting expression of RRM2 genes

Zusammensetzungen und Verfahren zur Hemmung der Expression von RRM2-Genen

Compositions et procédés d'inhibition de l'expression de gènes RRM2

(84) Designated Contracting States: **AL AT BE BG CH CY CZ DE DK EE ES FI FR GB GR HR HU IE IS IT LI LT LU LV MC MK MT NL NO PL PT RO RS SE SI SK SM TR**

(30) Priority: **18.10.2010 EP 10187851**

(43) Date of publication of application: **25.03.2015 Bulletin 2015/13**

(62) Document number(s) of the earlier application(s) in accordance with Art. 76 EPC: **11767402.8 / 2 630 240**

(73) Proprietor: **Arrowhead Pharmaceuticals, Inc. Pasadena, CA 91101 (US)**

(72) Inventors:
- **Boylan, John Frederick San Diego, CA 92128-4189 (US)**
- **Bramlage, Birgit 95326 Kulmbach (DE)**
- **Hossbach, Markus 95326 Kulmbach (DE)**
- **Reidhaar-Olson, John New York, NY 10019 (US)**

(74) Representative: **van Kooij, Adriaan et al Arnold & Siedsma Bezuidenhoutseweg 57 2594 AC The Hague (NL)**

(56) References cited:
**WO-A1-2006/017932 US-A1- 2008 227 967**

- **AVOLIO TINA M ET AL: "RNA interference targeting the R2 subunit of ribonucleotide reductase inhibits growth of tumor cells in vitro and in vivo", ANTI-CANCER DRUGS, LIPPINCOTT WILLIAMS & WILKINS, US; NL, vol. 18, no. 4, 1 April 2007 (2007-04-01), pages 377-388, XP009103016, ISSN: 0959-4973, DOI: 10.1097/CAD.0B013E328013C04F**
- **GLEN REID ET AL: "Potent subunit-specific effects on cell growth and drug sensitivity from optimised siRNA-mediated silencing of ribonucleotide reductase.", JOURNAL OF RNAI AND GENE SILENCING, vol. 5, no. 1, 1 January 2009 (2009-01-01) , pages 321-330, XP055013968, ISSN: 1747-0854**
- **DUXBURY M S ET AL: "Retrovirally mediated RNA interference targeting the M2 subunit of ribonucleotide reductase: A novel therapeutic strategy in pancreatic cancer", SURGERY, C.V. MOSBY CO., ST. LOUIS, US, vol. 136, no. 2, 1 August 2004 (2004-08-01), pages 261-269, XP004541864, ISSN: 0039-6060, DOI: 10.1016/J.SURG.2004.04.029**
- **HEIDEL JEREMY D ET AL: "Potent siRNA inhibitors of ribonucleotide reductase subunit RRM2 reduce cell proliferation in vitro and in vivo.", CLINICAL CANCER RESEARCH : AN OFFICIAL JOURNAL OF THE AMERICAN ASSOCIATION FOR CANCER RESEARCH 1 APR 2007 LNKD- PUBMED:17404105, vol. 13, no. 7, 1 April 2007 (2007-04-01), pages 2207-2215, XP002665151, ISSN: 1078-0432**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

This invention relates to double-stranded ribonucleic acids (dsRNAs), and their use in mediating RNA interference (RNAi) to inhibit the expression of the RRM2 gene.

Cancer remains an important area of high unmet medical need. The majority of current treatments provide small gains in overall survival requiring a delicate balance between efficacy and toxicity. Cancer is a disease characterized by uncontrolled growth and survival driven by improper regulation of the cell cycle. The cell cycle is divided up into four stages culminating in cytokinesis with checkpoint controls ensuring accurate completion of each phase. The cell cycle is designed to duplicate cellular material equally partitioning this material into what will become two new cells. DNA replication occurs during S-phase requiring pools of nucleic acid as the building blocks (dNTP) for new DNA as well as to repair DNA damage. Ribonucleotide reductase (RR) converts ribonucleoside 5'-diphosphates into 2'-deoxyribonucleotides which serve as the dNTP source for DNA synthesis and repair. RR catalyzes the rate-limiting step in the generation of dNTPs and represents an important part of cancer cell growth and repair. (RR) is made up of two subunits called RRM1 and RRM2 both of which are required for catalytic RR activity. RRM2 is overexpressed in a range of tumor types and elevated expression is associated with malignant transformation and metastasis. Overexpression of RRM2 cooperates with other oncogenes to drive the transformation and progression of normal cells. Tumor cells are particularly sensitive to changes in their dNTP pools because they have a high proliferation rate and lack checkpoint controls to monitor and repair DNA damage. Inhibition of RRM2 in tumor cells is expected to enhance DNA damage from a lack of sufficient dNTPs producing an apoptotic response. In nontransformed cells, checkpoint controls monitor the level of dNTPs and have the signaling ability to arrest cells before completing DNA replication and repair protecting cells from incurring incompletely replicated DNA or poorly repaired DNA damage.

Despite significant advances in the field of RNAi and advances in the treatment of fibrosis and proliferative disorders, like cancers, there remains a need for an agent that can selectively and efficiently silence the RRM2 gene. A specific RRM2 inhibitor is expected to provide an improved therapeutic index over existing inhibitors because it is more selective and tumors cells lacking checkpoint controls are dependent on large dNTP pools to support their rapid proliferation and DNA repair. Also, preclinical data supports the potent tumor cell killing effects following RRM2 inhibition.

RRM2 mRNA overexpression is associated with rapidly proliferating tumor cells. RRM2 expression is cell cycle regulated peaking at S-phase when DNA replication and repair occur followed by rapid degradation during mitosis. RRM2 is overexpressed in Acute myeloid leukemia (AML), bladder cancer, prostate cancer, Non Small Cell Lung Cancer (NSCLC), breast cancer, Hepatocellular Carcinoma (HCC), and colorectal cancers to name a few.

Double-stranded ribonucleic acid (dsRNA) molecules have been shown to block gene expression in a highly conserved regulatory mechanism known as RNA interference (RNAi), which is a viable pathway in the development of therapeutically active substances for the treatment of a wide range of proliferating diseases. Accordingly, inhibition of RRM2 expression with the dsRNA molecules of this invention may be used in the treatment of cancer including but not limited to Hepatocellular Carcinoma (HCC) and leukemia as well as other solid tumor types.

US 2008/227967 discloses that efficient sequence specific gene silencing is possible through the use of siRNA technology. By selecting particular siRNAs by rational design, one can maximize the generation of an effective gene silencing reagent, as well as methods for silencing genes. Methods, compositions, and kits generated through rational design of siRNAs are disclosed including those directed to RRM2. The invention provides double-stranded ribonucleic acid molecules (dsRNA) comprising a sense strand and an antisense strand wherein:

said sense strand and said antisense strand form a fully complimentary sequence of 19 base pairs,
said sense strand comprises SEQ ID NO. 9 and has at least one modified nucleotide,
said antisense strand comprises SEQ ID NO. 242 and has at least one modifiednucleotide, and preferably wherein said sense strand and said antisense strand are each less than 30 nucleotides in length, as well as compositions comprising said dsRNA for inhibiting the expression of the RRM2 gene. The invention also provides said compositions for treating pathological conditions and diseases caused by the expression of the RRM2 gene such as in proliferative disorders like cancer and inflammation.

The double-stranded ribonucleic acid (dsRNA) molecules is able to selectively and efficiently decrease the expression of RRM2. The use of RRM2 RNAi provides the therapeutic and/or prophylactic treatment of diseases/disorders which are associated with inflammation and proliferative disorders, like cancers. Particular disease/disorder states include the therapeutic and/or prophylactic treatment of inflammation and proliferative disorders, like cancers, particularly HCC, leukemia and solid tumors.

The invention provides said compositions for specifically targeting the liver with RRM2 dsRNA, for treating pathological conditions and diseases caused by the expression of the RRM2 gene including those described above.

The invention provides said double-stranded ribonucleic acid (dsRNA) molecules for inhibiting the expression of a RRM2 gene, in particular the expression of the mammalian or human RRM2 gene.

Tables 3 and 4 provide for selective biological, clinical and pharmaceutical relevant parameters of certain dsRNA molecules disclosed herein. In one embodiment the dsRNA molecules comprise an antisense strand with a 3' overhang of 1-5 nucleotides in length, preferably 1-2 nucleotides in length. Preferably said overhang of the antisense strand comprises uracil or nucleotides which are complementary to the mRNA encoding RRM2. In another preferred embodiment, said dsRNA molecules comprise a sense strand with a 3' overhang of 1-5 nucleotides in length, preferably 1-2 nucleotides in length. Preferably said overhang of the sense strand comprises uracil or nucleotides which are identical to the mRNA encoding RRM2.

The dsRNA molecules of the invention comprise modified nucleotides, such as a 2'-O-methyl modified nucleotide, inverted deoxythymidine, a nucleotide comprising a 5'-phosphorothioate group, and a terminal nucleotide linked to a cholesteryl derivative or dodecanoic acid bisdecylamide group. 2' modified nucleotides may have the additional advantage that certain immunostimulatory factors or cytokines are suppressed when the inventive dsRNA molecules are employed *in vivo,* for example in a medical setting. Alternatively the modified nucleotide may be chosen from the group of: a 2'-deoxy-2'-fluoro modified nucleotide, a 2'-deoxy-modified nucleotide, a locked nucleotide, an abasic nucleotide, 2'-amino-modified nucleotide, 2'-alkyl-modified nucleotide, morpholino nucleotide, a phosphoramidate, and a non-natural base comprising nucleotide. In one preferred embodiment the dsRNA molecules comprises at least one of the following modified nucleotides: a 2'-O-methyl modified nucleotide, a nucleotide comprising a 5'-phosphorothioate group and a deoxythymidine. In another preferred embodiment one of those deoxythymidine nucleotides at the 3' of both strand is a inverted deoxythymidine.

In one embodiment the dsRNA molecules of the invention comprise a sense and an antisense strand wherein both strands have a half-life of at least 0.9 hours. In one preferred embodiment the dsRNA molecules of the invention comprise a sense and an antisense strand wherein both strands have a half-life of at least 48 hours, preferably in human serum. In another embodiment the dsRNA molecules of the invention are non-immunostimulatory, e.g. do not stimulate INF-alpha and TNF-alpha *in vitro.* In another embodiment, the dsRNA molecules of the invention do stimulate INF-alpha and TNF-alpha *in vitro* to a very minor degree.

The invention also provides for cells comprising at least one of the dsRNAs of the invention. The cell is preferably a mammalian cell, such as a human cell. Furthermore, tissues and/or non-human organisms comprising the dsRNA molecules of the invention are an embodiment of this invention, whereby said non-human organisms are particularly useful for research purposes or as research tools, for example in drug testing.

Disclosed is a method for inhibiting the expression of a RRM2 gene, in particular a mammalian or human RRM2 gene, in a cell, tissue or organism comprising the following steps:

(a) introducing into the cell, tissue or organism a double-stranded ribonucleic acid (dsRNA) as defined herein; and
(b) maintaining said cell, tissue or organism produced in step (a) for a time sufficient to obtain degradation of the mRNA transcript of a RRM2 gene, thereby inhibiting expression of a RRM2 gene in a given cell.

The invention also relates to pharmaceutical compositions comprising the inventive dsRNAs of the invention. These pharmaceutical compositions are particularly useful in the inhibition of the expression of a RRM2 gene in a cell, a tissue or an organism. The pharmaceutical composition comprising one or more of the dsRNA of the invention may also comprise (a) pharmaceutically acceptable carrier(s), diluent(s) and/or excipient(s).

Disclosed are methods for treating, preventing or managing inflammation and / or proliferative disorders like cancers which are associated with RRM2, said method comprising administering to a subject in need of such treatment, prevention or management a therapeutically or prophylactically effective amount of one or more of the dsRNAs of the invention. Preferably, said subject is a mammal, most preferably a human patient.

Disclosed is a method for treating a subject having a pathological condition mediated by the expression of a RRM2 gene. Such conditions comprise disorders associated with inflammation and proliferative disorders, like cancers, as described above. In this instance, the dsRNA acts as a therapeutic agent for controlling the expression of a RRM2 gene. The method comprises administering a pharmaceutical composition of the invention to the patient (e.g., human), such that expression of a RRM2 gene is silenced. Because of their high specificity, the dsRNAs described specifically target mRNAs of a RRM2 gene. In one preferred instance, the described dsRNAs specifically decrease RRM2 mRNA levels and do not directly affect the expression and /or mRNA levels of off-target genes in the cell.

The described dsRNA can decrease RRM2 mRNA levels in the liver by at least 60%, preferably by at least 70%, and most preferably by at least 80% *in vivo.* The described dsRNAs can decrease RRM2 mRNA levels *in vivo* for at least 4 days. The dsRNAs of the invention can be used for the preparation of a pharmaceutical composition for the treatment of inflammation and proliferative disorders, like cancer. Cancers to be treated with said pharmaceutical composition comprise but are not limited to: HCC, AML, leukemia, bladder cancer, prostate cancer, NSCLC, breast cancer and colorectal cancer.

Disclosed are vectors for inhibiting the expression of a RRM2 gene in a cell, in particular a RRM2 gene comprising a regulatory sequence operably linked to a nucleotide sequence that encodes at least one strand of the dsRNA molecules

of the invention.

Disclosed is a cell comprising a vector for inhibiting the expression of a RRM2 gene in a cell. Said vector comprises a regulatory sequence operably linked to a nucleotide sequence that encodes at least one strand of the dsRNA molecule of the invention. Yet, said vector can comprise, besides said regulatory sequence a sequence that encodes at least one "sense strand" of the inventive dsRNA and at least one "anti sense strand" of said dsRNA. It is also envisaged that the cell comprises two or more vectors comprising, besides said regulatory sequences, the herein defined sequence(s) that encode(s) at least one strand of the dsRNA molecules of the invention.

The disclosed method can comprise administering a composition comprising a dsRNA, wherein the dsRNA comprises a nucleotide sequence which is complementary to at least a part of an RNA transcript of a RRM2 gene of the mammal to be treated. As pointed out above, also vectors and cells comprising nucleic acid molecules that encode for at least one strand of the herein defined dsRNA molecules can be used as pharmaceutical compositions and may, therefore, also be employed in the herein disclosed methods of treating a subject in need of medical intervention. It is also of note that these instances relating to pharmaceutical compositions also relate to approaches like gene therapy approaches. RRM2 specific dsRNA molecules as provided herein or nucleic acid molecules encoding individual strands of these inventive dsRNA molecules may also be inserted into vectors and used as gene therapy vectors for human patients. Gene therapy vectors can be delivered to a subject by, for example, intravenous injection, local administration (see U.S. Patent 5,328,470) or by stereotactic injection (see e.g., Chen et al. (1994) Proc. Natl. Acad. Sci. USA 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells which produce the gene delivery system.

Disclosed is that RRM2 specific dsRNA molecules that modulate RRM2 gene expression activity are expressed from transcription units inserted into DNA or RNA vectors (see, e.g., Skillern, A., et al., International PCT Publication No. WO 00/22113). These transgenes can be introduced as a linear construct, a circular plasmid, or a viral vector, which can be incorporated and inherited as a transgene integrated into the host genome. The transgene can also be constructed to permit it to be inherited as an extrachromosomal plasmid (Gassmann, et al., Proc. Natl. Acad. Sci. USA (1995) 92:1292).

The individual strands of a dsRNA can be transcribed by promoters on two separate expression vectors and co-transfected into a target cell. Alternatively, each individual strand of the dsRNA can be transcribed by promoters both of which are located on the same expression plasmid. A dsRNA can be expressed as an inverted repeat joined by a linker polynucleotide sequence such that the dsRNA has a stem and loop structure.

The recombinant dsRNA expression vectors can be DNA plasmids or viral vectors. dsRNA expressing viral vectors can be constructed based on adeno-associated virus (for a review, see Muzyczka, et al., Curr. Topics Micro. Immunol. (1992) 158:97-129)); adenovirus (see, for example, Berkner, et al., BioTechniques (1998) 6:616), Rosenfeld et al. (1991, Science 252:431-434), and Rosenfeld et al. (1992), Cell 68:143-155)); or alphavirus as well as others known in the art. Retroviruses have been used to introduce a variety of genes into many different cell types, including epithelial cells, *in vitro* and/or *in vivo* (see, e.g., Danos and Mulligan, Proc. Natl. Acad. Sci. USA (1998) 85:6460-6464). Recombinant retroviral vectors capable of transducing and expressing genes inserted into the genome of a cell can be produced by transfecting the recombinant retroviral genome into suitable packaging cell lines such as PA317 and Psi-CRIP (Comette et al., 1991, Human Gene Therapy 2:5-10; Cone et al., 1984, Proc. Natl. Acad. Sci. USA 81:6349). Recombinant adenoviral vectors can be used to infect a wide variety of cells and tissues in susceptible hosts (e.g., rat, hamster, dog, and chimpanzee) (Hsu et al., 1992, J. Infectious Disease, 166:769), and also have the advantage of not requiring mitotically active cells for infection.

The promoter driving dsRNA expression in either a DNA plasmid or viral vector may be a eukaryotic RNA polymerase I (e.g. ribosomal RNA promoter), RNA polymerase II (e.g. CMV early promoter or actin promoter or U1 snRNA promoter) or preferably RNA polymerase III promoter (e.g. U6 snRNA or 7SK RNA promoter) or a prokaryotic promoter, for example the T7 promoter, provided the expression plasmid also encodes T7 RNA polymerase required for transcription from a T7 promoter. The promoter can also direct transgene expression to the pancreas (see, e.g. the insulin regulatory sequence for pancreas (Bucchini et al., 1986, Proc. Natl. Acad. Sci. USA 83:2511-2515)).

In addition, expression of the transgene can be precisely regulated, for example, by using an inducible regulatory sequence and expression systems such as a regulatory sequence that is sensitive to certain physiological regulators, e.g., circulating glucose levels, or hormones (Docherty et al., 1994, FASEB J. 8:20-24). Such inducible expression systems, suitable for the control of transgene expression in cells or in mammals include regulation by ecdysone, by estrogen, progesterone, tetracycline, chemical inducers of dimerization, and isopropyl-beta-DI - thiogalactopyranoside (EPTG). A person skilled in the art would be able to choose the appropriate regulatory/promoter sequence based on the intended use of the dsRNA transgene.

Recombinant vectors capable of expressing dsRNA molecules can be delivered as described below, and persist in target cells. Alternatively, viral vectors can be used that provide for transient expression of dsRNA molecules. Such vectors can be repeatedly administered as necessary. Once expressed, the dsRNAs bind to target RNA and modulate

its function or expression. Delivery of dsRNA expressing vectors can be systemic, such as by intravenous or intramuscular administration, by administration to target cells ex-planted from the patient followed by reintroduction into the patient, or by any other means that allows for introduction into a desired target cell.

dsRNA expression DNA plasmids are typically transfected into target cells as a complex with cationic lipid carriers (e.g. Oligofectamine) or non-cationic lipid-based carriers (e.g. Transit-TKO™). Multiple lipid transfections for dsRNA-mediated knockdowns targeting different regions of a single RRM2 gene or multiple RRM2 genes over a period of a week or more can also be contemplated. Successful introduction of the vectors into host cells can be monitored using various known methods. For example, transient transfection can be signaled with a reporter, such as a fluorescent marker, such as Green Fluorescent Protein (GFP). Stable transfection of ex vivo cells can be ensured using markers that provide the transfected cell with resistance to specific environmental factors (e.g., antibiotics and drugs), such as hygromycin B resistance.

The following detailed description discloses how to make and use the dsRNA and compositions containing dsRNA to inhibit the expression of a target RRM2 gene, as well as compositions and methods for treating diseases and disorders caused by the expression of said RRM2 gene.

DEFINITIONS

For convenience, the meaning of certain terms and phrases used in the specification, examples, and appended claims, are provided below. If there is an apparent discrepancy between the usage of a term in other parts of this specification and its definition provided in this section, the definition in this section shall prevail.

"G," "C," "A", "U" and "T" or "dT" respectively, each generally stand for a nucleotide that contains guanine, cytosine, adenine, uracil and deoxythymidine as a base, respectively. However, the term "ribonucleotide" or "nucleotide" can also refer to a modified nucleotide, as further detailed below, or a surrogate replacement moiety. As detailed below, the herein described dsRNA molecules may also comprise "overhangs", i.e. unpaired, overhanging nucleotides which are not directly involved in the RNA double helical structure normally formed by the herein defined pair of "sense strand" and "anti sense strand". Often, such an overhanging stretch comprises the deoxythymidine nucleotide, in most embodiments, 2 deoxythymidines in the 3’ end. Such overhangs will be described and illustrated below.

The term "RRM2" as used herein relates in particular to the ribonucleotide reductase M2, also known as the ribonucleotide reductase M2 polypeptide, ribonucleoside-diphosphate reductase subunit M2, ribonucleotide reductase small chain, ribonucleotide reductase small subunit, with synonyms R2, RR2, RR2M, and the like and said term relates to the corresponding gene, encoded mRNA, encoded protein/polypeptide as well as functional fragments of the same. Preferred is the human RRM2 gene. The dsRNAs of the invention can target the RRM2 gene of human (*H.sapiens*) and cynomolgous monkey (*Macaca fascicularis*) RRM2 gene. The term "RRM2 gene/sequence" does not only relate to (the) wild-type sequence(s) but also to mutations and alterations which may be comprised in said gene/sequence.

As used herein, "target sequence" refers to a contiguous portion of the nucleotide sequence of an mRNA molecule formed during the transcription of a RRM2 gene, including mRNA that is a product of RNA processing of a primary transcription product.

As used herein, the term "strand comprising a sequence" refers to an oligonucleotide comprising a chain of nucleotides that is described by the sequence referred to using the standard nucleotide nomenclature. However, as detailed herein, such a "strand comprising a sequence" may also comprise modifications, like modified nucleotides.

As used herein, and unless otherwise indicated, the term "complementary," when used to describe a first nucleotide sequence in relation to a second nucleotide sequence, refers to the ability of an oligonucleotide or polynucleotide comprising the first nucleotide sequence to hybridize and form a duplex structure under certain conditions with an oligonucleotide or polynucleotide comprising the second nucleotide sequence. "Complementary" sequences, as used herein, may also include, or be formed entirely from, non-Watson-Crick base pairs and/or base pairs formed from non-natural and modified nucleotides, in as far as the above requirements with respect to their ability to hybridize are fulfilled.

Sequences referred to as "fully complementary" comprise base-pairing of the oligonucleotide or polynucleotide comprising the first nucleotide sequence to the oligonucleotide or polynucleotide comprising the second nucleotide sequence over the entire length of the first and second nucleotide sequence.

However, where a first sequence is referred to as "substantially complementary" with respect to a second sequence herein, the two sequences can be fully complementary, or they may form one or more, but preferably not more than 13 mismatched base pairs upon hybridization.

The terms "complementary", "fully complementary" and "substantially complementary" herein may be used with respect to the base matching between the sense strand and the antisense strand of a dsRNA, or between the antisense strand of a dsRNA and a target sequence, as will be understood from the context of their use.

The term "double-stranded RNA", "dsRNA molecule", or "dsRNA", as used herein, refers to a ribonucleic acid molecule, or complex of ribonucleic acid molecules, having a duplex structure comprising two anti-parallel and substantially complementary nucleic acid strands. The two strands forming the duplex structure may be different portions of one

larger RNA molecule, or they may be separate RNA molecules. Where the two strands are part of one larger molecule, and therefore are connected by an uninterrupted chain of nucleotides between the 3'-end of one strand and the 5' end of the respective other strand forming the duplex structure, the connecting RNA chain is referred to as a "hairpin loop". Where the two strands are connected covalently by means other than an uninterrupted chain of nucleotides between the 3'-end of one strand and the 5'end of the respective other strand forming the duplex structure, the connecting structure is referred to as a "linker". The RNA strands may have the same or a different number of nucleotides. In addition to the duplex structure, a dsRNA may comprise one or more nucleotide overhangs. The nucleotides in said "overhangs" may comprise between 0 and 5 nucleotides, whereby "0" means no additional nucleotide(s) that form(s) an "overhang" and whereas "5" means five additional nucleotides on the individual strands of the dsRNA duplex. These optional "overhangs" are located in the 3' end of the individual strands. As will be detailed below, also dsRNA molecules which comprise only an "overhang" in one of the two strands may be useful and even advantageous. Most preferably 2 "dT" (deoxythymidine) nucleotides are found at the 3' end of both strands of the dsRNA. Also 2 "U"(uracil) nucleotides can be used as overhangs at the 3' end of both strands of the dsRNA. Accordingly, a "nucleotide overhang" refers to the unpaired nucleotide or nucleotides that protrude from the duplex structure of a dsRNA when a 3'-end of one strand of the dsRNA extends beyond the 5'-end of the other strand, or vice versa. For example the antisense strand comprises 23 nucleotides and the sense strand comprises 21 nucleotides, forming a 2 nucleotide overhang at the 3' end of the antisense strand. Preferably, the 2 nucleotide overhang is fully complementary to the mRNA of the target gene. "Blunt" or "blunt end" means that there are no unpaired nucleotides at that end of the dsRNA, i.e., no nucleotide overhang. A "blunt ended" dsRNA is a dsRNA that is double-stranded over its entire length, i.e., no nucleotide overhang at either end of the molecule.

The term "antisense strand" refers to the strand of a dsRNA which includes a region that is substantially complementary to a target sequence. As used herein, the term "region of complementarity" refers to the region on the antisense strand that is substantially complementary to a sequence, for example a target sequence. Where the region of complementarity is not fully complementary to the target sequence, the mismatches are most tolerated outside nucleotides 2-7 of the 5' terminus of the antisense strand

The term "sense strand," as used herein, refers to the strand of a dsRNA that includes a region that is substantially complementary to a region of the antisense strand. "Substantially complementary" means preferably at least 85% of the overlapping nucleotides in sense and antisense strand are complementary.

"Introducing into a cell", when referring to a dsRNA, means facilitating uptake or absorption into the cell, as is understood by those skilled in the art. Absorption or uptake of dsRNA can occur through unaided diffusive or active cellular processes, or by auxiliary agents or devices. For example, for *in vivo* delivery, dsRNA can be injected into a tissue site or administered systemically. It is, for example envisaged that the dsRNA molecules of this invention be administered to a subject in need of medical intervention. Such an administration may comprise the injection of the dsRNA, the vector or a cell into a diseased site in said subject, for example into liver tissue/cells or into cancerous tissues/cells, like liver cancer tissue. In addition, the injection can be in close proximity to the diseased tissue envisaged. *In vitro* introduction into a cell includes methods known in the art such as electroporation and lipofection.

As used herein, "proliferating" and "proliferation" refer to cells undergoing mitosis. Throughout this application, the term "proliferative disorder" refers to any disease/disorder marked by unwanted or aberrant proliferation of tissue. As used herein, the term " proliferative disorder" also refers to conditions in which the unregulated and/or abnormal growth of cells can lead to the development of an unwanted condition or disease, which can be cancerous or non-cancerous.

The term "inflammation" as used herein refers to the biologic response of body tissue to injury, irritation, or disease which can be caused by harmful stimuli, for example, pathogens, damaged cells, or irritants. Inflammation is typically characterized by pain and swelling. Inflammation is intended to encompass both acute responses, in which inflammatory processes are active (e.g., neutrophils and leukocytes), and chronic responses, which are marked by slow progress, a shift in the type of cell present at the site of inflammation, and the formation of connective tissue.

Cancers to be treated comprise, but are again not limited to leukemia, AML, solid tumors, liver cancer, brain cancer, breast cancer, lung cancer, NSCLC, colorectal cancer, bladder cancer and prostate cancer, whereby said liver cancer may, inter alia, be selected from the group consisting of hepatocellular carcinoma (HCC), hepatoblastoma, a mixed liver cancer, a cancer derived from mesenchymal tissue, a liver sarcoma or a cholangiocarcinoma.

The terms "silence", "inhibit the expression of' and "knock down", in as far as they refer to a RRM2 gene, herein refer to the at least partial suppression of the expression of a RRM2 gene, as manifested by a reduction of the amount of mRNA transcribed from a RRM2 gene which may be isolated from a first cell or group of cells in which a RRM2 gene is transcribed and which has or have been treated such that the expression of a RRM2 gene is inhibited, as compared to a second cell or group of cells substantially identical to the first cell or group of cells but which has or have not been so treated (control cells). The degree of inhibition is usually expressed in terms of

$$\frac{(\text{mRNA in control cells}) - (\text{mRNA in treated cells})}{(\text{mRNA in control cells})} \bullet 100\%$$

Alternatively, the degree of inhibition may be given in terms of a reduction of a parameter that is functionally linked to the RRM2 gene transcription, e.g. the amount of protein encoded by a RRM2 gene which is secreted by a cell, or the number of cells displaying a certain phenotype.

As illustrated in the appended examples and in the appended tables provided herein, the disclosed dsRNA molecules are capable of inhibiting the expression of a human RRM2 by at least about 60%, preferably by at least 70%, most preferably by at least 80% *in vitro* assays, i.e. *in vitro.* The term *"in vitro"* as used herein includes but is not limited to cell culture assays. The disclosed dsRNA molecules can be capable of inhibiting the expression of a mouse or rat RRM2 by at least 60 %.preferably by at least 70%, most preferably by at least 80%. The person skilled in the art can readily determine such an inhibition rate and related effects, in particular in light of the assays provided herein.

The term "off target" as used herein refers to all non-target mRNAs of the transcriptome that are predicted by in silico methods to hybridize to the described dsRNAs based on sequence complementarity. The dsRNAs of the present invention preferably do specifically inhibit the expression of RRM2, i.e. do not inhibit the expression of any off-target.

The term "half-life" as used herein is a measure of stability of a compound or molecule and can be assessed by methods known to a person skilled in the art, especially in light of the assays provided herein.

The term "non-immunostimulatory" as used herein refers to the absence of any induction of a immune response by the invented dsRNA molecules. Methods to determine immune responses are well known to a person skilled in the art, for example by assessing the release of cytokines, as described in the examples section.

The terms "treat", "treatment", and the like, mean in context of this invention the relief from or alleviation of a disorder related to RRM2 expression, like inflammation and proliferative disorders, like cancers.

As used herein, a "pharmaceutical composition" comprises a pharmacologically effective amount of a dsRNA and a pharmaceutically acceptable carrier. However, such a "pharmaceutical composition" may also comprise individual strands of such a dsRNA molecule or the herein described vector(s) comprising a regulatory sequence operably linked to a nucleotide sequence that encodes at least one strand of a sense or an antisense strand comprised in the dsRNAs of this invention. It is also envisaged that cells, tissues or isolated organs that express or comprise the herein defined dsRNAs may be used as "pharmaceutical compositions". As used herein, "pharmacologically effective amount," "therapeutically effective amount" or simply "effective amount" refers to that amount of an RNA effective to produce the intended pharmacological, therapeutic or preventive result.

The term "pharmaceutically acceptable carrier" refers to a carrier for administration of a therapeutic agent. Such carriers include, but are not limited to, saline, buffered saline, dextrose, water, glycerol, ethanol, and combinations thereof. The term specifically excludes cell culture medium. For drugs administered orally, pharmaceutically acceptable carriers include, but are not limited to pharmaceutically acceptable excipients such as inert diluents, disintegrating agents, binding agents, lubricating agents, sweetening agents, flavoring agents, coloring agents and preservatives as known to persons skilled in the art.

It is in particular envisaged that the pharmaceutically acceptable carrier allows for the systemic administration of the dsRNAs, vectors or cells of this invention. Whereas also the enteric administration is envisaged the parenteral administration and also transdermal or transmucosal (e.g. insufflation, buccal, vaginal, anal) administration as well as inhalation of the drug are feasible ways of administering to a patient in need of medical intervention. When parenteral administration is employed, this can comprise the direct injection of the compounds into the diseased tissue or at least in close proximity. However, also intravenous, intraarterial, subcutaneous, intramuscular, intraperitoneal, intradermal, intrathecal and other administrations of the compounds are within the skill of the artisan, for example the attending physician.

For intramuscular, subcutaneous and intravenous use, the pharmaceutical compositions will generally be provided in sterile aqueous solutions or suspensions, buffered to an appropriate pH and isotonicity. The carrier can consist exclusively of an aqueous buffer. In this context, "exclusively" means no auxiliary agents or encapsulating substances are present which might affect or mediate uptake of dsRNA in the cells that express a RRM2 gene. Aqueous suspensions may include suspending agents such as cellulose derivatives, sodium alginate, polyvinylpyrrolidone and gum tragacanth, and a wetting agent such as lecithin. Suitable preservatives for aqueous suspensions include ethyl and n-propyl p-hydroxybenzoate. The pharmaceutical compositions also include encapsulated formulations to protect the dsRNA against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. Liposomal suspensions can also be used as pharmaceutically acceptable carriers. These can be prepared according to methods known to those skilled in the art, for example, as described in PCT publication WO 91/06309.

As used herein, a "transformed cell" is a cell into which at least one vector has been introduced from which a dsRNA molecule or at least one strand of such a dsRNA molecule may be expressed. Such a vector can be a vector comprising a regulatory sequence operably linked to nucleotide sequence that encodes at least one sense strand or antisense strand of a dsRNA of the present invention.

It can be reasonably expected that shorter dsRNAs comprising one of the sequences in Table 1 and 4 minus only a few nucleotides on one or both ends may be similarly effective as compared to the dsRNAs described above.

As mentioned above, at least one end/strand of the dsRNA may have a single-stranded nucleotide overhang of 1 to 5, preferably 1 or 2 nucleotides. dsRNAs having at least one nucleotide overhang have unexpectedly superior inhibitory properties than their blunt-ended counterparts. Moreover, the present inventors have discovered that the presence of only one nucleotide overhang strengthens the interference activity of the dsRNA, without affecting its overall stability. dsRNA having only one overhang has proven particularly stable and effective *in vivo,* as well as in a variety of cells, cell culture mediums, blood, and serum. Preferably, the single-stranded overhang is located at the 3'-terminal end of the antisense strand or, alternatively, at the 3'-terminal end of the sense strand. The dsRNA may also have a blunt end, preferably located at the 5'-end of the antisense strand. Preferably, the antisense strand of the dsRNA has a nucleotide overhang at the 3'-end, and the 5'-end is blunt. In another embodiment, one or more of the nucleotides in the overhang is replaced with a nucleoside thiophosphate.

The dsRNA of the present invention may also be chemically modified to enhance stability. The nucleic acids may be synthesized and/or modified by methods well established in the art, such as those described in "Current protocols in nucleic acid chemistry", Beaucage, S.L. et al. (Edrs.), John Wiley & Sons, Inc., New York, NY, USA. Chemical modifications may include, but are not limited to 2' modifications, introduction of non-natural bases, covalent attachment to a ligand, and replacement of phosphate linkages with thiophosphate linkages, inverted deoxythymidines. In this embodiment, the integrity of the duplex structure is strengthened by at least one, and preferably two, chemical linkages. Chemical linking may be achieved by any of a variety of well-known techniques, for example by introducing covalent, ionic or hydrogen bonds; hydrophobic interactions, van der Waals or stacking interactions; by means of metal-ion coordination, or through use of purine analogues. Preferably, the chemical groups that can be used to modify the dsRNA include, without limitation, methylene blue; bifunctional groups, preferably bis-(2-chloroethyl)amine; N-acetyl-N'-(p-glyoxylbenzoyl)cystamine; 4-thiouracil; and psoralen. In one preferred embodiment, the linker is a hexa-ethylene glycol linker. In this case, the dsRNA are produced by solid phase synthesis and the hexa-ethylene glycol linker is incorporated according to standard methods (e.g., Williams, D.J., and K.B. Hall, Biochem. (1996) 35:14665-14670). In a particular embodiment, the 5'-end of the antisense strand and the 3'-end of the sense strand are chemically linked via a hexaethylene glycol linker. In another embodiment, at least one nucleotide of the dsRNA comprises a phosphorothioate or phosphorodithioate groups. The chemical bond at the ends of the dsRNA is preferably formed by triple-helix bonds.

In certain embodiments, a chemical bond may be formed by means of one or several bonding groups, wherein such bonding groups are preferably poly-(oxyphosphinicooxy-1,3-propandiol)- and/or polyethylene glycol chains. In other embodiments, a chemical bond may also be formed by means of purine analogs introduced into the double-stranded structure instead of purines. In further embodiments, a chemical bond may be formed by azabenzene units introduced into the double-stranded structure. In still further embodiments, a chemical bond may be formed by branched nucleotide analogs instead of nucleotides introduced into the double-stranded structure. In certain embodiments, a chemical bond may be induced by ultraviolet light.

In yet another embodiment, the nucleotides at one or both of the two single strands may be modified to prevent or inhibit the activation of cellular enzymes, for example certain nucleases. Techniques for inhibiting the activation of cellular enzymes are known in the art including, but not limited to, 2'-amino modifications, 2'-amino sugar modifications, 2'-F sugar modifications, 2'-F modifications, 2'-alkyl sugar modifications, uncharged backbone modifications, morpholino modifications, 2'-O-methyl modifications, and phosphoramidate (see, e.g., Wagner, Nat. Med. (1995) 1:1116-8). Thus, at least one 2'-hydroxyl group of the nucleotides on a dsRNA is replaced by a chemical group, preferably by a 2'-amino or a 2'-methyl group. Also, at least one nucleotide may be modified to form a locked nucleotide. Such locked nucleotide contains a methylene bridge that connects the 2'-oxygen of ribose with the 4'-carbon of ribose. Introduction of a locked nucleotide into an oligonucleotide improves the affinity for complementary sequences and increases the melting temperature by several degrees.

Modifications of dsRNA molecules provided herein may positively influence their stability *in vivo* as well as *in vitro* and also improve their delivery to the (diseased) target side. Furthermore, such structural and chemical modifications may positively influence physiological reactions towards the dsRNA molecules upon administration, e.g. the cytokine release which is preferably suppressed. Such chemical and structural modifications are known in the art and are, inter alia, illustrated in Nawrot (2006) Current Topics in Med Chem, 6, 913-925.

Conjugating a ligand to a dsRNA can enhance its cellular absorption as well as targeting to a particular tissue. In certain instances, a hydrophobic ligand is conjugated to the dsRNA to facilitate direct permeation of the cellular membrane. Alternatively, the ligand conjugated to the dsRNA is a substrate for receptor-mediated endocytosis. These approaches have been used to facilitate cell permeation of antisense oligonucleotides. For example, cholesterol has

been conjugated to various antisense oligonucleotides resulting in compounds that are substantially more active compared to their non-conjugated analogs. See M. Manoharan Antisense & Nucleic Acid Drug Development 2002, 12, 103. Other lipophilic compounds that have been conjugated to oligonucleotides include 1-pyrene butyric acid, 1,3-bis-O-(hexadecyl)glycerol, and menthol. One example of a ligand for receptor-mediated endocytosis is folic acid. Folic acid enters the cell by folate-receptor-mediated endocytosis. dsRNA compounds bearing folic acid would be efficiently transported into the cell via the folate-receptor-mediated endocytosis. Attachment of folic acid to the 3'-terminus of an oligonucleotide results in increased cellular uptake of the oligonucleotide (Li, S.; Deshmukh, H. M.; Huang, L. Pharm. Res. 1998, 15, 1540). Other ligands that have been conjugated to oligonucleotides include polyethylene glycols, carbohydrate clusters, cross-linking agents, porphyrin conjugates, and delivery peptides.

In certain instances, conjugation of a cationic ligand to oligonucleotides often results in improved resistance to nucleases. Representative examples of cationic ligands are propylammonium and dimethylpropylammonium. Interestingly, antisense oligonucleotides were reported to retain their high binding affinity to mRNA when the cationic ligand was dispersed throughout the oligonucleotide. See M. Manoharan Antisense & Nucleic Acid Drug Development 2002, 12, 103 and references therein.

The ligand-conjugated dsRNA of the invention may be synthesized by the use of a dsRNA that bears a pendant reactive functionality, such as that derived from the attachment of a linking molecule onto the dsRNA. This reactive oligonucleotide may be reacted directly with commercially-available ligands, ligands that are synthesized bearing any of a variety of protecting groups, or ligands that have a linking moiety attached thereto. The methods of the invention facilitate the synthesis of ligand-conjugated dsRNA by the use of, in some preferred embodiments, nucleoside monomers that have been appropriately conjugated with ligands and that may further be attached to a solid-support material. Such ligand-nucleoside conjugates, optionally attached to a solid-support material, are prepared according to some preferred embodiments of the methods of the invention via reaction of a selected serum-binding ligand with a linking moiety located on the 5' position of a nucleoside or oligonucleotide. In certain instances, an dsRNA bearing an aralkyl ligand attached to the 3'-terminus of the dsRNA is prepared by first covalently attaching a monomer building block to a controlled-pore-glass support via a long-chain aminoalkyl group. Then, nucleotides are bonded via standard solid-phase synthesis techniques to the monomer building-block bound to the solid support. The monomer building block may be a nucleoside or other organic compound that is compatible with solid-phase synthesis.

The dsRNA used in the conjugates may be conveniently and routinely made through the well-known technique of solid-phase synthesis. It is also known to use similar techniques to prepare other oligonucleotides, such as the phosphorothioates and alkylated derivatives.

Teachings regarding the synthesis of particular modified oligonucleotides may be found in the following U.S. patents: U.S. Pat. No. 5,218,105, drawn to polyamine conjugated oligonucleotides; U.S. Pat. Nos. 5,541,307, drawn to oligonucleotides having modified backbones; U.S. Pat. No. 5,521,302, drawn to processes for preparing oligonucleotides having chiral phosphorus linkages; U.S. Pat. No. 5,539,082, drawn to peptide nucleic acids; U.S. Pat. No. 5,554,746, drawn to oligonucleotides having β-lactam backbones; U.S. Pat. No. 5,571,902, drawn to methods and materials for the synthesis of oligonucleotides; U.S. Pat. No. 5,578,718, drawn to nucleosides having alkylthio groups, wherein such groups may be used as linkers to other moieties attached at any of a variety of positions of the nucleoside; U.S. Pat. No 5,587,361 drawn to oligonucleotides having phosphorothioate linkages of high chiral purity; U.S. Pat. No. 5,506,351, drawn to processes for the preparation of 2'-O-alkyl guanosine and related compounds, including 2,6-diaminopurine compounds; U.S. Pat. No. 5,587,469, drawn to oligonucleotides having N-2 substituted purines; U.S. Pat. No. 5,587,470, drawn to oligonucleotides having 3-deazapurines; U.S. Pat. No. 5,608,046, both drawn to conjugated 4'-desmethyl nucleoside analogs; U.S. Pat. No. 5,610,289, drawn to backbone-modified oligonucleotide analogs; U.S. Pat. No 6,262,241 drawn to, inter alia, methods of synthesizing 2'-fluoro-oligonucleotides.

In the ligand-conjugated dsRNA and ligand-molecule bearing sequence-specific linked nucleosides, the oligonucleotides and oligonucleosides may be assembled on a suitable DNA synthesizer utilizing standard nucleotide or nucleoside precursors, or nucleotide or nucleoside conjugate precursors that already bear the linking moiety, ligand-nucleotide or nucleoside-conjugate precursors that already bear the ligand molecule, or non-nucleoside ligand-bearing building blocks.

When using nucleotide-conjugate precursors that already bear a linking moiety, the synthesis of the sequence-specific linked nucleosides is typically completed, and the ligand molecule is then reacted with the linking moiety to form the ligand-conjugated oligonucleotide. Oligonucleotide conjugates bearing a variety of molecules such as steroids, vitamins, lipids and reporter molecules, has previously been described (see Manoharan et al., PCT Application WO 93/07883). In a preferred embodiment, the oligonucleotides or linked nucleosides are synthesized by an automated synthesizer using phosphoramidites derived from ligand-nucleoside conjugates in addition to commercially available phosphoramidites.

The incorporation of a 2'-O-methyl, 2'-O-ethyl, 2'-O-propyl, 2'-O-allyl, 2'-O-aminoalkyl or 2'-deoxy-2'-fluoro group in nucleosides of an oligonucleotide confers enhanced hybridization properties to the oligonucleotide. Further, oligonucleotides containing phosphorothioate backbones have enhanced nuclease stability. Thus, functionalized, linked

nucleosides of the invention can be augmented to include either or both a phosphorothioate backbone or a 2'-O-methyl, 2'-O-ethyl, 2'-O-propyl, 2'-O-aminoalkyl, 2'-O-allyl or 2'-deoxy-2'-fluoro group.

Functionalized nucleoside sequences possessing an amino group at the 5'-terminus can be prepared using a DNA synthesizer, and then reacted with an active ester derivative of a selected ligand. Active ester derivatives are well known to those skilled in the art. Representative active esters include N-hydrosuccinimide esters, tetrafluorophenolic esters, pentafluorophenolic esters and pentachlorophenolic esters. The reaction of the amino group and the active ester produces an oligonucleotide in which the selected ligand is attached to the 5'-position through a linking group. The amino group at the 5'-terminus can be prepared utilizing a 5'-Amino-Modifier C6 reagent. Ligand molecules may be conjugated to oligonucleotides at the 5'-position by the use of a ligand-nucleoside phosphoramidite wherein the ligand is linked to the 5'-hydroxy group directly or indirectly via a linker. Such ligand-nucleoside phosphoramidites are typically used at the end of an automated synthesis procedure to provide a ligand-conjugated oligonucleotide bearing the ligand at the 5'-terminus.

The preparation of ligand conjugated oligonucleotides can commence with the selection of appropriate precursor molecules upon which to construct the ligand molecule. Typically, the precursor is an appropriately-protected derivative of the commonly-used nucleosides. For example, the synthetic precursors for the synthesis of the ligand-conjugated oligonucleotides of the invention include, but are not limited to, 2'-aminoalkoxy-5'-ODMT-nucleosides, 2'-6-aminoalkylamino-5'-ODMT-nucleosides, 5'-6-aminoalkoxy-2'-deoxy-nucleosides, 5'-6-aminoalkoxy-2-protected-nucleosides, 3'-6-aminoalkoxy-5'-ODMT-nucleosides, and 3'-aminoalkylamino-5'-ODMT-nucleosides that may be protected in the nucleobase portion of the molecule. Methods for the synthesis of such amino-linked protected nucleoside precursors are known to those of ordinary skill in the art.

In many cases, protecting groups are used during the preparation of the compounds of the invention. As used herein, the term "protected" means that the indicated moiety has a protecting group appended thereon. In some preferred embodiments of the invention, compounds contain one or more protecting groups. A wide variety of protecting groups can be employed in the methods of the invention. In general, protecting groups render chemical functionalities inert to specific reaction conditions, and can be appended to and removed from such functionalities in a molecule without substantially damaging the remainder of the molecule.

Representative hydroxyl protecting groups, as well as other representative protecting groups, are disclosed in Greene and Wuts, Protective Groups in Organic Synthesis, Chapter 2, 2d ed., John Wiley & Sons, New York, 1991, and Oligonucleotides And Analogues A Practical Approach, Ekstein, F. Ed., IRL Press, N.Y, 1991.

Amino-protecting groups stable to acid treatment are selectively removed with base treatment, and are used to make reactive amino groups selectively available for substitution. Examples of such groups are the Fmoc (E. Atherton and R. C. Sheppard in The Peptides, S. Udenfriend, J. Meienhofer, Eds., Academic Press, Orlando, 1987, volume 9, p.1) and various substituted sulfonylethyl carbamates exemplified by the Nsc group (Samukov et al., Tetrahedron Lett., 1994, 35:7821.

Additional amino-protecting groups include, but are not limited to, carbamate protecting groups, such as 2-trimethylsilylethoxycarbonyl (Teoc), 1-methyl-1-(4-biphenylyl)ethoxycarbonyl (Bpoc), t-butoxycarbonyl (BOC), allyloxycarbonyl (Alloc), 9-fluorenylmethyloxycarbonyl (Fmoc), and benzyloxycarbonyl (Cbz); amide protecting groups, such as formyl, acetyl, trihaloacetyl, benzoyl, and nitrophenylacetyl; sulfonamide protecting groups, such as 2-nitrobenzenesulfonyl; and imine and cyclic imide protecting groups, such as phthalimido and dithiasuccinoyl. Equivalents of these amino-protecting groups are also encompassed.

Many solid supports are commercially available and one of ordinary skill in the art can readily select a solid support to be used in the solid-phase synthesis steps. In certain embodiments, a universal support is used. A universal support allows for the preparation of oligonucleotides having unusual or modified nucleotides located at the 3'-terminus of the oligonucleotide. For further details about universal supports see Scott et al., Innovations and Perspectives in solid-phase Synthesis, 3rd International Symposium, 1994, Ed. Roger Epton, Mayflower Worldwide, 115-124]. In addition, it has been reported that the oligonucleotide can be cleaved from the universal support under milder reaction conditions when the oligonucleotide is bonded to the solid support via a *syn*-1,2-acetoxyphosphate group which more readily undergoes basic hydrolysis. See Guzaev, A. I.; Manoharan, M. J. Am. Chem. Soc. 2003, 125, 2380.

The nucleosides are linked by phosphorus-containing or non-phosphorus-containing covalent internucleoside linkages. For the purposes of identification, such conjugated nucleosides can be characterized as ligand-bearing nucleosides or ligand-nucleoside conjugates. The linked nucleosides having an aralkyl ligand conjugated to a nucleoside within their sequence will demonstrate enhanced dsRNA activity when compared to like dsRNA compounds that are not conjugated.

The aralkyl-ligand-conjugated oligonucleotides also include conjugates of oligonucleotides and linked nucleosides wherein the ligand is attached directly to the nucleoside or nucleotide without the intermediacy of a linker group. The ligand may be attached, via linking groups, at a carboxyl, amino or oxo group of the ligand. Typical linking groups may be ester, amide or carbamate groups.

Specific examples of preferred modified oligonucleotides envisioned for use in the ligand-conjugated oligonucleotides include oligonucleotides containing modified backbones or non-natural internucleoside linkages. As defined here, oligonucleotides having modified backbones or internucleoside linkages include those that retain a phosphorus atom in the backbone and those that do not have a phosphorus atom in the backbone. For the purposes of the invention, modified oligonucleotides that do not have a phosphorus atom in their intersugar backbone can also be considered to be oligonucleosides.

Specific oligonucleotide chemical modifications are described below.

Preferred modified internucleoside linkages or backbones include, for example, phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'. Various salts, mixed salts and free-acid forms are also included.

Representative United States Patents relating to the preparation of the above phosphorus-atom-containing linkages include U.S. Pat. Nos. 4,469,863; 5,023,243; 5,264,423; 5,321,131; 5,399,676; 5,405,939; 5,453,496; 5,455,233 and 5,466,677.

Preferred modified internucleoside linkages or backbones that do not include a phosphorus atom therein (i.e., oligonucleosides) have backbones that are formed by short chain alkyl or cycloalkyl intersugar linkages, mixed heteroatom and alkyl or cycloalkyl intersugar linkages, or one or more short chain heteroatomic or heterocyclic intersugar linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and $CH_2$ component parts.

Representative United States patents relating to the preparation of the above oligonucleosides include U.S. Pat. Nos. 5,034,506; 5,214,134; 5,216,141; 5,264,562; 5,466,677; 5,470,967; 5,489,677; 5,602,240 and 5,663,312.

In other oligonucleotide mimetics, both the sugar and the internucleoside linkage, i.e., the backbone, of the nucleoside units are replaced with novel groups. The nucleobase units are maintained for hybridization with an appropriate nucleic acid target compound. One such oligonucleotide, an oligonucleotide mimetic, that has been shown to have excellent hybridization properties, is referred to as a peptide nucleic acid (PNA). In PNA compounds, the sugar-backbone of an oligonucleotide is replaced with an amide-containing backbone, in particular an aminoethylglycine backbone. The nucleobases are retained and are bound directly or indirectly to atoms of the amide portion of the backbone. Teaching of PNA compounds can be found for example in U.S. Pat. No. 5,539,082.

Disclosed are oligonucleotides with phosphorothioate linkages and oligonucleosides with heteroatom backbones, and in particular $--CH_2--NH--O--CH_2--$, $--CH_2--N(CH_3)--O--CH_2--$ [known as a methylene (methylimino) or MMI backbone], $--CH_2--O--N(CH_3)--CH_2--$, $--CH_2--N(CH_3)-N(CH_3)--CH_2--$, and $--O--N(CH_3)--CH_2--CH_2--$ [wherein the native phosphodiester backbone is represented as $--O--P--O--CH_2--$] of the above referenced U.S. Pat. No. 5,489,677, and the amide backbones of the above referenced U.S. Pat. No. 5,602,240. Also disclosed are oligonucleotides having morpholino backbone structures of the above-referenced U.S. Pat. No. 5,034,506.

The oligonucleotides employed in the ligand-conjugated oligonucleotides may additionally or alternatively comprise nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C), and uracil (U). Modified nucleobases include other synthetic and natural nucleobases, such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine and 3-deazaadenine.

Further nucleobases include those disclosed in U.S. Pat. No. 3,687,808, those disclosed in the Concise Encyclopedia Of Polymer Science And Engineering, pages 858-859, Kroschwitz, J. I., ed. John Wiley & Sons, 1990, those disclosed by Englisch et al., Angewandte Chemie, International Edition, 1991, 30, 613, and those disclosed by Sanghvi, Y. S., Chapter 15, Antisense Research and Applications, pages 289-302, Crooke, S. T. and Lebleu, B., ed., CRC Press, 1993. Certain of these nucleobases are particularly useful for increasing the binding affinity of the oligonucleotides of the invention. These include 5-substituted pyrimidines, 6-azapyrimidines and N-2, N-6 and O-6 substituted purines, including 2-aminopropyladenine, 5-propynyluracil and 5-propynylcytosine. 5-Methylcytosine substitutions have been shown to increase nucleic acid duplex stability by 0.6-1.2 °C. (Id., pages 276-278) and are presently preferred base

substitutions, even more particularly when combined with 2'-methoxyethyl sugar modifications.

Representative United States patents relating to the preparation of certain of the above-noted modified nucleobases as well as other modified nucleobases include, but are not limited to, the above noted U.S. Pat. No. 3,687,808, as well as U.S. Pat. Nos 5,134,066; 5,459,255; 5,552,540; 5,594,121 and 5,596,091.

In certain instances, the oligonucleotides employed in the ligand-conjugated oligonucleotides may additionally or alternatively comprise one or more substituted sugar moieties. Oligonucleotides can comprise one of the following at the 2' position: OH; F; O-, S-, or N-alkyl, O-, S-, or N-alkenyl, or O, S- or N-alkynyl, wherein the alkyl, alkenyl and alkynyl may be substituted or unsubstituted $C_1$ to $C_{10}$ alkyl or $C_2$ to $C_{10}$ alkenyl and alkynyl. Particularly preferred are $O[(CH_2)_nO]_mCH_3$, $O(CH_2)_nOCH_3$, $O(CH_2)_nNH_2$, $O(CH_2)_nCH_3$, $O(CH_2)_nONH_2$, and $O(CH_2)_nON[(CH_2)_nCH_3)]_2$, where n and m are from 1 to about 10. Other oligonucleotides can comprise one of the following at the 2' position: $C_1$ to $C_{10}$ lower alkyl, substituted lower alkyl, alkaryl, aralkyl, O-alkaryl or O-aralkyl, SH, $SCH_3$, OCN, Cl, Br, CN, $CF_3$, $OCF_3$, $SOCH_3$, $SO_2\ CH_3$, $ONO_2$, $NO_2$, $N_3$, $NH_2$, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. A preferred modification includes 2'-methoxyethoxy [2'-O--$CH_2CH_2OCH_3$, also known as 2'-O-(2-methoxyethyl) or 2'-MOE], i.e., an alkoxyalkoxy group. A further preferred modification includes 2'-dimethylaminooxyethoxy, i.e., a $O(CH_2)_2ON(CH_3)_2$ group, also known as 2'-DMAOE, as described in U.S. Pat. No. 6,127,533, filed on Jan. 30, 1998.

Other modifications include 2'-methoxy (2'-O--$CH_3$), 2'-aminopropoxy (2'-$OCH_2CH_2CH_2NH_2$) and 2'-fluoro (2'-F). Similar modifications may also be made at other positions on the oligonucleotide, particularly the 3' position of the sugar on the 3' terminal nucleotide or in 2'-5' linked oligonucleotides.

As used herein, the term "sugar substituent group" or "2'-substituent group" includes groups attached to the 2'-position of the ribofuranosyl moiety with or without an oxygen atom. Sugar substituent groups include, but are not limited to, fluoro, O-alkyl, O-alkylamino, O-alkylalkoxy, protected O-alkylamino, O-alkylaminoalkyl, O-alkyl imidazole and polyethers of the formula $(O\text{-alkyl})_m$, wherein m is 1 to about 10. Preferred among these polyethers are linear and cyclic polyethylene glycols (PEGs), and (PEG)-containing groups, such as crown ethers and, inter alia, those which are disclosed by Delgardo et. al. (Critical Reviews in Therapeutic Drug Carrier Systems 1992, 9:249). Further sugar modifications are disclosed by Cook (Anti-fibrosis Drug Design, 1991, 6:585-607). Fluoro, O-alkyl, O-alkylamino, O-alkyl imidazole, O-alkylaminoalkyl, and alkyl amino substitution is described in U.S. Patent 6,166,197, entitled "Oligomeric Compounds having Pyrimidine Nucleotide(s) with 2' and 5' Substitutions,".

Additional sugar substituent groups amenable to the invention include 2'-SR and 2'-$NR_2$ groups, wherein each R is, independently, hydrogen, a protecting group or substituted or unsubstituted alkyl, alkenyl, or alkynyl. 2'-SR Nucleosides are disclosed in U.S. Pat. No. 5,670,633. The incorporation of 2'-SR monomer synthons is disclosed by Hamm et al. (J. Org. Chem., 1997, 62:3415-3420). 2'-NR nucleosides are disclosed by Goettingen, M., J. Org. Chem., 1996, 61, 6273-6281; and Polushin et al., Tetrahedron Lett., 1996, 37, 3227-3230. Further representative 2'-substituent groups include those having one of formula I or II:

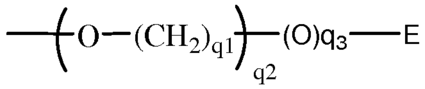

I

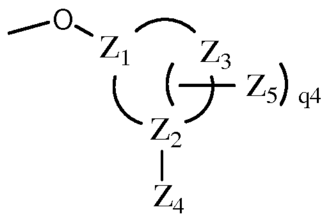

II

wherein,

E is $C_1$-$C_{10}$ alkyl, $N(Q_3)(Q_4)$ or $N=C(Q_3)(Q_4)$; each $Q_3$ and $Q_4$ is, independently, H, $C_1$-$C_{10}$ alkyl, dialkylaminoalkyl, a nitrogen protecting group, a tethered or untethered conjugate group, a linker to a solid support; or $Q_3$ and $Q_4$, together, form a nitrogen protecting group or a ring structure optionally including at least one additional heteroatom selected from N and O;

$q_1$ is an integer from 1 to 10;

$q_2$ is an integer from 1 to 10;

$q_3$ is 0 or 1;

$q_4$ is 0, 1 or 2;

each $Z_1$, $Z_2$ and $Z_3$ is, independently, $C_4$-$C_7$ cycloalkyl, $C_5$-$C_{14}$ aryl or $C_3$-$C_{15}$ heterocyclyl, wherein the heteroatom

in said heterocyclyl group is selected from oxygen, nitrogen and sulfur;

$Z_4$ is $OM_1$, $SM_1$, or $N(M_1)_2$; each $M_1$ is, independently, H, $C_1$-$C_8$ alkyl, $C_1$-$C_8$ haloalkyl, $C(=NH)N(H)M_2$, $C(=O)N(H)M_2$ or $OC(=O)N(H)M_2$; $M_2$ is H or $C_1$-$C_8$ alkyl; and

$Z_5$ is $C_1$-$C_{10}$ alkyl, $C_1$-$C_{10}$ haloalkyl, $C_2$-$C_{10}$ alkenyl, $C_2$-$C_{10}$ alkynyl, $C_6$-$C_{14}$ aryl, $N(Q_3)(Q_4)$, $OQ_3$, halo, $SQ_3$ or CN.

Representative 2'-O-sugar substituent groups of formula I are disclosed in U.S. Pat. No. 6,172,209, entitled "Capped 2'-Oxyethoxy Oligonucleotides,". Representative cyclic 2'-O-sugar substituent groups of formula II are disclosed in U.S. Patent 6,271,358, entitled "RNA Targeted 2'-Modified Oligonucleotides that are Conformationally Preorganized".

Sugars having O-substitutions on the ribosyl ring are also amenable to the invention. Representative substitutions for ring O include, but are not limited to, S, $CH_2$, CHF, and $CF_2$.

Oligonucleotides may also have sugar mimetics, such as cyclobutyl moieties, in place of the pentofuranosyl sugar. Representative United States patents relating to the preparation of such modified sugars include, but are not limited to, U.S. Pat. Nos. 5,359,044; 5,466,786; 5,519,134; 5,591,722; 5,597,909; 5,646,265 and 5,700,920.

Additional modifications may also be made at other positions on the oligonucleotide, particularly the 3' position of the sugar on the 3' terminal nucleotide. For example, one additional modification of the ligand-conjugated oligonucleotides of the invention involves chemically linking to the oligonucleotide one or more additional non-ligand moieties or conjugates which enhance the activity, cellular distribution or cellular uptake of the oligonucleotide. Such moieties include but are not limited to lipid moieties, such as a cholesterol moiety (Letsinger et al., Proc. Natl. Acad. Sci. USA, 1989, 86, 6553), cholic acid (Manoharan et al., Bioorg. Med. Chem. Lett., 1994, 4, 1053), a thioether, e.g., hexyl-S-tritylthiol (Manoharan et al., Ann. N.Y. Acad. Sci., 1992, 660, 306; Manoharan et al., Bioorg. Med. Chem. Let., 1993, 3, 2765), a thiocholesterol (Oberhauser et al., Nucl. Acids Res., 1992, 20, 533), an aliphatic chain, e.g., dodecandiol or undecyl residues (Saison-Behmoaras et al., EMBO J., 1991, 10, 111; Kabanov et al., FEBS Lett., 1990, 259, 327; Svinarchuk et al., Biochimie, 1993, 75, 49), a phospholipid, e.g., di-hexadecyl-rac-glycerol or triethylammonium 1,2-di-O-hexadecyl-rac-glycero-3-H-phosphonate (Manoharan et al., Tetrahedron Lett., 1995, 36, 3651; Shea et al., Nucl. Acids Res., 1990, 18, 3777), a polyamine or a polyethylene glycol chain (Manoharan et al., Nucleosides & Nucleotides, 1995, 14, 969), or adamantane acetic acid (Manoharan et al., Tetrahedron Lett., 1995, 36, 3651), a palmityl moiety (Mishra et al., Biochim. Biophys. Acta, 1995, 1264, 229), or an octadecylamine or hexylamino-carbonyl-oxycholesterol moiety (Crooke et al., J. Pharmacol. Exp. Ther., 1996, 277, 923).

The disclosure also includes compositions employing oligonucleotides that are substantially chirally pure with regard to particular positions within the oligonucleotides. Examples of substantially chirally pure oligonucleotides include those having phosphorothioate linkages that are at least 75% Sp or Rp (Cook et al., U.S. Pat. No. 5,587,361) and those having substantially chirally pure (Sp or Rp) alkylphosphonate, phosphoramidate or phosphotriester linkages (Cook, U.S. Pat. Nos. 5,212,295 and 5,521,302).

In certain instances, the oligonucleotide may be modified by a non-ligand group. A number of non-ligand molecules have been conjugated to oligonucleotides in order to enhance the activity, cellular distribution or cellular uptake of the oligonucleotide, and procedures for performing such conjugations are available in the scientific literature. Such non-ligand moieties have included lipid moieties, such as cholesterol (Letsinger et al., Proc. Natl. Acad. Sci. USA, 1989, 86:6553), cholic acid (Manoharan et al., Bioorg. Med. Chem. Lett., 1994, 4:1053), a thioether, e.g., hexyl-S-tritylthiol (Manoharan et al., Ann. N.Y. Acad. Sci., 1992, 660:306; Manoharan et al., Bioorg. Med. Chem. Let., 1993, 3:2765), a thiocholesterol (Oberhauser et al., Nucl. Acids Res., 1992, 20:533), an aliphatic chain, e.g., dodecandiol or undecyl residues (Saison-Behmoaras et al., EMBO J., 1991, 10:111; Kabanov et al., FEBS Lett., 1990, 259:327; Svinarchuk et al., Biochimie, 1993, 75:49), a phospholipid, e.g., di-hexadecyl-rac-glycerol or triethylammonium 1,2-di-O-hexadecyl-rac-glycero-3-H-phosphonate (Manoharan et al., Tetrahedron Lett., 1995, 36:3651; Shea et al., Nucl. Acids Res., 1990, 18:3777), a polyamine or a polyethylene glycol chain (Manoharan et al., Nucleosides & Nucleotides, 1995, 14:969), or adamantane acetic acid (Manoharan et al., Tetrahedron Lett., 1995, 36:3651), a palmityl moiety (Mishra et al., Biochim. Biophys. Acta, 1995, 1264:229), or an octadecylamine or hexylamino-carbonyl-oxycholesterol moiety (Crooke et al., J. Pharmacol. Exp. Ther., 1996, 277:923). Typical conjugation protocols involve the synthesis of oligonucleotides bearing an aminolinker at one or more positions of the sequence. The amino group is then reacted with the molecule being conjugated using appropriate coupling or activating reagents. The conjugation reaction may be performed either with the oligonucleotide still bound to the solid support or following cleavage of the oligonucleotide in solution phase. Purification of the oligonucleotide conjugate by HPLC typically affords the pure conjugate.

Alternatively, the molecule being conjugated may be converted into a building block, such as a phosphoramidite, via an alcohol group present in the molecule or by attachment of a linker bearing an alcohol group that may be phosphorylated.

Importantly, each of these approaches may be used for the synthesis of ligand conjugated oligonucleotides. Amino linked oligonucleotides may be coupled directly with ligand via the use of coupling reagents or following activation of the ligand as an NHS or pentfluorophenolate ester. Ligand phosphoramidites may be synthesized via the attachment of an aminohexanol linker to one of the carboxyl groups followed by phosphitylation of the terminal alcohol functionality.

Other linkers, such as cysteamine, may also be utilized for conjugation to a chloroacetyl linker present on a synthesized oligonucleotide.

The person skilled in the art is readily aware of methods to introduce the molecules disclosed into cells, tissues or organisms. Corresponding examples have also been provided in the detailed description of the invention above. For example, the nucleic acid molecules or the vectors encoding for at least one strand of the inventive dsRNAs may be introduced into cells or tissues by methods known in the art, like transfections etc.

Also for the introduction of dsRNA molecules, means and methods have been provided. For example, targeted delivery by glycosylated and folate-modified molecules, including the use of polymeric carriers with ligands, such as galactose and lactose or the attachment of folic acid to various macromolecules allows the binding of molecules to be delivered to folate receptors. Targeted delivery by peptides and proteins other than antibodies, for example, including RGD-modified nanoparticles to deliver siRNA *in vivo* or multicomponent (nonviral) delivery systems including short cyclodextrins, adamantine-PEG are known. Yet, also the targeted delivery using antibodies or antibody fragments, including (monovalent) Fab-fragments of an antibody (or other fragments of such an antibody) or single-chain antibodies are envisaged. Injection approaches for target directed delivery comprise, inter alia, hydrodynamic i.v. injection. Also cholesterol conjugates of dsRNA may be used for targeted delivery, whereby the conjugation to lipohilic groups enhances cell uptake and improve pharmacokinetics and tissue biodistribution of oligonucleotides. Also cationic delivery systems are known, whereby synthetic vectors with net positive (cationic) charge to facilitate the complex formation with the polyanionic nucleic acid and interaction with the negatively charged cell membrane. Such cationic delivery systems comprise also cationic liposomal delivery systems, cationic polymer and peptide delivery systems. Other delivery systems for the cellular uptake of dsRNA/siRNA are aptamer-ds/siRNA. Also gene therapy approaches can be used to deliver the inventive dsRNA molecules or nucleic acid molecules encoding the same. Such systems comprise the use of non-pathogenic virus, modified viral vectors, as well as deliveries with nanoparticles or liposomes. Other delivery methods for the cellular uptake of dsRNA are extracorporeal, for example *ex vivo* treatments of cells, organs or tissues. Certain of these technologies are described and summarized in publications, like Akhtar (2007), Journal of Clinical Investigation 117, 3623-3632, Nguyen et al. (2008), Current Opinion in Moleculare Therapeutics 10, 158-167, Zamboni (2005), Clin. Cancer Res. 11, 8230-8234 or Ikeda et al. (2006), Pharmaceutical Research 23, 1631-1640.

Unless otherwise defined, all technical and scientific terms used herein have the same meaning as commonly understood by one of ordinary skill in the art to which this invention belongs.

The above provided embodiments and items are now illustrated with the following examples.

**Description of figures and appended tables:**

**Table 1** - Core sequences of dsRNAs targeting human RRM2 gene Letters in capitals represent RNA nucleotides.

**Table 2 -** Characterization of dsRNAs targeting human RRM2: Activity testing with single dose in HeLa-S3 cells. Letters in capitals represent RNA nucleotides, lower case letters "c", "g", "a" and "u" represent 2' O-methyl-modified nucleotides, "s" represents phosphorothioate, and "dT" deoxythymidine. S.d.= standard deviation, %mRNA = mean mRNA knockdown.

**Table 3 -** Characterization of dsRNAs targeting human RRM2: Activity testing for dose response in HeLa-S3 cells. IC 50: 50 % inhibitory concentration, IC 80: 80 % inhibitory concentration, IC 20: 20 % inhibitory concentration.

**Table 4** - Characterization of dsRNAs targeting human RRM2: Stability and Cytokine Induction, t ½: half-life of a strand as defined in examples, PBMC: Human peripheral blood mononuclear cells.

**Table 5** - Core sequences of dsRNAs targeting human RRM2 gene and their modified counterparts. Letters in capitals represent RNA nucleotides, lower case letters "c", "g", "a" and "u" represent 2' O-methyl-modified nucleotides, "s" represents phosphorothioate, "dT" deoxythymidine.

**Table 6 -** Sequences of bDNA probes for determination of human RRM2. LE= label extender, CE= capture extender, BL= blocking probe.

**Table 7 -** Sequences of bDNA probes for determination of human GAPDH. LE= label extender, CE= capture extender, BL= blocking probe.

**Table 8 -** mRNA knockdown and cell viability dose-response curves, IC50 summary.

**Table 9 -** Time course of mRNA knockdown: >80% mRNA knockdown observed at 24 hr, Percent mRNA knockdown with 5 nM siRNA,
relative to mock transfection.

**Figure 1 -** RRM2 protein knockdown dose-response 24h post-transfection. A: Western blot B: Quantitation of RRM2 protein in Western blot.

**Figure 2 -** Cell cycle analysis of HepG2 cells with dsRNA 477/839: FACS analysis following propidium iodide

staining indicates that following siRNA treatment cells accumulate in S-phase.

**Figure 3 -** Cell cycle analysis of HepG2 cells with dsRNA 477/839: FACS analysis following propidium iodide staining indicates that following siRNA treatment cells accumulate in S-phase.

**Figure 4 -** Cell cycle analysis of HLF cells with dsRNA 477/839: FACS analysis following propidium iodide staining indicates that following siRNA treatment cells accumulate in S-phase.

**Figure 5 -** Cell cycle analysis of HLF cells with dsRNA 477/839: FACS analysis following propidium iodide staining indicates that following siRNA treatment cells accumulate in S-phase.

**Figure 6 -** Apoptosis assay in HepG2 cells. dsRNA 477/839 activates caspase-3/7.

**Figure 7 -** Apoptosis assay in HLF cells. dsRNA 477/839 activates caspase-3/7.

**Figure 8 -** Effects on components of DNA damage pathway 48 hr post-transfection with RRM2 siRNA (5 nM). RRM2 knockdown activates the pathway (pChk1 and γ-H2AX) even in the absence of phleomycin-induced DNA damage.

## EXAMPLES

### Identification of dsRNAs for therapeutic use

dsRNA design was carried out to identify dsRNAs specifically targeting human RRM2 for therapeutic use. First, the known mRNA sequences of human (Homo sapiens) RRM2 (NM_001034.3 and NM_001165931.1 listed as SEQ ID NO. 1013 and 1014) were downloaded from NCBI Genbank.

The cynomolgous monkey (Macaca fascicularis) RRM2 gene was sequenced (see SEQ ID NO. 1015)

The cynomolgus monkey sequence (SEQ ID NO. 1015) was examined together with the human RRM2 mRNA sequences (SEQ ID NO. 1013 and 1014) by computer analysis to identify homologous sequences of 19 nucleotides that yield RNA interference (RNAi) agents cross-reactive to both sequences.

In identifying RNAi agents, the selection was limited to 19mer sequences having at least 2 mismatches to any other sequence in the human RefSeq database (release 38), which we assumed to represent the comprehensive human transcriptome, by using a proprietary algorithm.

All sequences containing 4 or more consecutive U's (poly-U sequences) or G's (poly-G sequences) were excluded from the synthesis.

The sequences thus identified formed the basis for the synthesis of the RNAi agents in appended Tables 1, 2 and 5. dsRNAs cross-reactive to human as well as cynomolgous monkey were defined as most preferable for therapeutic use.

### dsRNA synthesis

Where the source of a reagent is not specifically given herein, such reagent may be obtained from any supplier of reagents for molecular biology at a quality/purity standard for application in molecular biology.

Single-stranded RNAs were produced by solid phase synthesis on a scale of 1 μmole using an Expedite 8909 synthesizer (Applied Biosystems, Applera Deutschland GmbH, Darmstadt, Germany) and controlled pore glass (CPG, 500Å, Proligo Biochemie GmbH, Hamburg, Germany) as solid support. RNA and RNA containing *2'-O*-methyl nucleotides were generated by solid phase synthesis employing the corresponding phosphoramidites and *2'-O*-methyl phosphoramidites, respectively (Proligo Biochemie GmbH, Hamburg, Germany). These building blocks were incorporated at selected sites within the sequence of the oligoribonucleotide chain using standard nucleoside phosphoramidite chemistry such as described in Current protocols in nucleic acid chemistry, Beaucage, S.L. et al. (Edrs.), John Wiley & Sons, Inc., New York, NY, USA. Phosphorothioate linkages were introduced by replacement of the iodine oxidizer solution with a solution of the Beaucage reagent (Chruachem Ltd, Glasgow, UK) in acetonitrile (1%). Further ancillary reagents were obtained from Mallinckrodt Baker (Griesheim, Germany).

Deprotection and purification of the crude oligoribonucleotides by anion exchange HPLC were carried out according to established procedures. Yields and concentrations were determined by UV absorption of a solution of the respective RNA at a wavelength of 260 nm using a spectral photometer (DU 640B, Beckman Coulter GmbH, UnterschleiBheim, Germany). Double stranded RNA was generated by mixing an equimolar solution of complementary strands in annealing buffer (20 mM sodium phosphate, pH 6.8; 100 mM sodium chloride), heated in a water bath at 85 - 90°C for 3 minutes and cooled to room temperature over a period of 3 - 4 hours. The annealed RNA solution was stored at -20 °C until use.

### Activity testing

The activity of the RRM2 dsRNAs for therapeutic use described above was tested in HeLa-S3 cells. Cells in

culture were used for quantitation of RRM2 mRNA by branched DNA in total mRNA derived from cells incubated with RRM2-targeting dsRNAs.

HeLa-S3 cells were obtained from American Type Culture Collection (Rockville, Md., cat. No. CCL-2.2) and cultured in Ham's F12 (Biochrom AG, Berlin, Germany, cat. No. FG 0815) supplemented to contain 10% fetal calf serum (FCS) (Biochrom AG, Berlin, Germany, cat. No. S0115), Penicillin 100 U/ml, Streptomycin 100 mg/ml (Biochrom AG, Berlin, Germany, cat. No. A2213) at 37°C in an atmosphere with 5% CO2 in a humidified incubator (Heraeus HERAcell, Kendro Laboratory Products, Langenselbold, Germany).

Cell seeding and transfection of dsRNA were performed at the same time. For transfection with dsRNA, cells were seeded at a density of 2.0 times. 10.sup.4 cells/well in 96-well plates. Transfection with dsRNA was carried out with lipofectamine 2000 (Invitrogen GmbH, Karlsruhe, Germany, cat.No. 11668-019) as described by the manufacturer. In a first single dose experiment dsRNAs were transfected at a concentration of 50nM. In a second single dose experiment most active dsRNAs were reanalyzed at 500pM. Most potent dsRNAs and modification variants thereof were tested for improved silencing of RRM2 in single dose at 30pM. Very effective dsRNAs from single dose screens were further characterized by dose response curves. For this, transfections were performed as described for the single dose screen above, but with the following concentrations of dsRNA (nM): 24, 6, 1.5, 0.375, 0.0938, 0.0234, 0.0059, 0.0015, 0.0004 and 0.0001 nM . After transfection cells were incubated for 24 h at 37°C and 5 % CO2 in a humidified incubator (Heraeus HERAcell, Kendro Laboratory Products, Langenselbold, Germany). bDNA Assay Kit QuantiGene 2.0 (Panomics/Affymetrix, Fremont, USA, Cat-No: 15735) was used for quantification of RRM2 mRNA, while QuantiGene Assay 1.0 (Panomics/Affymetrix, Fremont, USA, Cat-No: QG0004) was used for quantification of GAPDH mRNA. 24 hours after transfection cells were harvested and lysed at 53°C following procedures recommended by the manufacturer Panomics/Affymetrix for bDNA quantitation of mRNA. Afterwards, 50 μl of the lysates were incubated with probesets specific to human RRM2 and human GAPDH (sequence of probesets see appended tables 7 and 8) and processed according to the manufacturer's protocol for QuantiGene Assay Kit 1 or 2, respectively. Chemoluminescence was measured in a Victor2-Light (Perkin Elmer, Wiesbaden, Germany) as RLUs (relative light units) and values obtained with the human RRM2 probeset were normalized to the respective human GAPDH values for each well. Unrelated control dsRNAs were used as a negative control.

Inhibition data are given in appended tables 2 and 3.

## Stability of dsRNAs

Stability of dsRNAs targeting human RRM2 was determined in *in vitro* assays with either human or mouse serum by measuring the half-life of each single strand.

Measurements were carried out in triplicates for each time point, using 3μl 50μM dsRNA sample mixed with 30μl human serum (Sigma) or mouse serum (Sigma). Mixtures were incubated for either 0min, 30min, 1h, 3h, 6h, 24h, or 48h at 37°C. As control for unspecific degradation dsRNA was incubated with 30μl 1x PBS pH 6.8 for 48h. Reactions were stopped by the addition of 4μl proteinase K (20mg/ml), 25μl of "Tissue and Cell Lysis Solution" (Epicentre) and 38μl Millipore water for 30 min at 65°C. Samples were afterwards spin filtered through a 0.2 μm 96 well filter plate at 1400 rpm for 8 min, washed with 55μl Millipore water twice and spin filtered again.

For separation of single strands and analysis of remaining full length product (FLP), samples were run through an ion exchange Dionex Summit HPLC under denaturing conditions using as eluent A 20mM Na3PO4 in 10% ACN pH=11 and for eluent B 1 M NaBr in eluent A.

The following gradient was applied:

| Time | %A | %B |
|---|---|---|
| -1.0 min | 75 | 25 |
| 1.00 min | 75 | 25 |
| 19.0 min | 38 | 62 |
| 19.5 min | 0 | 100 |
| 21.5 min | 0 | 100 |
| 22.0 min | 75 | 25 |
| 24.0 min | 75 | 25 |

For every injection, the chromatograms were integrated automatically by the Dionex Chromeleon 6.60 HPLC software, and were adjusted manually if necessary. All peak areas were corrected to the internal standard (IS) peak and normalized to the incubation at t=0 min. The area under the peak and resulting remaining FLP was calculated for each single strand and triplicate separately. Half-life (t1/2) of a strand was defined by the average time point [h] for triplicates

at which half of the FLP was degraded. Results are given in appended table 4.

**Cytokine induction**

Potential cytokine induction of dsRNAs was determined by measuring the release of INF-a and TNF-a in an *in vitro* PBMC assay.

Human peripheral blood mononuclear cells (PBMC) were isolated from buffy coat blood of two donors by Ficoll centrifugation at the day of transfection. Cells were transfected in quadruplicates with dsRNA and cultured for 24h at 37°C at a final concentration of 130nM in Opti-MEM, using either Gene Porter 2 (GP2) or DOTAP. dsRNA sequences that were known to induce INF-a and TNF-a in this assay, as well as a CpG oligo, were used as positive controls. Chemical conjugated dsRNA or CpG oligonucleotides that did not need a transfection reagent for cytokine induction, were incubated at a concentration of 500nM in culture medium. At the end of incubation, the quadruplicate culture supernatant were pooled.

INF-a and TNF-a was then measured in these pooled supernatants by standard sandwich ELISA with two data points per pool. The degree of cytokine induction was expressed relative to positive controls using a score from 0 to 5, with 5 indicating maximum induction. Results are given in appended table 4.

**Cell culture and siRNA transfections.** HepG2, HLF and A549 cells were obtained from ATCC and maintained in the recommended media, supplemented with 10% fetal bovine serum and 2mM 1-Glutamine (HepG2: MEME; HLF and A549: DMEM). Cells were transfected using 0.1 μl DharmaFect 1 (Thermo Fisher) per well of a 96-well plate, with each well containing a final volume of 100 μl growth media. Transfections in 6-well plates were carried out in a similar manner, with volumes adjusted for the larger well size. Transfections were performed using a "reverse transfection" protocol, in which cells (HepG2: 5,000 cells; HLF: 2,000 cells; A540: 4,000 cells) were mixed with transfection mix immediately prior to plating.

**bDNA assay.** QuantiGene branched DNA assays (Affymetrix) for mRNA quantitation were run according to the manufacturer's directions using probe sets designed and synthesized by Affymetrix. 40 ul of lysate were used, and signal was normalized to expression of cyclophin B. Results are summarized in table 8 and 9.

**Cell growth assays.** Cell-Titer Glo assays (Promega) were performed according to the manufacturer's directions. Real-time growth assays were performed on the xCELLigence instrument (Roche) using E-Plate 96 plates. Cell index was measured every hour, and area under the curve calculated at particular time points. Results are summarized in table 8.

**Western blots.** Antibodies for Western blots were obtained from the following sources: Santa Cruz Biotechnology, Inc. (goat polyclonal anti-RRM2, catalog number sc-10844; goat anti-mouse-HRP, catalog number sc-2005); Cell Signaling Technology (rabbit polyclonal anti-Chkl, catalog number 2345; rabbit polyclonal anti-pChkl (S317), catalog number 2344; goat anti-rabbit-HRP, catalog number 7074; loading control antibody sampler kit (HRP conjugate), catalog number 4670); R&D Systems (mouse monoclonal anti-H2AX, catalog number MAB3406; rabbit polyclonal anti-gamma-H2AX, catalog number AF2288); Promega (donkey anti-goat-HRP, catalog number V8051).

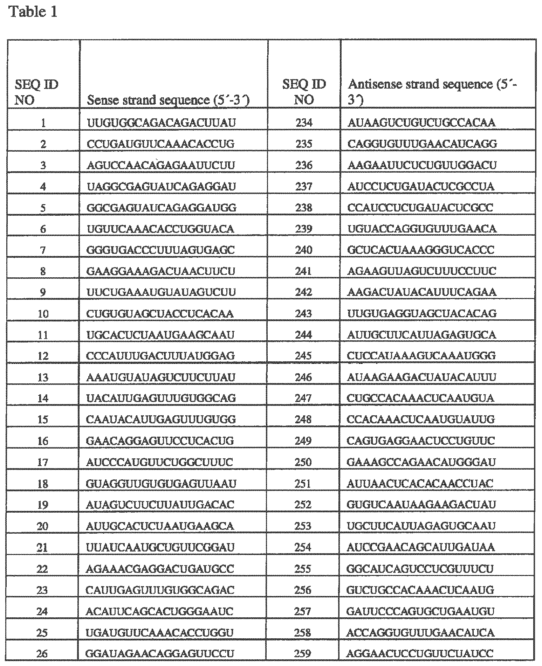

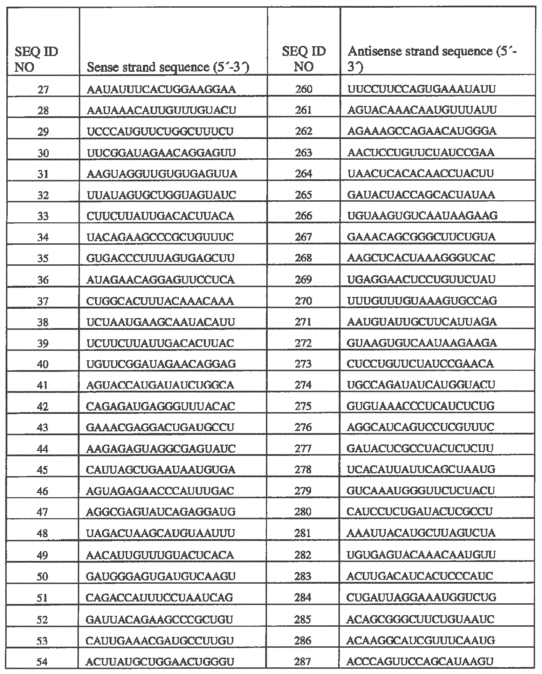

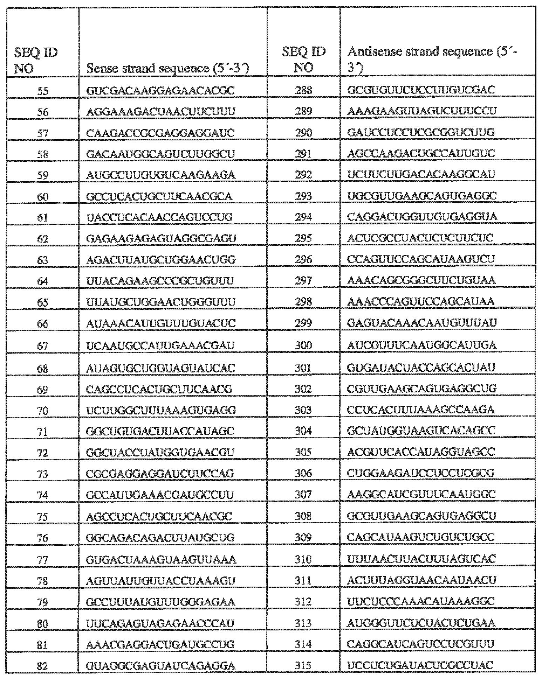

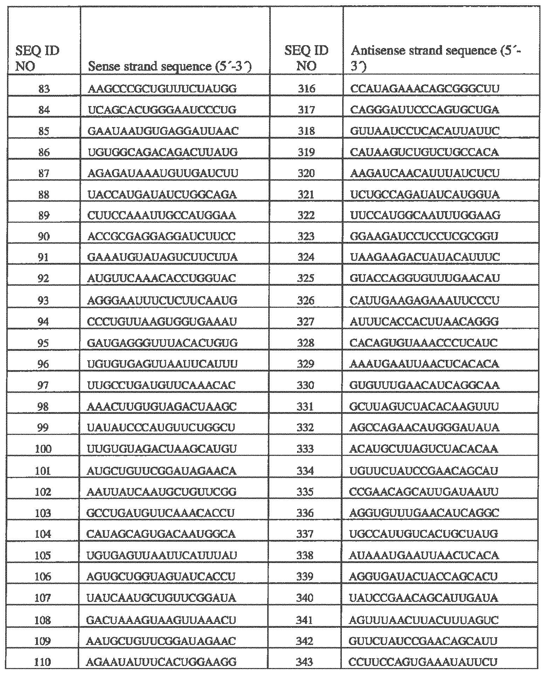

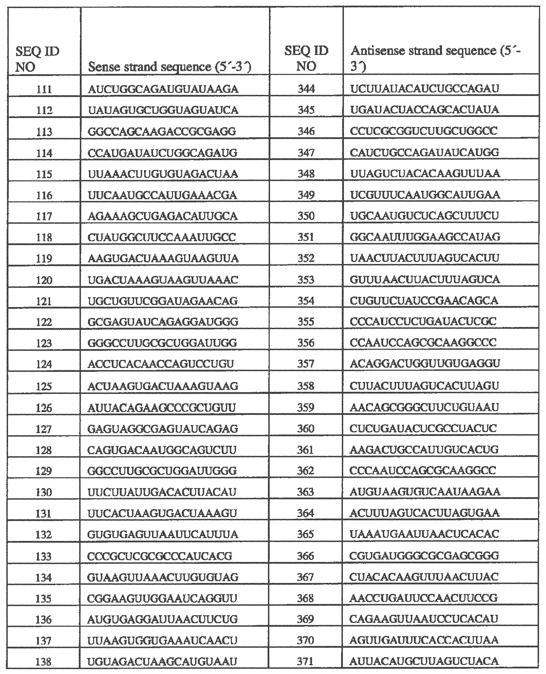

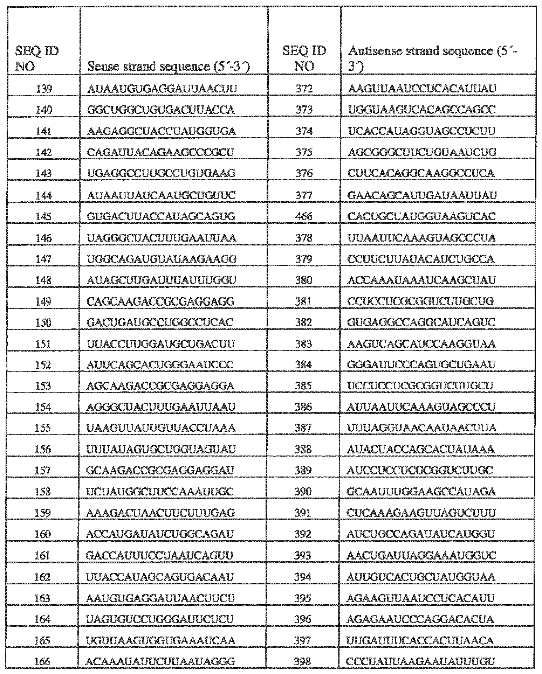

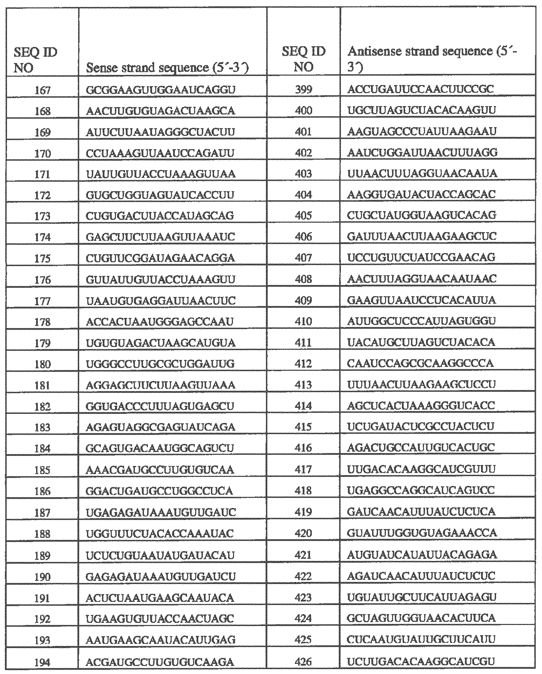

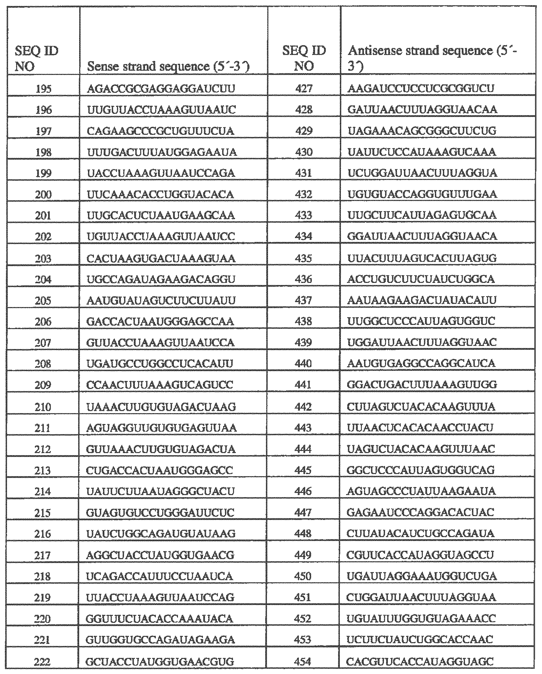

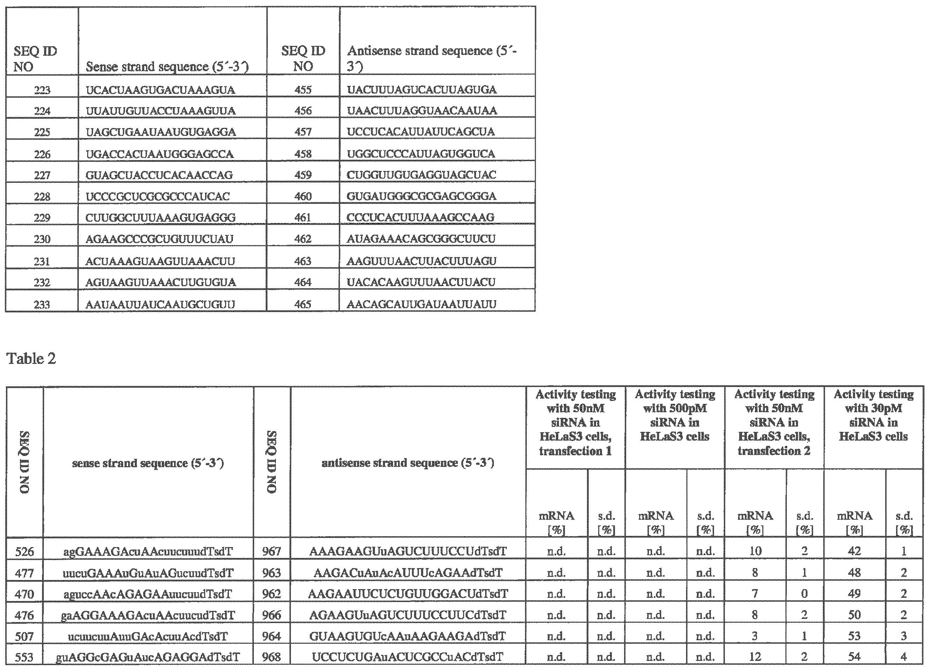

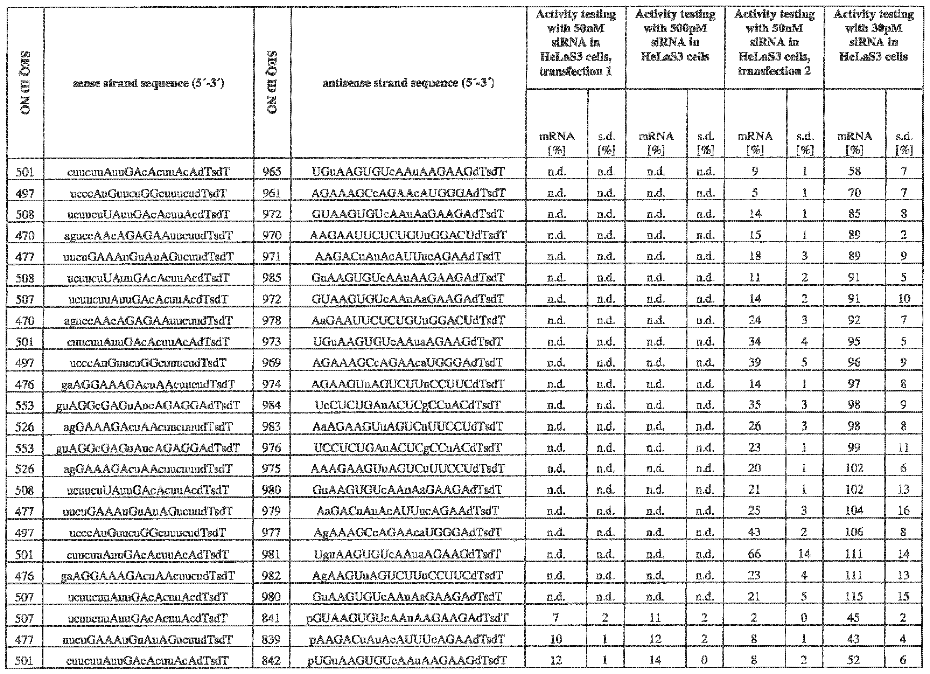

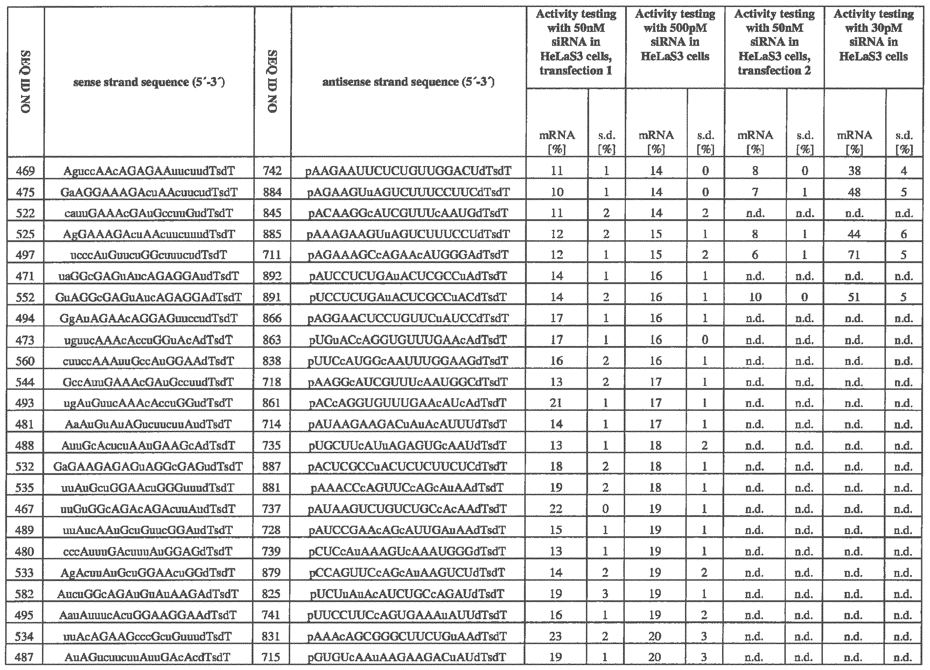

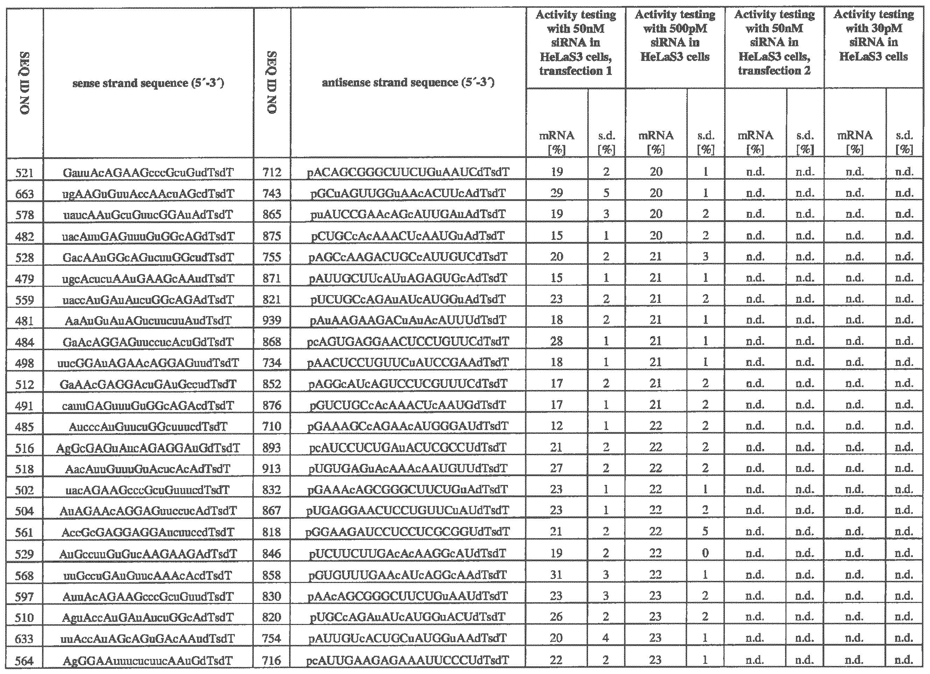

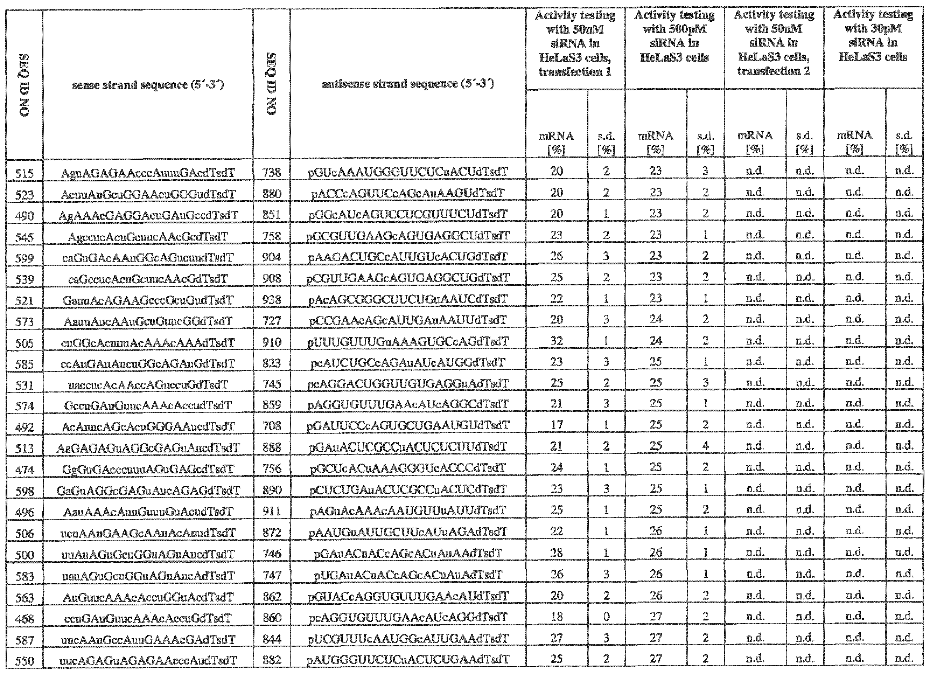

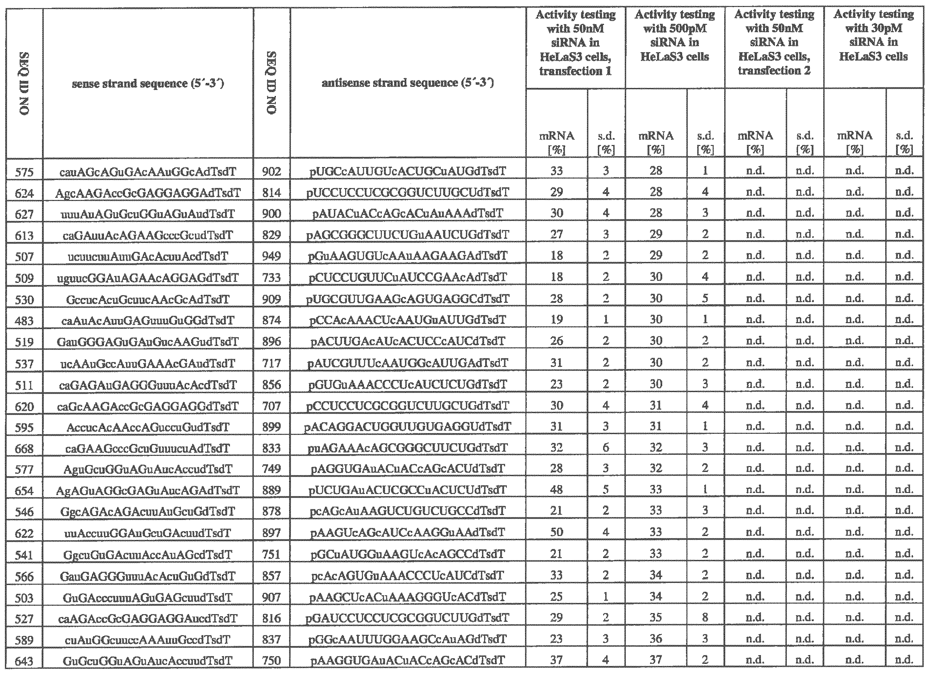

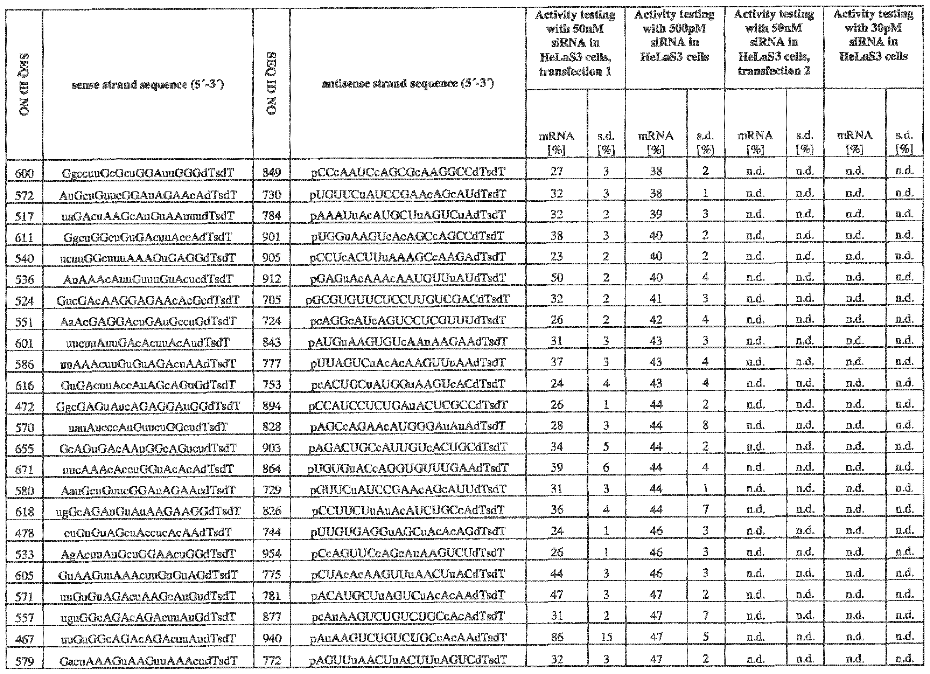

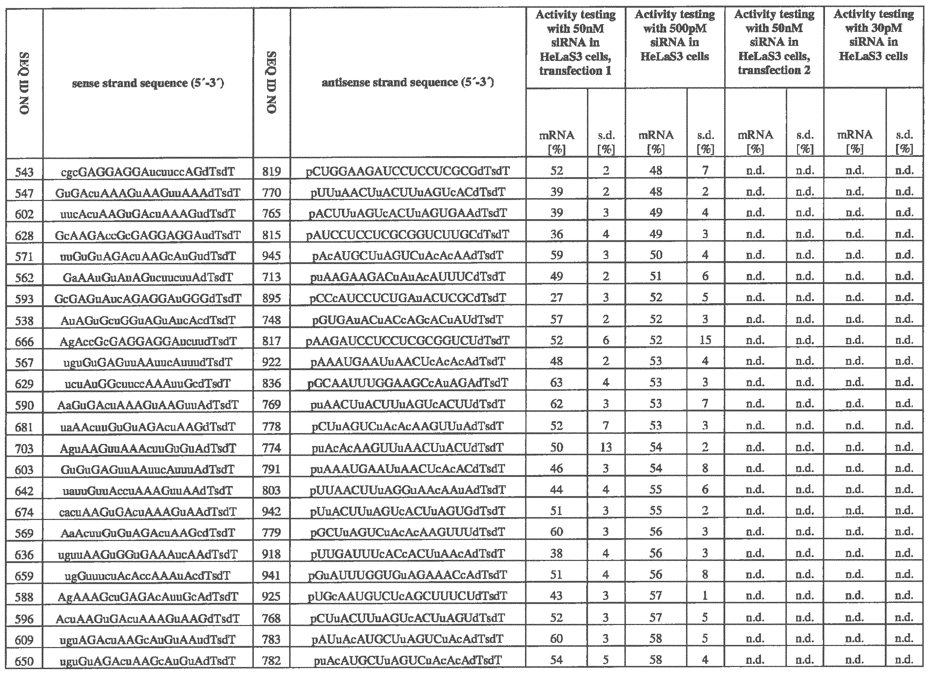

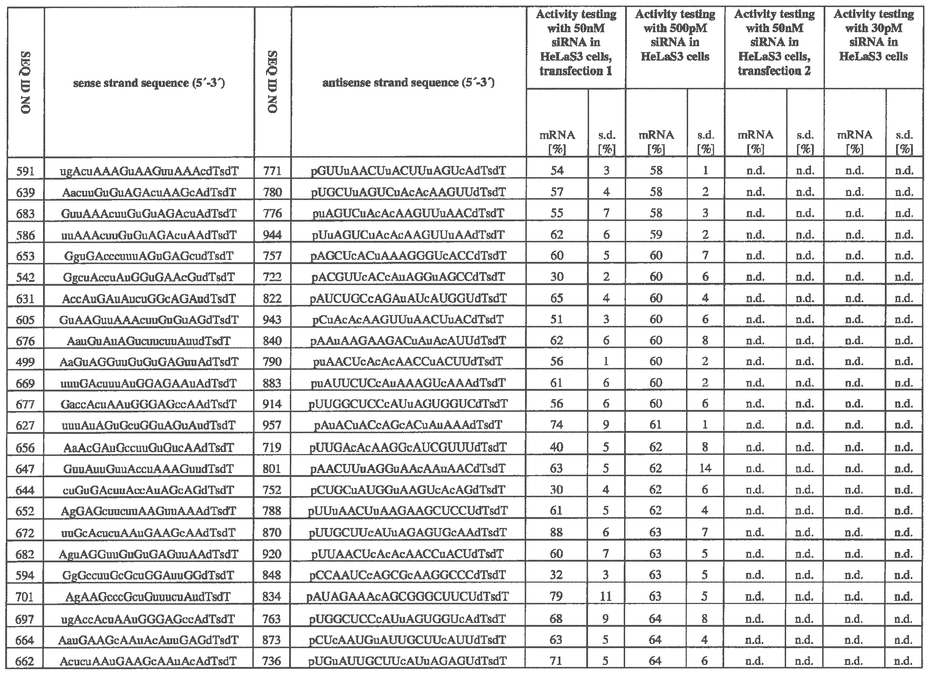

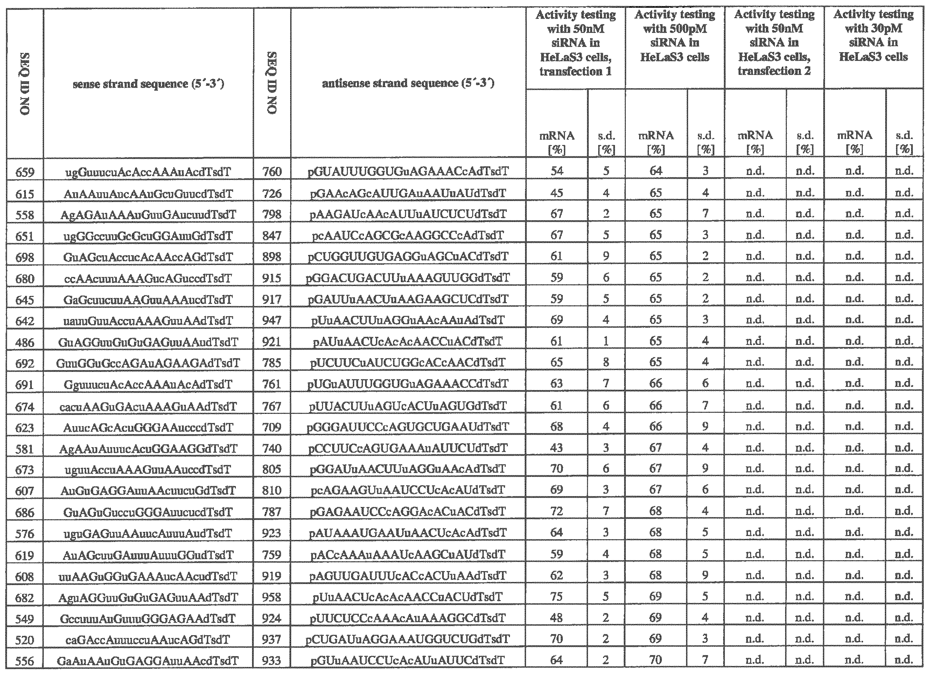

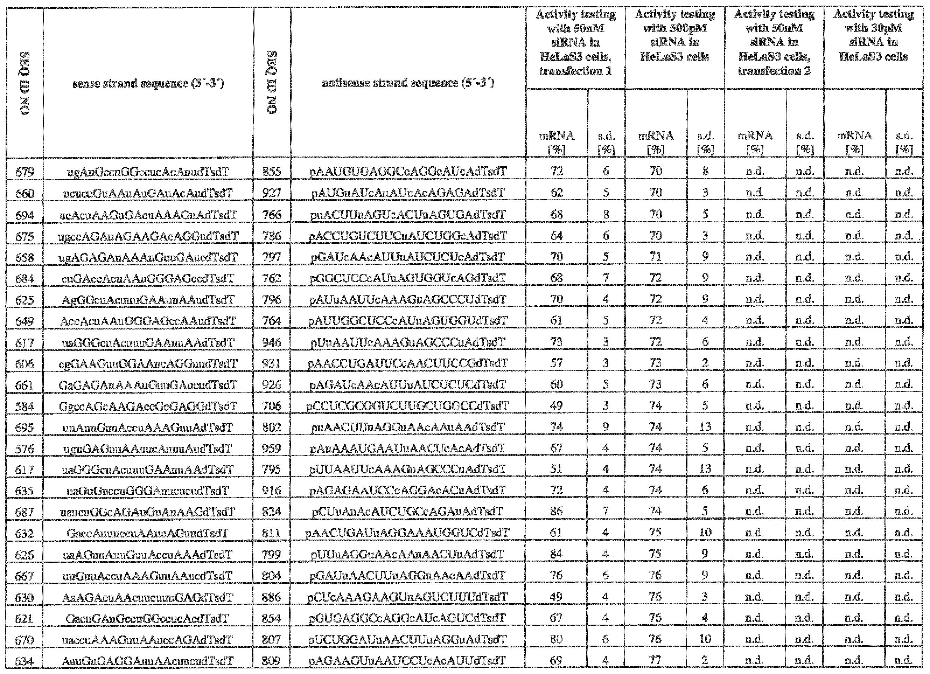

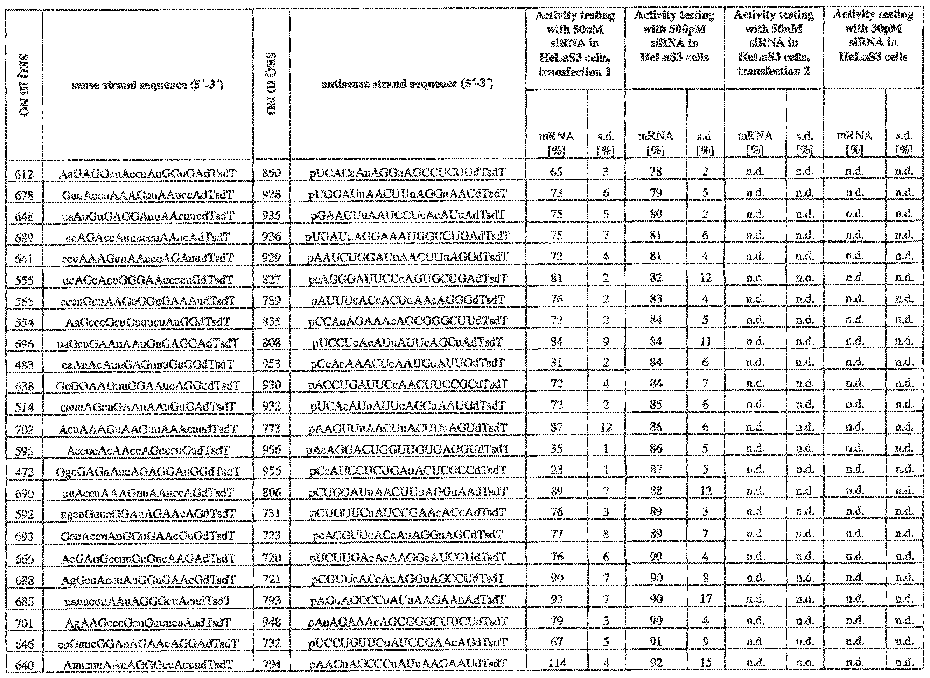

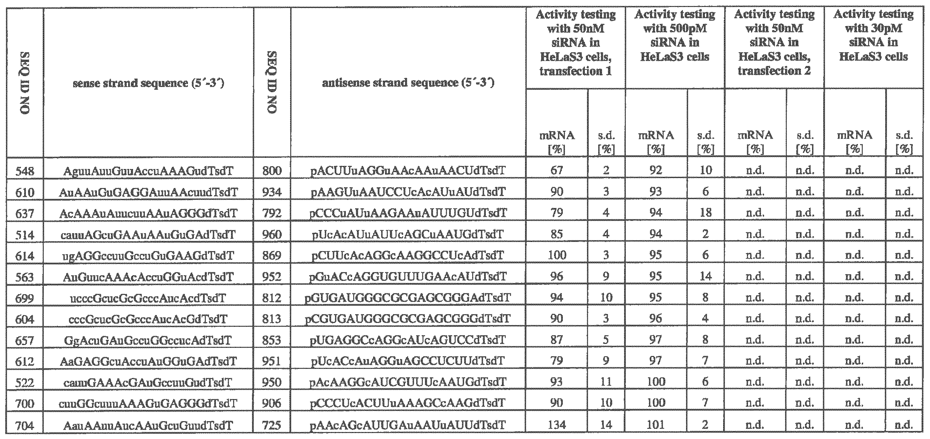

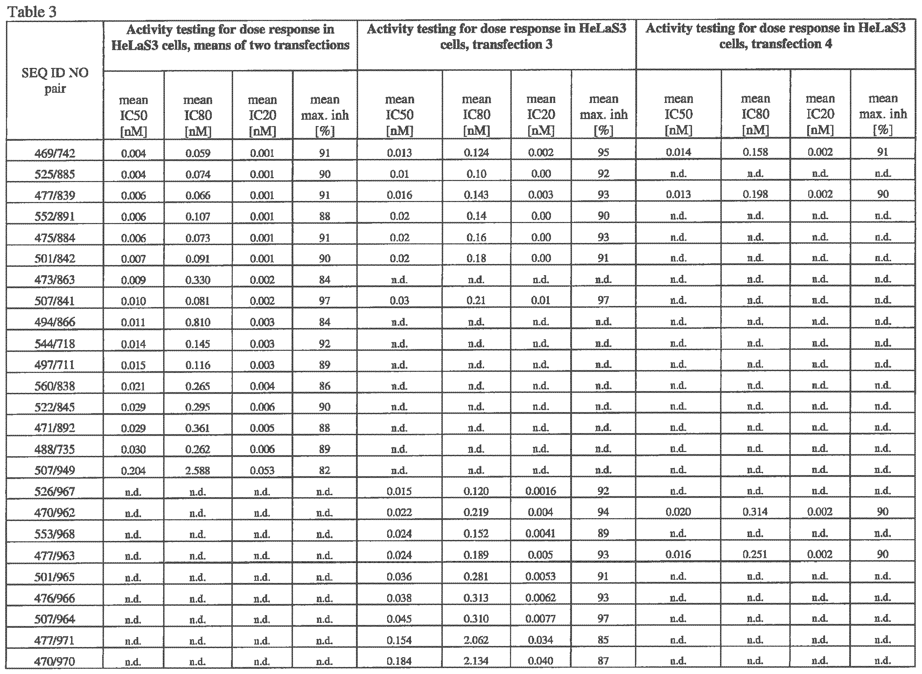

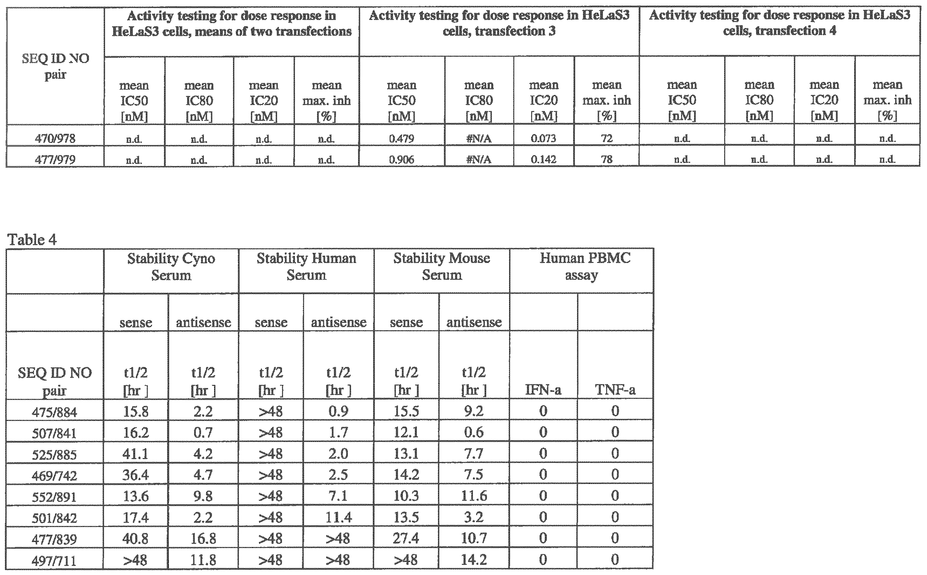

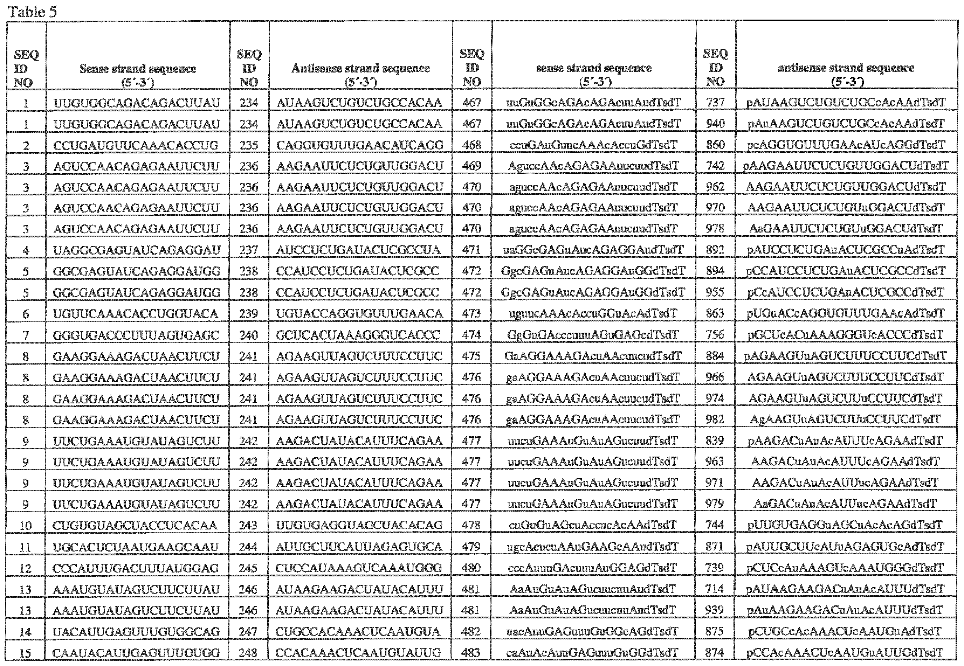

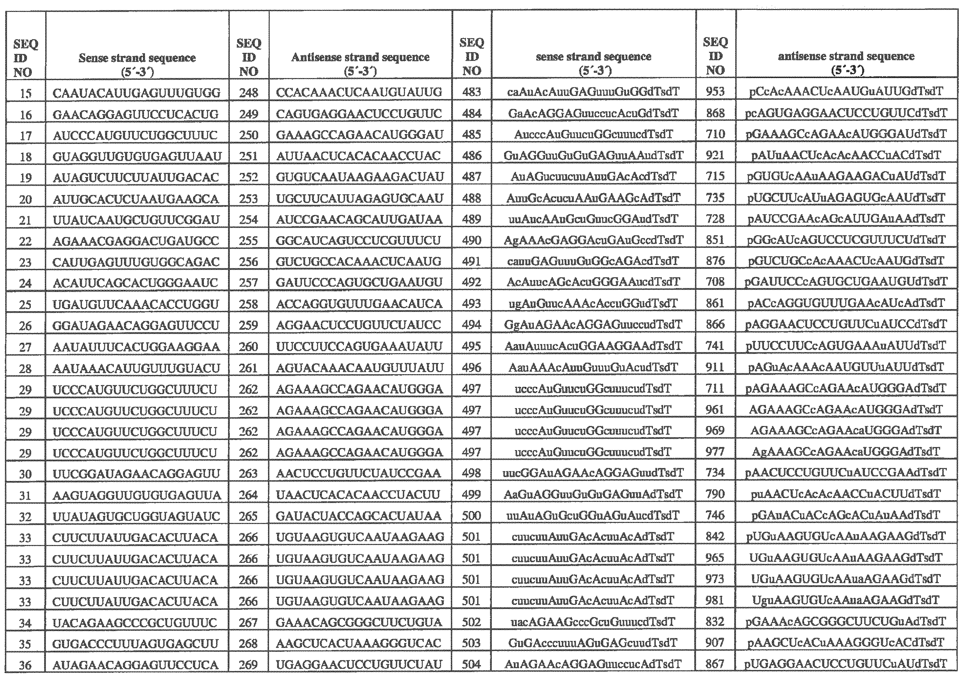

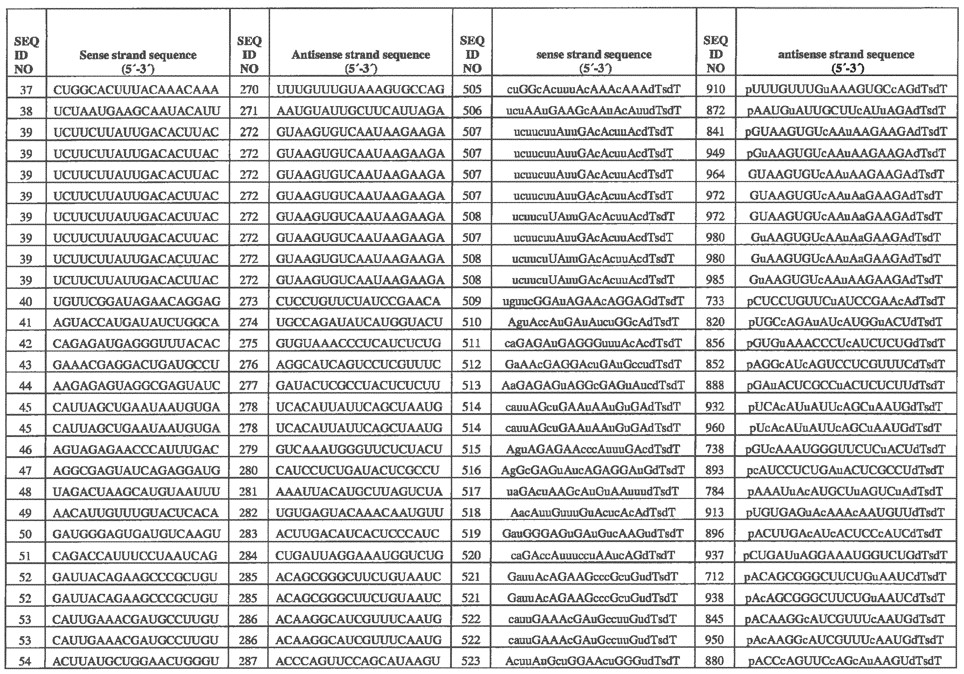

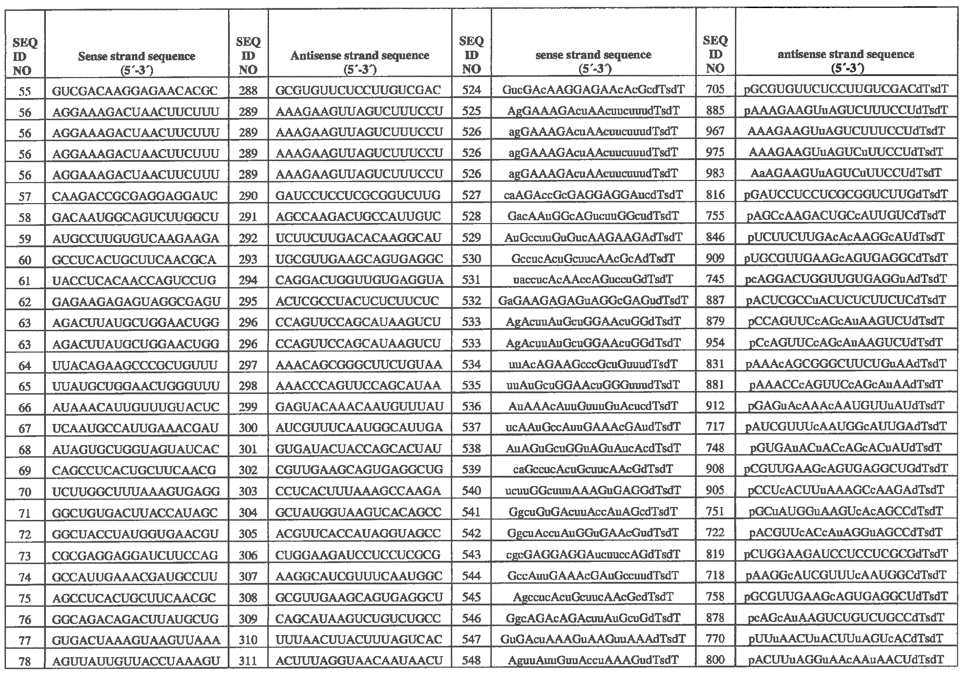

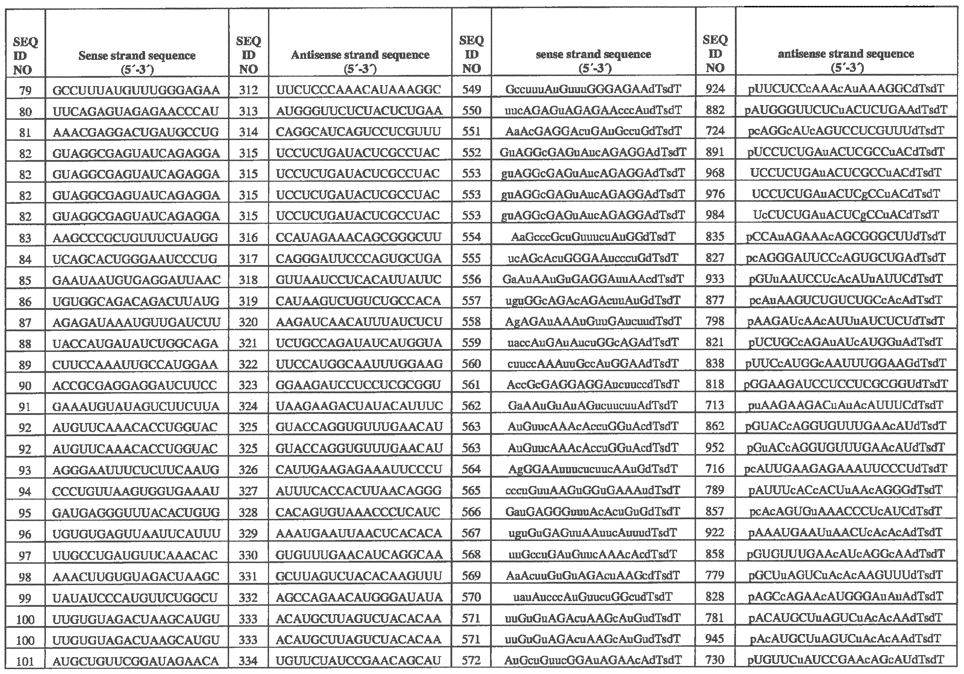

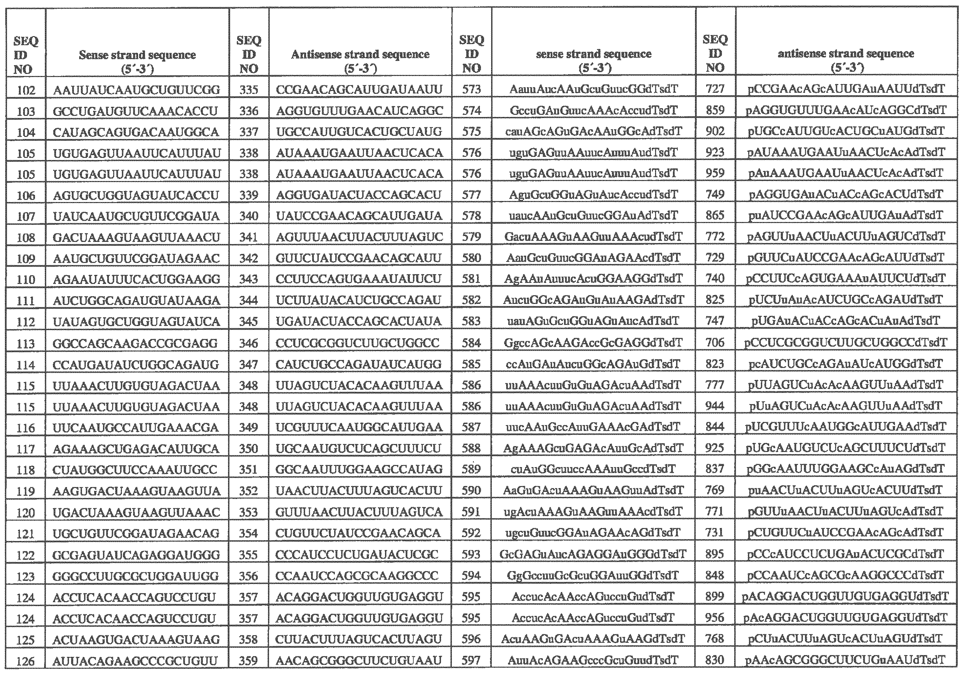

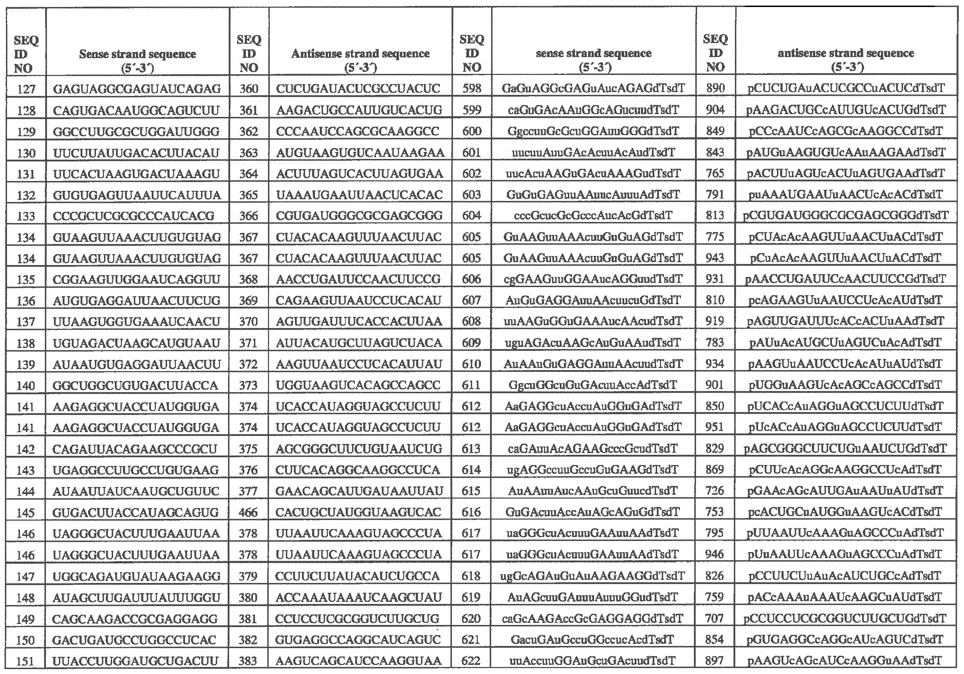

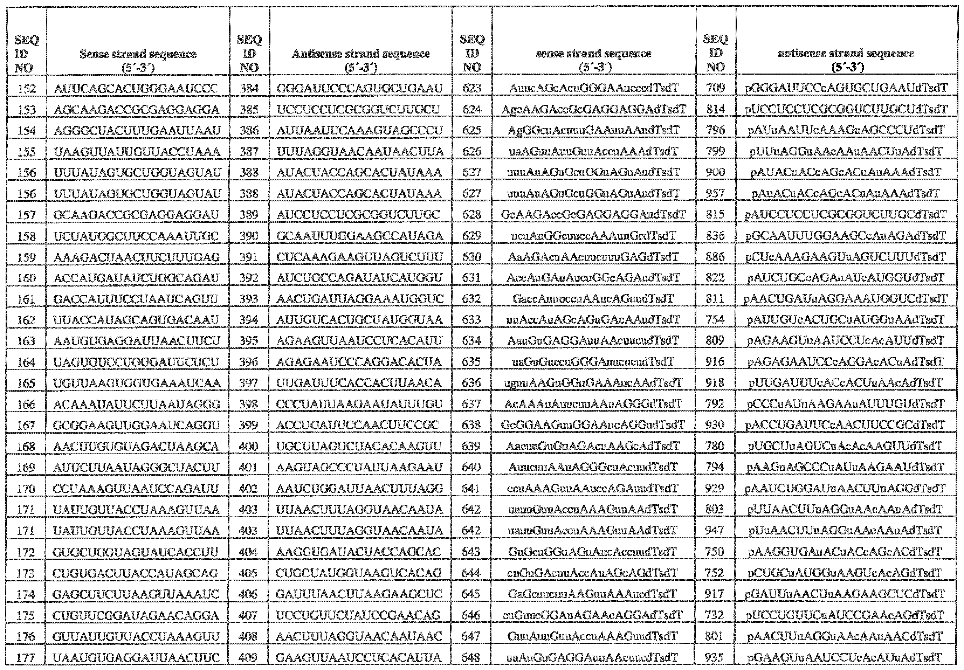

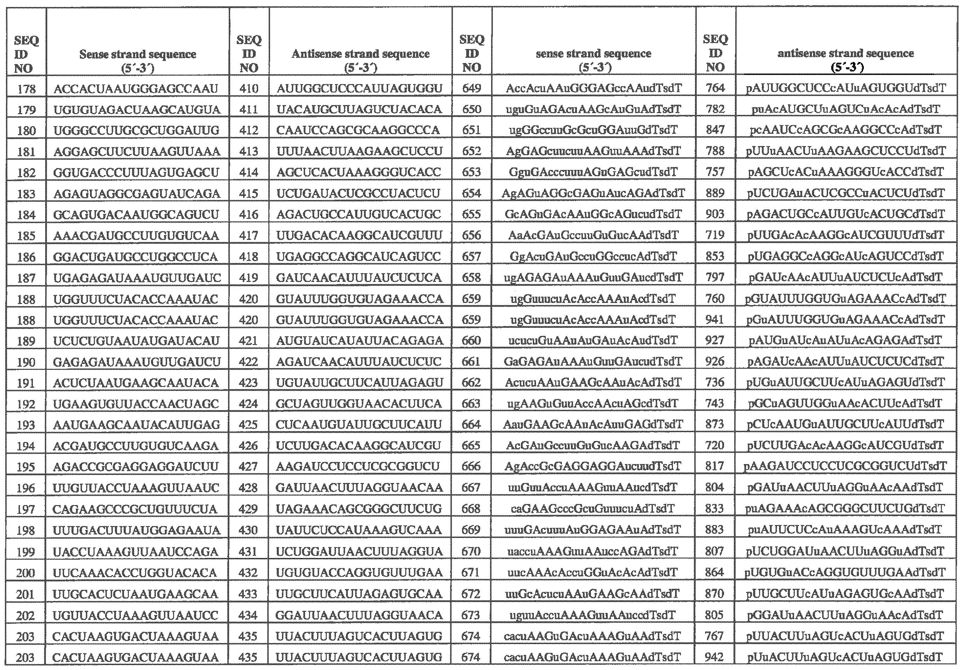

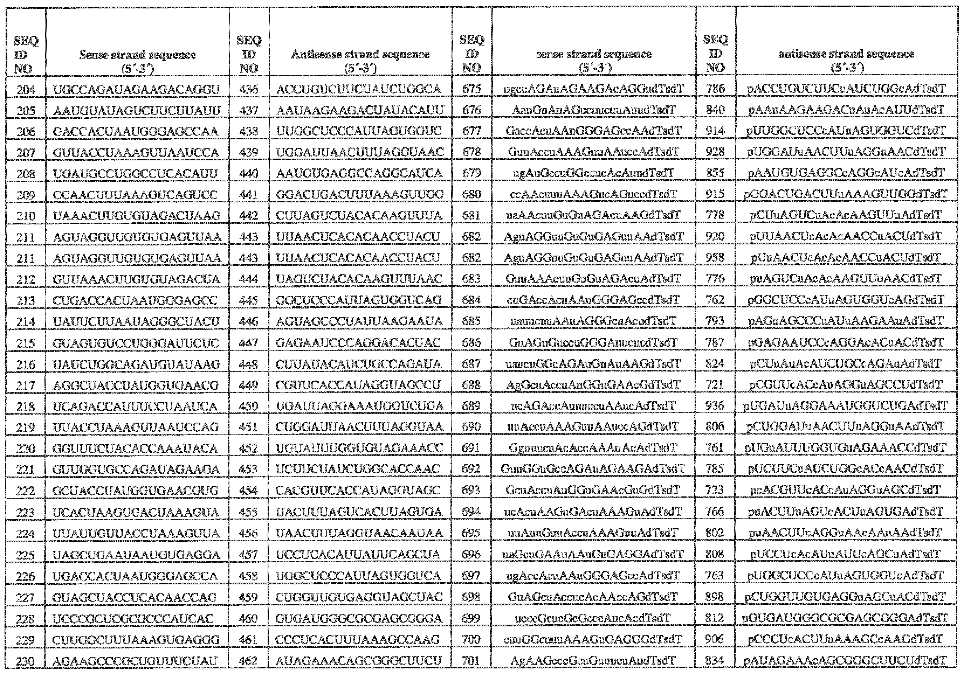

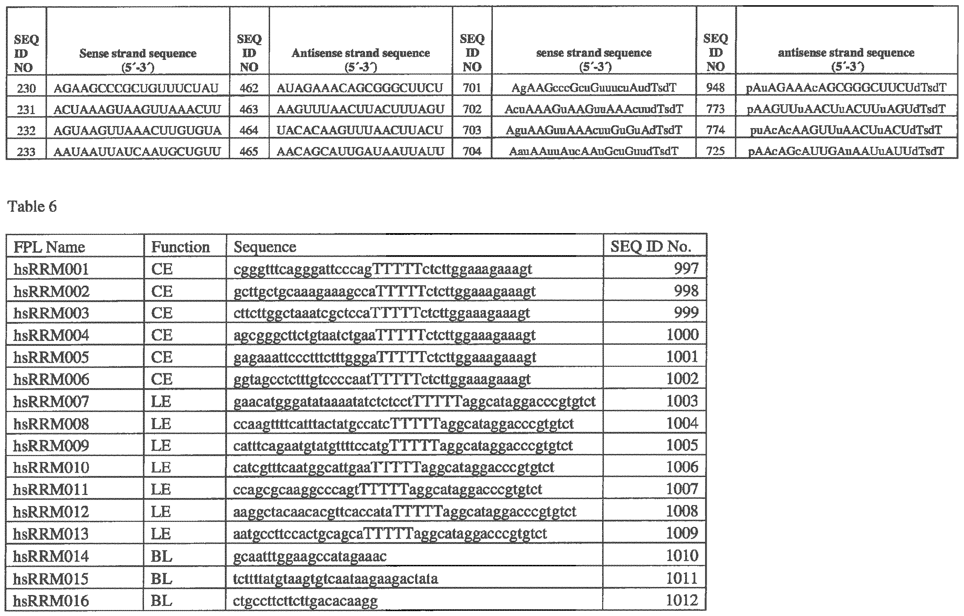

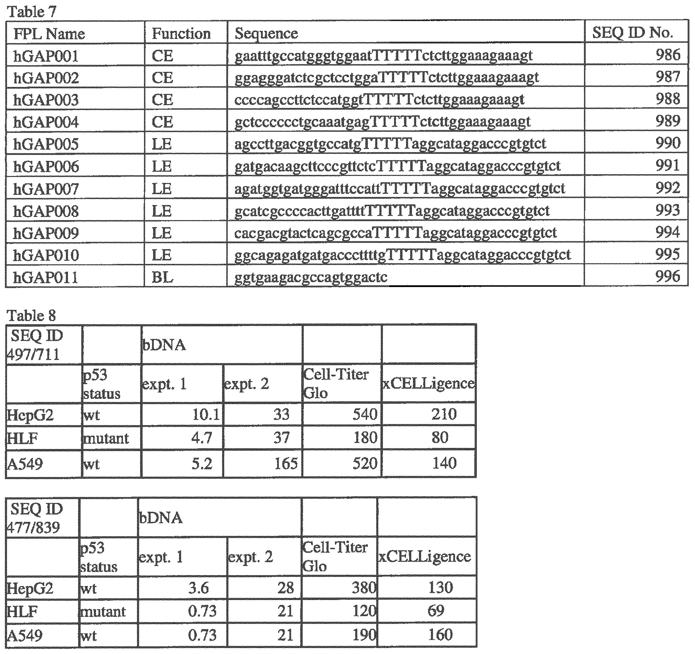

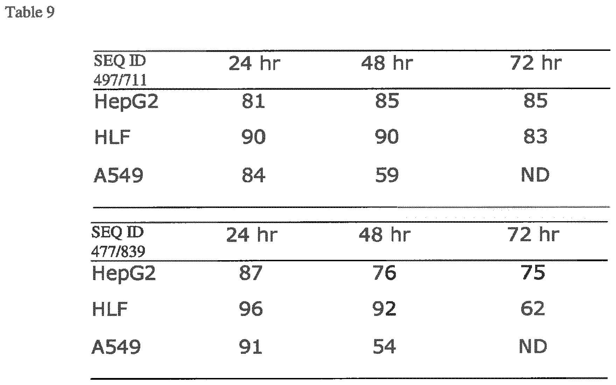

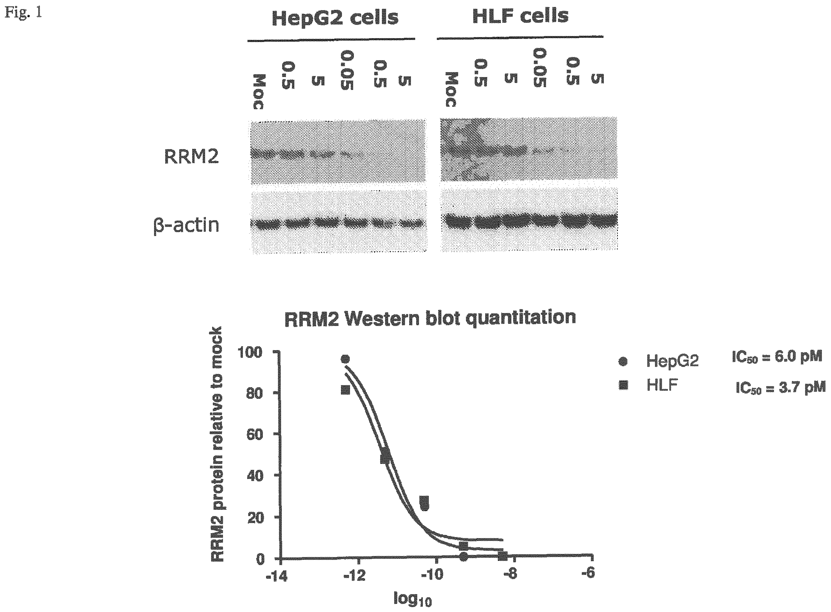

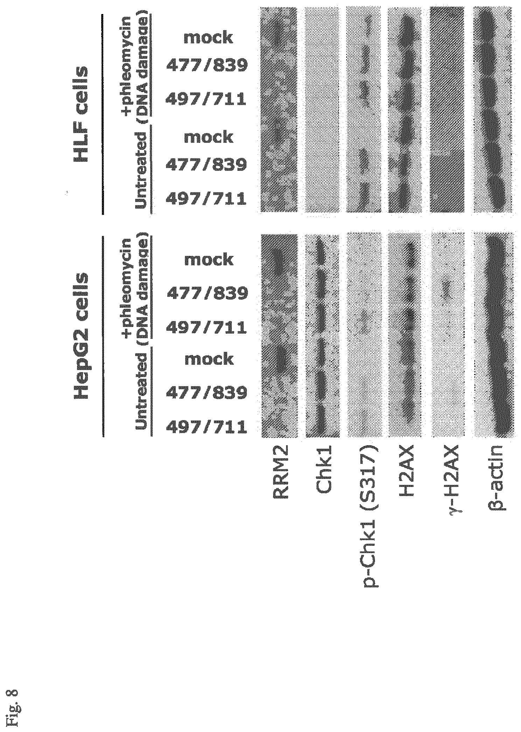

Cells were transfected in 6-well plates and lysed with Pierce M-PER Mammalian Protein Extraction Reagent containing Pierce Halt Protease and Phosphatase Inhibitor Cocktail, according to the manufacturer's recommendations. Protein concentrations were determined using the Pierce Micro BCA Protein Assay Kit. Lysates were run on Novex NuPAGE 4-12% Bis-Tris gels. Protein was transferred to nitrocellulose using the Invitrogen iBlot Dry Blotting System. Western blots were probed using dilutions of antibodies recommended by the manufacturers, and detected using Amersham ECL Plus Western Blotting Detection Reagents. Gel images were collected on a FujiFilm LAS-4000 instrument and quantitated using Multi Gauge v3.1 software. RRM2 protein knock-down dose-response results are shown in figure 1. Effects on components of DNA damage pathway (*in vivo* pharmacodynamic markers) are shown in figure 8.

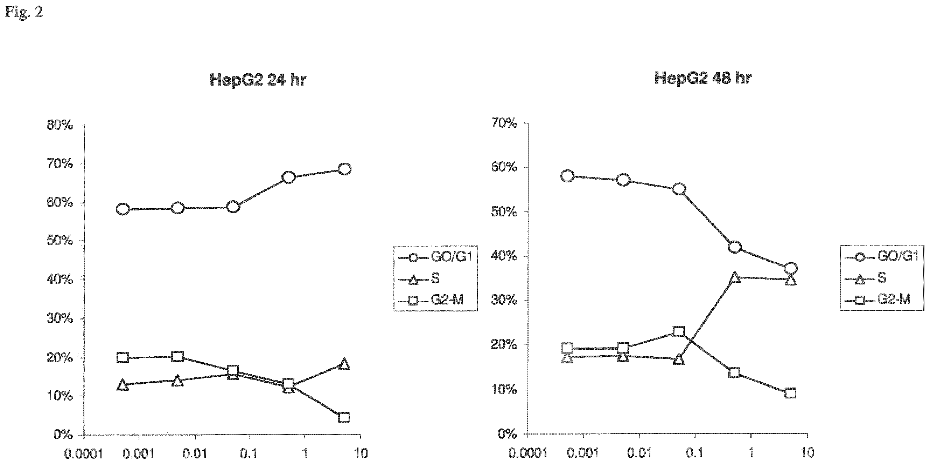

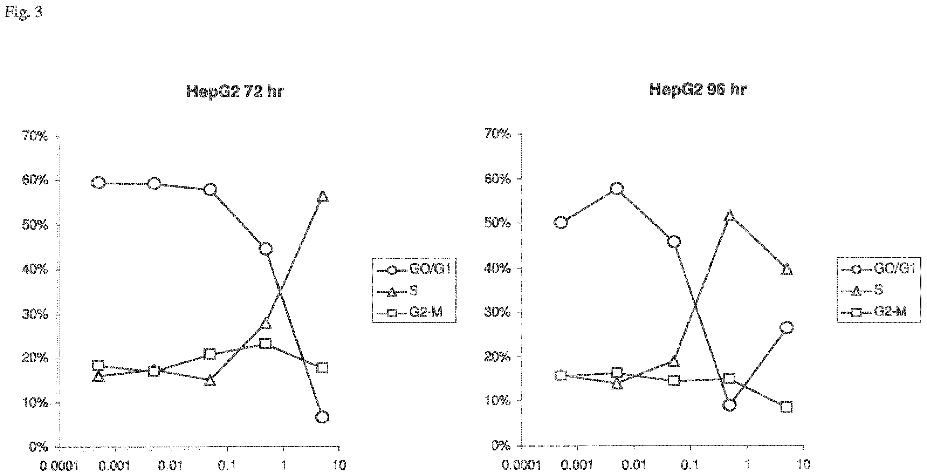

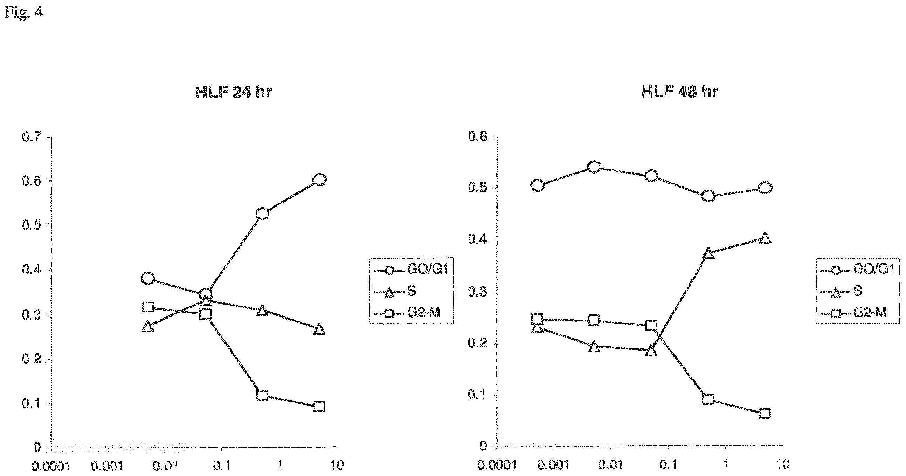

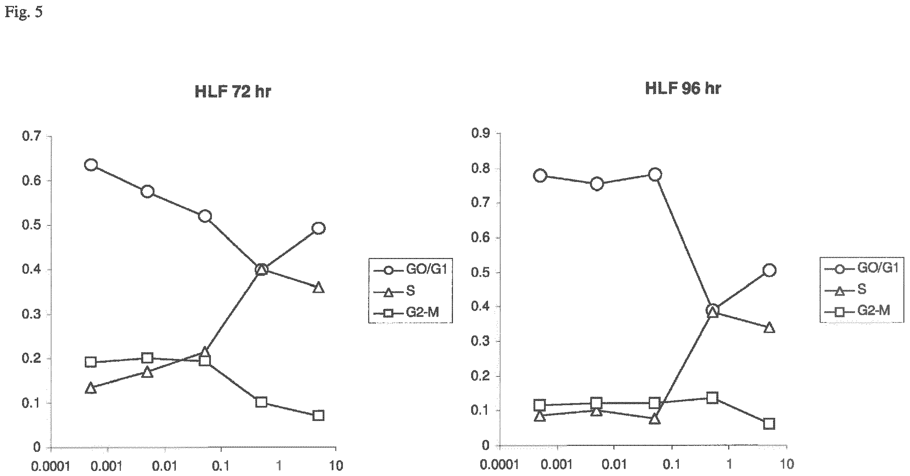

**Cell cycle assay.** Cells were transfected in 6-well plates and harvested at 24, 48, 72 and 96 hours after transfection. Media was removed and collected in a 50-ml conical tube. Wells were washed with 2 ml PBS and added to the corresponding tubes. Cells were trypsinized, and, once displaced, added to the appropriate tubes. Tubes were centrifuged at 2,000 rpm for 10 min, washed once with 2 ml PBS, and then centrifuged again at 2,000 rpm for 5 min. Supernatants were carefully removed. To fix cells, tubes were tapped to loosen the pellets, and 1.2 ml cold 70% ETOH was added. After vortexing, samples were stored at -20 C overnight. After thawing cells at room temperature for 20-30 min, 1.2 ml cold PBS was added, and tubes were centrifuged at 2,000 rpm for 10 min. Pellets were washed with 2 ml PBS and centrifuged again at 2,000 rpm. To the pellets was added 0.5 ml propidium iodide/RNase staining buffer (BD Pharmingen, catalog number 550825). Following a 15-min incubation at 37 C, data was collected on FACS LSRII instrument (BD) using DIVA software (10,000 events per sample) and analyzed using FlowJo software. Results are shown in figures 2 - 5.

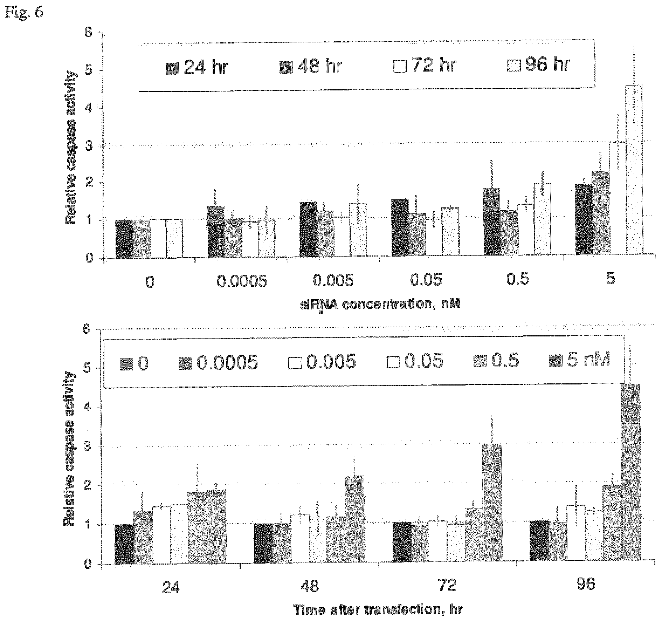

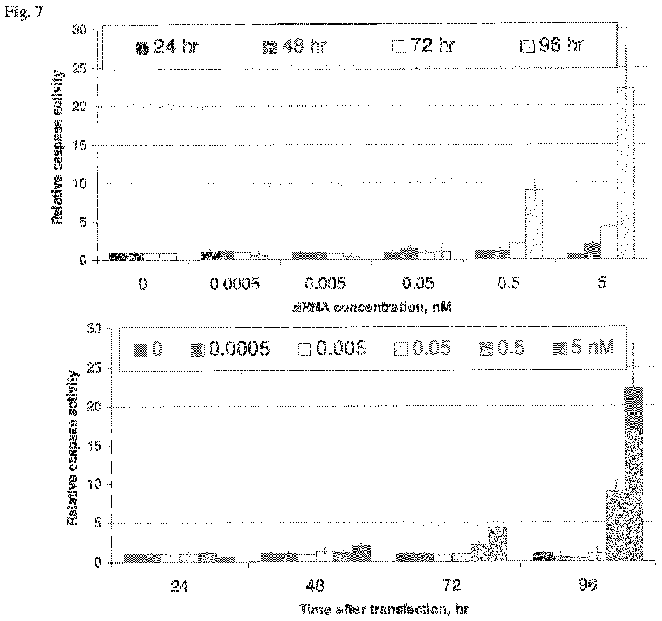

**Caspase assay.** Activation of caspase 3/7 was determined using the Apo-ONE Homogeneous Caspase-3/7 assay (Promega), according to the manufacturer's recommendations. Results are shown in figures 6 and 7.

## Claims

1. A double-stranded ribonucleic acid molecule comprising a sense strand and an antisense strand wherein:

said sense strand and said antisense strand form a fully complimentary sequence of 19 base pairs,
said sense strand comprises SEQ ID NO. 9 and has at least one modified nucleotide,
said antisense strand comprises SEQ ID NO. 242 and has at least one modified nucleotide, and

preferably wherein said sense strand and said antisense strand are each less than 30 nucleotides in length.

**2.** The double strand ribonucleic acid molecule of claim 1, wherein said sense strand or said antisense strand further comprises a 3' overhang of 1-5 nucleotides in length

**3.** The double stranded ribonucleic acid molecule of claim 2, wherein said 3' overhang comprises two deoxythymidine nucleotides, preferably wherein said 3' overhang comprises two deoxythymidine nucleotides linked by a phosphorothioate group.

**4.** The double strand ribonucleic acid molecule of claim 2, wherein said 3' overhang comprises an inverted deoxythymidine.

**5.** The double-stranded ribonucleic acid molecule of claim 1, wherein said modified nucleotide is selected from the group consisting of: a 2'-O-methyl modified nucleotide, a nucleotide comprising a 5'-phosphorothioate group, and a terminal nucleotide linked to a cholesteryl derivative or dodecanoic acid bisdecylamide group, a 2'-deoxy-2'-fluoro modified nucleotide, a 2'-deoxy-modified nucleotide, a locked nucleotide, an abasic nucleotide, inverted deoxythymidine, 2'-amino-modified nucleotide, 2'-alkyl-modified nucleotide, morpholino nucleotide, a phosphoramidate, and a non-natural base comprising nucleotide.

**6.** The double strand ribonucleic acid molecule of claim 5, wherein said sense strand and said antisense strand each contains more than one modified nucleotide.

**7.** The double-stranded ribonucleic acid molecule of claim 6 wherein said sense strand 5 comprises the modified nucleic acid sequence of SEQ ID No. 477 and said antisense strand comprises a modified nucleic acid sequence selected from_the group consisting of SEQ ID NOs. 839, 963, 971 and 979, preferably wherein said double-stranded ribonucleic acid molecule comprises a sequence pair selected from the group consisting of SEQ ID NOs: 477/839, 477/963, 477/971, 477/979.

**8.** A cell, tissue or non-human organism comprising a double-stranded ribonucleic acid molecule according to any of the claims 1 to 7.

**9.** A pharmaceutical composition comprising a double-stranded ribonucleic acid molecule according to any of the claims 1 to 7 and a pharmaceutically acceptable carrier.

**Patentansprüche**

**1.** Doppelsträngiges Ribonukleinsäuremolekül, das einen Sense-Strang und einen Antisense-Strang aufweist, wobei:

der Sense-Strang und der Antisense-Strang eine vollständig komplementäre Sequenz aus 19 Basenpaaren bilden,
der Sense-Strang SEQ ID NO. 9 aufweist und wenigstens ein modifiziertes Nukleotid hat,
der Antisense-Strang SEQ ID NO. 242 aufweist und wenigstens ein modifiziertes Nukleotid hat, und
wobei der Sense-Strang und der Antisense-Strang vorzugsweise jeweils weniger als 30 Nukleotide lang sind.

**2.** Doppelsträngiges Ribonukleinsäuremolekül nach Anspruch 1, wobei der Sense-Strang oder der Antisense-Strang ferner einen 3'-Überhang mit einer Länge von 1 - 5 Nukleotiden aufweisen.

**3.** Doppelsträngiges Ribonukleinsäuremolekül nach Anspruch 2, wobei der 3'-Überhang zwei Deoxythymidin-Nukleotide aufweist, wobei der 3'-Überhang vorzugsweise zwei Deoxythymidin-Nukleotide aufweist, die durch eine Phosphorothioatgruppe verbunden sind.

**4.** Doppelsträngiges Ribonukleinsäuremolekül nach Anspruch 2, wobei der 3'-Überhang ein invertiertes Deoxythymidin aufweist.

5. Doppelsträngiges Ribonukleinsäuremolekül nach Anspruch 1, wobei das modifizierte Nukleotid aus der Gruppe ausgewählt ist, die besteht aus: einem 2'-O-Methylmodifizierten Nukleotid, einem Nukleotid, das eine 5'-Phosphorothioatgruppe aufweist, und einem terminalen Nukleotid, das mit einem Cholesterinderivat oder einer *Hydroxydodekansäure-Bisdecylamid*-Gruppe verbunden ist, einem 2'-Deoxy-2'-fluormodifizierten Nukleotid, einem 2'-Deoxy-modifzierten Nukleotid, einem verbrückten Nukleotid, einem abasischen Nukleotid, invertiertem Deoxythymidin, einem 2'-Amino-modifiziertem Nukleotid, einem 2'-Alkyl-modifizierten Nukleotid, einem Morpholino-Nukleotid, Phosphoramidat, und eine nicht-natürlichen Base, die Nukleotid aufweist.

6. Doppelsträngiges Ribonukleinsäuremolekül nach Anspruch 5, wobei der Sense-Strang und der Antisense-Strang jeweils mehr als ein modifiziertes Nukleotid enthalten.

7. Doppelsträngiges Ribonukleinsäuremolekül nach Anspruch 6, wobei der Sense-Strang die modifizierte Nukleinsäuresequenz der SEQ ID No. 477 aufweist und der Antisense-Strang eine modifizierte Nukleinsäuresequenz aufweist, die aus der Gruppe ausgewählt ist, die aus den SEQ ID NOs. 839, 963, 971 und 979 besteht, wobei das doppelsträngige Ribonukleinsäuremolekül vorzugsweise ein Sequenzpaar aufweist, das aus der Gruppe ausgewählt ist, die besteht aus SEQ ID NOs: 477/839, 477/963, 477/971, 477/979.

8. Zelle, Gewebe oder nicht menschlicher Organismus, der ein doppelsträngiges Ribonukleinsäuremolekül nach einem der Ansprüche 1 bis 7 aufweist.

9. Pharmazeutische Zusammensetzung, die ein doppelsträngiges Ribonukleinsäuremolekül nach einem der Ansprüche 1 bis 7 und einen pharmazeutisch akzeptablen Träger aufweist.

## Revendications

1. Molécule d'acide ribonucléique double brin comprenant un brin sens et un brin antisens dans laquelle :

    ledit brin sens et ledit brin antisens forment une séquence totalement complémentaire de 19 paires de bases,
    ledit brin sens comprend SEQ ID NO : 9 et comporte au moins un nucléotide modifié,
    ledit brin antisens comprend SEQ ID NO : 242 et comporte au moins un nucléotide modifié, et

   de préférence dans laquelle ledit brin sens et ledit brin antisens ont chacun une longueur inférieure à 30 nucléotides.

2. Molécule d'acide ribonucléique double brin selon la revendication 1, dans laquelle ledit brin sens ou ledit brin antisens comprend en outre une extrémité sortante en 3' d'une longueur de 1 à 5 nucléotides.

3. Molécule d'acide ribonucléique double brin selon la revendication 2, dans laquelle ladite extrémité sortante en 3' comprend deux nucléotides de désoxythymidine, de préférence dans laquelle ladite extrémité sortante en 3' comprend deux nucléotides de désoxythymidine liés par un groupe phosphorothioate.

4. Molécule d'acide ribonucléique double brin selon la revendication 2, dans laquelle ladite extrémité sortante en 3' comprend une désoxythymidine inversée.

5. Molécule d'acide ribonucléique double brin selon la revendication 1, dans laquelle ledit nucléotide modifié est choisi dans le groupe constitué de : un nucléotide modifié par un groupe 2'-O-méthyle, un nucléotide comprenant un groupe 5'-phosphorothioate, et un nucléotide terminal lié à un dérivé de cholestéryle ou un groupe bisdécylamide d'acide dodécanoïque, un nucléotide modifié par un groupe 2'-désoxy-2'-fluoro, un nucléotide modifié par un groupe 2'-désoxy, un nucléotide verrouillé, un nucléotide abasique, une désoxythymidine inversée, un nucléotide modifié par un groupe 2'-amino, un nucléotide modifié par un groupe 2'-alkyle, un morpholino-nucléotide, un phosphoramidate, et un nucléotide comprenant une base non naturelle.

6. Molécule d'acide ribonucléique double brin selon la revendication 5, dans laquelle ledit brin sens et ledit brin antisens contiennent chacun plus d'un nucléotide modifié.

7. Molécule d'acide ribonucléique double brin selon la revendication 6 dans laquelle ledit brin sens comprend la séquence d'acide nucléique modifiée de SEQ ID NO : 477 et ledit brin antisens comprend une séquence d'acide nucléique modifiée choisie dans le groupe constitué de SEQ ID NO : 839, 963, 971 et 979, de préférence dans

laquelle ladite molécule d'acide ribonucléique double brin comprend une paire de séquences choisie dans le groupe constitué de SEQ ID NO : 477/839, 477/963, 477/971, 477/979.

8. Cellule, tissu ou organisme non humain comprenant une molécule d'acide ribonucléique double brin selon l'une quelconque des revendications 1 à 7.

9. Composition pharmaceutique comprenant une molécule d'acide ribonucléique double brin selon l'une quelconque des revendications 1 à 7 et un support pharmaceutiquement acceptable.

Table 1

| SEQ ID NO | Sense strand sequence (5´-3´) | SEQ ID NO | Antisense strand sequence (5´-3´) |
|---|---|---|---|
| 1 | UUGUGGCAGACAGACUUAU | 234 | AUAAGUCUGUCUGCCACAA |
| 2 | CCUGAUGUUCAAACACCUG | 235 | CAGGUGUUUGAACAUCAGG |
| 3 | AGUCCAACAGAGAAUUCUU | 236 | AAGAAUUCUCUGUUGGACU |
| 4 | UAGGCGAGUAUCAGAGGAU | 237 | AUCCUCUGAUACUCGCCUA |
| 5 | GGCGAGUAUCAGAGGAUGG | 238 | CCAUCCUCUGAUACUCGCC |
| 6 | UGUUCAAACACCUGGUACA | 239 | UGUACCAGGUGUUUGAACA |
| 7 | GGGUGACCCUUUAGUGAGC | 240 | GCUCACUAAAGGGUCACCC |
| 8 | GAAGGAAAGACUAACUUCU | 241 | AGAAGUUAGUCUUUCCUUC |
| 9 | UUCUGAAAUGUAUAGUCUU | 242 | AAGACUAUACAUUUCAGAA |
| 10 | CUGUGUAGCUACCUCACAA | 243 | UUGUGAGGUAGCUACACAG |
| 11 | UGCACUCUAAUGAAGCAAU | 244 | AUUGCUUCAUUAGAGUGCA |
| 12 | CCCAUUUGACUUUAUGGAG | 245 | CUCCAUAAAGUCAAAUGGG |
| 13 | AAAUGUAUAGUCUUCUUAU | 246 | AUAAGAAGACUAUACAUUU |
| 14 | UACAUUGAGUUUGUGGCAG | 247 | CUGCCACAAACUCAAUGUA |
| 15 | CAAUACAUUGAGUUUGUGG | 248 | CCACAAACUCAAUGUAUUG |
| 16 | GAACAGGAGUUCCUCACUG | 249 | CAGUGAGGAACUCCUGUUC |
| 17 | AUCCCAUGUUCUGGCUUUC | 250 | GAAAGCCAGAACAUGGGAU |
| 18 | GUAGGUUGUGUGAGUUAAU | 251 | AUUAACUCACACAACCUAC |
| 19 | AUAGUCUUCUUAUUGACAC | 252 | GUGUCAAUAAGAAGACUAU |
| 20 | AUUGCACUCUAAUGAAGCA | 253 | UGCUUCAUUAGAGUGCAAU |
| 21 | UUAUCAAUGCUGUUCGGAU | 254 | AUCCGAACAGCAUUGAUAA |
| 22 | AGAAACGAGGACUGAUGCC | 255 | GGCAUCAGUCCUCGUUUCU |
| 23 | CAUUGAGUUUGUGGCAGAC | 256 | GUCUGCCACAAACUCAAUG |
| 24 | ACAUUCAGCACUGGGAAUC | 257 | GAUUCCCAGUGCUGAAUGU |
| 25 | UGAUGUUCAAACACCUGGU | 258 | ACCAGGUGUUUGAACAUCA |
| 26 | GGAUAGAACAGGAGUUCCU | 259 | AGGAACUCCUGUUCUAUCC |

| SEQ ID NO | Sense strand sequence (5´-3´) | SEQ ID NO | Antisense strand sequence (5´-3´) |
|---|---|---|---|
| 27 | AAUAUUUCACUGGAAGGAA | 260 | UUCCUUCCAGUGAAAUAUU |
| 28 | AAUAAACAUUGUUUGUACU | 261 | AGUACAAACAAUGUUUAUU |
| 29 | UCCCAUGUUCUGGCUUUCU | 262 | AGAAAGCCAGAACAUGGGA |
| 30 | UUCGGAUAGAACAGGAGUU | 263 | AACUCCUGUUCUAUCCGAA |
| 31 | AAGUAGGUUGUGUGAGUUA | 264 | UAACUCACACAACCUACUU |
| 32 | UUAUAGUGCUGGUAGUAUC | 265 | GAUACUACCAGCACUAUAA |
| 33 | CUUCUUAUUGACACUUACA | 266 | UGUAAGUGUCAAUAAGAAG |
| 34 | UACAGAAGCCCGCUGUUUC | 267 | GAAACAGCGGGCUUCUGUA |
| 35 | GUGACCCUUUAGUGAGCUU | 268 | AAGCUCACUAAAGGGUCAC |
| 36 | AUAGAACAGGAGUUCCUCA | 269 | UGAGGAACUCCUGUUCUAU |
| 37 | CUGGCACUUUACAAACAAA | 270 | UUUGUUUGUAAAGUGCCAG |
| 38 | UCUAAUGAAGCAAUACAUU | 271 | AAUGUAUUGCUUCAUUAGA |
| 39 | UCUUCUUAUUGACACUUAC | 272 | GUAAGUGUCAAUAAGAAGA |
| 40 | UGUUCGGAUAGAACAGGAG | 273 | CUCCUGUUCUAUCCGAACA |
| 41 | AGUACCAUGAUAUCUGGCA | 274 | UGCCAGAUAUCAUGGUACU |
| 42 | CAGAGAUGAGGGUUUACAC | 275 | GUGUAAACCCUCAUCUCUG |
| 43 | GAAACGAGGACUGAUGCCU | 276 | AGGCAUCAGUCCUCGUUUC |
| 44 | AAGAGAGUAGGCGAGUAUC | 277 | GAUACUCGCCUACUCUCUU |
| 45 | CAUUAGCUGAAUAAUGUGA | 278 | UCACAUUAUUCAGCUAAUG |
| 46 | AGUAGAGAACCCAUUUGAC | 279 | GUCAAAUGGGUUCUCUACU |
| 47 | AGGCGAGUAUCAGAGGAUG | 280 | CAUCCUCUGAUACUCGCCU |
| 48 | UAGACUAAGCAUGUAAUUU | 281 | AAAUUACAUGCUUAGUCUA |
| 49 | AACAUUGUUUGUACUCACA | 282 | UGUGAGUACAAACAAUGUU |
| 50 | GAUGGGAGUGAUGUCAAGU | 283 | ACUUGACAUCACUCCCAUC |
| 51 | CAGACCAUUUCCUAAUCAG | 284 | CUGAUUAGGAAAUGGUCUG |
| 52 | GAUUACAGAAGCCCGCUGU | 285 | ACAGCGGGCUUCUGUAAUC |
| 53 | CAUUGAAACGAUGCCUUGU | 286 | ACAAGGCAUCGUUUCAAUG |
| 54 | ACUUAUGCUGGAACUGGGU | 287 | ACCCAGUUCCAGCAUAAGU |

| SEQ ID NO | Sense strand sequence (5´-3´) | SEQ ID NO | Antisense strand sequence (5´-3´) |
|---|---|---|---|
| 55 | GUCGACAAGGAGAACACGC | 288 | GCGUGUUCUCCUUGUCGAC |
| 56 | AGGAAAGACUAACUUCUUU | 289 | AAAGAAGUUAGUCUUUCCU |
| 57 | CAAGACCGCGAGGAGGAUC | 290 | GAUCCUCCUCGCGGUCUUG |
| 58 | GACAAUGGCAGUCUUGGCU | 291 | AGCCAAGACUGCCAUUGUC |
| 59 | AUGCCUUGUGUCAAGAAGA | 292 | UCUUCUUGACACAAGGCAU |
| 60 | GCCUCACUGCUUCAACGCA | 293 | UGCGUUGAAGCAGUGAGGC |
| 61 | UACCUCACAACCAGUCCUG | 294 | CAGGACUGGUUGUGAGGUA |
| 62 | GAGAAGAGAGUAGGCGAGU | 295 | ACUCGCCUACUCUCUUCUC |
| 63 | AGACUUAUGCUGGAACUGG | 296 | CCAGUUCCAGCAUAAGUCU |
| 64 | UUACAGAAGCCCGCUGUUU | 297 | AAACAGCGGGCUUCUGUAA |
| 65 | UUAUGCUGGAACUGGGUUU | 298 | AAACCCAGUUCCAGCAUAA |
| 66 | AUAAACAUUGUUUGUACUC | 299 | GAGUACAAACAAUGUUUAU |
| 67 | UCAAUGCCAUUGAAACGAU | 300 | AUCGUUUCAAUGGCAUUGA |
| 68 | AUAGUGCUGGUAGUAUCAC | 301 | GUGAUACUACCAGCACUAU |
| 69 | CAGCCUCACUGCUUCAACG | 302 | CGUUGAAGCAGUGAGGCUG |
| 70 | UCUUGGCUUUAAAGUGAGG | 303 | CCUCACUUUAAAGCCAAGA |
| 71 | GGCUGUGACUUACCAUAGC | 304 | GCUAUGGUAAGUCACAGCC |
| 72 | GGCUACCUAUGGUGAACGU | 305 | ACGUUCACCAUAGGUAGCC |
| 73 | CGCGAGGAGGAUCUUCCAG | 306 | CUGGAAGAUCCUCCUCGCG |
| 74 | GCCAUUGAAACGAUGCCUU | 307 | AAGGCAUCGUUUCAAUGGC |
| 75 | AGCCUCACUGCUUCAACGC | 308 | GCGUUGAAGCAGUGAGGCU |
| 76 | GGCAGACAGACUUAUGCUG | 309 | CAGCAUAAGUCUGUCUGCC |
| 77 | GUGACUAAAGUAAGUUAAA | 310 | UUUAACUUACUUUAGUCAC |
| 78 | AGUUAUUGUUACCUAAAGU | 311 | ACUUUAGGUAACAAUAACU |
| 79 | GCCUUUAUGUUUGGGAGAA | 312 | UUCUCCCAAACAUAAAGGC |
| 80 | UUCAGAGUAGAGAACCCAU | 313 | AUGGGUUCUCUACUCUGAA |
| 81 | AAACGAGGACUGAUGCCUG | 314 | CAGGCAUCAGUCCUCGUUU |
| 82 | GUAGGCGAGUAUCAGAGGA | 315 | UCCUCUGAUACUCGCCUAC |

| SEQ ID NO | Sense strand sequence (5´-3´) | SEQ ID NO | Antisense strand sequence (5´-3´) |
|---|---|---|---|
| 83 | AAGCCCGCUGUUUCUAUGG | 316 | CCAUAGAAACAGCGGGCUU |
| 84 | UCAGCACUGGGAAUCCCUG | 317 | CAGGGAUUCCCAGUGCUGA |
| 85 | GAAUAAUGUGAGGAUUAAC | 318 | GUUAAUCCUCACAUUAUUC |
| 86 | UGUGGCAGACAGACUUAUG | 319 | CAUAAGUCUGUCUGCCACA |
| 87 | AGAGAUAAAUGUUGAUCUU | 320 | AAGAUCAACAUUUAUCUCU |
| 88 | UACCAUGAUAUCUGGCAGA | 321 | UCUGCCAGAUAUCAUGGUA |
| 89 | CUUCCAAAUUGCCAUGGAA | 322 | UUCCAUGGCAAUUUGGAAG |
| 90 | ACCGCGAGGAGGAUCUUCC | 323 | GGAAGAUCCUCCUCGCGGU |
| 91 | GAAAUGUAUAGUCUUCUUA | 324 | UAAGAAGACUAUACAUUUC |
| 92 | AUGUUCAAACACCUGGUAC | 325 | GUACCAGGUGUUUGAACAU |
| 93 | AGGGAAUUUCUCUUCAAUG | 326 | CAUUGAAGAGAAAUUCCCU |
| 94 | CCCUGUUAAGUGGUGAAAU | 327 | AUUUCACCACUUAACAGGG |
| 95 | GAUGAGGGUUUACACUGUG | 328 | CACAGUGUAAACCCUCAUC |
| 96 | UGUGUGAGUUAAUUCAUUU | 329 | AAAUGAAUUAACUCACACA |
| 97 | UUGCCUGAUGUUCAAACAC | 330 | GUGUUUGAACAUCAGGCAA |
| 98 | AAACUUGUGUAGACUAAGC | 331 | GCUUAGUCUACACAAGUUU |
| 99 | UAUAUCCCAUGUUCUGGCU | 332 | AGCCAGAACAUGGGAUAUA |
| 100 | UUGUGUAGACUAAGCAUGU | 333 | ACAUGCUUAGUCUACACAA |
| 101 | AUGCUGUUCGGAUAGAACA | 334 | UGUUCUAUCCGAACAGCAU |
| 102 | AAUUAUCAAUGCUGUUCGG | 335 | CCGAACAGCAUUGAUAAUU |
| 103 | GCCUGAUGUUCAAACACCU | 336 | AGGUGUUUGAACAUCAGGC |
| 104 | CAUAGCAGUGACAAUGGCA | 337 | UGCCAUUGUCACUGCUAUG |
| 105 | UGUGAGUUAAUUCAUUUAU | 338 | AUAAAUGAAUUAACUCACA |
| 106 | AGUGCUGGUAGUAUCACCU | 339 | AGGUGAUACUACCAGCACU |
| 107 | UAUCAAUGCUGUUCGGAUA | 340 | UAUCCGAACAGCAUUGAUA |
| 108 | GACUAAAGUAAGUUAAACU | 341 | AGUUUAACUUACUUUAGUC |
| 109 | AAUGCUGUUCGGAUAGAAC | 342 | GUUCUAUCCGAACAGCAUU |
| 110 | AGAAUAUUUCACUGGAAGG | 343 | CCUUCCAGUGAAAUAUUCU |

| SEQ ID NO | Sense strand sequence (5´-3´) | SEQ ID NO | Antisense strand sequence (5´-3´) |
|---|---|---|---|
| 111 | AUCUGGCAGAUGUAUAAGA | 344 | UCUUAUACAUCUGCCAGAU |
| 112 | UAUAGUGCUGGUAGUAUCA | 345 | UGAUACUACCAGCACUAUA |
| 113 | GGCCAGCAAGACCGCGAGG | 346 | CCUCGCGGUCUUGCUGGCC |
| 114 | CCAUGAUAUCUGGCAGAUG | 347 | CAUCUGCCAGAUAUCAUGG |
| 115 | UUAAACUUGUGUAGACUAA | 348 | UUAGUCUACACAAGUUUAA |
| 116 | UUCAAUGCCAUUGAAACGA | 349 | UCGUUUCAAUGGCAUUGAA |
| 117 | AGAAAGCUGAGACAUUGCA | 350 | UGCAAUGUCUCAGCUUUCU |
| 118 | CUAUGGCUUCCAAAUUGCC | 351 | GGCAAUUUGGAAGCCAUAG |
| 119 | AAGUGACUAAAGUAAGUUA | 352 | UAACUUACUUUAGUCACUU |
| 120 | UGACUAAAGUAAGUUAAAC | 353 | GUUUAACUUACUUUAGUCA |
| 121 | UGCUGUUCGGAUAGAACAG | 354 | CUGUUCUAUCCGAACAGCA |
| 122 | GCGAGUAUCAGAGGAUGGG | 355 | CCCAUCCUCUGAUACUCGC |
| 123 | GGGCCUUGCGCUGGAUUGG | 356 | CCAAUCCAGCGCAAGGCCC |
| 124 | ACCUCACAACCAGUCCUGU | 357 | ACAGGACUGGUUGUGAGGU |
| 125 | ACUAAGUGACUAAAGUAAG | 358 | CUUACUUUAGUCACUUAGU |
| 126 | AUUACAGAAGCCCGCUGUU | 359 | AACAGCGGGCUUCUGUAAU |
| 127 | GAGUAGGCGAGUAUCAGAG | 360 | CUCUGAUACUCGCCUACUC |
| 128 | CAGUGACAAUGGCAGUCUU | 361 | AAGACUGCCAUUGUCACUG |
| 129 | GGCCUUGCGCUGGAUUGGG | 362 | CCCAAUCCAGCGCAAGGCC |
| 130 | UUCUUAUUGACACUUACAU | 363 | AUGUAAGUGUCAAUAAGAA |
| 131 | UUCACUAAGUGACUAAAGU | 364 | ACUUUAGUCACUUAGUGAA |
| 132 | GUGUGAGUUAAUUCAUUUA | 365 | UAAAUGAAUUAACUCACAC |
| 133 | CCCGCUCGCGCCCAUCACG | 366 | CGUGAUGGGCGCGAGCGGG |
| 134 | GUAAGUUAAACUUGUGUAG | 367 | CUACACAAGUUUAACUUAC |
| 135 | CGGAAGUUGGAAUCAGGUU | 368 | AACCUGAUUCCAACUUCCG |
| 136 | AUGUGAGGAUUAACUUCUG | 369 | CAGAAGUUAAUCCUCACAU |
| 137 | UUAAGUGGUGAAAUCAACU | 370 | AGUUGAUUUCACCACUUAA |
| 138 | UGUAGACUAAGCAUGUAAU | 371 | AUUACAUGCUUAGUCUACA |

| SEQ ID NO | Sense strand sequence (5´-3´) | SEQ ID NO | Antisense strand sequence (5´-3´) |
|---|---|---|---|
| 139 | AUAAUGUGAGGAUUAACUU | 372 | AAGUUAAUCCUCACAUUAU |
| 140 | GGCUGGCUGUGACUUACCA | 373 | UGGUAAGUCACAGCCAGCC |
| 141 | AAGAGGCUACCUAUGGUGA | 374 | UCACCAUAGGUAGCCUCUU |
| 142 | CAGAUUACAGAAGCCCGCU | 375 | AGCGGGCUUCUGUAAUCUG |
| 143 | UGAGGCCUUGCCUGUGAAG | 376 | CUUCACAGGCAAGGCCUCA |
| 144 | AUAAUUAUCAAUGCUGUUC | 377 | GAACAGCAUUGAUAAUUAU |
| 145 | GUGACUUACCAUAGCAGUG | 466 | CACUGCUAUGGUAAGUCAC |
| 146 | UAGGGCUACUUUGAAUUAA | 378 | UUAAUUCAAAGUAGCCCUA |
| 147 | UGGCAGAUGUAUAAGAAGG | 379 | CCUUCUUAUACAUCUGCCA |
| 148 | AUAGCUUGAUUUAUUUGGU | 380 | ACCAAAUAAAUCAAGCUAU |
| 149 | CAGCAAGACCGCGAGGAGG | 381 | CCUCCUCGCGGUCUUGCUG |
| 150 | GACUGAUGCCUGGCCUCAC | 382 | GUGAGGCCAGGCAUCAGUC |
| 151 | UUACCUUGGAUGCUGACUU | 383 | AAGUCAGCAUCCAAGGUAA |
| 152 | AUUCAGCACUGGGAAUCCC | 384 | GGGAUUCCCAGUGCUGAAU |
| 153 | AGCAAGACCGCGAGGAGGA | 385 | UCCUCCUCGCGGUCUUGCU |
| 154 | AGGGCUACUUUGAAUUAAU | 386 | AUUAAUUCAAAGUAGCCCU |
| 155 | UAAGUUAUUGUUACCUAAA | 387 | UUUAGGUAACAAUAACUUA |
| 156 | UUUAUAGUGCUGGUAGUAU | 388 | AUACUACCAGCACUAUAAA |
| 157 | GCAAGACCGCGAGGAGGAU | 389 | AUCCUCCUCGCGGUCUUGC |
| 158 | UCUAUGGCUUCCAAAUUGC | 390 | GCAAUUUGGAAGCCAUAGA |
| 159 | AAAGACUAACUUCUUUGAG | 391 | CUCAAAGAAGUUAGUCUUU |
| 160 | ACCAUGAUAUCUGGCAGAU | 392 | AUCUGCCAGAUAUCAUGGU |
| 161 | GACCAUUUCCUAAUCAGUU | 393 | AACUGAUUAGGAAAUGGUC |
| 162 | UUACCAUAGCAGUGACAAU | 394 | AUUGUCACUGCUAUGGUAA |
| 163 | AAUGUGAGGAUUAACUUCU | 395 | AGAAGUUAAUCCUCACAUU |
| 164 | UAGUGUCCUGGGAUUCUCU | 396 | AGAGAAUCCCAGGACACUA |
| 165 | UGUUAAGUGGUGAAAUCAA | 397 | UUGAUUUCACCACUUAACA |
| 166 | ACAAAUAUUCUUAAUAGGG | 398 | CCCUAUUAAGAAUAUUUGU |

| SEQ ID NO | Sense strand sequence (5´-3´) | SEQ ID NO | Antisense strand sequence (5´-3´) |
|---|---|---|---|
| 167 | GCGGAAGUUGGAAUCAGGU | 399 | ACCUGAUUCCAACUUCCGC |
| 168 | AACUUGUGUAGACUAAGCA | 400 | UGCUUAGUCUACACAAGUU |
| 169 | AUUCUUAAUAGGGCUACUU | 401 | AAGUAGCCCUAUUAAGAAU |
| 170 | CCUAAAGUUAAUCCAGAUU | 402 | AAUCUGGAUUAACUUUAGG |
| 171 | UAUUGUUACCUAAAGUUAA | 403 | UUAACUUUAGGUAACAAUA |
| 172 | GUGCUGGUAGUAUCACCUU | 404 | AAGGUGAUACUACCAGCAC |
| 173 | CUGUGACUUACCAUAGCAG | 405 | CUGCUAUGGUAAGUCACAG |
| 174 | GAGCUUCUUAAGUUAAAUC | 406 | GAUUUAACUUAAGAAGCUC |
| 175 | CUGUUCGGAUAGAACAGGA | 407 | UCCUGUUCUAUCCGAACAG |
| 176 | GUUAUUGUUACCUAAAGUU | 408 | AACUUUAGGUAACAAUAAC |
| 177 | UAAUGUGAGGAUUAACUUC | 409 | GAAGUUAAUCCUCACAUUA |
| 178 | ACCACUAAUGGGAGCCAAU | 410 | AUUGGCUCCCAUUAGUGGU |
| 179 | UGUGUAGACUAAGCAUGUA | 411 | UACAUGCUUAGUCUACACA |
| 180 | UGGGCCUUGCGCUGGAUUG | 412 | CAAUCCAGCGCAAGGCCCA |
| 181 | AGGAGCUUCUUAAGUUAAA | 413 | UUUAACUUAAGAAGCUCCU |
| 182 | GGUGACCCUUUAGUGAGCU | 414 | AGCUCACUAAAGGGUCACC |
| 183 | AGAGUAGGCGAGUAUCAGA | 415 | UCUGAUACUCGCCUACUCU |
| 184 | GCAGUGACAAUGGCAGUCU | 416 | AGACUGCCAUUGUCACUGC |
| 185 | AAACGAUGCCUUGUGUCAA | 417 | UUGACACAAGGCAUCGUUU |
| 186 | GGACUGAUGCCUGGCCUCA | 418 | UGAGGCCAGGCAUCAGUCC |
| 187 | UGAGAGAUAAAUGUUGAUC | 419 | GAUCAACAUUUAUCUCUCA |
| 188 | UGGUUUCUACACCAAAUAC | 420 | GUAUUUGGUGUAGAAACCA |
| 189 | UCUCUGUAAUAUGAUACAU | 421 | AUGUAUCAUAUUACAGAGA |
| 190 | GAGAGAUAAAUGUUGAUCU | 422 | AGAUCAACAUUUAUCUCUC |
| 191 | ACUCUAAUGAAGCAAUACA | 423 | UGUAUUGCUUCAUUAGAGU |
| 192 | UGAAGUGUUACCAACUAGC | 424 | GCUAGUUGGUAACACUUCA |
| 193 | AAUGAAGCAAUACAUUGAG | 425 | CUCAAUGUAUUGCUUCAUU |
| 194 | ACGAUGCCUUGUGUCAAGA | 426 | UCUUGACACAAGGCAUCGU |

| SEQ ID NO | Sense strand sequence (5´-3´) | SEQ ID NO | Antisense strand sequence (5´-3´) |
|---|---|---|---|
| 195 | AGACCGCGAGGAGGAUCUU | 427 | AAGAUCCUCCUCGCGGUCU |
| 196 | UUGUUACCUAAAGUUAAUC | 428 | GAUUAACUUUAGGUAACAA |
| 197 | CAGAAGCCCGCUGUUUCUA | 429 | UAGAAACAGCGGGCUUCUG |
| 198 | UUUGACUUUAUGGAGAAUA | 430 | UAUUCUCCAUAAAGUCAAA |
| 199 | UACCUAAAGUUAAUCCAGA | 431 | UCUGGAUUAACUUUAGGUA |
| 200 | UUCAAACACCUGGUACACA | 432 | UGUGUACCAGGUGUUUGAA |
| 201 | UUGCACUCUAAUGAAGCAA | 433 | UUGCUUCAUUAGAGUGCAA |
| 202 | UGUUACCUAAAGUUAAUCC | 434 | GGAUUAACUUUAGGUAACA |
| 203 | CACUAAGUGACUAAAGUAA | 435 | UUACUUUAGUCACUUAGUG |
| 204 | UGCCAGAUAGAAGACAGGU | 436 | ACCUGUCUUCUAUCUGGCA |
| 205 | AAUGUAUAGUCUUCUUAUU | 437 | AAUAAGAAGACUAUACAUU |
| 206 | GACCACUAAUGGGAGCCAA | 438 | UUGGCUCCCAUUAGUGGUC |
| 207 | GUUACCUAAAGUUAAUCCA | 439 | UGGAUUAACUUUAGGUAAC |
| 208 | UGAUGCCUGGCCUCACAUU | 440 | AAUGUGAGGCCAGGCAUCA |
| 209 | CCAACUUUAAAGUCAGUCC | 441 | GGACUGACUUUAAAGUUGG |
| 210 | UAAACUUGUGUAGACUAAG | 442 | CUUAGUCUACACAAGUUUA |
| 211 | AGUAGGUUGUGUGAGUUAA | 443 | UUAACUCACACAACCUACU |
| 212 | GUUAAACUUGUGUAGACUA | 444 | UAGUCUACACAAGUUUAAC |
| 213 | CUGACCACUAAUGGGAGCC | 445 | GGCUCCCAUUAGUGGUCAG |
| 214 | UAUUCUUAAUAGGGCUACU | 446 | AGUAGCCCUAUUAAGAAUA |
| 215 | GUAGUGUCCUGGGAUUCUC | 447 | GAGAAUCCCAGGACACUAC |
| 216 | UAUCUGGCAGAUGUAUAAG | 448 | CUUAUACAUCUGCCAGAUA |
| 217 | AGGCUACCUAUGGUGAACG | 449 | CGUUCACCAUAGGUAGCCU |
| 218 | UCAGACCAUUUCCUAAUCA | 450 | UGAUUAGGAAAUGGUCUGA |
| 219 | UUACCUAAAGUUAAUCCAG | 451 | CUGGAUUAACUUUAGGUAA |
| 220 | GGUUUCUACACCAAAUACA | 452 | UGUAUUUGGUGUAGAAACC |
| 221 | GUUGGUGCCAGAUAGAAGA | 453 | UCUUCUAUCUGGCACCAAC |
| 222 | GCUACCUAUGGUGAACGUG | 454 | CACGUUCACCAUAGGUAGC |

| SEQ ID NO | Sense strand sequence (5´-3´) | SEQ ID NO | Antisense strand sequence (5´-3´) |
|---|---|---|---|
| 223 | UCACUAAGUGACUAAAGUA | 455 | UACUUUAGUCACUUAGUGA |
| 224 | UUAUUGUUACCUAAAGUUA | 456 | UAACUUUAGGUAACAAUAA |
| 225 | UAGCUGAAUAAUGUGAGGA | 457 | UCCUCACAUUAUUCAGCUA |
| 226 | UGACCACUAAUGGGAGCCA | 458 | UGGCUCCCAUUAGUGGUCA |
| 227 | GUAGCUACCUCACAACCAG | 459 | CUGGUUGUGAGGUAGCUAC |
| 228 | UCCCGCUCGCGCCCAUCAC | 460 | GUGAUGGGCGCGAGCGGGA |
| 229 | CUUGGCUUUAAAGUGAGGG | 461 | CCCUCACUUUAAAGCCAAG |
| 230 | AGAAGCCCGCUGUUUCUAU | 462 | AUAGAAACAGCGGGCUUCU |
| 231 | ACUAAAGUAAGUUAAACUU | 463 | AAGUUUAACUUACUUUAGU |
| 232 | AGUAAGUUAAACUUGUGUA | 464 | UACACAAGUUUAACUUACU |
| 233 | AAUAAUUAUCAAUGCUGUU | 465 | AACAGCAUUGAUAAUUAUU |

Table 2

| SEQ ID NO | sense strand sequence (5´-3´) | SEQ ID NO | antisense strand sequence (5´-3´) | Activity testing with 50nM siRNA in HeLaS3 cells, transfection 1 | | Activity testing with 500pM siRNA in HeLaS3 cells | | Activity testing with 50nM siRNA in HeLaS3 cells, transfection 2 | | Activity testing with 30pM siRNA in HeLaS3 cells | |
|---|---|---|---|---|---|---|---|---|---|---|---|
| | | | | mRNA [%] | s.d. [%] | mRNA [%] | s.d. [%] | mRNA [%] | s.d. [%] | mRNA [%] | s.d. [%] |
| 526 | agGAAAGAcuAAcuucuuudTsdT | 967 | AAAGAAGUuAGUCUUUCCUdTsdT | n.d. | n.d. | n.d. | n.d. | 10 | 2 | 42 | 1 |
| 477 | uucuGAAAuGuAuAGucuudTsdT | 963 | AAGACuAuAcAUUUcAGAAdTsdT | n.d. | n.d. | n.d. | n.d. | 8 | 1 | 48 | 2 |
| 470 | aguccAAcAGAGAAuucuudTsdT | 962 | AAGAAUUCUCUGUUGGACUdTsdT | n.d. | n.d. | n.d. | n.d. | 7 | 0 | 49 | 2 |
| 476 | gaAGGAAAGAcuAAcuucudTsdT | 966 | AGAAGUuAGUCUUUCCUUCdTsdT | n.d. | n.d. | n.d. | n.d. | 8 | 2 | 50 | 2 |
| 507 | ucuucuuAuuGAcAcuuAcdTsdT | 964 | GUAAGUGUcAAuAAGAAGAdTsdT | n.d. | n.d. | n.d. | n.d. | 3 | 1 | 53 | 3 |
| 553 | guAGGcGAGuAucAGAGGAdTsdT | 968 | UCCUCUGAuACUCGCCuACdTsdT | n.d. | n.d. | n.d. | n.d. | 12 | 2 | 54 | 4 |

| SEQ ID NO | sense strand sequence (5'-3') | SEQ ID NO | antisense strand sequence (5'-3') | Activity testing with 50nM siRNA in HeLaS3 cells, transfection 1 | | Activity testing with 500pM siRNA in HeLaS3 cells | | Activity testing with 50nM siRNA in HeLaS3 cells, transfection 2 | | Activity testing with 30pM siRNA in HeLaS3 cells | |
|---|---|---|---|---|---|---|---|---|---|---|---|
| | | | | mRNA [%] | s.d. [%] | mRNA [%] | s.d. [%] | mRNA [%] | s.d. [%] | mRNA [%] | s.d. [%] |
| 501 | cuucuuAuuGAcAcuuAcAdTsdT | 965 | UGuAAGUGUcAAuAAGAAGdTsdT | n.d. | n.d. | n.d. | n.d. | 9 | 1 | 58 | 7 |
| 497 | ucccAuGuucuGGcuuucudTsdT | 961 | AGAAAGCcAGAAcAUGGGAdTsdT | n.d. | n.d. | n.d. | n.d. | 5 | 1 | 70 | 7 |
| 508 | ucuucuUAuuGAcAcuuAcdTsdT | 972 | GUAAGUGUcAAuAaGAAGAdTsdT | n.d. | n.d. | n.d. | n.d. | 14 | 1 | 85 | 8 |
| 470 | aguccAAcAGAGAAuucuudTsdT | 970 | AAGAAUUCUCUGUuGGACUdTsdT | n.d. | n.d. | n.d. | n.d. | 15 | 1 | 89 | 2 |
| 477 | uucuGAAAuGuAuAGucuudTsdT | 971 | AAGACuAuAcAUUucAGAAdTsdT | n.d. | n.d. | n.d. | n.d. | 18 | 3 | 89 | 9 |
| 508 | ucuucuUAuuGAcAcuuAcdTsdT | 985 | GuAAGUGUcAAuAAGAAGAdTsdT | n.d. | n.d. | n.d. | n.d. | 11 | 2 | 91 | 5 |
| 507 | ucuucuuAuuGAcAcuuAcdTsdT | 972 | GUAAGUGUcAAuAaGAAGAdTsdT | n.d. | n.d. | n.d. | n.d. | 14 | 2 | 91 | 10 |
| 470 | aguccAAcAGAGAAuucuudTsdT | 978 | AaGAAUUCUCUGUuGGACUdTsdT | n.d. | n.d. | n.d. | n.d. | 24 | 3 | 92 | 7 |
| 501 | cuucuuAuuGAcAcuuAcAdTsdT | 973 | UGuAAGUGUcAAuaAGAAGdTsdT | n.d. | n.d. | n.d. | n.d. | 34 | 4 | 95 | 5 |
| 497 | ucccAuGuucuGGcuuucudTsdT | 969 | AGAAAGCcAGAAcaUGGGAdTsdT | n.d. | n.d. | n.d. | n.d. | 39 | 5 | 96 | 9 |
| 476 | gaAGGAAAGAcuAAcuucudTsdT | 974 | AGAAGUuAGUCUUuCCUUCdTsdT | n.d. | n.d. | n.d. | n.d. | 14 | 1 | 97 | 8 |
| 553 | guAGGcGAGuAucAGAGGAdTsdT | 984 | UcCUCUGAuACUCgCCuACdTsdT | n.d. | n.d. | n.d. | n.d. | 35 | 3 | 98 | 9 |
| 526 | agGAAAGAcuAAcuucuuudTsdT | 983 | AaAGAAGUuAGUCuUUCCUdTsdT | n.d. | n.d. | n.d. | n.d. | 26 | 3 | 98 | 8 |
| 553 | guAGGcGAGuAucAGAGGAdTsdT | 976 | UCCUCUGAuACUCgCCuACdTsdT | n.d. | n.d. | n.d. | n.d. | 23 | 1 | 99 | 11 |
| 526 | agGAAAGAcuAAcuucuuudTsdT | 975 | AAAGAAGUuAGUCuUUCCUdTsdT | n.d. | n.d. | n.d. | n.d. | 20 | 1 | 102 | 6 |
| 508 | ucuucuUAuuGAcAcuuAcdTsdT | 980 | GuAAGUGUcAAuAaGAAGAdTsdT | n.d. | n.d. | n.d. | n.d. | 21 | 1 | 102 | 13 |
| 477 | uucuGAAAuGuAuAGucuudTsdT | 979 | AaGACuAuAcAUUucAGAAdTsdT | n.d. | n.d. | n.d. | n.d. | 25 | 3 | 104 | 16 |
| 497 | ucccAuGuucuGGcuuucudTsdT | 977 | AgAAAGCcAGAAcaUGGGAdTsdT | n.d. | n.d. | n.d. | n.d. | 43 | 2 | 106 | 8 |
| 501 | cuucuuAuuGAcAcuuAcAdTsdT | 981 | UguAAGUGUcAAuaAGAAGdTsdT | n.d. | n.d. | n.d. | n.d. | 66 | 14 | 111 | 14 |
| 476 | gaAGGAAAGAcuAAcuucudTsdT | 982 | AgAAGUuAGUCUUuCCUUCdTsdT | n.d. | n.d. | n.d. | n.d. | 23 | 4 | 111 | 13 |
| 507 | ucuucuuAuuGAcAcuuAcdTsdT | 980 | GuAAGUGUcAAuAaGAAGAdTsdT | n.d. | n.d. | n.d. | n.d. | 21 | 5 | 115 | 15 |
| 507 | ucuucuuAuuGAcAcuuAcdTsdT | 841 | pGUAAGUGUcAAuAAGAAGAdTsdT | 7 | 2 | 11 | 2 | 2 | 0 | 45 | 2 |
| 477 | uucuGAAAuGuAuAGucuudTsdT | 839 | pAAGACuAuAcAUUUcAGAAdTsdT | 10 | 1 | 12 | 2 | 8 | 1 | 43 | 4 |
| 501 | cuucuuAuuGAcAcuuAcAdTsdT | 842 | pUGuAAGUGUcAAuAAGAAGdTsdT | 12 | 1 | 14 | 0 | 8 | 2 | 52 | 6 |

| SEQ ID NO | sense strand sequence (5'-3') | SEQ ID NO | antisense strand sequence (5'-3') | Activity testing with 50nM siRNA in HeLaS3 cells, transfection 1 | | Activity testing with 500pM siRNA in HeLaS3 cells | | Activity testing with 50nM siRNA in HeLaS3 cells, transfection 2 | | Activity testing with 30pM siRNA in HeLaS3 cells | |
|---|---|---|---|---|---|---|---|---|---|---|---|
| | | | | mRNA [%] | s.d. [%] | mRNA [%] | s.d. [%] | mRNA [%] | s.d. [%] | mRNA [%] | s.d. [%] |
| 469 | AguccAAcAGAGAAuucuudTsdT | 742 | pAAGAAUUCUCUGUUGGACUdTsdT | 11 | 1 | 14 | 0 | 8 | 0 | 38 | 4 |
| 475 | GaAGGAAAGAcuAAcuucudTsdT | 884 | pAGAAGUuAGUCUUUCCUUCdTsdT | 10 | 1 | 14 | 0 | 7 | 1 | 48 | 5 |
| 522 | cauuGAAAcGAuGccuuGudTsdT | 845 | pACAAGGcAUCGUUUcAAUGdTsdT | 11 | 2 | 14 | 2 | n.d. | n.d. | n.d. | n.d. |
| 525 | AgGAAAGAcuAAcuucuuudTsdT | 885 | pAAAGAAGUuAGUCUUUCCUdTsdT | 12 | 2 | 15 | 1 | 8 | 1 | 44 | 6 |
| 497 | ucccAuGuucuGGcuuucudTsdT | 711 | pAGAAAGCcAGAAcAUGGGAdTsdT | 12 | 1 | 15 | 2 | 6 | 1 | 71 | 5 |
| 471 | uaGGcGAGuAucAGAGGAudTsdT | 892 | pAUCCUCUGAuACUCGCCuAdTsdT | 14 | 1 | 16 | 1 | n.d. | n.d. | n.d. | n.d. |
| 552 | GuAGGcGAGuAucAGAGGAdTsdT | 891 | pUCCUCUGAuACUCGCCuACdTsdT | 14 | 2 | 16 | 1 | 10 | 0 | 51 | 5 |
| 494 | GgAuAGAAcAGGAGuuccudTsdT | 866 | pAGGAACUCCUGUUCuAUCCdTsdT | 17 | 1 | 16 | 1 | n.d. | n.d. | n.d. | n.d. |
| 473 | uguucAAAcAccuGGuAcAdTsdT | 863 | pUGuACcAGGUGUUUGAAcAdTsdT | 17 | 1 | 16 | 0 | n.d. | n.d. | n.d. | n.d. |
| 560 | cuuccAAAuuGccAuGGAAdTsdT | 838 | pUUCcAUGGcAAUUUGGAAGdTsdT | 16 | 2 | 16 | 1 | n.d. | n.d. | n.d. | n.d. |
| 544 | GccAuuGAAAcGAuGccuudTsdT | 718 | pAAGGcAUCGUUUcAAUGGCdTsdT | 13 | 2 | 17 | 1 | n.d. | n.d. | n.d. | n.d. |
| 493 | ugAuGuucAAAcAccuGGudTsdT | 861 | pACcAGGUGUUUGAAcAUcAdTsdT | 21 | 1 | 17 | 1 | n.d. | n.d. | n.d. | n.d. |
| 481 | AaAuGuAuAGucuucuuAudTsdT | 714 | pAUAAGAAGACuAuAcAUUUdTsdT | 14 | 1 | 17 | 1 | n.d. | n.d. | n.d. | n.d. |
| 488 | AuuGcAcucuAAuGAAGcAdTsdT | 735 | pUGCUUcAUuAGAGUGcAAUdTsdT | 13 | 1 | 18 | 2 | n.d. | n.d. | n.d. | n.d. |
| 532 | GaGAAGAGAGuAGGcGAGudTsdT | 887 | pACUCGCCuACUCUCUUCUCdTsdT | 18 | 2 | 18 | 1 | n.d. | n.d. | n.d. | n.d. |
| 535 | uuAuGcuGGAAcuGGGuuudTsdT | 881 | pAAACCcAGUUCcAGcAuAAdTsdT | 19 | 2 | 18 | 1 | n.d. | n.d. | n.d. | n.d. |
| 467 | uuGuGGcAGAcAGAcuuAudTsdT | 737 | pAUAAGUCUGUCUGCcAcAAdTsdT | 22 | 0 | 19 | 1 | n.d. | n.d. | n.d. | n.d. |
| 489 | uuAucAAuGcuGuucGGAudTsdT | 728 | pAUCCGAAcAGcAUUGAuAAdTsdT | 15 | 1 | 19 | 1 | n.d. | n.d. | n.d. | n.d. |
| 480 | cccAuuuGAcuuuAuGGAGdTsdT | 739 | pCUCcAuAAAGUcAAAUGGGdTsdT | 13 | 1 | 19 | 1 | n.d. | n.d. | n.d. | n.d. |
| 533 | AgAcuuAuGcuGGAAcuGGdTsdT | 879 | pCCAGUUCcAGcAuAAGUCUdTsdT | 14 | 2 | 19 | 2 | n.d. | n.d. | n.d. | n.d. |
| 582 | AucuGGcAGAuGuAuAAGAdTsdT | 825 | pUCUuAuAcAUCUGCcAGAUdTsdT | 19 | 3 | 19 | 1 | n.d. | n.d. | n.d. | n.d. |
| 495 | AauAuuucAcuGGAAGGAAdTsdT | 741 | pUUCCUUCcAGUGAAAuAUUdTsdT | 16 | 1 | 19 | 2 | n.d. | n.d. | n.d. | n.d. |
| 534 | uuAcAGAAGcccGcuGuuudTsdT | 831 | pAAAcAGCGGGCUUCUGuAAdTsdT | 23 | 2 | 20 | 3 | n.d. | n.d. | n.d. | n.d. |
| 487 | AuAGucuucuuAuuGAcAcdTsdT | 715 | pGUGUcAAuAAGAAGACuAUdTsdT | 19 | 1 | 20 | 3 | n.d. | n.d. | n.d. | n.d. |

| SEQ ID NO | sense strand sequence (5'-3') | SEQ ID NO | antisense strand sequence (5'-3') | Activity testing with 50nM siRNA in HeLaS3 cells, transfection 1 | | Activity testing with 500pM siRNA in HeLaS3 cells | | Activity testing with 50nM siRNA in HeLaS3 cells, transfection 2 | | Activity testing with 30pM siRNA in HeLaS3 cells | |
|---|---|---|---|---|---|---|---|---|---|---|---|
| | | | | mRNA [%] | s.d. [%] | mRNA [%] | s.d. [%] | mRNA [%] | s.d. [%] | mRNA [%] | s.d. [%] |
| 521 | GauuAcAGAAGcccGcuGudTsdT | 712 | pACAGCGGGCUUCUGuAAUCdTsdT | 19 | 2 | 20 | 1 | n.d. | n.d. | n.d. | n.d. |
| 663 | ugAAGuGuuAccAAcuAGcdTsdT | 743 | pGCuAGUUGGuAAcACUUcAdTsdT | 29 | 5 | 20 | 1 | n.d. | n.d. | n.d. | n.d. |
| 578 | uaucAAuGcuGuucGGAuAdTsdT | 865 | puAUCCGAAcAGcAUUGAuAdTsdT | 19 | 3 | 20 | 2 | n.d. | n.d. | n.d. | n.d. |
| 482 | uacAuuGAGuuuGuGGcAGdTsdT | 875 | pCUGCcAcAAACUcAAUGuAdTsdT | 15 | 1 | 20 | 2 | n.d. | n.d. | n.d. | n.d. |
| 528 | GacAAuGGcAGucuuGGcudTsdT | 755 | pAGCcAAGACUGCcAUUGUCdTsdT | 20 | 2 | 21 | 3 | n.d. | n.d. | n.d. | n.d. |
| 479 | ugcAcucuAAuGAAGcAAudTsdT | 871 | pAUUGCUUcAUuAGAGUGcAdTsdT | 15 | 1 | 21 | 1 | n.d. | n.d. | n.d. | n.d. |
| 559 | uaccAuGAuAucuGGcAGAdTsdT | 821 | pUCUGCcAGAuAUcAUGGuAdTsdT | 23 | 2 | 21 | 2 | n.d. | n.d. | n.d. | n.d. |
| 481 | AaAuGuAuAGucuucuuAudTsdT | 939 | pAuAAGAAGACuAuAcAUUUdTsdT | 18 | 2 | 21 | 1 | n.d. | n.d. | n.d. | n.d. |
| 484 | GaAcAGGAGuuccucAcuGdTsdT | 868 | pcAGUGAGGAACUCCUGUUCdTsdT | 28 | 1 | 21 | 1 | n.d. | n.d. | n.d. | n.d. |
| 498 | uucGGAuAGAAcAGGAGuudTsdT | 734 | pAACUCCUGUUCuAUCCGAAdTsdT | 18 | 1 | 21 | 1 | n.d. | n.d. | n.d. | n.d. |
| 512 | GaAAcGAGGAcuGAuGccudTsdT | 852 | pAGGcAUcAGUCCUCGUUUCdTsdT | 17 | 2 | 21 | 2 | n.d. | n.d. | n.d. | n.d. |
| 491 | cauuGAGuuuGuGGcAGAcdTsdT | 876 | pGUCUGCcAcAAACUcAAUGdTsdT | 17 | 1 | 21 | 2 | n.d. | n.d. | n.d. | n.d. |
| 485 | AucccAuGuucuGGcuuucdTsdT | 710 | pGAAAGCcAGAAcAUGGGAUdTsdT | 12 | 1 | 22 | 2 | n.d. | n.d. | n.d. | n.d. |
| 516 | AgGcGAGuAucAGAGGAuGdTsdT | 893 | pcAUCCUCUGAuACUCGCCUdTsdT | 21 | 2 | 22 | 2 | n.d. | n.d. | n.d. | n.d. |
| 518 | AacAuuGuuuGuAcucAcAdTsdT | 913 | pUGUGAGuAcAAAcAAUGUUdTsdT | 27 | 2 | 22 | 2 | n.d. | n.d. | n.d. | n.d. |
| 502 | uacAGAAGcccGcuGuuucdTsdT | 832 | pGAAAcAGCGGGCUUCUGuAdTsdT | 23 | 1 | 22 | 1 | n.d. | n.d. | n.d. | n.d. |
| 504 | AuAGAAcAGGAGuuccucAdTsdT | 867 | pUGAGGAACUCCUGUUCuAUdTsdT | 23 | 1 | 22 | 2 | n.d. | n.d. | n.d. | n.d. |
| 561 | AccGcGAGGAGGAucuuccdTsdT | 818 | pGGAAGAUCCUCCUCGCGGUdTsdT | 21 | 2 | 22 | 5 | n.d. | n.d. | n.d. | n.d. |
| 529 | AuGccuuGuGucAAGAAGAdTsdT | 846 | pUCUUCUUGAcAcAAGGcAUdTsdT | 19 | 2 | 22 | 0 | n.d. | n.d. | n.d. | n.d. |
| 568 | uuGccuGAuGuucAAAcAcdTsdT | 858 | pGUGUUUGAAcAUcAGGcAAdTsdT | 31 | 3 | 22 | 1 | n.d. | n.d. | n.d. | n.d. |
| 597 | AuuAcAGAAGcccGcuGuudTsdT | 830 | pAAcAGCGGGCUUCUGuAAUdTsdT | 23 | 3 | 23 | 2 | n.d. | n.d. | n.d. | n.d. |
| 510 | AguAccAuGAuAucuGGcAdTsdT | 820 | pUGCcAGAuAUcAUGGuACUdTsdT | 26 | 2 | 23 | 2 | n.d. | n.d. | n.d. | n.d. |
| 633 | uuAccAuAGcAGuGAcAAudTsdT | 754 | pAUUGUcACUGCuAUGGuAAdTsdT | 20 | 4 | 23 | 1 | n.d. | n.d. | n.d. | n.d. |
| 564 | AgGGAAuuucucuucAAuGdTsdT | 716 | pcAUUGAAGAGAAAUUCCCUdTsdT | 22 | 2 | 23 | 1 | n.d. | n.d. | n.d. | n.d. |

| SEQ ID NO | sense strand sequence (5´-3´) | SEQ ID NO | antisense strand sequence (5´-3´) | Activity testing with 50nM siRNA in HeLaS3 cells, transfection 1 | | Activity testing with 500pM siRNA in HeLaS3 cells | | Activity testing with 50nM siRNA in HeLaS3 cells, transfection 2 | | Activity testing with 30pM siRNA in HeLaS3 cells | |
|---|---|---|---|---|---|---|---|---|---|---|---|
| | | | | mRNA [%] | s.d. [%] | mRNA [%] | s.d. [%] | mRNA [%] | s.d. [%] | mRNA [%] | s.d. [%] |
| 515 | AguAGAGAAcccAuuuGAcdTsdT | 738 | pGUcAAAUGGGUUCUCuACUdTsdT | 20 | 2 | 23 | 3 | n.d. | n.d. | n.d. | n.d. |
| 523 | AcuuAuGcuGGAAcuGGGudTsdT | 880 | pACCcAGUUCcAGcAuAAGUdTsdT | 20 | 2 | 23 | 2 | n.d. | n.d. | n.d. | n.d. |
| 490 | AgAAAcGAGGAcuGAuGccdTsdT | 851 | pGGcAUcAGUCCUCGUUUCUdTsdT | 20 | 1 | 23 | 2 | n.d. | n.d. | n.d. | n.d. |
| 545 | AgccucAcuGcuucAAcGcdTsdT | 758 | pGCGUUGAAGcAGUGAGGCUdTsdT | 23 | 2 | 23 | 1 | n.d. | n.d. | n.d. | n.d. |
| 599 | caGuGAcAAuGGcAGucuudTsdT | 904 | pAAGACUGCcAUUGUcACUGdTsdT | 26 | 3 | 23 | 2 | n.d. | n.d. | n.d. | n.d. |
| 539 | caGccucAcuGcuucAAcGdTsdT | 908 | pCGUUGAAGcAGUGAGGCUGdTsdT | 25 | 2 | 23 | 2 | n.d. | n.d. | n.d. | n.d. |
| 521 | GauuAcAGAAGcccGcuGudTsdT | 938 | pAcAGCGGGCUUCUGuAAUCdTsdT | 22 | 1 | 23 | 1 | n.d. | n.d. | n.d. | n.d. |
| 573 | AauuAucAAuGcuGuucGGdTsdT | 727 | pCCGAAcAGcAUUGAuAAUUdTsdT | 20 | 3 | 24 | 2 | n.d. | n.d. | n.d. | n.d. |
| 505 | cuGGcAcuuuAcAAAcAAAdTsdT | 910 | pUUUGUUUGuAAAGUGCcAGdTsdT | 32 | 1 | 24 | 2 | n.d. | n.d. | n.d. | n.d. |
| 585 | ccAuGAuAucuGGcAGAuGdTsdT | 823 | pcAUCUGCcAGAuAUcAUGGdTsdT | 23 | 3 | 25 | 1 | n.d. | n.d. | n.d. | n.d. |
| 531 | uaccucAcAAccAGuccuGdTsdT | 745 | pcAGGACUGGUUGUGAGGuAdTsdT | 25 | 2 | 25 | 3 | n.d. | n.d. | n.d. | n.d. |
| 574 | GccuGAuGuucAAAcAccudTsdT | 859 | pAGGUGUUUGAAcAUcAGGCdTsdT | 21 | 3 | 25 | 1 | n.d. | n.d. | n.d. | n.d. |
| 492 | AcAuucAGcAcuGGGAAucdTsdT | 708 | pGAUUCCcAGUGCUGAAUGUdTsdT | 17 | 1 | 25 | 2 | n.d. | n.d. | n.d. | n.d. |
| 513 | AaGAGAGuAGGcGAGuAucdTsdT | 888 | pGAuACUCGCCuACUCUCUUdTsdT | 21 | 2 | 25 | 4 | n.d. | n.d. | n.d. | n.d. |
| 474 | GgGuGAcccuuuAGuGAGcdTsdT | 756 | pGCUcACuAAAGGGUcACCCdTsdT | 24 | 1 | 25 | 2 | n.d. | n.d. | n.d. | n.d. |
| 598 | GaGuAGGcGAGuAucAGAGdTsdT | 890 | pCUCUGAuACUCGCCuACUCdTsdT | 23 | 3 | 25 | 1 | n.d. | n.d. | n.d. | n.d. |
| 496 | AauAAAcAuuGuuuGuAcudTsdT | 911 | pAGuAcAAAcAAUGUUuAUUdTsdT | 25 | 1 | 25 | 2 | n.d. | n.d. | n.d. | n.d. |
| 506 | ucuAAuGAAGcAAuAcAuudTsdT | 872 | pAAUGuAUUGCUUcAUuAGAdTsdT | 22 | 1 | 26 | 1 | n.d. | n.d. | n.d. | n.d. |
| 500 | uuAuAGuGcuGGuAGuAucdTsdT | 746 | pGAuACuACcAGcACuAuAAdTsdT | 28 | 1 | 26 | 1 | n.d. | n.d. | n.d. | n.d. |
| 583 | uauAGuGcuGGuAGuAucAdTsdT | 747 | pUGAuACuACcAGcACuAuAdTsdT | 26 | 3 | 26 | 1 | n.d. | n.d. | n.d. | n.d. |
| 563 | AuGuucAAAcAccuGGuAcdTsdT | 862 | pGUACcAGGUGUUUGAAcAUdTsdT | 20 | 2 | 26 | 2 | n.d. | n.d. | n.d. | n.d. |
| 468 | ccuGAuGuucAAAcAccuGdTsdT | 860 | pcAGGUGUUUGAAcAUcAGGdTsdT | 18 | 0 | 27 | 2 | n.d. | n.d. | n.d. | n.d. |
| 587 | uucAAuGccAuuGAAAcGAdTsdT | 844 | pUCGUUUcAAUGGcAUUGAAdTsdT | 27 | 3 | 27 | 2 | n.d. | n.d. | n.d. | n.d. |
| 550 | uucAGAGuAGAGAAcccAudTsdT | 882 | pAUGGGUUCUCuACUCUGAAdTsdT | 25 | 2 | 27 | 2 | n.d. | n.d. | n.d. | n.d. |

| SEQ ID NO | sense strand sequence (5'-3') | SEQ ID NO | antisense strand sequence (5'-3') | Activity testing with 50nM siRNA in HeLaS3 cells, transfection 1 | | Activity testing with 500pM siRNA in HeLaS3 cells | | Activity testing with 50nM siRNA in HeLaS3 cells, transfection 2 | | Activity testing with 30pM siRNA in HeLaS3 cells | |
|---|---|---|---|---|---|---|---|---|---|---|---|
| | | | | mRNA [%] | s.d. [%] | mRNA [%] | s.d. [%] | mRNA [%] | s.d. [%] | mRNA [%] | s.d. [%] |
| 575 | cauAGcAGuGAcAAuGGcAdTsdT | 902 | pUGCcAUUGUcACUGCuAUGdTsdT | 33 | 3 | 28 | 1 | n.d. | n.d. | n.d. | n.d. |
| 624 | AgcAAGAccGcGAGGAGGAdTsdT | 814 | pUCCUCCUCGCGGUCUUGCUdTsdT | 29 | 4 | 28 | 4 | n.d. | n.d. | n.d. | n.d. |
| 627 | uuuAuAGuGcuGGuAGuAudTsdT | 900 | pAUACuACcAGcACuAuAAAdTsdT | 30 | 4 | 28 | 3 | n.d. | n.d. | n.d. | n.d. |
| 613 | caGAuuAcAGAAGcccGcudTsdT | 829 | pAGCGGGCUUCUGuAAUCUGdTsdT | 27 | 3 | 29 | 2 | n.d. | n.d. | n.d. | n.d. |
| 507 | ucuucuuAuuGAcAcuuAcdTsdT | 949 | pGuAAGUGUcAAuAAGAAGAdTsdT | 18 | 2 | 29 | 2 | n.d. | n.d. | n.d. | n.d. |
| 509 | uguucGGAuAGAAcAGGAGdTsdT | 733 | pCUCCUGUUCuAUCCGAAcAdTsdT | 18 | 2 | 30 | 4 | n.d. | n.d. | n.d. | n.d. |
| 530 | GccucAcuGcuucAAcGcAdTsdT | 909 | pUGCGUUGAAGcAGUGAGGCdTsdT | 28 | 2 | 30 | 5 | n.d. | n.d. | n.d. | n.d. |
| 483 | caAuAcAuuGAGuuuGuGGdTsdT | 874 | pCCAcAAACUcAAUGuAUUGdTsdT | 19 | 1 | 30 | 1 | n.d. | n.d. | n.d. | n.d. |
| 519 | GauGGGAGuGAuGucAAGudTsdT | 896 | pACUUGAcAUcACUCCcAUCdTsdT | 26 | 2 | 30 | 2 | n.d. | n.d. | n.d. | n.d. |
| 537 | ucAAuGccAuuGAAAcGAudTsdT | 717 | pAUCGUUUcAAUGGcAUUGAdTsdT | 31 | 2 | 30 | 2 | n.d. | n.d. | n.d. | n.d. |
| 511 | caGAGAuGAGGGuuuAcAcdTsdT | 856 | pGUGuAAACCCUcAUCUCUGdTsdT | 23 | 2 | 30 | 3 | n.d. | n.d. | n.d. | n.d. |
| 620 | caGcAAGAccGcGAGGAGGdTsdT | 707 | pCCUCCUCGCGGUCUUGCUGdTsdT | 30 | 4 | 31 | 4 | n.d. | n.d. | n.d. | n.d. |
| 595 | AccucAcAAccAGuccuGudTsdT | 899 | pACAGGACUGGUUGUGAGGUdTsdT | 31 | 3 | 31 | 1 | n.d. | n.d. | n.d. | n.d. |
| 668 | caGAAGcccGcuGuuucuAdTsdT | 833 | puAGAAAcAGCGGGCUUCUGdTsdT | 32 | 6 | 32 | 3 | n.d. | n.d. | n.d. | n.d. |
| 577 | AguGcuGGuAGuAucAccudTsdT | 749 | pAGGUGAuACuACcAGcACUdTsdT | 28 | 3 | 32 | 2 | n.d. | n.d. | n.d. | n.d. |
| 654 | AgAGuAGGcGAGuAucAGAdTsdT | 889 | pUCUGAuACUCGCCuACUCUdTsdT | 48 | 5 | 33 | 1 | n.d. | n.d. | n.d. | n.d. |
| 546 | GgcAGAcAGAcuuAuGcuGdTsdT | 878 | pcAGcAuAAGUCUGUCUGCCdTsdT | 21 | 2 | 33 | 3 | n.d. | n.d. | n.d. | n.d. |
| 622 | uuAccuuGGAuGcuGAcuudTsdT | 897 | pAAGUcAGcAUCcAAGGuAAdTsdT | 50 | 4 | 33 | 2 | n.d. | n.d. | n.d. | n.d. |
| 541 | GgcuGuGAcuuAccAuAGcdTsdT | 751 | pGCuAUGGuAAGUcAcAGCCdTsdT | 21 | 2 | 33 | 2 | n.d. | n.d. | n.d. | n.d. |
| 566 | GauGAGGGuuuAcAcuGuGdTsdT | 857 | pcAcAGUGuAAACCCUcAUCdTsdT | 33 | 2 | 34 | 2 | n.d. | n.d. | n.d. | n.d. |
| 503 | GuGAcccuuuAGuGAGcuudTsdT | 907 | pAAGCUcACuAAAGGGUcACdTsdT | 25 | 1 | 34 | 2 | n.d. | n.d. | n.d. | n.d. |
| 527 | caAGAccGcGAGGAGGAucdTsdT | 816 | pGAUCCUCCUCGCGGUCUUGdTsdT | 29 | 2 | 35 | 8 | n.d. | n.d. | n.d. | n.d. |
| 589 | cuAuGGcuucc AAAuuGccdTsdT | 837 | pGGcAAUUUGGAAGCcAuAGdTsdT | 23 | 3 | 36 | 3 | n.d. | n.d. | n.d. | n.d. |
| 643 | GuGcuGGuAGuAucAccuudTsdT | 750 | pAAGGUGAuACuACcAGcACdTsdT | 37 | 4 | 37 | 2 | n.d. | n.d. | n.d. | n.d. |

| SEQ ID NO | sense strand sequence (5´-3´) | SEQ ID NO | antisense strand sequence (5´-3´) | Activity testing with 50nM siRNA in HeLaS3 cells, transfection 1 | | Activity testing with 500pM siRNA in HeLaS3 cells | | Activity testing with 50nM siRNA in HeLaS3 cells, transfection 2 | | Activity testing with 30pM siRNA in HeLaS3 cells | |
|---|---|---|---|---|---|---|---|---|---|---|---|
| | | | | mRNA [%] | s.d. [%] | mRNA [%] | s.d. [%] | mRNA [%] | s.d. [%] | mRNA [%] | s.d. [%] |
| 600 | GgccuuGcGcuGGAuuGGGdTsdT | 849 | pCCcAAUCcAGCGcAAGGCCdTsdT | 27 | 3 | 38 | 2 | n.d. | n.d. | n.d. | n.d. |
| 572 | AuGcuGuucGGAuAGAAcAdTsdT | 730 | pUGUUCuAUCCGAAcAGcAUdTsdT | 32 | 3 | 38 | 1 | n.d. | n.d. | n.d. | n.d. |
| 517 | uaGAcuAAGcAuGuAAuuudTsdT | 784 | pAAAUuAcAUGCUuAGUCuAdTsdT | 32 | 2 | 39 | 3 | n.d. | n.d. | n.d. | n.d. |
| 611 | GgcuGGcuGuGAcuuAccAdTsdT | 901 | pUGGuAAGUcAcAGCcAGCCdTsdT | 38 | 3 | 40 | 2 | n.d. | n.d. | n.d. | n.d. |
| 540 | ucuuGGcuuuAAAGuGAGGdTsdT | 905 | pCCUcACUUuAAAGCcAAGAdTsdT | 23 | 2 | 40 | 2 | n.d. | n.d. | n.d. | n.d. |
| 536 | AuAAAcAuuGuuuGuAcucdTsdT | 912 | pGAGuAcAAAcAAUGUUuAUdTsdT | 50 | 2 | 40 | 4 | n.d. | n.d. | n.d. | n.d. |
| 524 | GucGAcAAGGAGAAcAcGcdTsdT | 705 | pGCGUGUUCUCCUUGUCGACdTsdT | 32 | 2 | 41 | 3 | n.d. | n.d. | n.d. | n.d. |
| 551 | AaAcGAGGAcuGAuGccuGdTsdT | 724 | pcAGGcAUcAGUCCUCGUUUdTsdT | 26 | 2 | 42 | 4 | n.d. | n.d. | n.d. | n.d. |
| 601 | uucuuAuuGAcAcuuAcAudTsdT | 843 | pAUGuAAGUGUcAAuAAGAAdTsdT | 31 | 3 | 43 | 3 | n.d. | n.d. | n.d. | n.d. |
| 586 | uuAAAcuuGuGuAGAcuAAdTsdT | 777 | pUUAGUCuAcAcAAGUUuAAdTsdT | 37 | 3 | 43 | 4 | n.d. | n.d. | n.d. | n.d. |
| 616 | GuGAcuuAccAuAGcAGuGdTsdT | 753 | pcACUGCuAUGGuAAGUcACdTsdT | 24 | 4 | 43 | 4 | n.d. | n.d. | n.d. | n.d. |
| 472 | GgcGAGuAucAGAGGAuGGdTsdT | 894 | pCCAUCCUCUGAuACUCGCCdTsdT | 26 | 1 | 44 | 2 | n.d. | n.d. | n.d. | n.d. |
| 570 | uauAucccAuGuucuGGcudTsdT | 828 | pAGCcAGAAcAUGGGAuAuAdTsdT | 28 | 3 | 44 | 8 | n.d. | n.d. | n.d. | n.d. |
| 655 | GcAGuGAcAAuGGcAGucudTsdT | 903 | pAGACUGCcAUUGUcACUGCdTsdT | 34 | 5 | 44 | 2 | n.d. | n.d. | n.d. | n.d. |
| 671 | uucAAAcAccuGGuAcAcAdTsdT | 864 | pUGUGuACcAGGUGUUUGAAdTsdT | 59 | 6 | 44 | 4 | n.d. | n.d. | n.d. | n.d. |
| 580 | AauGcuGuucGGAuAGAAcdTsdT | 729 | pGUUCuAUCCGAAcAGcAUUdTsdT | 31 | 3 | 44 | 1 | n.d. | n.d. | n.d. | n.d. |
| 618 | ugGcAGAuGuAuAAGAAGGdTsdT | 826 | pCCUUCUuAuAcAUCUGCcAdTsdT | 36 | 4 | 44 | 7 | n.d. | n.d. | n.d. | n.d. |
| 478 | cuGuGuAGcuAccucAcAAdTsdT | 744 | pUUGUGAGGuAGCuAcAcAGdTsdT | 24 | 1 | 46 | 3 | n.d. | n.d. | n.d. | n.d. |
| 533 | AgAcuuAuGcuGGAAcuGGdTsdT | 954 | pCcAGUUCcAGcAuAAGUCUdTsdT | 26 | 1 | 46 | 3 | n.d. | n.d. | n.d. | n.d. |
| 605 | GuAAGuuAAAcuuGuGuAGdTsdT | 775 | pCUAcAcAAGUUuAACUuACdTsdT | 44 | 3 | 46 | 3 | n.d. | n.d. | n.d. | n.d. |
| 571 | uuGuGuAGAcuAAGcAuGudTsdT | 781 | pACAUGCUuAGUCuAcAcAAdTsdT | 47 | 3 | 47 | 2 | n.d. | n.d. | n.d. | n.d. |
| 557 | uguGGcAGAcAGAcuuAuGdTsdT | 877 | pcAuAAGUCUGUCUGCcAcAdTsdT | 31 | 2 | 47 | 7 | n.d. | n.d. | n.d. | n.d. |
| 467 | uuGuGGcAGAcAGAcuuAudTsdT | 940 | pAuAAGUCUGUCUGCcAcAAdTsdT | 86 | 15 | 47 | 5 | n.d. | n.d. | n.d. | n.d. |
| 579 | GacuAAAGuAAGuuAAAcudTsdT | 772 | pAGUUuAACUuACUUuAGUCdTsdT | 32 | 3 | 47 | 2 | n.d. | n.d. | n.d. | n.d. |

| SEQ ID NO | sense strand sequence (5´-3´) | SEQ ID NO | antisense strand sequence (5´-3´) | Activity testing with 50nM siRNA in HeLaS3 cells, transfection 1 | | Activity testing with 500pM siRNA in HeLaS3 cells | | Activity testing with 50nM siRNA in HeLaS3 cells, transfection 2 | | Activity testing with 30pM siRNA in HeLaS3 cells | |
|---|---|---|---|---|---|---|---|---|---|---|---|
| | | | | mRNA [%] | s.d. [%] | mRNA [%] | s.d. [%] | mRNA [%] | s.d. [%] | mRNA [%] | s.d. [%] |
| 543 | cgcGAGGAGGAucuuccAGdTsdT | 819 | pCUGGAAGAUCCUCCUCGCGdTsdT | 52 | 2 | 48 | 7 | n.d. | n.d. | n.d. | n.d. |
| 547 | GuGAcuAAAGuAAGuuAAAdTsdT | 770 | pUUuAACUuACUUuAGUcACdTsdT | 39 | 2 | 48 | 2 | n.d. | n.d. | n.d. | n.d. |
| 602 | uucAcuAAGuGAcuAAAGudTsdT | 765 | pACUUuAGUcACUuAGUGAAdTsdT | 39 | 3 | 49 | 4 | n.d. | n.d. | n.d. | n.d. |
| 628 | GcAAGAccGcGAGGAGGAudTsdT | 815 | pAUCCUCCUCGCGGUCUUGCdTsdT | 36 | 4 | 49 | 3 | n.d. | n.d. | n.d. | n.d. |
| 571 | uuGuGuAGAcuAAGcAuGudTsdT | 945 | pAcAUGCUuAGUCuAcAcAAdTsdT | 59 | 3 | 50 | 4 | n.d. | n.d. | n.d. | n.d. |
| 562 | GaAAuGuAuAGucuucuuAdTsdT | 713 | puAAGAAGACuAuAcAUUUCdTsdT | 49 | 2 | 51 | 6 | n.d. | n.d. | n.d. | n.d. |
| 593 | GcGAGuAucAGAGGAuGGGdTsdT | 895 | pCCcAUCCUCUGAuACUCGCdTsdT | 27 | 3 | 52 | 5 | n.d. | n.d. | n.d. | n.d. |
| 538 | AuAGuGcuGGuAGuAucAcdTsdT | 748 | pGUGAuACuACcAGcACuAUdTsdT | 57 | 2 | 52 | 3 | n.d. | n.d. | n.d. | n.d. |
| 666 | AgAccGcGAGGAGGAucuudTsdT | 817 | pAAGAUCCUCCUCGCGGUCUdTsdT | 52 | 6 | 52 | 15 | n.d. | n.d. | n.d. | n.d. |
| 567 | uguGuGAGuuAAucAuuudTsdT | 922 | pAAAUGAAUuAACUcAcAcAdTsdT | 48 | 2 | 53 | 4 | n.d. | n.d. | n.d. | n.d. |
| 629 | ucuAuGGcuuccAAAuuGcdTsdT | 836 | pGCAAUUUGGAAGCcAuAGAdTsdT | 63 | 4 | 53 | 3 | n.d. | n.d. | n.d. | n.d. |
| 590 | AaGuGAcuAAAGuAAGuuAdTsdT | 769 | puAACUuACUUuAGUcACUUdTsdT | 62 | 3 | 53 | 7 | n.d. | n.d. | n.d. | n.d. |
| 681 | uaAAcuuGuGuAGAcuAAGdTsdT | 778 | pCUuAGUCuAcAcAAGUUuAdTsdT | 52 | 7 | 53 | 3 | n.d. | n.d. | n.d. | n.d. |
| 703 | AguAAGuuAAAcuuGuGuAdTsdT | 774 | puAcAcAAGUUuAACUuACUdTsdT | 50 | 13 | 54 | 2 | n.d. | n.d. | n.d. | n.d. |
| 603 | GuGuGAGuuAAucAuuuAdTsdT | 791 | puAAAUGAAUuAACUcAcACdTsdT | 46 | 3 | 54 | 8 | n.d. | n.d. | n.d. | n.d. |
| 642 | uauuGuuAccuAAAGuuAAdTsdT | 803 | pUUAACUUuAGGuAAcAAuAdTsdT | 44 | 4 | 55 | 6 | n.d. | n.d. | n.d. | n.d. |
| 674 | cacuAAGuGAcuAAAGuAAdTsdT | 942 | pUuACUUuAGUcACUuAGUGdTsdT | 51 | 3 | 55 | 2 | n.d. | n.d. | n.d. | n.d. |
| 569 | AaAcuuGuGuAGAcuAAGcdTsdT | 779 | pGCUuAGUCuAcAcAAGUUUdTsdT | 60 | 3 | 56 | 3 | n.d. | n.d. | n.d. | n.d. |
| 636 | uguuAAGuGGuGAAAucAAdTsdT | 918 | pUUGAUUUcACcACUuAAcAdTsdT | 38 | 4 | 56 | 3 | n.d. | n.d. | n.d. | n.d. |
| 659 | ugGuuucuAcAccAAAuAcdTsdT | 941 | pGuAUUUGGUGuAGAAACcAdTsdT | 51 | 4 | 56 | 8 | n.d. | n.d. | n.d. | n.d. |
| 588 | AgAAAGcuGAGAcAuuGcAdTsdT | 925 | pUGcAAUGUCUcAGCUUUCUdTsdT | 43 | 3 | 57 | 1 | n.d. | n.d. | n.d. | n.d. |
| 596 | AcuAAGuGAcuAAAGuAAGdTsdT | 768 | pCUuACUUuAGUcACUuAGUdTsdT | 52 | 3 | 57 | 5 | n.d. | n.d. | n.d. | n.d. |
| 609 | uguAGAcuAAGcAuGuAAudTsdT | 783 | pAUuAcAUGCUuAGUCuAcAdTsdT | 60 | 3 | 58 | 5 | n.d. | n.d. | n.d. | n.d. |
| 650 | uguGuAGAcuAAGcAuGuAdTsdT | 782 | puAcAUGCUuAGUCuAcAcAdTsdT | 54 | 5 | 58 | 4 | n.d. | n.d. | n.d. | n.d. |

| SEQ ID NO | sense strand sequence (5´-3´) | SEQ ID NO | antisense strand sequence (5´-3´) | Activity testing with 50nM siRNA in HeLaS3 cells, transfection 1 | | Activity testing with 500pM siRNA in HeLaS3 cells | | Activity testing with 50nM siRNA in HeLaS3 cells, transfection 2 | | Activity testing with 30pM siRNA in HeLaS3 cells | |
|---|---|---|---|---|---|---|---|---|---|---|---|
| | | | | mRNA [%] | s.d. [%] | mRNA [%] | s.d. [%] | mRNA [%] | s.d. [%] | mRNA [%] | s.d. [%] |
| 591 | ugAcuAAAGuAAGuuAAAcdTsdT | 771 | pGUUuAACUuACUUuAGUcAdTsdT | 54 | 3 | 58 | 1 | n.d. | n.d. | n.d. | n.d. |
| 639 | AacuuGuGuAGAcuAAGcAdTsdT | 780 | pUGCUuAGUCuAcAcAAGUUdTsdT | 57 | 4 | 58 | 2 | n.d. | n.d. | n.d. | n.d. |
| 683 | GuuAAAcuuGuGuAGAcuAdTsdT | 776 | puAGUCuAcAcAAGUUuAACdTsdT | 55 | 7 | 58 | 3 | n.d. | n.d. | n.d. | n.d. |
| 586 | uuAAAcuuGuGuAGAcuAAdTsdT | 944 | pUuAGUCuAcAcAAGUUuAAdTsdT | 62 | 6 | 59 | 2 | n.d. | n.d. | n.d. | n.d. |
| 653 | GguGAcccuuuAGuGAGcudTsdT | 757 | pAGCUcACuAAAGGGUcACCdTsdT | 60 | 5 | 60 | 7 | n.d. | n.d. | n.d. | n.d. |
| 542 | GgcuAccuAuGGuGAAcGudTsdT | 722 | pACGUUcACcAuAGGuAGCCdTsdT | 30 | 2 | 60 | 6 | n.d. | n.d. | n.d. | n.d. |
| 631 | AccAuGAuAucuGGcAGAudTsdT | 822 | pAUCUGCcAGAuAUcAUGGUdTsdT | 65 | 4 | 60 | 4 | n.d. | n.d. | n.d. | n.d. |
| 605 | GuAAGuuAAAcuuGuGuAGdTsdT | 943 | pCuAcAcAAGUUuAACUuACdTsdT | 51 | 3 | 60 | 6 | n.d. | n.d. | n.d. | n.d. |
| 676 | AauGuAuAGucuucuuAuudTsdT | 840 | pAAuAAGAAGACuAuAcAUUdTsdT | 62 | 6 | 60 | 8 | n.d. | n.d. | n.d. | n.d. |
| 499 | AaGuAGGuuGuGuGAGuuAdTsdT | 790 | puAACUcAcAcAACCuACUUdTsdT | 56 | 1 | 60 | 2 | n.d. | n.d. | n.d. | n.d. |
| 669 | uuuGAcuuuAuGGAGAAuAdTsdT | 883 | puAUUCUCcAuAAAGUcAAAdTsdT | 61 | 6 | 60 | 2 | n.d. | n.d. | n.d. | n.d. |
| 677 | GaccAcuAAuGGGAGccAAdTsdT | 914 | pUUGGCUCCcAUuAGUGGUCdTsdT | 56 | 6 | 60 | 6 | n.d. | n.d. | n.d. | n.d. |
| 627 | uuuAuAGuGcuGGuAGuAudTsdT | 957 | pAuACuACcAGcACuAuAAAdTsdT | 74 | 9 | 61 | 1 | n.d. | n.d. | n.d. | n.d. |
| 656 | AaAcGAuGccuuGuGucAAdTsdT | 719 | pUUGAcAcAAGGcAUCGUUUdTsdT | 40 | 5 | 62 | 8 | n.d. | n.d. | n.d. | n.d. |
| 647 | GuuAuuGuuAccuAAAGuudTsdT | 801 | pAACUUuAGGuAAcAAuAACdTsdT | 63 | 5 | 62 | 14 | n.d. | n.d. | n.d. | n.d. |
| 644 | cuGuGAcuuAccAuAGcAGdTsdT | 752 | pCUGCuAUGGuAAGUcAcAGdTsdT | 30 | 4 | 62 | 6 | n.d. | n.d. | n.d. | n.d. |
| 652 | AgGAGcuucuuAAGuuAAAdTsdT | 788 | pUUuAACUuAAGAAGCUCCUdTsdT | 61 | 5 | 62 | 4 | n.d. | n.d. | n.d. | n.d. |
| 672 | uuGcAcucuAAuGAAGcAAdTsdT | 870 | pUUGCUUcAUuAGAGUGcAAdTsdT | 88 | 6 | 63 | 7 | n.d. | n.d. | n.d. | n.d. |
| 682 | AguAGGuuGuGuGAGuuAAdTsdT | 920 | pUUAACUcAcAcAACCuACUdTsdT | 60 | 7 | 63 | 5 | n.d. | n.d. | n.d. | n.d. |
| 594 | GgGccuuGcGcuGGAuuGGdTsdT | 848 | pCCAAUCcAGCGcAAGGCCCdTsdT | 32 | 3 | 63 | 5 | n.d. | n.d. | n.d. | n.d. |
| 701 | AgAAGcccGcuGuuucuAudTsdT | 834 | pAUAGAAAcAGCGGGCUUCUdTsdT | 79 | 11 | 63 | 5 | n.d. | n.d. | n.d. | n.d. |
| 697 | ugAccAcuAAuGGGAGccAdTsdT | 763 | pUGGCUCCcAUuAGUGGUcAdTsdT | 68 | 9 | 64 | 8 | n.d. | n.d. | n.d. | n.d. |
| 664 | AauGAAGcAAuAcAuuGAGdTsdT | 873 | pCUcAAUGuAUUGCUUcAUUdTsdT | 63 | 5 | 64 | 4 | n.d. | n.d. | n.d. | n.d. |
| 662 | AcucuAAuGAAGcAAuAcAdTsdT | 736 | pUGuAUUGCUUcAUuAGAGUdTsdT | 71 | 5 | 64 | 6 | n.d. | n.d. | n.d. | n.d. |

| SEQ ID NO | sense strand sequence (5´-3´) | SEQ ID NO | antisense strand sequence (5´-3´) | Activity testing with 50nM siRNA in HeLaS3 cells, transfection 1 | | Activity testing with 500pM siRNA in HeLaS3 cells | | Activity testing with 50nM siRNA in HeLaS3 cells, transfection 2 | | Activity testing with 30pM siRNA in HeLaS3 cells | |
|---|---|---|---|---|---|---|---|---|---|---|---|
| | | | | mRNA [%] | s.d. [%] | mRNA [%] | s.d. [%] | mRNA [%] | s.d. [%] | mRNA [%] | s.d. [%] |
| 659 | ugGuuucuAcAccAAAuAcdTsdT | 760 | pGUAUUUGGUGuAGAAACcAdTsdT | 54 | 5 | 64 | 3 | n.d. | n.d. | n.d. | n.d. |
| 615 | AuAAuuAucAAuGcuGuucdTsdT | 726 | pGAAcAGcAUUGAuAAUuAUdTsdT | 45 | 4 | 65 | 4 | n.d. | n.d. | n.d. | n.d. |
| 558 | AgAGAuAAAuGuuGAucuudTsdT | 798 | pAAGAUcAAcAUUuAUCUCUdTsdT | 67 | 2 | 65 | 7 | n.d. | n.d. | n.d. | n.d. |
| 651 | ugGGccuuGcGcuGGAuuGdTsdT | 847 | pcAAUCcAGCGcAAGGCCcAdTsdT | 67 | 5 | 65 | 3 | n.d. | n.d. | n.d. | n.d. |
| 698 | GuAGcuAccucAcAAccAGdTsdT | 898 | pCUGGUUGUGAGGuAGCuACdTsdT | 61 | 9 | 65 | 2 | n.d. | n.d. | n.d. | n.d. |
| 680 | ccAAcuuuAAAGucAGuccdTsdT | 915 | pGGACUGACUUuAAAGUUGGdTsdT | 59 | 6 | 65 | 2 | n.d. | n.d. | n.d. | n.d. |
| 645 | GaGcuucuuAAGuuAAAucdTsdT | 917 | pGAUUuAACUuAAGAAGCUCdTsdT | 59 | 5 | 65 | 2 | n.d. | n.d. | n.d. | n.d. |
| 642 | uauuGuuAccuAAAGuuAAdTsdT | 947 | pUuAACUUuAGGuAAcAAuAdTsdT | 69 | 4 | 65 | 3 | n.d. | n.d. | n.d. | n.d. |
| 486 | GuAGGuuGuGuGAGuuAAudTsdT | 921 | pAUuAACUcAcAcAACCuACdTsdT | 61 | 1 | 65 | 4 | n.d. | n.d. | n.d. | n.d. |
| 692 | GuuGGuGccAGAuAGAAGAdTsdT | 785 | pUCUUCuAUCUGGcACcAACdTsdT | 65 | 8 | 65 | 4 | n.d. | n.d. | n.d. | n.d. |
| 691 | GguuucuAcAccAAAuAcAdTsdT | 761 | pUGuAUUUGGUGuAGAAACCdTsdT | 63 | 7 | 66 | 6 | n.d. | n.d. | n.d. | n.d. |
| 674 | cacuAAGuGAcuAAAGuAAdTsdT | 767 | pUUACUUuAGUcACUuAGUGdTsdT | 61 | 6 | 66 | 7 | n.d. | n.d. | n.d. | n.d. |
| 623 | AuucAGcAcuGGGAAucccdTsdT | 709 | pGGGAUUCCcAGUGCUGAAUdTsdT | 68 | 4 | 66 | 9 | n.d. | n.d. | n.d. | n.d. |
| 581 | AgAAuAuuucAcuGGAAGGdTsdT | 740 | pCCUUCcAGUGAAAuAUUCUdTsdT | 43 | 3 | 67 | 4 | n.d. | n.d. | n.d. | n.d. |
| 673 | uguuAccuAAAGuuAAuccdTsdT | 805 | pGGAUuAACUUuAGGuAAcAdTsdT | 70 | 6 | 67 | 9 | n.d. | n.d. | n.d. | n.d. |
| 607 | AuGuGAGGAuuAAcuucuGdTsdT | 810 | pcAGAAGUuAAUCCUcAcAUdTsdT | 69 | 3 | 67 | 6 | n.d. | n.d. | n.d. | n.d. |
| 686 | GuAGuGuccuGGGAuucucdTsdT | 787 | pGAGAAUCCcAGGAcACuACdTsdT | 72 | 7 | 68 | 4 | n.d. | n.d. | n.d. | n.d. |
| 576 | uguGAGuuAAuucAuuuAudTsdT | 923 | pAUAAAUGAAUuAACUcAcAdTsdT | 64 | 3 | 68 | 5 | n.d. | n.d. | n.d. | n.d. |
| 619 | AuAGcuuGAuuuAuuuGGudTsdT | 759 | pACcAAAuAAAUcAAGCuAUdTsdT | 59 | 4 | 68 | 5 | n.d. | n.d. | n.d. | n.d. |
| 608 | uuAAGuGGuGAAAucAAcudTsdT | 919 | pAGUUGAUUUcACcACUuAAdTsdT | 62 | 3 | 68 | 9 | n.d. | n.d. | n.d. | n.d. |
| 682 | AguAGGuuGuGuGAGuuAAdTsdT | 958 | pUuAACUcAcAcAACCuACUdTsdT | 75 | 5 | 69 | 5 | n.d. | n.d. | n.d. | n.d. |
| 549 | GccuuuAuGuuuGGGAGAAdTsdT | 924 | pUUCUCCcAAAcAuAAAGGCdTsdT | 48 | 2 | 69 | 4 | n.d. | n.d. | n.d. | n.d. |
| 520 | caGAccAuuuccuAAucAGdTsdT | 937 | pCUGAUuAGGAAAUGGUCUGdTsdT | 70 | 2 | 69 | 3 | n.d. | n.d. | n.d. | n.d. |
| 556 | GaAuAAuGuGAGGAuuAAcdTsdT | 933 | pGUuAAUCCUcAcAUuAUUCdTsdT | 64 | 2 | 70 | 7 | n.d. | n.d. | n.d. | n.d. |

| SEQ ID NO | sense strand sequence (5´-3´) | SEQ ID NO | antisense strand sequence (5´-3´) | Activity testing with 50nM siRNA in HeLaS3 cells, transfection 1 | | Activity testing with 500pM siRNA in HeLaS3 cells | | Activity testing with 50nM siRNA in HeLaS3 cells, transfection 2 | | Activity testing with 30pM siRNA in HeLaS3 cells | |
|---|---|---|---|---|---|---|---|---|---|---|---|
| | | | | mRNA [%] | s.d. [%] | mRNA [%] | s.d. [%] | mRNA [%] | s.d. [%] | mRNA [%] | s.d. [%] |
| 679 | ugAuGccuGGccucAcAuudTsdT | 855 | pAAUGUGAGGCcAGGcAUcAdTsdT | 72 | 6 | 70 | 8 | n.d. | n.d. | n.d. | n.d. |
| 660 | ucucuGuAAuAuGAuAcAudTsdT | 927 | pAUGuAUcAuAUuAcAGAGAdTsdT | 62 | 5 | 70 | 3 | n.d. | n.d. | n.d. | n.d. |
| 694 | ucAcuAAGuGAcuAAAGuAdTsdT | 766 | puACUUuAGUcACUuAGUGAdTsdT | 68 | 8 | 70 | 5 | n.d. | n.d. | n.d. | n.d. |
| 675 | ugccAGAuAGAAGAcAGGudTsdT | 786 | pACCUGUCUUCuAUCUGGcAdTsdT | 64 | 6 | 70 | 3 | n.d. | n.d. | n.d. | n.d. |
| 658 | ugAGAGAuAAAuGuuGAucdTsdT | 797 | pGAUcAAcAUUuAUCUCUcAdTsdT | 70 | 5 | 71 | 9 | n.d. | n.d. | n.d. | n.d. |
| 684 | cuGAccAcuAAuGGGAGccdTsdT | 762 | pGGCUCCcAUuAGUGGUcAGdTsdT | 68 | 7 | 72 | 9 | n.d. | n.d. | n.d. | n.d. |
| 625 | AgGGcuAcuuuGAAuuAAudTsdT | 796 | pAUuAAUUcAAAGuAGCCCUdTsdT | 70 | 4 | 72 | 9 | n.d. | n.d. | n.d. | n.d. |
| 649 | AccAcuAAuGGGAGccAAudTsdT | 764 | pAUUGGCUCCcAUuAGUGGUdTsdT | 61 | 5 | 72 | 4 | n.d. | n.d. | n.d. | n.d. |
| 617 | uaGGGcuAcuuuGAAuuAAdTsdT | 946 | pUuAAUUcAAAGuAGCCCuAdTsdT | 73 | 3 | 72 | 6 | n.d. | n.d. | n.d. | n.d. |
| 606 | cgGAAGuuGGAAucAGGuudTsdT | 931 | pAACCUGAUUCcAACUUCCGdTsdT | 57 | 3 | 73 | 2 | n.d. | n.d. | n.d. | n.d. |
| 661 | GaGAGAuAAAuGuuGAucudTsdT | 926 | pAGAUcAAcAUUuAUCUCUCdTsdT | 60 | 5 | 73 | 6 | n.d. | n.d. | n.d. | n.d. |
| 584 | GgccAGcAAGAccGcGAGGdTsdT | 706 | pCCUCGCGGUCUUGCUGGCCdTsdT | 49 | 3 | 74 | 5 | n.d. | n.d. | n.d. | n.d. |
| 695 | uuAuuGuuAccuAAAGuuAdTsdT | 802 | puAACUUuAGGuAAcAAuAAdTsdT | 74 | 9 | 74 | 13 | n.d. | n.d. | n.d. | n.d. |
| 576 | uguGAGuuAAuucAuuuAudTsdT | 959 | pAuAAAUGAAUuAACUcAcAdTsdT | 67 | 4 | 74 | 5 | n.d. | n.d. | n.d. | n.d. |
| 617 | uaGGGcuAcuuuGAAuuAAdTsdT | 795 | pUUAAUUcAAAGuAGCCCuAdTsdT | 51 | 4 | 74 | 13 | n.d. | n.d. | n.d. | n.d. |
| 635 | uaGuGuccuGGGAuucucudTsdT | 916 | pAGAGAAUCCcAGGAcACuAdTsdT | 72 | 4 | 74 | 6 | n.d. | n.d. | n.d. | n.d. |
| 687 | uaucuGGcAGAuGuAuAAGdTsdT | 824 | pCUuAuAcAUCUGCcAGAuAdTsdT | 86 | 7 | 74 | 5 | n.d. | n.d. | n.d. | n.d. |
| 632 | GaccAuuuccuAAucAGuudTsdT | 811 | pAACUGAUuAGGAAAUGGUCdTsdT | 61 | 4 | 75 | 10 | n.d. | n.d. | n.d. | n.d. |
| 626 | uaAGuuAuuGuuAccuAAAdTsdT | 799 | pUUuAGGuAAcAAuAACUuAdTsdT | 84 | 4 | 75 | 9 | n.d. | n.d. | n.d. | n.d. |
| 667 | uuGuuAccuAAAGuuAAucdTsdT | 804 | pGAUuAACUUuAGGuAAcAAdTsdT | 76 | 6 | 76 | 9 | n.d. | n.d. | n.d. | n.d. |
| 630 | AaAGAcuAAcuucuuuGAGdTsdT | 886 | pCUcAAAGAAGUuAGUCUUUdTsdT | 49 | 4 | 76 | 3 | n.d. | n.d. | n.d. | n.d. |
| 621 | GacuGAuGccuGGccucAcdTsdT | 854 | pGUGAGGCcAGGcAUcAGUCdTsdT | 67 | 4 | 76 | 4 | n.d. | n.d. | n.d. | n.d. |
| 670 | uaccuAAAGuuAAuccAGAdTsdT | 807 | pUCUGGAUuAACUUuAGGuAdTsdT | 80 | 6 | 76 | 10 | n.d. | n.d. | n.d. | n.d. |
| 634 | AauGuGAGGAuuAAcuucudTsdT | 809 | pAGAAGUuAAUCCUcAcAUUdTsdT | 69 | 4 | 77 | 2 | n.d. | n.d. | n.d. | n.d. |

| SEQ ID NO | sense strand sequence (5´-3´) | SEQ ID NO | antisense strand sequence (5´-3´) | Activity testing with 50nM siRNA in HeLaS3 cells, transfection 1 | | Activity testing with 500pM siRNA in HeLaS3 cells | | Activity testing with 50nM siRNA in HeLaS3 cells, transfection 2 | | Activity testing with 30pM siRNA in HeLaS3 cells | |
|---|---|---|---|---|---|---|---|---|---|---|---|
| | | | | mRNA [%] | s.d. [%] | mRNA [%] | s.d. [%] | mRNA [%] | s.d. [%] | mRNA [%] | s.d. [%] |
| 612 | AaGAGGcuAccuAuGGuGAdTsdT | 850 | pUCACcAuAGGuAGCCUCUUdTsdT | 65 | 3 | 78 | 2 | n.d. | n.d. | n.d. | n.d. |
| 678 | GuuAccuAAAGuuAAuccAdTsdT | 928 | pUGGAUuAACUUuAGGuAACdTsdT | 73 | 6 | 79 | 5 | n.d. | n.d. | n.d. | n.d. |
| 648 | uaAuGuGAGGAuuAAcuucdTsdT | 935 | pGAAGUuAAUCCUcAcAUuAdTsdT | 75 | 5 | 80 | 2 | n.d. | n.d. | n.d. | n.d. |
| 689 | ucAGAccAuuuccuAAucAdTsdT | 936 | pUGAUuAGGAAAUGGUCUGAdTsdT | 75 | 7 | 81 | 6 | n.d. | n.d. | n.d. | n.d. |
| 641 | ccuAAAGuuAAuccAGAuudTsdT | 929 | pAAUCUGGAUuAACUUuAGGdTsdT | 72 | 4 | 81 | 4 | n.d. | n.d. | n.d. | n.d. |
| 555 | ucAGcAcuGGGAAucccuGdTsdT | 827 | pcAGGGAUUCCcAGUGCUGAdTsdT | 81 | 2 | 82 | 12 | n.d. | n.d. | n.d. | n.d. |
| 565 | cccuGuuAAGuGGuGAAAudTsdT | 789 | pAUUUcACcACUuAAcAGGGdTsdT | 76 | 2 | 83 | 4 | n.d. | n.d. | n.d. | n.d. |
| 554 | AaGcccGcuGuuucuAuGGdTsdT | 835 | pCCAuAGAAAcAGCGGGCUUdTsdT | 72 | 2 | 84 | 5 | n.d. | n.d. | n.d. | n.d. |
| 696 | uaGcuGAAuAAuGuGAGGAdTsdT | 808 | pUCCUcAcAUuAUUcAGCuAdTsdT | 84 | 9 | 84 | 11 | n.d. | n.d. | n.d. | n.d. |
| 483 | caAuAcAuuGAGuuuGuGGdTsdT | 953 | pCcAcAAACUcAAUGuAUUGdTsdT | 31 | 2 | 84 | 6 | n.d. | n.d. | n.d. | n.d. |
| 638 | GcGGAAGuuGGAAucAGGudTsdT | 930 | pACCUGAUUCcAACUUCCGCdTsdT | 72 | 4 | 84 | 7 | n.d. | n.d. | n.d. | n.d. |
| 514 | cauuAGcuGAAuAAuGuGAdTsdT | 932 | pUCAcAUuAUUcAGCuAAUGdTsdT | 72 | 2 | 85 | 6 | n.d. | n.d. | n.d. | n.d. |
| 702 | AcuAAAGuAAGuuAAAcuudTsdT | 773 | pAAGUUuAACUuACUUuAGUdTsdT | 87 | 12 | 86 | 6 | n.d. | n.d. | n.d. | n.d. |
| 595 | AccucAcAAccAGuccuGudTsdT | 956 | pAcAGGACUGGUUGUGAGGUdTsdT | 35 | 1 | 86 | 5 | n.d. | n.d. | n.d. | n.d. |
| 472 | GgcGAGuAucAGAGGAuGGdTsdT | 955 | pCcAUCCUCUGAuACUCGCCdTsdT | 23 | 1 | 87 | 5 | n.d. | n.d. | n.d. | n.d. |
| 690 | uuAccuAAAGuuAAuccAGdTsdT | 806 | pCUGGAUuAACUUuAGGuAAdTsdT | 89 | 7 | 88 | 12 | n.d. | n.d. | n.d. | n.d. |
| 592 | ugcuGuucGGAuAGAAcAGdTsdT | 731 | pCUGUUCuAUCCGAAcAGcAdTsdT | 76 | 3 | 89 | 3 | n.d. | n.d. | n.d. | n.d. |
| 693 | GcuAccuAuGGuGAAcGuGdTsdT | 723 | pcACGUUcACcAuAGGuAGCdTsdT | 77 | 8 | 89 | 7 | n.d. | n.d. | n.d. | n.d. |
| 665 | AcGAuGccuuGuGucAAGAdTsdT | 720 | pUCUUGAcAcAAGGcAUCGUdTsdT | 76 | 6 | 90 | 4 | n.d. | n.d. | n.d. | n.d. |
| 688 | AgGcuAccuAuGGuGAAcGdTsdT | 721 | pCGUUcACcAuAGGuAGCCUdTsdT | 90 | 7 | 90 | 8 | n.d. | n.d. | n.d. | n.d. |
| 685 | uauucuuAAuAGGGcuAcudTsdT | 793 | pAGuAGCCCuAUuAAGAAuAdTsdT | 93 | 7 | 90 | 17 | n.d. | n.d. | n.d. | n.d. |
| 701 | AgAAGcccGcuGuuucuAudTsdT | 948 | pAuAGAAAcAGCGGGCUUCUdTsdT | 79 | 3 | 90 | 4 | n.d. | n.d. | n.d. | n.d. |
| 646 | cuGuucGGAuAGAAcAGGAdTsdT | 732 | pUCCUGUUCuAUCCGAAcAGdTsdT | 67 | 5 | 91 | 9 | n.d. | n.d. | n.d. | n.d. |
| 640 | AuucuuAAuAGGGcuAcuudTsdT | 794 | pAAGuAGCCCuAUuAAGAAUdTsdT | 114 | 4 | 92 | 15 | n.d. | n.d. | n.d. | n.d. |

| SEQ ID NO | sense strand sequence (5´-3´) | SEQ ID NO | antisense strand sequence (5´-3´) | Activity testing with 50nM siRNA in HeLaS3 cells, transfection 1 | | Activity testing with 500pM siRNA in HeLaS3 cells | | Activity testing with 50nM siRNA in HeLaS3 cells, transfection 2 | | Activity testing with 30pM siRNA in HeLaS3 cells | |
|---|---|---|---|---|---|---|---|---|---|---|---|
| | | | | mRNA [%] | s.d. [%] | mRNA [%] | s.d. [%] | mRNA [%] | s.d. [%] | mRNA [%] | s.d. [%] |
| 548 | AguuAuuGuuAccuAAAGudTsdT | 800 | pACUUuAGGuAAcAAuAACUdTsdT | 67 | 2 | 92 | 10 | n.d. | n.d. | n.d. | n.d. |
| 610 | AuAAuGuGAGGAuuAAcuudTsdT | 934 | pAAGUuAAUCCUcAcAUuAUdTsdT | 90 | 3 | 93 | 6 | n.d. | n.d. | n.d. | n.d. |
| 637 | AcAAAuAuucuuAAuAGGGdTsdT | 792 | pCCCuAUuAAGAAuAUUUGUdTsdT | 79 | 4 | 94 | 18 | n.d. | n.d. | n.d. | n.d. |
| 514 | cauuAGcuGAAuAAuGuGAdTsdT | 960 | pUcAcAUuAUUcAGCuAAUGdTsdT | 85 | 4 | 94 | 2 | n.d. | n.d. | n.d. | n.d. |
| 614 | ugAGGccuuGccuGuGAAGdTsdT | 869 | pCUUcAcAGGcAAGGCCUcAdTsdT | 100 | 3 | 95 | 6 | n.d. | n.d. | n.d. | n.d. |
| 563 | AuGuucAAAcAccuGGuAcdTsdT | 952 | pGuACcAGGUGUUUGAAcAUdTsdT | 96 | 9 | 95 | 14 | n.d. | n.d. | n.d. | n.d. |
| 699 | ucccGcucGcGcccAucAcdTsdT | 812 | pGUGAUGGGCGCGAGCGGGAdTsdT | 94 | 10 | 95 | 8 | n.d. | n.d. | n.d. | n.d. |
| 604 | cccGcucGcGcccAucAcGdTsdT | 813 | pCGUGAUGGGCGCGAGCGGGdTsdT | 90 | 3 | 96 | 4 | n.d. | n.d. | n.d. | n.d. |
| 657 | GgAcuGAuGccuGGccucAdTsdT | 853 | pUGAGGCcAGGcAUcAGUCCdTsdT | 87 | 5 | 97 | 8 | n.d. | n.d. | n.d. | n.d. |
| 612 | AaGAGGcuAccuAuGGuGAdTsdT | 951 | pUcACcAuAGGuAGCCUCUUdTsdT | 79 | 9 | 97 | 7 | n.d. | n.d. | n.d. | n.d. |
| 522 | cauuGAAAcGAuGccuuGudTsdT | 950 | pAcAAGGcAUCGUUUcAAUGdTsdT | 93 | 11 | 100 | 6 | n.d. | n.d. | n.d. | n.d. |
| 700 | cuuGGcuuuAAAGuGAGGGdTsdT | 906 | pCCCUcACUUuAAAGCcAAGdTsdT | 90 | 10 | 100 | 7 | n.d. | n.d. | n.d. | n.d. |
| 704 | AauAAuuAucAAuGcuGuudTsdT | 725 | pAAcAGcAUUGAuAAUuAUUdTsdT | 134 | 14 | 101 | 2 | n.d. | n.d. | n.d. | n.d. |

Table 3

| SEQ ID NO pair | Activity testing for dose response in HeLaS3 cells, means of two transfections | | | | Activity testing for dose response in HeLaS3 cells, transfection 3 | | | | Activity testing for dose response in HeLaS3 cells, transfection 4 | | | |
|---|---|---|---|---|---|---|---|---|---|---|---|---|
| | mean IC50 [nM] | mean IC80 [nM] | mean IC20 [nM] | mean max. inh [%] | mean IC50 [nM] | mean IC80 [nM] | mean IC20 [nM] | mean max. inh [%] | mean IC50 [nM] | mean IC80 [nM] | mean IC20 [nM] | mean max. inh [%] |
| 469/742 | 0.004 | 0.059 | 0.001 | 91 | 0.013 | 0.124 | 0.002 | 95 | 0.014 | 0.158 | 0.002 | 91 |
| 525/885 | 0.004 | 0.074 | 0.001 | 90 | 0.01 | 0.10 | 0.00 | 92 | n.d. | n.d. | n.d. | n.d. |
| 477/839 | 0.006 | 0.066 | 0.001 | 91 | 0.016 | 0.143 | 0.003 | 93 | 0.013 | 0.198 | 0.002 | 90 |
| 552/891 | 0.006 | 0.107 | 0.001 | 88 | 0.02 | 0.14 | 0.00 | 90 | n.d. | n.d. | n.d. | n.d. |
| 475/884 | 0.006 | 0.073 | 0.001 | 91 | 0.02 | 0.16 | 0.00 | 93 | n.d. | n.d. | n.d. | n.d. |
| 501/842 | 0.007 | 0.091 | 0.001 | 90 | 0.02 | 0.18 | 0.00 | 91 | n.d. | n.d. | n.d. | n.d. |
| 473/863 | 0.009 | 0.330 | 0.002 | 84 | n.d. | n.d. | n.d. | n.d. | n.d. | n.d. | n.d. | n.d. |
| 507/841 | 0.010 | 0.081 | 0.002 | 97 | 0.03 | 0.21 | 0.01 | 97 | n.d. | n.d. | n.d. | n.d. |
| 494/866 | 0.011 | 0.810 | 0.003 | 84 | n.d. | n.d. | n.d. | n.d. | n.d. | n.d. | n.d. | n.d. |
| 544/718 | 0.014 | 0.145 | 0.003 | 92 | n.d. | n.d. | n.d. | n.d. | n.d. | n.d. | n.d. | n.d. |
| 497/711 | 0.015 | 0.116 | 0.003 | 89 | n.d. | n.d. | n.d. | n.d. | n.d. | n.d. | n.d. | n.d. |
| 560/838 | 0.021 | 0.265 | 0.004 | 86 | n.d. | n.d. | n.d. | n.d. | n.d. | n.d. | n.d. | n.d. |
| 522/845 | 0.029 | 0.295 | 0.006 | 90 | n.d. | n.d. | n.d. | n.d. | n.d. | n.d. | n.d. | n.d. |
| 471/892 | 0.029 | 0.361 | 0.005 | 88 | n.d. | n.d. | n.d. | n.d. | n.d. | n.d. | n.d. | n.d. |
| 488/735 | 0.030 | 0.262 | 0.006 | 89 | n.d. | n.d. | n.d. | n.d. | n.d. | n.d. | n.d. | n.d. |
| 507/949 | 0.204 | 2.588 | 0.053 | 82 | n.d. | n.d. | n.d. | n.d. | n.d. | n.d. | n.d. | n.d. |
| 526/967 | n.d. | n.d. | n.d. | n.d. | 0.015 | 0.120 | 0.0016 | 92 | n.d. | n.d. | n.d. | n.d. |
| 470/962 | n.d. | n.d. | n.d. | n.d. | 0.022 | 0.219 | 0.004 | 94 | 0.020 | 0.314 | 0.002 | 90 |
| 553/968 | n.d. | n.d. | n.d. | n.d. | 0.024 | 0.152 | 0.0041 | 89 | n.d. | n.d. | n.d. | n.d. |
| 477/963 | n.d. | n.d. | n.d. | n.d. | 0.024 | 0.189 | 0.005 | 93 | 0.016 | 0.251 | 0.002 | 90 |
| 501/965 | n.d. | n.d. | n.d. | n.d. | 0.036 | 0.281 | 0.0053 | 91 | n.d. | n.d. | n.d. | n.d. |
| 476/966 | n.d. | n.d. | n.d. | n.d. | 0.038 | 0.313 | 0.0062 | 93 | n.d. | n.d. | n.d. | n.d. |
| 507/964 | n.d. | n.d. | n.d. | n.d. | 0.045 | 0.310 | 0.0077 | 97 | n.d. | n.d. | n.d. | n.d. |
| 477/971 | n.d. | n.d. | n.d. | n.d. | 0.154 | 2.062 | 0.034 | 85 | n.d. | n.d. | n.d. | n.d. |
| 470/970 | n.d. | n.d. | n.d. | n.d. | 0.184 | 2.134 | 0.040 | 87 | n.d. | n.d. | n.d. | n.d. |

| SEQ ID NO pair | Activity testing for dose response in HeLaS3 cells, means of two transfections | | | | Activity testing for dose response in HeLaS3 cells, transfection 3 | | | | Activity testing for dose response in HeLaS3 cells, transfection 4 | | | |
|---|---|---|---|---|---|---|---|---|---|---|---|---|
| | mean IC50 [nM] | mean IC80 [nM] | mean IC20 [nM] | mean max. inh [%] | mean IC50 [nM] | mean IC80 [nM] | mean IC20 [nM] | mean max. inh [%] | mean IC50 [nM] | mean IC80 [nM] | mean IC20 [nM] | mean max. inh [%] |
| 470/978 | n.d. | n.d. | n.d. | n.d. | 0.479 | #N/A | 0.073 | 72 | n.d. | n.d. | n.d. | n.d. |
| 477/979 | n.d. | n.d. | n.d. | n.d. | 0.906 | #N/A | 0.142 | 78 | n.d. | n.d. | n.d. | n.d. |

Table 4

| | Stability Cyno Serum | | Stability Human Serum | | Stability Mouse Serum | | Human PBMC assay | |
|---|---|---|---|---|---|---|---|---|
| | sense | antisense | sense | antisense | sense | antisense | | |
| SEQ ID NO pair | t1/2 [hr ] | t1/2 [hr ] | t1/2 [hr ] | t1/2 [hr ] | t1/2 [hr ] | t1/2 [hr ] | IFN-a | TNF-a |
| 475/884 | 15.8 | 2.2 | >48 | 0.9 | 15.5 | 9.2 | 0 | 0 |
| 507/841 | 16.2 | 0.7 | >48 | 1.7 | 12.1 | 0.6 | 0 | 0 |
| 525/885 | 41.1 | 4.2 | >48 | 2.0 | 13.1 | 7.7 | 0 | 0 |
| 469/742 | 36.4 | 4.7 | >48 | 2.5 | 14.2 | 7.5 | 0 | 0 |
| 552/891 | 13.6 | 9.8 | >48 | 7.1 | 10.3 | 11.6 | 0 | 0 |
| 501/842 | 17.4 | 2.2 | >48 | 11.4 | 13.5 | 3.2 | 0 | 0 |
| 477/839 | 40.8 | 16.8 | >48 | >48 | 27.4 | 10.7 | 0 | 0 |
| 497/711 | >48 | 11.8 | >48 | >48 | >48 | 14.2 | 0 | 0 |

Table 5

| SEQ ID NO | Sense strand sequence (5'-3') | SEQ ID NO | Antisense strand sequence (5'-3') | SEQ ID NO | sense strand sequence (5'-3') | SEQ ID NO | antisense strand sequence (5'-3') |
|---|---|---|---|---|---|---|---|
| 1 | UUGUGGCAGACAGACUUAU | 234 | AUAAGUCUGUCUGCCACAA | 467 | uuGuGGcAGAcAGAcuuAudTsdT | 737 | pAUAAGUCUGUCUGCcAcAAdTsdT |
| 1 | UUGUGGCAGACAGACUUAU | 234 | AUAAGUCUGUCUGCCACAA | 467 | uuGuGGcAGAcAGAcuuAudTsdT | 940 | pAuAAGUCUGUCUGCcAcAAdTsdT |
| 2 | CCUGAUGUUCAAACACCUG | 235 | CAGGUGUUUGAACAUCAGG | 468 | ccuGAuGuucAAAcAccuGdTsdT | 860 | pcAGGUGUUUGAAcAUcAGGdTsdT |
| 3 | AGUCCAACAGAGAAUUCUU | 236 | AAGAAUUCUCUGUUGGACU | 469 | AguccAAcAGAGAAuucuudTsdT | 742 | pAAGAAUUCUCUGUUGGACUdTsdT |
| 3 | AGUCCAACAGAGAAUUCUU | 236 | AAGAAUUCUCUGUUGGACU | 470 | aguccAAcAGAGAAuucuudTsdT | 962 | AAGAAUUCUCUGUUGGACUdTsdT |
| 3 | AGUCCAACAGAGAAUUCUU | 236 | AAGAAUUCUCUGUUGGACU | 470 | aguccAAcAGAGAAuucuudTsdT | 970 | AAGAAUUCUCUGUuGGACUdTsdT |
| 3 | AGUCCAACAGAGAAUUCUU | 236 | AAGAAUUCUCUGUUGGACU | 470 | aguccAAcAGAGAAuucuudTsdT | 978 | AaGAAUUCUCUGUuGGACUdTsdT |
| 4 | UAGGCGAGUAUCAGAGGAU | 237 | AUCCUCUGAUACUCGCCUA | 471 | uaGGcGAGuAucAGAGGAudTsdT | 892 | pAUCCUCUGAuACUCGCCuAdTsdT |
| 5 | GGCGAGUAUCAGAGGAUGG | 238 | CCAUCCUCUGAUACUCGCC | 472 | GgcGAGuAucAGAGGAuGGdTsdT | 894 | pCCAUCCUCUGAuACUCGCCdTsdT |
| 5 | GGCGAGUAUCAGAGGAUGG | 238 | CCAUCCUCUGAUACUCGCC | 472 | GgcGAGuAucAGAGGAuGGdTsdT | 955 | pCcAUCCUCUGAuACUCGCCdTsdT |
| 6 | UGUUCAAACACCUGGUACA | 239 | UGUACCAGGUGUUUGAACA | 473 | uguucAAAcAccuGGuAcAdTsdT | 863 | pUGuACcAGGUGUUUGAAcAdTsdT |
| 7 | GGGUGACCCUUUAGUGAGC | 240 | GCUCACUAAAGGGUCACCC | 474 | GgGuGAcccuuuAGuGAGcdTsdT | 756 | pGCUcACuAAAGGGUcACCCdTsdT |
| 8 | GAAGGAAAGACUAACUUCU | 241 | AGAAGUUAGUCUUUCCUUC | 475 | GaAGGAAAGAcuAAcuucudTsdT | 884 | pAGAAGUuAGUCUUUCCUUCdTsdT |
| 8 | GAAGGAAAGACUAACUUCU | 241 | AGAAGUUAGUCUUUCCUUC | 476 | gaAGGAAAGAcuAAcuucudTsdT | 966 | AGAAGUuAGUCUUUCCUUCdTsdT |
| 8 | GAAGGAAAGACUAACUUCU | 241 | AGAAGUUAGUCUUUCCUUC | 476 | gaAGGAAAGAcuAAcuucudTsdT | 974 | AGAAGUuAGUCUUuCCUUCdTsdT |
| 8 | GAAGGAAAGACUAACUUCU | 241 | AGAAGUUAGUCUUUCCUUC | 476 | gaAGGAAAGAcuAAcuucudTsdT | 982 | AgAAGUuAGUCUUuCCUUCdTsdT |
| 9 | UUCUGAAAUGUAUAGUCUU | 242 | AAGACUAUACAUUUCAGAA | 477 | uucuGAAAuGuAuAGucuudTsdT | 839 | pAAGACuAuAcAUUUcAGAAdTsdT |
| 9 | UUCUGAAAUGUAUAGUCUU | 242 | AAGACUAUACAUUUCAGAA | 477 | uucuGAAAuGuAuAGucuudTsdT | 963 | AAGACuAuAcAUUUcAGAAdTsdT |
| 9 | UUCUGAAAUGUAUAGUCUU | 242 | AAGACUAUACAUUUCAGAA | 477 | uucuGAAAuGuAuAGucuudTsdT | 971 | AAGACuAuAcAUUucAGAAdTsdT |
| 9 | UUCUGAAAUGUAUAGUCUU | 242 | AAGACUAUACAUUUCAGAA | 477 | uucuGAAAuGuAuAGucuudTsdT | 979 | AaGACuAuAcAUUucAGAAdTsdT |
| 10 | CUGUGUAGCUACCUCACAA | 243 | UUGUGAGGUAGCUACACAG | 478 | cuGuGuAGcuAccucAcAAdTsdT | 744 | pUUGUGAGGuAGCuAcAcAGdTsdT |
| 11 | UGCACUCUAAUGAAGCAAU | 244 | AUUGCUUCAUUAGAGUGCA | 479 | ugcAcucuAAuGAAGcAAudTsdT | 871 | pAUUGCUUcAUuAGAGUGcAdTsdT |
| 12 | CCCAUUUGACUUUAUGGAG | 245 | CUCCAUAAAGUCAAAUGGG | 480 | cccAuuuGAcuuuAuGGAGdTsdT | 739 | pCUCcAuAAAGUcAAAUGGGdTsdT |
| 13 | AAAUGUAUAGUCUUCUUAU | 246 | AUAAGAAGACUAUACAUUU | 481 | AaAuGuAuAGucuucuuAudTsdT | 714 | pAUAAGAAGACuAuAcAUUUdTsdT |
| 13 | AAAUGUAUAGUCUUCUUAU | 246 | AUAAGAAGACUAUACAUUU | 481 | AaAuGuAuAGucuucuuAudTsdT | 939 | pAuAAGAAGACuAuAcAUUUdTsdT |
| 14 | UACAUUGAGUUUGUGGCAG | 247 | CUGCCACAAACUCAAUGUA | 482 | uacAuuGAGuuuGuGGcAGdTsdT | 875 | pCUGCcAcAAACUcAAUGuAdTsdT |
| 15 | CAAUACAUUGAGUUUGUGG | 248 | CCACAAACUCAAUGUAUUG | 483 | caAuAcAuuGAGuuuGuGGdTsdT | 874 | pCCAcAAACUcAAUGuAUUGdTsdT |

| SEQ ID NO | Sense strand sequence (5'-3') | SEQ ID NO | Antisense strand sequence (5'-3') | SEQ ID NO | sense strand sequence (5'-3') | SEQ ID NO | antisense strand sequence (5'-3') |
|---|---|---|---|---|---|---|---|
| 15 | CAAUACAUUGAGUUUGUGG | 248 | CCACAAACUCAAUGUAUUG | 483 | caAuAcAuuGAGuuuGuGGdTsdT | 953 | pCcAcAAACUcAAUGuAUUGdTsdT |
| 16 | GAACAGGAGUUCCUCACUG | 249 | CAGUGAGGAACUCCUGUUC | 484 | GaAcAGGAGuuccucAcuGdTsdT | 868 | pcAGUGAGGAACUCCUGUUCdTsdT |
| 17 | AUCCCAUGUUCUGGCUUUC | 250 | GAAAGCCAGAACAUGGGAU | 485 | AucccAuGuucuGGcuuucdTsdT | 710 | pGAAAGCcAGAAcAUGGGAUdTsdT |
| 18 | GUAGGUUGUGUGAGUUAAU | 251 | AUUAACUCACACAACCUAC | 486 | GuAGGuuGuGuGAGuuAAudTsdT | 921 | pAUuAACUcAcAcAACCuACdTsdT |
| 19 | AUAGUCUUCUUAUUGACAC | 252 | GUGUCAAUAAGAAGACUAU | 487 | AuAGucuucuuAuuGAcAcdTsdT | 715 | pGUGUcAAuAAGAAGACuAUdTsdT |
| 20 | AUUGCACUCUAAUGAAGCA | 253 | UGCUUCAUUAGAGUGCAAU | 488 | AuuGcAcucuAAuGAAGcAdTsdT | 735 | pUGCUUcAUuAGAGUGcAAUdTsdT |
| 21 | UUAUCAAUGCUGUUCGGAU | 254 | AUCCGAACAGCAUUGAUAA | 489 | uuAucAAuGcuGuucGGAudTsdT | 728 | pAUCCGAAcAGcAUUGAuAAdTsdT |
| 22 | AGAAACGAGGACUGAUGCC | 255 | GGCAUCAGUCCUCGUUUCU | 490 | AgAAAcGAGGAcuGAuGccdTsdT | 851 | pGGcAUcAGUCCUCGUUUCUdTsdT |
| 23 | CAUUGAGUUUGUGGCAGAC | 256 | GUCUGCCACAAACUCAAUG | 491 | cauuGAGuuuGuGGcAGAcdTsdT | 876 | pGUCUGCcAcAAACUcAAUGdTsdT |
| 24 | ACAUUCAGCACUGGGAAUC | 257 | GAUUCCCAGUGCUGAAUGU | 492 | AcAuucAGcAcuGGGAAucdTsdT | 708 | pGAUUCCcAGUGCUGAAUGUdTsdT |
| 25 | UGAUGUUCAAACACCUGGU | 258 | ACCAGGUGUUUGAACAUCA | 493 | ugAuGuucAAAcAccuGGudTsdT | 861 | pACcAGGUGUUUGAAcAUcAdTsdT |
| 26 | GGAUAGAACAGGAGUUCCU | 259 | AGGAACUCCUGUUCUAUCC | 494 | GgAuAGAAcAGGAGuuccudTsdT | 866 | pAGGAACUCCUGUUCuAUCCdTsdT |
| 27 | AAUAUUUCACUGGAAGGAA | 260 | UUCCUUCCAGUGAAAUAUU | 495 | AauAuuucAcuGGAAGGAAdTsdT | 741 | pUUCCUUCcAGUGAAAuAUUdTsdT |
| 28 | AAUAAACAUUGUUUGUACU | 261 | AGUACAAACAAUGUUUAUU | 496 | AauAAAcAuuGuuuGuAcudTsdT | 911 | pAGuAcAAAcAAUGUUuAUUdTsdT |
| 29 | UCCCAUGUUCUGGCUUUCU | 262 | AGAAAGCCAGAACAUGGGA | 497 | ucccAuGuucuGGcuuucudTsdT | 711 | pAGAAAGCcAGAAcAUGGGAdTsdT |
| 29 | UCCCAUGUUCUGGCUUUCU | 262 | AGAAAGCCAGAACAUGGGA | 497 | ucccAuGuucuGGcuuucudTsdT | 961 | AGAAAGCcAGAAcAUGGGAdTsdT |
| 29 | UCCCAUGUUCUGGCUUUCU | 262 | AGAAAGCCAGAACAUGGGA | 497 | ucccAuGuucuGGcuuucudTsdT | 969 | AGAAAGCcAGAAcaUGGGAdTsdT |
| 29 | UCCCAUGUUCUGGCUUUCU | 262 | AGAAAGCCAGAACAUGGGA | 497 | ucccAuGuucuGGcuuucudTsdT | 977 | AgAAAGCcAGAAcaUGGGAdTsdT |
| 30 | UUCGGAUAGAACAGGAGUU | 263 | AACUCCUGUUCUAUCCGAA | 498 | uucGGAuAGAAcAGGAGuudTsdT | 734 | pAACUCCUGUUCuAUCCGAAdTsdT |
| 31 | AAGUAGGUUGUGUGAGUUA | 264 | UAACUCACACAACCUACUU | 499 | AaGuAGGuuGuGuGAGuuAdTsdT | 790 | puAACUcAcAcAACCuACUUdTsdT |
| 32 | UUAUAGUGCUGGUAGUAUC | 265 | GAUACUACCAGCACUAUAA | 500 | uuAuAGuGcuGGuAGuAucdTsdT | 746 | pGAuACuACcAGcACuAuAAdTsdT |
| 33 | CUUCUUAUUGACACUUACA | 266 | UGUAAGUGUCAAUAAGAAG | 501 | cuucuuAuuGAcAcuuAcAdTsdT | 842 | pUGuAAGUGUcAAuAAGAAGdTsdT |
| 33 | CUUCUUAUUGACACUUACA | 266 | UGUAAGUGUCAAUAAGAAG | 501 | cuucuuAuuGAcAcuuAcAdTsdT | 965 | UGuAAGUGUcAAuAAGAAGdTsdT |
| 33 | CUUCUUAUUGACACUUACA | 266 | UGUAAGUGUCAAUAAGAAG | 501 | cuucuuAuuGAcAcuuAcAdTsdT | 973 | UGuAAGUGUcAAuaAGAAGdTsdT |
| 33 | CUUCUUAUUGACACUUACA | 266 | UGUAAGUGUCAAUAAGAAG | 501 | cuucuuAuuGAcAcuuAcAdTsdT | 981 | UguAAGUGUcAAuaAGAAGdTsdT |
| 34 | UACAGAAGCCCGCUGUUUC | 267 | GAAACAGCGGGCUUCUGUA | 502 | uacAGAAGcccGcuGuuucdTsdT | 832 | pGAAAcAGCGGGCUUCUGuAdTsdT |
| 35 | GUGACCCUUUAGUGAGCUU | 268 | AAGCUCACUAAAGGGUCAC | 503 | GuGAcccuuuAGuGAGcuudTsdT | 907 | pAAGCUcACuAAAGGGUcACdTsdT |
| 36 | AUAGAACAGGAGUUCCUCA | 269 | UGAGGAACUCCUGUUCUAU | 504 | AuAGAAcAGGAGuuccucAdTsdT | 867 | pUGAGGAACUCCUGUUCuAUdTsdT |

| SEQ ID NO | Sense strand sequence (5'-3') | SEQ ID NO | Antisense strand sequence (5'-3') | SEQ ID NO | sense strand sequence (5'-3') | SEQ ID NO | antisense strand sequence (5'-3') |
|---|---|---|---|---|---|---|---|
| 37 | CUGGCACUUUACAAACAAA | 270 | UUUGUUUGUAAAGUGCCAG | 505 | cuGGcAcuuuAcAAAcAAAdTsdT | 910 | pUUUGUUUGuAAAGUGCcAGdTsdT |
| 38 | UCUAAUGAAGCAAUACAUU | 271 | AAUGUAUUGCUUCAUUAGA | 506 | ucuAAuGAAGcAAuAcAuudTsdT | 872 | pAAUGuAUUGCUUcAUuAGAdTsdT |
| 39 | UCUUCUUAUUGACACUUAC | 272 | GUAAGUGUCAAUAAGAAGA | 507 | ucuucuuAuuGAcAcuuAcdTsdT | 841 | pGUAAGUGUcAAuAAGAAGAdTsdT |
| 39 | UCUUCUUAUUGACACUUAC | 272 | GUAAGUGUCAAUAAGAAGA | 507 | ucuucuuAuuGAcAcuuAcdTsdT | 949 | pGuAAGUGUcAAuAAGAAGAdTsdT |
| 39 | UCUUCUUAUUGACACUUAC | 272 | GUAAGUGUCAAUAAGAAGA | 507 | ucuucuuAuuGAcAcuuAcdTsdT | 964 | GUAAGUGUcAAuAAGAAGAdTsdT |
| 39 | UCUUCUUAUUGACACUUAC | 272 | GUAAGUGUCAAUAAGAAGA | 507 | ucuucuuAuuGAcAcuuAcdTsdT | 972 | GUAAGUGUcAAuAaGAAGAdTsdT |
| 39 | UCUUCUUAUUGACACUUAC | 272 | GUAAGUGUCAAUAAGAAGA | 508 | ucuucuUAuuGAcAcuuAcdTsdT | 972 | GUAAGUGUcAAuAaGAAGAdTsdT |
| 39 | UCUUCUUAUUGACACUUAC | 272 | GUAAGUGUCAAUAAGAAGA | 507 | ucuucuuAuuGAcAcuuAcdTsdT | 980 | GuAAGUGUcAAuAaGAAGAdTsdT |
| 39 | UCUUCUUAUUGACACUUAC | 272 | GUAAGUGUCAAUAAGAAGA | 508 | ucuucuUAuuGAcAcuuAcdTsdT | 980 | GuAAGUGUcAAuAaGAAGAdTsdT |
| 39 | UCUUCUUAUUGACACUUAC | 272 | GUAAGUGUCAAUAAGAAGA | 508 | ucuucuUAuuGAcAcuuAcdTsdT | 985 | GuAAGUGUcAAuAAGAAGAdTsdT |
| 40 | UGUUCGGAUAGAACAGGAG | 273 | CUCCUGUUCUAUCCGAACA | 509 | uguucGGAuAGAAcAGGAGdTsdT | 733 | pCUCCUGUUCuAUCCGAAcAdTsdT |
| 41 | AGUACCAUGAUAUCUGGCA | 274 | UGCCAGAUAUCAUGGUACU | 510 | AguAccAuGAuAucuGGcAdTsdT | 820 | pUGCcAGAuAUcAUGGuACUdTsdT |
| 42 | CAGAGAUGAGGGUUUACAC | 275 | GUGUAAACCCUCAUCUCUG | 511 | caGAGAuGAGGGuuuAcAcdTsdT | 856 | pGUGuAAACCCUcAUCUCUGdTsdT |
| 43 | GAAACGAGGACUGAUGCCU | 276 | AGGCAUCAGUCCUCGUUUC | 512 | GaAAcGAGGAcuGAuGccudTsdT | 852 | pAGGcAUcAGUCCUCGUUUCdTsdT |
| 44 | AAGAGAGUAGGCGAGUAUC | 277 | GAUACUCGCCUACUCUCUU | 513 | AaGAGAGuAGGcGAGuAucdTsdT | 888 | pGAuACUCGCCuACUCUCUUdTsdT |
| 45 | CAUUAGCUGAAUAAUGUGA | 278 | UCACAUUAUUCAGCUAAUG | 514 | cauuAGcuGAAuAAuGuGAdTsdT | 932 | pUCAcAUuAUUcAGCuAAUGdTsdT |
| 45 | CAUUAGCUGAAUAAUGUGA | 278 | UCACAUUAUUCAGCUAAUG | 514 | cauuAGcuGAAuAAuGuGAdTsdT | 960 | pUcAcAUuAUUcAGCuAAUGdTsdT |
| 46 | AGUAGAGAACCCAUUUGAC | 279 | GUCAAAUGGGUUCUCUACU | 515 | AguAGAGAAcccAuuuGAcdTsdT | 738 | pGUcAAAUGGGUUCUCuACUdTsdT |
| 47 | AGGCGAGUAUCAGAGGAUG | 280 | CAUCCUCUGAUACUCGCCU | 516 | AgGcGAGuAucAGAGGAuGdTsdT | 893 | pcAUCCUCUGAuACUCGCCUdTsdT |
| 48 | UAGACUAAGCAUGUAAUUU | 281 | AAAUUACAUGCUUAGUCUA | 517 | uaGAcuAAGcAuGuAAuuudTsdT | 784 | pAAAUuAcAUGCUuAGUCuAdTsdT |
| 49 | AACAUUGUUUGUACUCACA | 282 | UGUGAGUACAAACAAUGUU | 518 | AacAuuGuuuGuAcucAcAdTsdT | 913 | pUGUGAGuAcAAAcAAUGUUdTsdT |
| 50 | GAUGGGAGUGAUGUCAAGU | 283 | ACUUGACAUCACUCCCAUC | 519 | GauGGGAGuGAuGucAAGudTsdT | 896 | pACUUGAcAUcACUCCcAUCdTsdT |
| 51 | CAGACCAUUUCCUAAUCAG | 284 | CUGAUUAGGAAAUGGUCUG | 520 | caGAccAuuuccuAAucAGdTsdT | 937 | pCUGAUuAGGAAAUGGUCUGdTsdT |
| 52 | GAUUACAGAAGCCCGCUGU | 285 | ACAGCGGGCUUCUGUAAUC | 521 | GauuAcAGAAGcccGcuGudTsdT | 712 | pACAGCGGGCUUCUGuAAUCdTsdT |
| 52 | GAUUACAGAAGCCCGCUGU | 285 | ACAGCGGGCUUCUGUAAUC | 521 | GauuAcAGAAGcccGcuGudTsdT | 938 | pAcAGCGGGCUUCUGuAAUCdTsdT |
| 53 | CAUUGAAACGAUGCCUUGU | 286 | ACAAGGCAUCGUUUCAAUG | 522 | cauuGAAAcGAuGccuuGudTsdT | 845 | pACAAGGcAUCGUUUcAAUGdTsdT |
| 53 | CAUUGAAACGAUGCCUUGU | 286 | ACAAGGCAUCGUUUCAAUG | 522 | cauuGAAAcGAuGccuuGudTsdT | 950 | pAcAAGGcAUCGUUUcAAUGdTsdT |
| 54 | ACUUAUGCUGGAACUGGGU | 287 | ACCCAGUUCCAGCAUAAGU | 523 | AcuuAuGcuGGAAcuGGGudTsdT | 880 | pACCcAGUUCcAGcAuAAGUdTsdT |

| SEQ ID NO | Sense strand sequence (5'-3') | SEQ ID NO | Antisense strand sequence (5'-3') | SEQ ID NO | sense strand sequence (5'-3') | SEQ ID NO | antisense strand sequence (5'-3') |
|---|---|---|---|---|---|---|---|
| 55 | GUCGACAAGGAGAACACGC | 288 | GCGUGUUCUCCUUGUCGAC | 524 | GucGAcAAGGAGAAcAcGcdTsdT | 705 | pGCGUGUUCUCCUUGUCGACdTsdT |
| 56 | AGGAAAGACUAACUUCUUU | 289 | AAAGAAGUUAGUCUUUCCU | 525 | AgGAAAGAcuAAcuucuuudTsdT | 885 | pAAAGAAGUuAGUCUUUCCUdTsdT |
| 56 | AGGAAAGACUAACUUCUUU | 289 | AAAGAAGUUAGUCUUUCCU | 526 | agGAAAGAcuAAcuucuuudTsdT | 967 | AAAGAAGUuAGUCUUUCCUdTsdT |
| 56 | AGGAAAGACUAACUUCUUU | 289 | AAAGAAGUUAGUCUUUCCU | 526 | agGAAAGAcuAAcuucuuudTsdT | 975 | AAAGAAGUuAGUCuUUCCUdTsdT |
| 56 | AGGAAAGACUAACUUCUUU | 289 | AAAGAAGUUAGUCUUUCCU | 526 | agGAAAGAcuAAcuucuuudTsdT | 983 | AaAGAAGUuAGUCuUUCCUdTsdT |
| 57 | CAAGACCGCGAGGAGGAUC | 290 | GAUCCUCCUCGCGGUCUUG | 527 | caAGAccGcGAGGAGGAucdTsdT | 816 | pGAUCCUCCUCGCGGUCUUGdTsdT |
| 58 | GACAAUGGCAGUCUUGGCU | 291 | AGCCAAGACUGCCAUUGUC | 528 | GacAAuGGcAGucuuGGcudTsdT | 755 | pAGCcAAGACUGCcAUUGUCdTsdT |
| 59 | AUGCCUUGUGUCAAGAAGA | 292 | UCUUCUUGACACAAGGCAU | 529 | AuGccuuGuGucAAGAAGAdTsdT | 846 | pUCUUCUUGAcAcAAGGcAUdTsdT |
| 60 | GCCUCACUGCUUCAACGCA | 293 | UGCGUUGAAGCAGUGAGGC | 530 | GccucAcuGcuucAAcGcAdTsdT | 909 | pUGCGUUGAAGcAGUGAGGCdTsdT |
| 61 | UACCUCACAACCAGUCCUG | 294 | CAGGACUGGUUGUGAGGUA | 531 | uaccucAcAAccAGuccuGdTsdT | 745 | pcAGGACUGGUUGUGAGGuAdTsdT |
| 62 | GAGAAGAGAGUAGGCGAGU | 295 | ACUCGCCUACUCUCUUCUC | 532 | GaGAAGAGAGuAGGcGAGudTsdT | 887 | pACUCGCCuACUCUCUUCUCdTsdT |
| 63 | AGACUUAUGCUGGAACUGG | 296 | CCAGUUCCAGCAUAAGUCU | 533 | AgAcuuAuGcuGGAAcuGGdTsdT | 879 | pCCAGUUCcAGcAuAAGUCUdTsdT |
| 63 | AGACUUAUGCUGGAACUGG | 296 | CCAGUUCCAGCAUAAGUCU | 533 | AgAcuuAuGcuGGAAcuGGdTsdT | 954 | pCcAGUUCcAGcAuAAGUCUdTsdT |
| 64 | UUACAGAAGCCCGCUGUUU | 297 | AAACAGCGGGCUUCUGUAA | 534 | uuAcAGAAGcccGcuGuuudTsdT | 831 | pAAAcAGCGGGCUUCUGuAAdTsdT |
| 65 | UUAUGCUGGAACUGGGUUU | 298 | AAACCCAGUUCCAGCAUAA | 535 | uuAuGcuGGAAcuGGGuuudTsdT | 881 | pAAACCcAGUUCcAGcAuAAdTsdT |
| 66 | AUAAACAUUGUUUGUACUC | 299 | GAGUACAAACAAUGUUUAU | 536 | AuAAAcAuuGuuuGuAcucdTsdT | 912 | pGAGuAcAAAcAAUGUUuAUdTsdT |
| 67 | UCAAUGCCAUUGAAACGAU | 300 | AUCGUUUCAAUGGCAUUGA | 537 | ucAAuGccAuuGAAAcGAudTsdT | 717 | pAUCGUUUcAAUGGcAUUGAdTsdT |
| 68 | AUAGUGCUGGUAGUAUCAC | 301 | GUGAUACUACCAGCACUAU | 538 | AuAGuGcuGGuAGuAucAcdTsdT | 748 | pGUGAuACuACcAGcACuAUdTsdT |
| 69 | CAGCCUCACUGCUUCAACG | 302 | CGUUGAAGCAGUGAGGCUG | 539 | caGccucAcuGcuucAAcGdTsdT | 908 | pCGUUGAAGcAGUGAGGCUGdTsdT |
| 70 | UCUUGGCUUUAAAGUGAGG | 303 | CCUCACUUUAAAGCCAAGA | 540 | ucuuGGcuuuAAAGuGAGGdTsdT | 905 | pCCUcACUUuAAAGCcAAGAdTsdT |
| 71 | GGCUGUGACUUACCAUAGC | 304 | GCUAUGGUAAGUCACAGCC | 541 | GgcuGuGAcuuAccAuAGcdTsdT | 751 | pGCuAUGGuAAGUcAcAGCCdTsdT |
| 72 | GGCUACCUAUGGUGAACGU | 305 | ACGUUCACCAUAGGUAGCC | 542 | GgcuAccuAuGGuGAAcGudTsdT | 722 | pACGUUcACcAuAGGuAGCCdTsdT |
| 73 | CGCGAGGAGGAUCUUCCAG | 306 | CUGGAAGAUCCUCCUCGCG | 543 | cgcGAGGAGGAucuuccAGdTsdT | 819 | pCUGGAAGAUCCUCCUCGCGdTsdT |
| 74 | GCCAUUGAAACGAUGCCUU | 307 | AAGGCAUCGUUUCAAUGGC | 544 | GccAuuGAAAcGAuGccuudTsdT | 718 | pAAGGcAUCGUUUcAAUGGCdTsdT |
| 75 | AGCCUCACUGCUUCAACGC | 308 | GCGUUGAAGCAGUGAGGCU | 545 | AgccucAcuGcuucAAcGcdTsdT | 758 | pGCGUUGAAGcAGUGAGGCUdTsdT |
| 76 | GGCAGACAGACUUAUGCUG | 309 | CAGCAUAAGUCUGUCUGCC | 546 | GgcAGAcAGAcuuAuGcuGdTsdT | 878 | pcAGcAuAAGUCUGUCUGCCdTsdT |
| 77 | GUGACUAAAGUAAGUUAAA | 310 | UUUAACUUACUUUAGUCAC | 547 | GuGAcuAAAGuAAGuuAAAdTsdT | 770 | pUUuAACUuACUUuAGUcACdTsdT |
| 78 | AGUUAUUGUUACCUAAAGU | 311 | ACUUUAGGUAACAAUAACU | 548 | AguuAuuGuuAccuAAAGudTsdT | 800 | pACUUuAGGuAAcAAuAACUdTsdT |

| SEQ ID NO | Sense strand sequence (5'-3') | SEQ ID NO | Antisense strand sequence (5'-3') | SEQ ID NO | sense strand sequence (5'-3') | SEQ ID NO | antisense strand sequence (5'-3') |
|---|---|---|---|---|---|---|---|
| 79 | GCCUUUAUGUUUGGGAGAA | 312 | UUCUCCCAAACAUAAAGGC | 549 | GccuuuAuGuuuGGGAGAAdTsdT | 924 | pUUCUCCcAAAcAuAAAGGCdTsdT |
| 80 | UUCAGAGUAGAGAACCCAU | 313 | AUGGGUUCUCUACUCUGAA | 550 | uucAGAGuAGAGAAcccAudTsdT | 882 | pAUGGGUUCUCuACUCUGAAdTsdT |
| 81 | AAACGAGGACUGAUGCCUG | 314 | CAGGCAUCAGUCCUCGUUU | 551 | AaAcGAGGAcuGAuGccuGdTsdT | 724 | pcAGGcAUcAGUCCUCGUUUdTsdT |
| 82 | GUAGGCGAGUAUCAGAGGA | 315 | UCCUCUGAUACUCGCCUAC | 552 | GuAGGcGAGuAucAGAGGAdTsdT | 891 | pUCCUCUGAuACUCGCCuACdTsdT |
| 82 | GUAGGCGAGUAUCAGAGGA | 315 | UCCUCUGAUACUCGCCUAC | 553 | guAGGcGAGuAucAGAGGAdTsdT | 968 | UCCUCUGAuACUCGCCuACdTsdT |
| 82 | GUAGGCGAGUAUCAGAGGA | 315 | UCCUCUGAUACUCGCCUAC | 553 | guAGGcGAGuAucAGAGGAdTsdT | 976 | UCCUCUGAuACUCgCCuACdTsdT |
| 82 | GUAGGCGAGUAUCAGAGGA | 315 | UCCUCUGAUACUCGCCUAC | 553 | guAGGcGAGuAucAGAGGAdTsdT | 984 | UcCUCUGAuACUCgCCuACdTsdT |
| 83 | AAGCCCGCUGUUUCUAUGG | 316 | CCAUAGAAACAGCGGGCUU | 554 | AaGcccGcuGuuucuAuGGdTsdT | 835 | pCCAuAGAAAcAGCGGGCUUdTsdT |
| 84 | UCAGCACUGGGAAUCCCUG | 317 | CAGGGAUUCCCAGUGCUGA | 555 | ucAGcAcuGGGAAucccuGdTsdT | 827 | pcAGGGAUUCCcAGUGCUGAdTsdT |
| 85 | GAAUAAUGUGAGGAUUAAC | 318 | GUUAAUCCUCACAUUAUUC | 556 | GaAuAAuGuGAGGAuuAAcdTsdT | 933 | pGUuAAUCCUcAcAUuAUUCdTsdT |
| 86 | UGUGGCAGACAGACUUAUG | 319 | CAUAAGUCUGUCUGCCACA | 557 | uguGGcAGAcAGAcuuAuGdTsdT | 877 | pcAuAAGUCUGUCUGCcAcAdTsdT |
| 87 | AGAGAUAAAUGUUGAUCUU | 320 | AAGAUCAACAUUUAUCUCU | 558 | AgAGAuAAAuGuuGAucuudTsdT | 798 | pAAGAUcAAcAUUuAUCUCUdTsdT |
| 88 | UACCAUGAUAUCUGGCAGA | 321 | UCUGCCAGAUAUCAUGGUA | 559 | uaccAuGAuAucuGGcAGAdTsdT | 821 | pUCUGCcAGAuAUcAUGGuAdTsdT |
| 89 | CUUCCAAAUUGCCAUGGAA | 322 | UUCCAUGGCAAUUUGGAAG | 560 | cuuccAAAuuGccAuGGAAdTsdT | 838 | pUUCcAUGGcAAUUUGGAAGdTsdT |
| 90 | ACCGCGAGGAGGAUCUUCC | 323 | GGAAGAUCCUCCUCGCGGU | 561 | AccGcGAGGAGGAucuuccdTsdT | 818 | pGGAAGAUCCUCCUCGCGGUdTsdT |
| 91 | GAAAUGUAUAGUCUUCUUA | 324 | UAAGAAGACUAUACAUUUC | 562 | GaAAuGuAuAGucuucuuAdTsdT | 713 | puAAGAAGACuAuAcAUUUCdTsdT |
| 92 | AUGUUCAAACACCUGGUAC | 325 | GUACCAGGUGUUUGAACAU | 563 | AuGuucAAAcAccuGGuAcdTsdT | 862 | pGUACcAGGUGUUUGAAcAUdTsdT |
| 92 | AUGUUCAAACACCUGGUAC | 325 | GUACCAGGUGUUUGAACAU | 563 | AuGuucAAAcAccuGGuAcdTsdT | 952 | pGuACcAGGUGUUUGAAcAUdTsdT |
| 93 | AGGGAAUUUCUCUUCAAUG | 326 | CAUUGAAGAGAAAUUCCCU | 564 | AgGGAAuuucucuucAAuGdTsdT | 716 | pcAUUGAAGAGAAAUUCCCUdTsdT |
| 94 | CCCUGUUAAGUGGUGAAAU | 327 | AUUUCACCACUUAACAGGG | 565 | cccuGuuAAGuGGuGAAAudTsdT | 789 | pAUUUcACcACUuAAcAGGGdTsdT |
| 95 | GAUGAGGGUUUACACUGUG | 328 | CACAGUGUAAACCCUCAUC | 566 | GauGAGGGuuuAcAcuGuGdTsdT | 857 | pcAcAGUGuAAACCCUcAUCdTsdT |
| 96 | UGUGUGAGUUAAUUCAUUU | 329 | AAAUGAAUUAACUCACACA | 567 | uguGuGAGuuAAuucAuuudTsdT | 922 | pAAAUGAAUuAACUcAcAcAdTsdT |
| 97 | UUGCCUGAUGUUCAAACAC | 330 | GUGUUUGAACAUCAGGCAA | 568 | uuGccuGAuGuucAAAcAcdTsdT | 858 | pGUGUUUGAAcAUcAGGcAAdTsdT |
| 98 | AAACUUGUGUAGACUAAGC | 331 | GCUUAGUCUACACAAGUUU | 569 | AaAcuuGuGuAGAcuAAGcdTsdT | 779 | pGCUuAGUCuAcAcAAGUUUdTsdT |
| 99 | UAUAUCCCAUGUUCUGGCU | 332 | AGCCAGAACAUGGGAUAUA | 570 | uauAucccAuGuucuGGcudTsdT | 828 | pAGCcAGAAcAUGGGAuAuAdTsdT |
| 100 | UUGUGUAGACUAAGCAUGU | 333 | ACAUGCUUAGUCUACACAA | 571 | uuGuGuAGAcuAAGcAuGudTsdT | 781 | pACAUGCUuAGUCuAcAcAAdTsdT |
| 100 | UUGUGUAGACUAAGCAUGU | 333 | ACAUGCUUAGUCUACACAA | 571 | uuGuGuAGAcuAAGcAuGudTsdT | 945 | pAcAUGCUuAGUCuAcAcAAdTsdT |
| 101 | AUGCUGUUCGGAUAGAACA | 334 | UGUUCUAUCCGAACAGCAU | 572 | AuGcuGuucGGAuAGAAcAdTsdT | 730 | pUGUUCuAUCCGAAcAGcAUdTsdT |

| SEQ ID NO | Sense strand sequence (5'-3') | SEQ ID NO | Antisense strand sequence (5'-3') | SEQ ID NO | sense strand sequence (5'-3') | SEQ ID NO | antisense strand sequence (5'-3') |
|---|---|---|---|---|---|---|---|
| 102 | AAUUAUCAAUGCUGUUCGG | 335 | CCGAACAGCAUUGAUAAUU | 573 | AauuAucAAuGcuGuucGGdTsdT | 727 | pCCGAAcAGcAUUGAuAAUUdTsdT |
| 103 | GCCUGAUGUUCAAACACCU | 336 | AGGUGUUUGAACAUCAGGC | 574 | GccuGAuGuucAAAcAccudTsdT | 859 | pAGGUGUUUGAAcAUcAGGCdTsdT |
| 104 | CAUAGCAGUGACAAUGGCA | 337 | UGCCAUUGUCACUGCUAUG | 575 | cauAGcAGuGAcAAuGGcAdTsdT | 902 | pUGCcAUUGUcACUGCuAUGdTsdT |
| 105 | UGUGAGUUAAUUCAUUUAU | 338 | AUAAAUGAAUUAACUCACA | 576 | uguGAGuuAAuucAuuuAudTsdT | 923 | pAUAAAUGAAUuAACUcAcAdTsdT |
| 105 | UGUGAGUUAAUUCAUUUAU | 338 | AUAAAUGAAUUAACUCACA | 576 | uguGAGuuAAuucAuuuAudTsdT | 959 | pAuAAAUGAAUuAACUcAcAdTsdT |
| 106 | AGUGCUGGUAGUAUCACCU | 339 | AGGUGAUACUACCAGCACU | 577 | AguGcuGGuAGuAucAccudTsdT | 749 | pAGGUGAuACuACcAGcACUdTsdT |
| 107 | UAUCAAUGCUGUUCGGAUA | 340 | UAUCCGAACAGCAUUGAUA | 578 | uaucAAuGcuGuucGGAuAdTsdT | 865 | puAUCCGAAcAGcAUUGAuAdTsdT |
| 108 | GACUAAAGUAAGUUAAACU | 341 | AGUUUAACUUACUUUAGUC | 579 | GacuAAAGuAAGuuAAAcudTsdT | 772 | pAGUUuAACUuACUUuAGUCdTsdT |
| 109 | AAUGCUGUUCGGAUAGAAC | 342 | GUUCUAUCCGAACAGCAUU | 580 | AauGcuGuucGGAuAGAAcdTsdT | 729 | pGUUCuAUCCGAAcAGcAUUdTsdT |
| 110 | AGAAUAUUUCACUGGAAGG | 343 | CCUUCCAGUGAAAUAUUCU | 581 | AgAAuAuuucAcuGGAAGGdTsdT | 740 | pCCUUCcAGUGAAAuAUUCUdTsdT |
| 111 | AUCUGGCAGAUGUAUAAGA | 344 | UCUUAUACAUCUGCCAGAU | 582 | AucuGGcAGAuGuAuAAGAdTsdT | 825 | pUCUuAuAcAUCUGCcAGAUdTsdT |
| 112 | UAUAGUGCUGGUAGUAUCA | 345 | UGAUACUACCAGCACUAUA | 583 | uauAGuGcuGGuAGuAucAdTsdT | 747 | pUGAuACuACcAGcACuAuAdTsdT |
| 113 | GGCCAGCAAGACCGCGAGG | 346 | CCUCGCGGUCUUGCUGGCC | 584 | GgccAGcAAGAccGcGAGGdTsdT | 706 | pCCUCGCGGUCUUGCUGGCCdTsdT |
| 114 | CCAUGAUAUCUGGCAGAUG | 347 | CAUCUGCCAGAUAUCAUGG | 585 | ccAuGAuAucuGGcAGAuGdTsdT | 823 | pcAUCUGCcAGAuAUcAUGGdTsdT |
| 115 | UUAAACUUGUGUAGACUAA | 348 | UUAGUCUACACAAGUUUAA | 586 | uuAAAcuuGuGuAGAcuAAdTsdT | 777 | pUUAGUCuAcAcAAGUUuAAdTsdT |
| 115 | UUAAACUUGUGUAGACUAA | 348 | UUAGUCUACACAAGUUUAA | 586 | uuAAAcuuGuGuAGAcuAAdTsdT | 944 | pUuAGUCuAcAcAAGUUuAAdTsdT |
| 116 | UUCAAUGCCAUUGAAACGA | 349 | UCGUUUCAAUGGCAUUGAA | 587 | uucAAuGccAuuGAAAcGAdTsdT | 844 | pUCGUUUcAAUGGcAUUGAAdTsdT |
| 117 | AGAAAGCUGAGACAUUGCA | 350 | UGCAAUGUCUCAGCUUUCU | 588 | AgAAAGcuGAGAcAuuGcAdTsdT | 925 | pUGcAAUGUCUcAGCUUUCUdTsdT |
| 118 | CUAUGGCUUCCAAAUUGCC | 351 | GGCAAUUUGGAAGCCAUAG | 589 | cuAuGGcuuccAAAuuGccdTsdT | 837 | pGGcAAUUUGGAAGCcAuAGdTsdT |
| 119 | AAGUGACUAAAGUAAGUUA | 352 | UAACUUACUUUAGUCACUU | 590 | AaGuGAcuAAAGuAAGuuAdTsdT | 769 | puAACUuACUUuAGUcACUUdTsdT |
| 120 | UGACUAAAGUAAGUUAAAC | 353 | GUUUAACUUACUUUAGUCA | 591 | ugAcuAAAGuAAGuuAAAcdTsdT | 771 | pGUUuAACUuACUUuAGUcAdTsdT |
| 121 | UGCUGUUCGGAUAGAACAG | 354 | CUGUUCUAUCCGAACAGCA | 592 | ugcuGuucGGAuAGAAcAGdTsdT | 731 | pCUGUUCuAUCCGAAcAGcAdTsdT |
| 122 | GCGAGUAUCAGAGGAUGGG | 355 | CCCAUCCUCUGAUACUCGC | 593 | GcGAGuAucAGAGGAuGGGdTsdT | 895 | pCCcAUCCUCUGAuACUCGCdTsdT |
| 123 | GGGCCUUGCGCUGGAUUGG | 356 | CCAAUCCAGCGCAAGGCCC | 594 | GgGccuuGcGcuGGAuuGGdTsdT | 848 | pCCAAUCcAGCGcAAGGCCCdTsdT |
| 124 | ACCUCACAACCAGUCCUGU | 357 | ACAGGACUGGUUGUGAGGU | 595 | AccucAcAAccAGuccuGudTsdT | 899 | pACAGGACUGGUUGUGAGGUdTsdT |
| 124 | ACCUCACAACCAGUCCUGU | 357 | ACAGGACUGGUUGUGAGGU | 595 | AccucAcAAccAGuccuGudTsdT | 956 | pAcAGGACUGGUUGUGAGGUdTsdT |
| 125 | ACUAAGUGACUAAAGUAAG | 358 | CUUACUUUAGUCACUUAGU | 596 | AcuAAGuGAcuAAAGuAAGdTsdT | 768 | pCUuACUUuAGUcACUuAGUdTsdT |
| 126 | AUUACAGAAGCCCGCUGUU | 359 | AACAGCGGGCUUCUGUAAU | 597 | AuuAcAGAAGcccGcuGuudTsdT | 830 | pAAcAGCGGGCUUCUGuAAUdTsdT |

| SEQ ID NO | Sense strand sequence (5'-3') | SEQ ID NO | Antisense strand sequence (5'-3') | SEQ ID NO | sense strand sequence (5'-3') | SEQ ID NO | antisense strand sequence (5'-3') |
|---|---|---|---|---|---|---|---|
| 127 | GAGUAGGCGAGUAUCAGAG | 360 | CUCUGAUACUCGCCUACUC | 598 | GaGuAGGcGAGuAucAGAGdTsdT | 890 | pCUCUGAuACUCGCCuACUCdTsdT |
| 128 | CAGUGACAAUGGCAGUCUU | 361 | AAGACUGCCAUUGUCACUG | 599 | caGuGAcAAuGGcAGucuudTsdT | 904 | pAAGACUGCcAUUGUcACUGdTsdT |
| 129 | GGCCUUGCGCUGGAUUGGG | 362 | CCCAAUCCAGCGCAAGGCC | 600 | GgccuuGcGcuGGAuuGGGdTsdT | 849 | pCCcAAUCcAGCGcAAGGCCdTsdT |
| 130 | UUCUUAUUGACACUUACAU | 363 | AUGUAAGUGUCAAUAAGAA | 601 | uucuuAuuGAcAcuuAcAudTsdT | 843 | pAUGuAAGUGUcAAuAAGAAdTsdT |
| 131 | UUCACUAAGUGACUAAAGU | 364 | ACUUUAGUCACUUAGUGAA | 602 | uucAcuAAGuGAcuAAAGudTsdT | 765 | pACUUuAGUcACUuAGUGAAdTsdT |
| 132 | GUGUGAGUUAAUUCAUUUA | 365 | UAAAUGAAUUAACUCACAC | 603 | GuGuGAGuuAAuucAuuuAdTsdT | 791 | puAAAUGAAUuAACUcAcACdTsdT |
| 133 | CCCGCUCGCGCCCAUCACG | 366 | CGUGAUGGGCGCGAGCGGG | 604 | cccGcucGcGcccAucAcGdTsdT | 813 | pCGUGAUGGGCGCGAGCGGGdTsdT |
| 134 | GUAAGUUAAACUUGUGUAG | 367 | CUACACAAGUUUAACUUAC | 605 | GuAAGuuAAAcuuGuGuAGdTsdT | 775 | pCUAcAcAAGUUuAACUuACdTsdT |
| 134 | GUAAGUUAAACUUGUGUAG | 367 | CUACACAAGUUUAACUUAC | 605 | GuAAGuuAAAcuuGuGuAGdTsdT | 943 | pCuAcAcAAGUUuAACUuACdTsdT |
| 135 | CGGAAGUUGGAAUCAGGUU | 368 | AACCUGAUUCCAACUUCCG | 606 | cgGAAGuuGGAAucAGGuudTsdT | 931 | pAACCUGAUUCcAACUUCCGdTsdT |
| 136 | AUGUGAGGAUUAACUUCUG | 369 | CAGAAGUUAAUCCUCACAU | 607 | AuGuGAGGAuuAAcuucuGdTsdT | 810 | pcAGAAGUuAAUCCUcAcAUdTsdT |
| 137 | UUAAGUGGUGAAAUCAACU | 370 | AGUUGAUUUCACCACUUAA | 608 | uuAAGuGGuGAAAucAAcudTsdT | 919 | pAGUUGAUUUcACcACUuAAdTsdT |
| 138 | UGUAGACUAAGCAUGUAAU | 371 | AUUACAUGCUUAGUCUACA | 609 | uguAGAcuAAGcAuGuAAudTsdT | 783 | pAUuAcAUGCUuAGUCuAcAdTsdT |
| 139 | AUAAUGUGAGGAUUAACUU | 372 | AAGUUAAUCCUCACAUUAU | 610 | AuAAuGuGAGGAuuAAcuudTsdT | 934 | pAAGUuAAUCCUcAcAUuAUdTsdT |
| 140 | GGCUGGCUGUGACUUACCA | 373 | UGGUAAGUCACAGCCAGCC | 611 | GgcuGGcuGuGAcuuAccAdTsdT | 901 | pUGGuAAGUcAcAGCcAGCCdTsdT |
| 141 | AAGAGGCUACCUAUGGUGA | 374 | UCACCAUAGGUAGCCUCUU | 612 | AaGAGGcuAccuAuGGuGAdTsdT | 850 | pUCACcAuAGGuAGCCUCUUdTsdT |
| 141 | AAGAGGCUACCUAUGGUGA | 374 | UCACCAUAGGUAGCCUCUU | 612 | AaGAGGcuAccuAuGGuGAdTsdT | 951 | pUcACcAuAGGuAGCCUCUUdTsdT |
| 142 | CAGAUUACAGAAGCCCGCU | 375 | AGCGGGCUUCUGUAAUCUG | 613 | caGAuuAcAGAAGcccGcudTsdT | 829 | pAGCGGGCUUCUGuAAUCUGdTsdT |
| 143 | UGAGGCCUUGCCUGUGAAG | 376 | CUUCACAGGCAAGGCCUCA | 614 | ugAGGccuuGccuGuGAAGdTsdT | 869 | pCUUcAcAGGcAAGGCCUcAdTsdT |
| 144 | AUAAUUAUCAAUGCUGUUC | 377 | GAACAGCAUUGAUAAUUAU | 615 | AuAAuuAucAAuGcuGuucdTsdT | 726 | pGAAcAGcAUUGAuAAUuAUdTsdT |
| 145 | GUGACUUACCAUAGCAGUG | 466 | CACUGCUAUGGUAAGUCAC | 616 | GuGAcuuAccAuAGcAGuGdTsdT | 753 | pcACUGCuAUGGuAAGUcACdTsdT |
| 146 | UAGGGCUACUUUGAAUUAA | 378 | UUAAUUCAAAGUAGCCCUA | 617 | uaGGGcuAcuuuGAAuuAAdTsdT | 795 | pUUAAUUcAAAGuAGCCCuAdTsdT |
| 146 | UAGGGCUACUUUGAAUUAA | 378 | UUAAUUCAAAGUAGCCCUA | 617 | uaGGGcuAcuuuGAAuuAAdTsdT | 946 | pUuAAUUcAAAGuAGCCCuAdTsdT |
| 147 | UGGCAGAUGUAUAAGAAGG | 379 | CCUUCUUAUACAUCUGCCA | 618 | ugGcAGAuGuAuAAGAAGGdTsdT | 826 | pCCUUCUuAuAcAUCUGCcAdTsdT |
| 148 | AUAGCUUGAUUUAUUUGGU | 380 | ACCAAAUAAAUCAAGCUAU | 619 | AuAGcuuGAuuuAuuuGGudTsdT | 759 | pACcAAAuAAAUcAAGCuAUdTsdT |
| 149 | CAGCAAGACCGCGAGGAGG | 381 | CCUCCUCGCGGUCUUGCUG | 620 | caGcAAGAccGcGAGGAGGdTsdT | 707 | pCCUCCUCGCGGUCUUGCUGdTsdT |
| 150 | GACUGAUGCCUGGCCUCAC | 382 | GUGAGGCCAGGCAUCAGUC | 621 | GacuGAuGccuGGccucAcdTsdT | 854 | pGUGAGGCcAGGcAUcAGUCdTsdT |
| 151 | UUACCUUGGAUGCUGACUU | 383 | AAGUCAGCAUCCAAGGUAA | 622 | uuAccuuGGAuGcuGAcuudTsdT | 897 | pAAGUcAGcAUCcAAGGuAAdTsdT |

| SEQ ID NO | Sense strand sequence (5'-3') | SEQ ID NO | Antisense strand sequence (5'-3') | SEQ ID NO | sense strand sequence (5'-3') | SEQ ID NO | antisense strand sequence (5'-3') |
|---|---|---|---|---|---|---|---|
| 152 | AUUCAGCACUGGGAAUCCC | 384 | GGGAUUCCCAGUGCUGAAU | 623 | AuucAGcAcuGGGAAucccdTsdT | 709 | pGGGAUUCCcAGUGCUGAAUdTsdT |
| 153 | AGCAAGACCGCGAGGAGGA | 385 | UCCUCCUCGCGGUCUUGCU | 624 | AgcAAGAccGcGAGGAGGAdTsdT | 814 | pUCCUCCUCGCGGUCUUGCUdTsdT |
| 154 | AGGGCUACUUUGAAUUAAU | 386 | AUUAAUUCAAAGUAGCCCU | 625 | AgGGcuAcuuuGAAuuAAudTsdT | 796 | pAUuAAUUcAAAGuAGCCCUdTsdT |
| 155 | UAAGUUAUUGUUACCUAAA | 387 | UUUAGGUAACAAUAACUUA | 626 | uaAGuuAuuGuuAccuAAAdTsdT | 799 | pUUuAGGuAAcAAuAACUuAdTsdT |
| 156 | UUUAUAGUGCUGGUAGUAU | 388 | AUACUACCAGCACUAUAAA | 627 | uuuAuAGuGcuGGuAGuAudTsdT | 900 | pAUACuACcAGcACuAuAAAdTsdT |
| 156 | UUUAUAGUGCUGGUAGUAU | 388 | AUACUACCAGCACUAUAAA | 627 | uuuAuAGuGcuGGuAGuAudTsdT | 957 | pAuACuACcAGcACuAuAAAdTsdT |
| 157 | GCAAGACCGCGAGGAGGAU | 389 | AUCCUCCUCGCGGUCUUGC | 628 | GcAAGAccGcGAGGAGGAudTsdT | 815 | pAUCCUCCUCGCGGUCUUGCdTsdT |
| 158 | UCUAUGGCUUCCAAAUUGC | 390 | GCAAUUUGGAAGCCAUAGA | 629 | ucuAuGGcuuccAAAuuGcdTsdT | 836 | pGCAAUUUGGAAGCcAuAGAdTsdT |
| 159 | AAAGACUAACUUCUUUGAG | 391 | CUCAAAGAAGUUAGUCUUU | 630 | AaAGAcuAAcuucuuuGAGdTsdT | 886 | pCUcAAAGAAGUuAGUCUUUdTsdT |
| 160 | ACCAUGAUAUCUGGCAGAU | 392 | AUCUGCCAGAUAUCAUGGU | 631 | AccAuGAuAucuGGcAGAudTsdT | 822 | pAUCUGCcAGAuAUcAUGGUdTsdT |
| 161 | GACCAUUUCCUAAUCAGUU | 393 | AACUGAUUAGGAAAUGGUC | 632 | GaccAuuuccuAAucAGuudTsdT | 811 | pAACUGAUuAGGAAAUGGUCdTsdT |
| 162 | UUACCAUAGCAGUGACAAU | 394 | AUUGUCACUGCUAUGGUAA | 633 | uuAccAuAGcAGuGAcAAudTsdT | 754 | pAUUGUcACUGCuAUGGuAAdTsdT |
| 163 | AAUGUGAGGAUUAACUUCU | 395 | AGAAGUUAAUCCUCACAUU | 634 | AauGuGAGGAuuAAcuucudTsdT | 809 | pAGAAGUuAAUCCUcAcAUUdTsdT |
| 164 | UAGUGUCCUGGGAUUCUCU | 396 | AGAGAAUCCCAGGACACUA | 635 | uaGuGuccuGGGAuucucudTsdT | 916 | pAGAGAAUCCcAGGAcACuAdTsdT |
| 165 | UGUUAAGUGGUGAAAUCAA | 397 | UUGAUUUCACCACUUAACA | 636 | uguuAAGuGGuGAAAucAAdTsdT | 918 | pUUGAUUUcACcACUuAAcAdTsdT |
| 166 | ACAAAUAUUCUUAAUAGGG | 398 | CCCUAUUAAGAAUAUUUGU | 637 | AcAAAuAuucuuAAuAGGGdTsdT | 792 | pCCCuAUuAAGAAuAUUUGUdTsdT |
| 167 | GCGGAAGUUGGAAUCAGGU | 399 | ACCUGAUUCCAACUUCCGC | 638 | GcGGAAGuuGGAAucAGGudTsdT | 930 | pACCUGAUUCcAACUUCCGCdTsdT |
| 168 | AACUUGUGUAGACUAAGCA | 400 | UGCUUAGUCUACACAAGUU | 639 | AacuuGuGuAGAcuAAGcAdTsdT | 780 | pUGCUuAGUCuAcAcAAGUUdTsdT |
| 169 | AUUCUUAAUAGGGCUACUU | 401 | AAGUAGCCCUAUUAAGAAU | 640 | AuucuuAAuAGGGcuAcuudTsdT | 794 | pAAGuAGCCCuAUuAAGAAUdTsdT |
| 170 | CCUAAAGUUAAUCCAGAUU | 402 | AAUCUGGAUUAACUUUAGG | 641 | ccuAAAGuuAAuccAGAuudTsdT | 929 | pAAUCUGGAUuAACUUuAGGdTsdT |
| 171 | UAUUGUUACCUAAAGUUAA | 403 | UUAACUUUAGGUAACAAUA | 642 | uauuGuuAccuAAAGuuAAdTsdT | 803 | pUUAACUUuAGGuAAcAAuAdTsdT |
| 171 | UAUUGUUACCUAAAGUUAA | 403 | UUAACUUUAGGUAACAAUA | 642 | uauuGuuAccuAAAGuuAAdTsdT | 947 | pUuAACUUuAGGuAAcAAuAdTsdT |
| 172 | GUGCUGGUAGUAUCACCUU | 404 | AAGGUGAUACUACCAGCAC | 643 | GuGcuGGuAGuAucAccuudTsdT | 750 | pAAGGUGAuACuACcAGcACdTsdT |
| 173 | CUGUGACUUACCAUAGCAG | 405 | CUGCUAUGGUAAGUCACAG | 644 | cuGuGAcuuAccAuAGcAGdTsdT | 752 | pCUGCuAUGGuAAGUcAcAGdTsdT |
| 174 | GAGCUUCUUAAGUUAAAUC | 406 | GAUUUAACUUAAGAAGCUC | 645 | GaGcuucuuAAGuuAAAucdTsdT | 917 | pGAUUuAACUuAAGAAGCUCdTsdT |
| 175 | CUGUUCGGAUAGAACAGGA | 407 | UCCUGUUCUAUCCGAACAG | 646 | cuGuucGGAuAGAAcAGGAdTsdT | 732 | pUCCUGUUCuAUCCGAAcAGdTsdT |
| 176 | GUUAUUGUUACCUAAAGUU | 408 | AACUUUAGGUAACAAUAAC | 647 | GuuAuuGuuAccuAAAGuudTsdT | 801 | pAACUUuAGGuAAcAAuAACdTsdT |
| 177 | UAAUGUGAGGAUUAACUUC | 409 | GAAGUUAAUCCUCACAUUA | 648 | uaAuGuGAGGAuuAAcuucdTsdT | 935 | pGAAGUuAAUCCUcAcAUuAdTsdT |

| SEQ ID NO | Sense strand sequence (5'-3') | SEQ ID NO | Antisense strand sequence (5'-3') | SEQ ID NO | sense strand sequence (5'-3') | SEQ ID NO | antisense strand sequence (5'-3') |
|---|---|---|---|---|---|---|---|
| 178 | ACCACUAAUGGGAGCCAAU | 410 | AUUGGCUCCCAUUAGUGGU | 649 | AccAcuAAuGGGAGccAAudTsdT | 764 | pAUUGGCUCCcAUuAGUGGUdTsdT |
| 179 | UGUGUAGACUAAGCAUGUA | 411 | UACAUGCUUAGUCUACACA | 650 | uguGuAGAcuAAGcAuGuAdTsdT | 782 | puAcAUGCUuAGUCuAcAcAdTsdT |
| 180 | UGGGCCUUGCGCUGGAUUG | 412 | CAAUCCAGCGCAAGGCCCA | 651 | ugGGccuuGcGcuGGAuuGdTsdT | 847 | pcAAUCcAGCGcAAGGCCcAdTsdT |
| 181 | AGGAGCUUCUUAAGUUAAA | 413 | UUUAACUUAAGAAGCUCCU | 652 | AgGAGcuucuuAAGuuAAAdTsdT | 788 | pUUuAACUuAAGAAGCUCCUdTsdT |
| 182 | GGUGACCCUUUAGUGAGCU | 414 | AGCUCACUAAAGGGUCACC | 653 | GguGAcccuuuAGuGAGcudTsdT | 757 | pAGCUcACuAAAGGGUcACCdTsdT |
| 183 | AGAGUAGGCGAGUAUCAGA | 415 | UCUGAUACUCGCCUACUCU | 654 | AgAGuAGGcGAGuAucAGAdTsdT | 889 | pUCUGAuACUCGCCuACUCUdTsdT |
| 184 | GCAGUGACAAUGGCAGUCU | 416 | AGACUGCCAUUGUCACUGC | 655 | GcAGuGAcAAuGGcAGucudTsdT | 903 | pAGACUGCcAUUGUcACUGCdTsdT |
| 185 | AAACGAUGCCUUGUGUCAA | 417 | UUGACACAAGGCAUCGUUU | 656 | AaAcGAuGccuuGuGucAAdTsdT | 719 | pUUGAcAcAAGGcAUCGUUUdTsdT |
| 186 | GGACUGAUGCCUGGCCUCA | 418 | UGAGGCCAGGCAUCAGUCC | 657 | GgAcuGAuGccuGGccucAdTsdT | 853 | pUGAGGCcAGGcAUcAGUCCdTsdT |
| 187 | UGAGAGAUAAAUGUUGAUC | 419 | GAUCAACAUUUAUCUCUCA | 658 | ugAGAGAuAAAuGuuGAucdTsdT | 797 | pGAUcAAcAUUuAUCUCUcAdTsdT |
| 188 | UGGUUUCUACACCAAAUAC | 420 | GUAUUUGGUGUAGAAACCA | 659 | ugGuuucuAcAccAAAuAcdTsdT | 760 | pGUAUUUGGUGuAGAAACcAdTsdT |
| 188 | UGGUUUCUACACCAAAUAC | 420 | GUAUUUGGUGUAGAAACCA | 659 | ugGuuucuAcAccAAAuAcdTsdT | 941 | pGuAUUUGGUGuAGAAACcAdTsdT |
| 189 | UCUCUGUAAUAUGAUACAU | 421 | AUGUAUCAUAUUACAGAGA | 660 | ucucuGuAAuAuGAuAcAudTsdT | 927 | pAUGuAUcAuAUuAcAGAGAdTsdT |
| 190 | GAGAGAUAAAUGUUGAUCU | 422 | AGAUCAACAUUUAUCUCUC | 661 | GaGAGAuAAAuGuuGAucudTsdT | 926 | pAGAUcAAcAUUuAUCUCUCdTsdT |
| 191 | ACUCUAAUGAAGCAAUACA | 423 | UGUAUUGCUUCAUUAGAGU | 662 | AcucuAAuGAAGcAAuAcAdTsdT | 736 | pUGuAUUGCUUcAUuAGAGUdTsdT |
| 192 | UGAAGUGUUACCAACUAGC | 424 | GCUAGUUGGUAACACUUCA | 663 | ugAAGuGuuAccAAcuAGcdTsdT | 743 | pGCuAGUUGGuAAcACUUcAdTsdT |
| 193 | AAUGAAGCAAUACAUUGAG | 425 | CUCAAUGUAUUGCUUCAUU | 664 | AauGAAGcAAuAcAuuGAGdTsdT | 873 | pCUcAAUGuAUUGCUUcAUUdTsdT |
| 194 | ACGAUGCCUUGUGUCAAGA | 426 | UCUUGACACAAGGCAUCGU | 665 | AcGAuGccuuGuGucAAGAdTsdT | 720 | pUCUUGAcAcAAGGcAUCGUdTsdT |
| 195 | AGACCGCGAGGAGGAUCUU | 427 | AAGAUCCUCCUCGCGGUCU | 666 | AgAccGcGAGGAGGAucuudTsdT | 817 | pAAGAUCCUCCUCGCGGUCUdTsdT |
| 196 | UUGUUACCUAAAGUUAAUC | 428 | GAUUAACUUUAGGUAACAA | 667 | uuGuuAccuAAAGuuAAucdTsdT | 804 | pGAUuAACUUuAGGuAAcAAdTsdT |
| 197 | CAGAAGCCCGCUGUUUCUA | 429 | UAGAAACAGCGGGCUUCUG | 668 | caGAAGcccGcuGuuucuAdTsdT | 833 | puAGAAAcAGCGGGCUUCUGdTsdT |
| 198 | UUUGACUUUAUGGAGAAUA | 430 | UAUUCUCCAUAAAGUCAAA | 669 | uuuGAcuuuAuGGAGAAuAdTsdT | 883 | puAUUCUCcAuAAAGUcAAAdTsdT |
| 199 | UACCUAAAGUUAAUCCAGA | 431 | UCUGGAUUAACUUUAGGUA | 670 | uaccuAAAGuuAAuccAGAdTsdT | 807 | pUCUGGAUuAACUUuAGGuAdTsdT |
| 200 | UUCAAACACCUGGUACACA | 432 | UGUGUACCAGGUGUUUGAA | 671 | uucAAAcAccuGGuAcAcAdTsdT | 864 | pUGUGuACcAGGUGUUUGAAdTsdT |
| 201 | UUGCACUCUAAUGAAGCAA | 433 | UUGCUUCAUUAGAGUGCAA | 672 | uuGcAcucuAAuGAAGcAAdTsdT | 870 | pUUGCUUcAUuAGAGUGcAAdTsdT |
| 202 | UGUUACCUAAAGUUAAUCC | 434 | GGAUUAACUUUAGGUAACA | 673 | uguuAccuAAAGuuAAuccdTsdT | 805 | pGGAUuAACUUuAGGuAAcAdTsdT |
| 203 | CACUAAGUGACUAAAGUAA | 435 | UUACUUUAGUCACUUAGUG | 674 | cacuAAGuGAcuAAAGuAAdTsdT | 767 | pUUACUUuAGUcACUuAGUGdTsdT |
| 203 | CACUAAGUGACUAAAGUAA | 435 | UUACUUUAGUCACUUAGUG | 674 | cacuAAGuGAcuAAAGuAAdTsdT | 942 | pUuACUUuAGUcACUuAGUGdTsdT |

| SEQ ID NO | Sense strand sequence (5'-3') | SEQ ID NO | Antisense strand sequence (5'-3') | SEQ ID NO | sense strand sequence (5'-3') | SEQ ID NO | antisense strand sequence (5'-3') |
|---|---|---|---|---|---|---|---|
| 204 | UGCCAGAUAGAAGACAGGU | 436 | ACCUGUCUUCUAUCUGGCA | 675 | ugccAGAuAGAAGAcAGGudTsdT | 786 | pACCUGUCUUCuAUCUGGcAdTsdT |
| 205 | AAUGUAUAGUCUUCUUAUU | 437 | AAUAAGAAGACUAUACAUU | 676 | AauGuAuAGucuucuuAuudTsdT | 840 | pAAuAAGAAGACuAuAcAUUdTsdT |
| 206 | GACCACUAAUGGGAGCCAA | 438 | UUGGCUCCCAUUAGUGGUC | 677 | GaccAcuAAuGGGAGccAAdTsdT | 914 | pUUGGCUCCcAUuAGUGGUCdTsdT |
| 207 | GUUACCUAAAGUUAAUCCA | 439 | UGGAUUAACUUUAGGUAAC | 678 | GuuAccuAAAGuuAAuccAdTsdT | 928 | pUGGAUuAACUUuAGGuAACdTsdT |
| 208 | UGAUGCCUGGCCUCACAUU | 440 | AAUGUGAGGCCAGGCAUCA | 679 | ugAuGccuGGccucAcAuudTsdT | 855 | pAAUGUGAGGCcAGGcAUcAdTsdT |
| 209 | CCAACUUUAAAGUCAGUCC | 441 | GGACUGACUUUAAAGUUGG | 680 | ccAAcuuuAAAGucAGuccdTsdT | 915 | pGGACUGACUUuAAAGUUGGdTsdT |
| 210 | UAAACUUGUGUAGACUAAG | 442 | CUUAGUCUACACAAGUUUA | 681 | uaAAcuuGuGuAGAcuAAGdTsdT | 778 | pCUuAGUCuAcAcAAGUUuAdTsdT |
| 211 | AGUAGGUUGUGUGAGUUAA | 443 | UUAACUCACACAACCUACU | 682 | AguAGGuuGuGuGAGuuAAdTsdT | 920 | pUUAACUcAcAcAACCuACUdTsdT |
| 211 | AGUAGGUUGUGUGAGUUAA | 443 | UUAACUCACACAACCUACU | 682 | AguAGGuuGuGuGAGuuAAdTsdT | 958 | pUuAACUcAcAcAACCuACUdTsdT |
| 212 | GUUAAACUUGUGUAGACUA | 444 | UAGUCUACACAAGUUUAAC | 683 | GuuAAAcuuGuGuAGAcuAdTsdT | 776 | puAGUCuAcAcAAGUUuAACdTsdT |
| 213 | CUGACCACUAAUGGGAGCC | 445 | GGCUCCCAUUAGUGGUCAG | 684 | cuGAccAcuAAuGGGAGccdTsdT | 762 | pGGCUCCcAUuAGUGGUcAGdTsdT |
| 214 | UAUUCUUAAUAGGGCUACU | 446 | AGUAGCCCUAUUAAGAAUA | 685 | uauucuuAAuAGGGcuAcudTsdT | 793 | pAGuAGCCCuAUuAAGAAuAdTsdT |
| 215 | GUAGUGUCCUGGGAUUCUC | 447 | GAGAAUCCCAGGACACUAC | 686 | GuAGuGuccuGGGAuucucdTsdT | 787 | pGAGAAUCCcAGGAcACuACdTsdT |
| 216 | UAUCUGGCAGAUGUAUAAG | 448 | CUUAUACAUCUGCCAGAUA | 687 | uaucuGGcAGAuGuAuAAGdTsdT | 824 | pCUuAuAcAUCUGCcAGAuAdTsdT |
| 217 | AGGCUACCUAUGGUGAACG | 449 | CGUUCACCAUAGGUAGCCU | 688 | AgGcuAccuAuGGuGAAcGdTsdT | 721 | pCGUUcACcAuAGGuAGCCUdTsdT |
| 218 | UCAGACCAUUUCCUAAUCA | 450 | UGAUUAGGAAAUGGUCUGA | 689 | ucAGAccAuuuccuAAucAdTsdT | 936 | pUGAUuAGGAAAUGGUCUGAdTsdT |
| 219 | UUACCUAAAGUUAAUCCAG | 451 | CUGGAUUAACUUUAGGUAA | 690 | uuAccuAAAGuuAAuccAGdTsdT | 806 | pCUGGAUuAACUUuAGGuAAdTsdT |
| 220 | GGUUUCUACACCAAAUACA | 452 | UGUAUUUGGUGUAGAAACC | 691 | GguuucuAcAccAAAuAcAdTsdT | 761 | pUGuAUUUGGUGuAGAAACCdTsdT |
| 221 | GUUGGUGCCAGAUAGAAGA | 453 | UCUUCUAUCUGGCACCAAC | 692 | GuuGGuGccAGAuAGAAGAdTsdT | 785 | pUCUUCuAUCUGGcACcAACdTsdT |
| 222 | GCUACCUAUGGUGAACGUG | 454 | CACGUUCACCAUAGGUAGC | 693 | GcuAccuAuGGuGAAcGuGdTsdT | 723 | pcACGUUcACcAuAGGuAGCdTsdT |
| 223 | UCACUAAGUGACUAAAGUA | 455 | UACUUUAGUCACUUAGUGA | 694 | ucAcuAAGuGAcuAAAGuAdTsdT | 766 | puACUUuAGUcACUuAGUGAdTsdT |
| 224 | UUAUUGUUACCUAAAGUUA | 456 | UAACUUUAGGUAACAAUAA | 695 | uuAuuGuuAccuAAAGuuAdTsdT | 802 | puAACUUuAGGuAAcAAuAAdTsdT |
| 225 | UAGCUGAAUAAUGUGAGGA | 457 | UCCUCACAUUAUUCAGCUA | 696 | uaGcuGAAuAAuGuGAGGAdTsdT | 808 | pUCCUcAcAUuAUUcAGCuAdTsdT |
| 226 | UGACCACUAAUGGGAGCCA | 458 | UGGCUCCCAUUAGUGGUCA | 697 | ugAccAcuAAuGGGAGccAdTsdT | 763 | pUGGCUCCcAUuAGUGGUcAdTsdT |
| 227 | GUAGCUACCUCACAACCAG | 459 | CUGGUUGUGAGGUAGCUAC | 698 | GuAGcuAccucAcAAccAGdTsdT | 898 | pCUGGUUGUGAGGuAGCuACdTsdT |
| 228 | UCCCGCUCGCGCCCAUCAC | 460 | GUGAUGGGCGCGAGCGGGA | 699 | ucccGcucGcGcccAucAcdTsdT | 812 | pGUGAUGGGCGCGAGCGGGAdTsdT |
| 229 | CUUGGCUUUAAAGUGAGGG | 461 | CCCUCACUUUAAAGCCAAG | 700 | cuuGGcuuuAAAGuGAGGGdTsdT | 906 | pCCCUcACUUuAAAGCcAAGdTsdT |
| 230 | AGAAGCCCGCUGUUUCUAU | 462 | AUAGAAACAGCGGGCUUCU | 701 | AgAAGcccGcuGuuucuAudTsdT | 834 | pAUAGAAAcAGCGGGCUUCUdTsdT |

| SEQ ID NO | Sense strand sequence (5'-3') | SEQ ID NO | Antisense strand sequence (5'-3') | SEQ ID NO | sense strand sequence (5'-3') | SEQ ID NO | antisense strand sequence (5'-3') |
|---|---|---|---|---|---|---|---|
| 230 | AGAAGCCCGCUGUUUCUAU | 462 | AUAGAAACAGCGGGCUUCU | 701 | AgAAGcccGcuGuuucuAudTsdT | 948 | pAuAGAAAcAGCGGGCUUCUdTsdT |
| 231 | ACUAAAGUAAGUUAAACUU | 463 | AAGUUUAACUUACUUUAGU | 702 | AcuAAAGuAAGuuAAAcuudTsdT | 773 | pAAGUUuAACUuACUUuAGUdTsdT |
| 232 | AGUAAGUUAAACUUGUGUA | 464 | UACACAAGUUUAACUUACU | 703 | AguAAGuuAAAcuuGuGuAdTsdT | 774 | puAcAcAAGUUuAACUuACUdTsdT |
| 233 | AAUAAUUAUCAAUGCUGUU | 465 | AACAGCAUUGAUAAUUAUU | 704 | AauAAuuAucAAuGcuGuudTsdT | 725 | pAAcAGcAUUGAuAAUuAUUdTsdT |

Table 6

| FPL Name | Function | Sequence | SEQ ID No. |
|---|---|---|---|
| hsRRM001 | CE | cgggtttcagggattcccagTTTTTctcttggaaagaaagt | 997 |
| hsRRM002 | CE | gcttgctgcaaagaaagccaTTTTTctcttggaaagaaagt | 998 |
| hsRRM003 | CE | cttcttggctaaatcgctccaTTTTTctcttggaaagaaagt | 999 |
| hsRRM004 | CE | agcgggcttctgtaatctgaaTTTTTctcttggaaagaaagt | 1000 |
| hsRRM005 | CE | gagaaattccctttctttgggaTTTTTctcttggaaagaaagt | 1001 |
| hsRRM006 | CE | ggtagcctctttgtccccaatTTTTTctcttggaaagaaagt | 1002 |
| hsRRM007 | LE | gaacatgggatataaaatatctctcctTTTTTaggcataggacccgtgtct | 1003 |
| hsRRM008 | LE | ccaagttttcatttactatgccatcTTTTTaggcataggacccgtgtct | 1004 |
| hsRRM009 | LE | catttcagaatgtatgttttccatgTTTTTaggcataggacccgtgtct | 1005 |
| hsRRM010 | LE | catcgtttcaatggcattgaaTTTTTaggcataggacccgtgtct | 1006 |
| hsRRM011 | LE | ccagcgcaaggcccagtTTTTTaggcataggacccgtgtct | 1007 |
| hsRRM012 | LE | aaggctacaacacgttcaccataTTTTTaggcataggacccgtgtct | 1008 |
| hsRRM013 | LE | aatgccttccactgcagcaTTTTTaggcataggacccgtgtct | 1009 |
| hsRRM014 | BL | gcaatttggaagccatagaaac | 1010 |
| hsRRM015 | BL | tcttttatgtaagtgtcaataagaagactata | 1011 |
| hsRRM016 | BL | ctgccttcttcttgacacaagg | 1012 |

Table 7

| FPL Name | Function | Sequence | SEQ ID No. |
|---|---|---|---|
| hGAP001 | CE | gaatttgccatgggtggaatTTTTTctcttggaaagaaagt | 986 |
| hGAP002 | CE | ggagggatctcgctcctggaTTTTTctcttggaaagaaagt | 987 |
| hGAP003 | CE | ccccagccttctccatggtTTTTTctcttggaaagaaagt | 988 |
| hGAP004 | CE | gctcccccctgcaaatgagTTTTTctcttggaaagaaagt | 989 |
| hGAP005 | LE | agccttgacggtgccatgTTTTTaggcataggacccgtgtct | 990 |
| hGAP006 | LE | gatgacaagcttcccgttctcTTTTTaggcataggacccgtgtct | 991 |
| hGAP007 | LE | agatggtgatgggatttccattTTTTTaggcataggacccgtgtct | 992 |
| hGAP008 | LE | gcatcgccccacttgattttTTTTTaggcataggacccgtgtct | 993 |
| hGAP009 | LE | cacgacgtactcagcgccaTTTTTaggcataggacccgtgtct | 994 |
| hGAP010 | LE | ggcagagatgatgacccttttgTTTTTaggcataggacccgtgtct | 995 |
| hGAP011 | BL | ggtgaagacgccagtggactc | 996 |

Table 8

| SEQ ID 497/711 | | bDNA | | | |
|---|---|---|---|---|---|
| | p53 status | expt. 1 | expt. 2 | Cell-Titer Glo | xCELLigence |
| HepG2 | wt | 10.1 | 33 | 540 | 210 |
| HLF | mutant | 4.7 | 37 | 180 | 80 |
| A549 | wt | 5.2 | 165 | 520 | 140 |

| SEQ ID 477/839 | | bDNA | | | |
|---|---|---|---|---|---|
| | p53 status | expt. 1 | expt. 2 | Cell-Titer Glo | xCELLigence |
| HepG2 | wt | 3.6 | 28 | 380 | 130 |
| HLF | mutant | 0.73 | 21 | 120 | 69 |
| A549 | wt | 0.73 | 21 | 190 | 160 |

Table 9

| SEQ ID 497/711 | 24 hr | 48 hr | 72 hr |
|---|---|---|---|
| HepG2 | 81 | 85 | 85 |
| HLF | 90 | 90 | 83 |
| A549 | 84 | 59 | ND |

| SEQ ID 477/839 | 24 hr | 48 hr | 72 hr |
|---|---|---|---|
| HepG2 | 87 | 76 | 75 |
| HLF | 96 | 92 | 62 |
| A549 | 91 | 54 | ND |

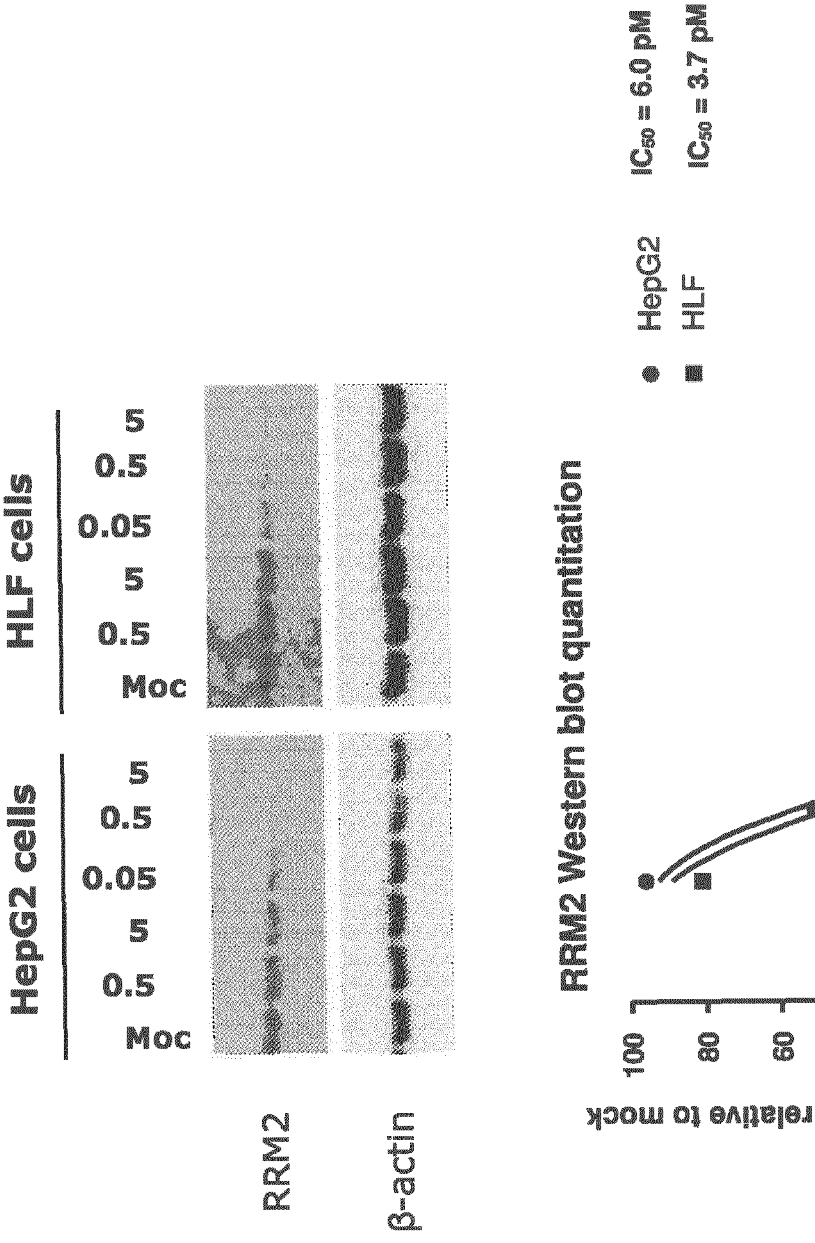
HepG2 cells
HLF cells
Moc
0.5
5
0.05
0.5
5
RRM2
β-actin
RRM2 Western blot quantitation
RRM2 protein relative to mock
log10
HepG2 IC50 = 6.0 pM
HLF IC50 = 3.7 pM

Fig. 1

Fig. 2

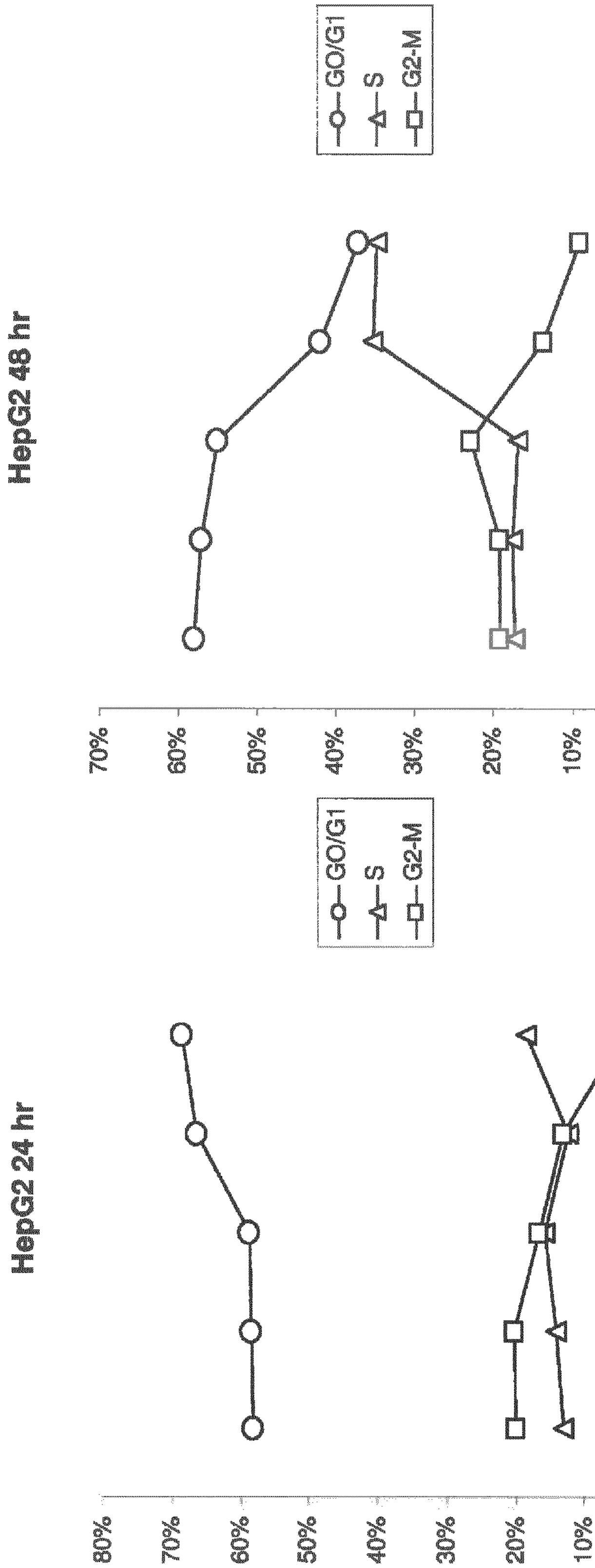
HepG2 24 hr
HepG2 48 hr
GO/G1
S
G2-M
0%
10%
20%
30%
40%
50%
60%
70%
80%
0.0001
0.001
0.01
0.1
1
10

Fig. 3

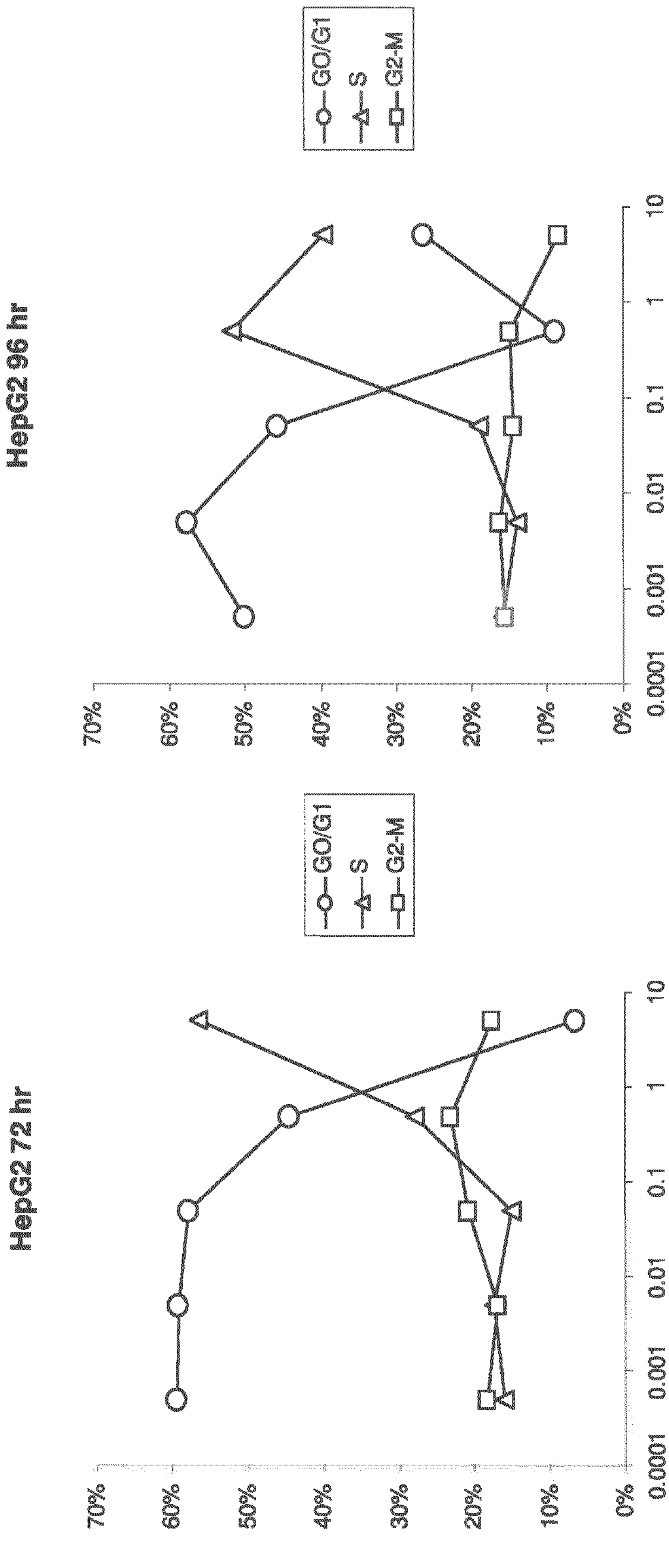
HepG2 72 hr
HepG2 96 hr
GO/G1
S
G2-M
70%
60%
50%
40%
30%
20%
10%
0%
0.0001
0.001
0.01
0.1
1
10

Fig. 4

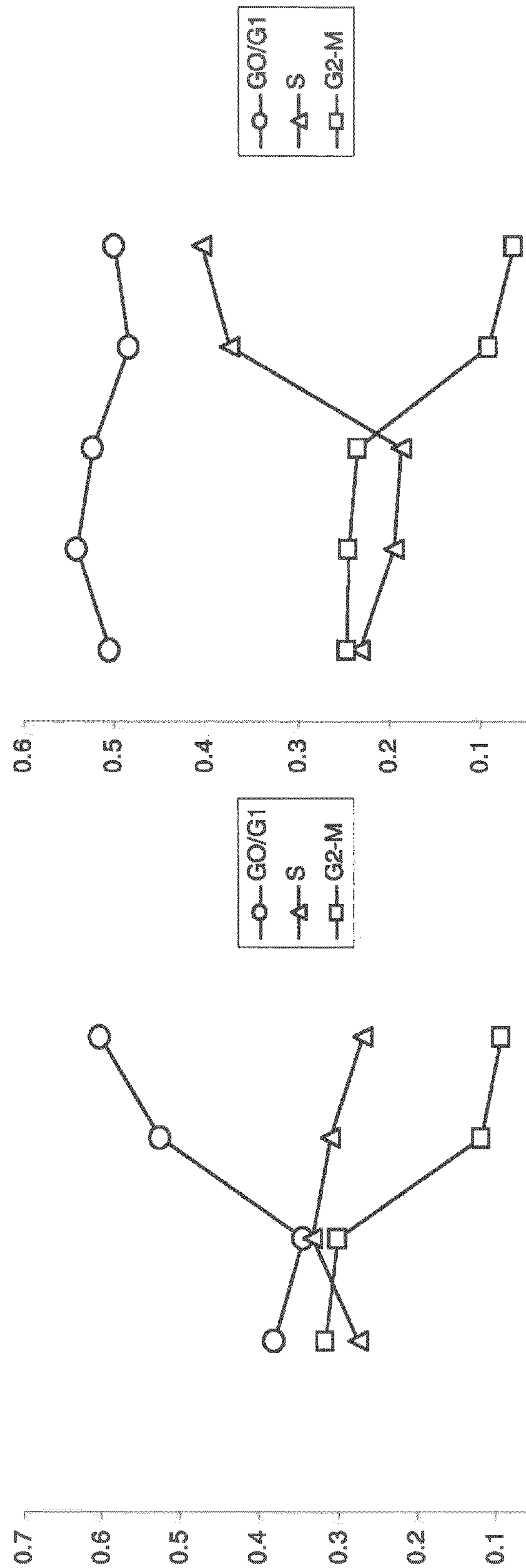
HLF 24 hr
HLF 48 hr
GO/G1
S
G2-M
0.7
0.6
0.5
0.4
0.3
0.2
0.1
0
0.0001
0.001
0.01
0.1
1
10

Fig. 5

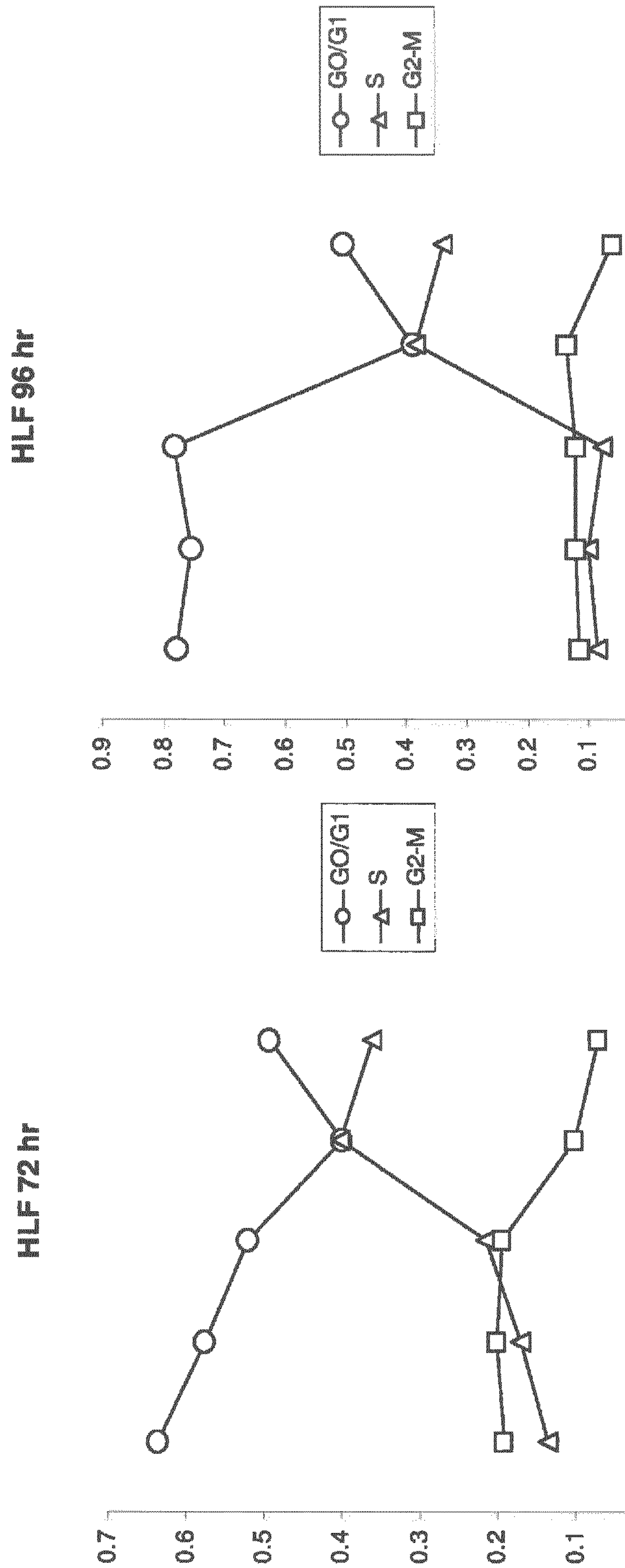
HLF 72 hr
GO/G1
S
G2-M
0.7
0.6
0.5
0.4
0.3
0.2
0.1
0
0.0001
0.001
0.01
0.1
1
10
HLF 96 hr
GO/G1
S
G2-M
0.9
0.8
0.7
0.6
0.5
0.4
0.3
0.2
0.1
0
0.0001
0.001
0.01
0.1
1
10

Fig. 6

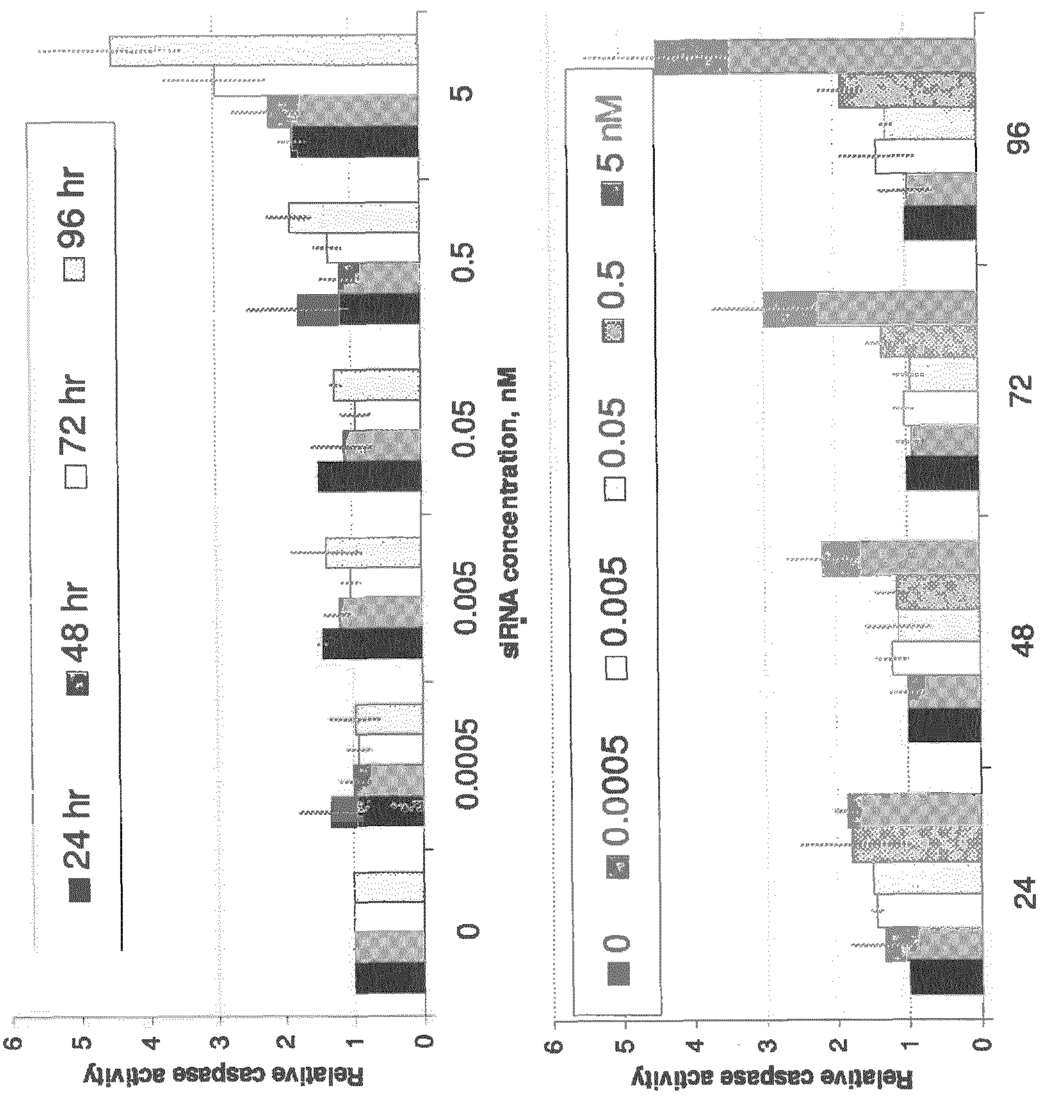

Relative caspase activity
24 hr
48 hr
72 hr
96 hr
siRNA concentration, nM
0
0.0005
0.005
0.05
0.5
5
Relative caspase activity
0
0.0005
0.005
0.05
0.5
5 nM
Time after transfection, hr
24
48
72
96

Fig. 7

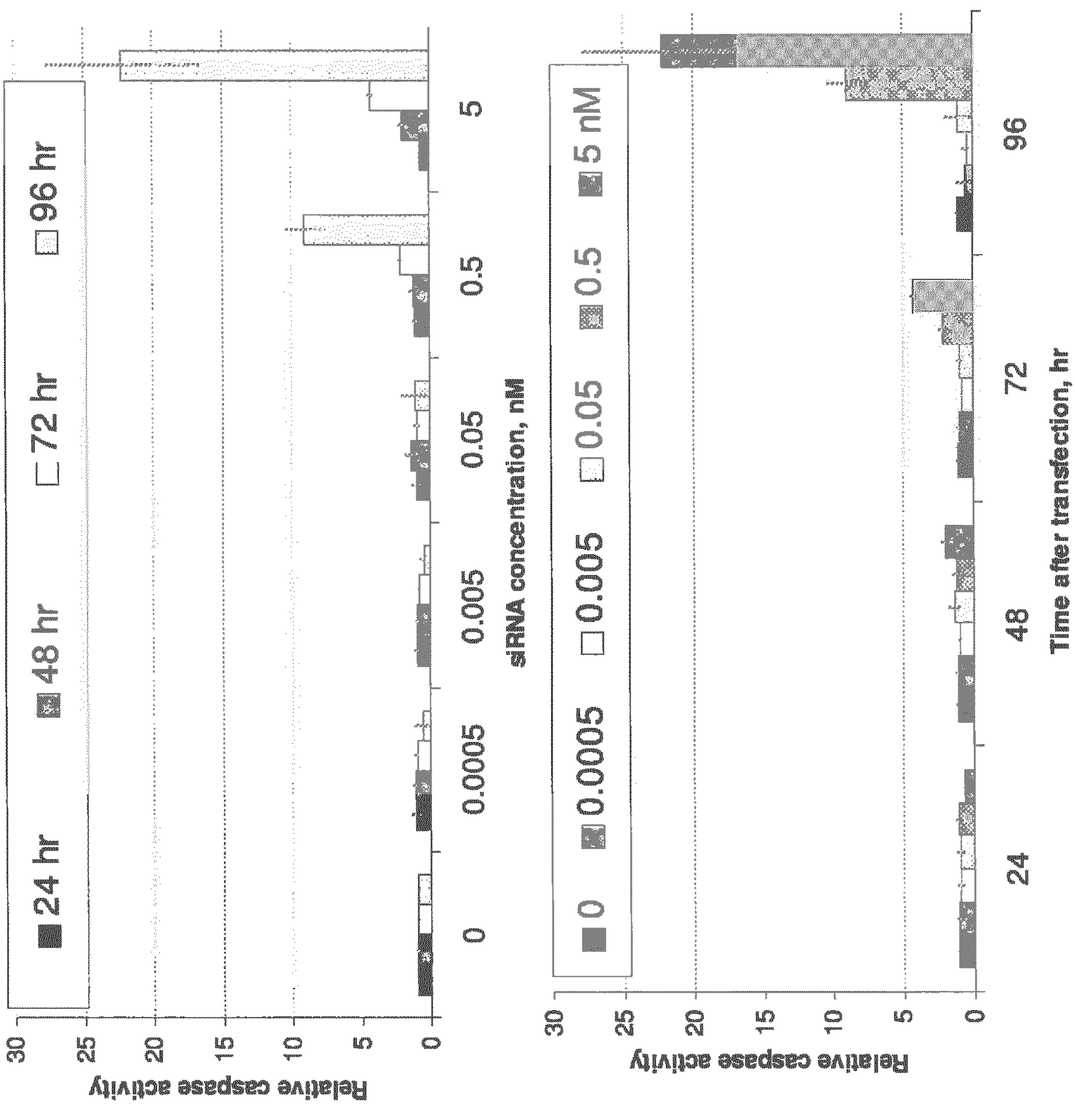
24 hr
48 hr
72 hr
96 hr
Relative caspase activity
30
25
20
15
10
5
0
0.0005
0.005
0.05
0.5
siRNA concentration, nM
0
0.0005
0.005
0.05
0.5
5 nM
24
48
72
96
Time after transfection, hr

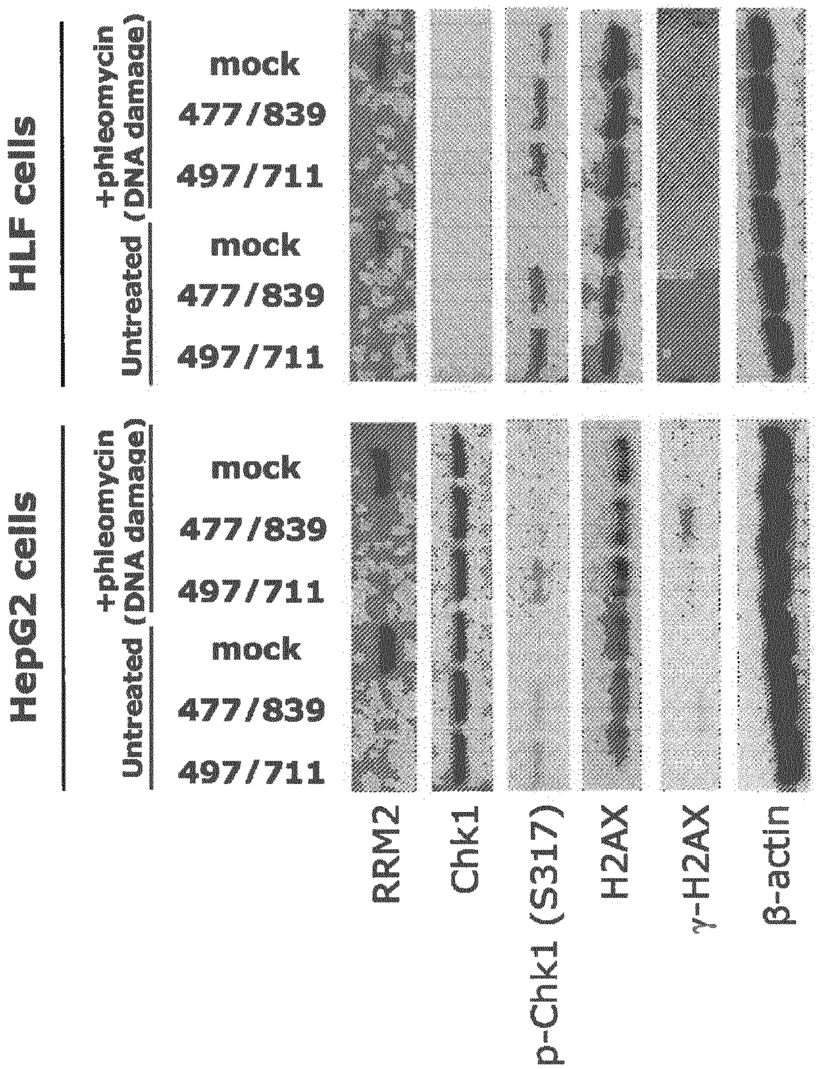
HepG2 cells
HLF cells
Untreated
+phleomycin (DNA damage)
497/711
477/839
mock
497/711
477/839
mock
RRM2
Chk1
p-Chk1 (S317)
H2AX
γ-H2AX
β-actin

Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2008227967 A
- US 5328470 A
- WO 0022113 A, Skillern, A.
- WO 9106309 A
- US 5218105 A
- US 5541307 A
- US 5521302 A
- US 5539082 A
- US 5554746 A
- US 5571902 A
- US 5578718 A
- US 5587361 A
- US 5506351 A
- US 5587469 A
- US 5587470 A
- US 5608046 A
- US 5610289 A
- US 6262241 B
- WO 9307883 A, Manoharan
- US 4469863 A
- US 5023243 A
- US 5264423 A
- US 5321131 A
- US 5399676 A
- US 5405939 A
- US 5453496 A
- US 5455233 A
- US 5466677 A
- US 5034506 A
- US 5214134 A
- US 5216141 A
- US 5264562 A
- US 5470967 A
- US 5489677 A
- US 5602240 A
- US 5663312 A
- US 3687808 A
- US 5134066 A
- US 5459255 A
- US 5552540 A
- US 5594121 A
- US 5596091 A
- US 6127533 A
- US 6166197 A
- US 5670633 A
- US 6172209 B
- US 6271358 B
- US 5359044 A
- US 5466786 A
- US 5519134 A
- US 5591722 A
- US 5597909 A
- US 5646265 A
- US 5700920 A
- US 5212295 A, Cook
- EP 10187851 A

### Non-patent literature cited in the description


- **CHEN et al.** *Proc. Natl. Acad. Sci. USA,* 1994, vol. 91, 3054-3057
- **GASSMANN et al.** *Proc. Natl. Acad. Sci. USA,* 1995, vol. 92, 1292
- **MUZYCZKA et al.** *Curr. Topics Micro. Immunol.,* 1992, vol. 158, 97-129
- **BERKNER et al.** *BioTechniques,* 1998, vol. 6, 616
- **ROSENFELD et al.** *Science,* 1991, vol. 252, 431-434
- **ROSENFELD et al.** *Cell,* 1992, vol. 68, 143-155
- **DANOS ; MULLIGAN.** *Proc. Natl. Acad. Sci. USA,* 1998, vol. 85, 6460-6464
- **COMETTE et al.** *Human Gene Therapy,* 1991, vol. 2, 5-10
- **CONE et al.** *Proc. Natl. Acad. Sci. USA,* 1984, vol. 81, 6349
- **HSU et al.** *J. Infectious Disease,* 1992, vol. 166, 769
- **BUCCHINI et al.** *Proc. Natl. Acad. Sci. USA,* 1986, vol. 83, 2511-2515
- **DOCHERTY et al.** *FASEB J.,* 1994, vol. 8, 20-24
- Current protocols in nucleic acid chemistry. John Wiley & Sons, Inc,
- **WILLIAMS, D.J. ; K.B. HALL.** *Biochem.,* 1996, vol. 35, 14665-14670
- **WAGNER.** *Nat. Med.,* 1995, vol. 1, 1116-8
- **NAWROT.** *Current Topics in Med Chem,* 2006, vol. 6, 913-925
- **M. MANOHARAN.** *Antisense & Nucleic Acid Drug Development,* 2002, vol. 12, 103

- **LI, S. ; DESHMUKH, H. M. ; HUANG, L.** *Pharm. Res.,* 1998, vol. 15, 1540
- **GREENE ; WUTS.** Protective Groups in Organic Synthesis. John Wiley & Sons, 1991
- Oligonucleotides And Analogues A Practical Approach. IRL Press, 1991
- **E. ATHERTON ; R. C. SHEPPARD.** The Peptides. Academic Press, 1987, vol. 9, 1
- **SAMUKOV et al.** *Tetrahedron Lett.,* 1994, vol. 35, 7821
- Innovations and Perspectives in solid-phase Synthesis. **SCOTT et al.** 3rd International Symposium. Mayflower Worldwide, 1994, 115-124
- **GUZAEV, A. I. ; MANOHARAN, M.** *J. Am. Chem. Soc.,* 2003, vol. 125, 2380
- Concise Encyclopedia Of Polymer Science And Engineering. John Wiley & Sons, 1990, 858-859
- **ENGLISCH et al.** *Angewandte Chemie, International Edition,* 1991, vol. 30, 613
- **SANGHVI, Y. S.** Antisense Research and Applications. CRC Press, 1993, 289-302
- **DELGARDO.** *Critical Reviews in Therapeutic Drug Carrier Systems,* 1992, vol. 9, 249
- **COOK.** *Anti-fibrosis Drug Design,* 1991, vol. 6, 585-607
- **HAMM et al.** *J. Org. Chem.,* 1997, vol. 62, 3415-3420
- **GOETTINGEN, M.** *J. Org. Chem.,* 1996, vol. 61, 6273-6281
- **POLUSHIN et al.** *Tetrahedron Lett.,* 1996, vol. 37, 3227-3230
- **LETSINGER et al.** *Proc. Natl. Acad. Sci. USA,* 1989, vol. 86, 6553
- **MANOHARAN et al.** *Bioorg. Med. Chem. Lett.,* 1994, vol. 4, 1053
- **MANOHARAN et al.** *Ann. N.Y. Acad. Sci.,* 1992, vol. 660, 306
- **MANOHARAN et al.** *Bioorg. Med. Chem. Let.,* 1993, vol. 3, 2765
- **OBERHAUSER et al.** *Nucl. Acids Res.,* 1992, vol. 20, 533
- **SAISON-BEHMOARAS et al.** *EMBO J.,* 1991, vol. 10, 111
- **KABANOV et al.** *FEBS Lett.,* 1990, vol. 259, 327
- **SVINARCHUK et al.** *Biochimie,* 1993, vol. 75, 49
- **MANOHARAN et al.** *Tetrahedron Lett.,* 1995, vol. 36, 3651
- **SHEA et al.** *Nucl. Acids Res.,* 1990, vol. 18, 3777
- **MANOHARAN et al.** *Nucleosides & Nucleotides,* 1995, vol. 14, 969
- **MISHRA et al.** *Biochim. Biophys. Acta,* 1995, vol. 1264, 229
- **CROOKE et al.** *J. Pharmacol. Exp. Ther.,* 1996, vol. 277, 923
- **AKHTAR.** *Journal of Clinical Investigation,* 2007, vol. 117, 3623-3632
- **NGUYEN et al.** *Current Opinion in Moleculare Therapeutics,* 2008, vol. 10, 158-167
- **ZAMBONI.** *Clin. Cancer Res.,* 2005, vol. 11, 8230-8234
- **IKEDA et al.** *Pharmaceutical Research,* 2006, vol. 23, 1631-1640

SEQUENCE LISTING

<110> ARROWHEAD RESEARCH CORPORATION

<120> COMPOSITIONS AND METHODS FOR INHIBITING EXPRESSION OF RRM2 GENES

<130> 4/2RB23/21A

<150> EP10187851.0
<151> 2010-10-18

<160> 1015

<210> 1
<211> 19
<212> RNA
<213> Artificial sequence

<220>
<223> Description of the artificial sequence: sense strand of dsRNA

<400> 1
uuguggcaga cagacuuau 19

<210> 2
<211> 19
<212> RNA
<213> Artificial sequence

<220>
<223> Description of the artificial sequence: sense strand of dsRNA

<400> 2
ccugauguuc aaacaccug 19

<210> 3
<211> 19
<212> RNA
<213> Artificial sequence

<220>
<223> Description of the artificial sequence: sense strand of dsRNA

<400> 3
aguccaacag agaauucuu 19

<210> 4
<211> 19
<212> RNA
<213> Artificial sequence

<220>
<223> Description of the artificial sequence: sense strand of dsRNA

<400> 4
uaggcgagua ucagaggau 19

<210> 5
<211> 19
<212> RNA
<213> Artificial sequence

<220>
<223> Description of the artificial sequence: sense strand of dsRNA

<400> 5
ggcgaguauc agaggaugg 19

<210> 6
<211> 19
<212> RNA
<213> Artificial sequence

<220>
<223> Description of the artificial sequence: sense strand of dsRNA

<400> 6
uguucaaaca ccugguaca 19

<210> 7
<211> 19
<212> RNA
<213> Artificial sequence

<220>
<223> Description of the artificial sequence: sense strand of dsRNA

<400> 7
gggugacccu uuagugagc 19

<210> 8
<211> 19
<212> RNA
<213> Artificial sequence

<220>
<223> Description of the artificial sequence: sense strand of dsRNA

<400> 8
gaaggaaaga cuaacuucu 19

<210> 9
<211> 19
<212> RNA
<213> Artificial sequence

<220>
<223> Description of the artificial sequence: sense strand of dsRNA

<400> 9
uucugaaaug uauagucuu 19

<210> 10
<211> 19
<212> RNA

<213> Artificial sequence

<220>
<223> Description of the artificial sequence: sense strand of dsRNA

<400> 10
cuguguagcu accucacaa 19

<210> 11
<211> 19
<212> RNA
<213> Artificial sequence

<220>
<223> Description of the artificial sequence: sense strand of dsRNA

<400> 11
ugcacucuaa ugaagcaau 19

<210> 12
<211> 19
<212> RNA
<213> Artificial sequence

<220>
<223> Description of the artificial sequence: sense strand of dsRNA

<400> 12
cccauuugac uuuauggag 19

<210> 13
<211> 19
<212> RNA
<213> Artificial sequence

<220>
<223> Description of the artificial sequence: sense strand of dsRNA

<400> 13
aaauguauag ucuucuuau 19

<210> 14
<211> 19
<212> RNA
<213> Artificial sequence

<220>
<223> Description of the artificial sequence: sense strand of dsRNA

<400> 14
uacauugagu uuguggcag 19

<210> 15
<211> 19
<212> RNA
<213> Artificial sequence

<220>

<223> Description of the artificial sequence: sense strand of dsRNA

<400> 15
caauacauug aguuugugg 19

<210> 16
<211> 19
<212> RNA
<213> Artificial sequence

<220>
<223> Description of the artificial sequence: sense strand of dsRNA

<400> 16
gaacaggagu uccucacug 19

<210> 17
<211> 19
<212> RNA
<213> Artificial sequence

<220>
<223> Description of the artificial sequence: sense strand of dsRNA

<400> 17
aucccauguu cuggcuuuc 19

<210> 18
<211> 19
<212> RNA
<213> Artificial sequence

<220>
<223> Description of the artificial sequence: sense strand of dsRNA

<400> 18
guagguugug ugaguuaau 19

<210> 19
<211> 19
<212> RNA
<213> Artificial sequence

<220>
<223> Description of the artificial sequence: sense strand of dsRNA

<400> 19
auagucuucu uauugacac 19

<210> 20
<211> 19
<212> RNA
<213> Artificial sequence

<220>
<223> Description of the artificial sequence: sense strand of dsRNA

<400> 20

auugcacucu aaugaagca 19

<210> 21
<211> 19
<212> RNA
<213> Artificial sequence

<220>
<223> Description of the artificial sequence: sense strand of dsRNA

<400> 21
uuaucaaugc uguucggau 19

<210> 22
<211> 19
<212> RNA
<213> Artificial sequence

<220>
<223> Description of the artificial sequence: sense strand of dsRNA

<400> 22
agaaacgagg acugaugcc 19

<210> 23
<211> 19
<212> RNA
<213> Artificial sequence

<220>
<223> Description of the artificial sequence: sense strand of dsRNA

<400> 23
cauugaguuu guggcagac 19

<210> 24
<211> 19
<212> RNA
<213> Artificial sequence

<220>
<223> Description of the artificial sequence: sense strand of dsRNA

<400> 24
acauucagca cugggaauc 19

<210> 25
<211> 19
<212> RNA
<213> Artificial sequence

<220>
<223> Description of the artificial sequence: sense strand of dsRNA

<400> 25
ugauguucaa acaccuggu 19

<210> 26

<211> 19
<212> RNA
<213> Artificial sequence

<220>
<223> Description of the artificial sequence: sense strand of dsRNA

<400> 26
ggauagaaca ggaguuccu 19

<210> 27
<211> 19
<212> RNA
<213> Artificial sequence

<220>
<223> Description of the artificial sequence: sense strand of dsRNA

<400> 27
aauauuucac uggaaggaa 19

<210> 28
<211> 19
<212> RNA
<213> Artificial sequence

<220>
<223> Description of the artificial sequence: sense strand of dsRNA

<400> 28
aauaaacauu guuuguacu 19

<210> 29
<211> 19
<212> RNA
<213> Artificial sequence

<220>
<223> Description of the artificial sequence: sense strand of dsRNA

<400> 29
ucccauguuc uggcuuucu 19

<210> 30
<211> 19
<212> RNA
<213> Artificial sequence

<220>
<223> Description of the artificial sequence: sense strand of dsRNA

<400> 30
uucggauaga acaggaguu 19

<210> 31
<211> 19
<212> RNA
<213> Artificial sequence

<220>
<223> Description of the artificial sequence: sense strand of dsRNA

<400> 31
aaguagguug ugugaguua 19

<210> 32
<211> 19
<212> RNA
<213> Artificial sequence

<220>
<223> Description of the artificial sequence: sense strand of dsRNA

<400> 32
uuauagugcu gguaguauc 19

<210> 33
<211> 19
<212> RNA
<213> Artificial sequence

<220>
<223> Description of the artificial sequence: sense strand of dsRNA

<400> 33
cuucuuauug acacuuaca 19

<210> 34
<211> 19
<212> RNA
<213> Artificial sequence

<220>
<223> Description of the artificial sequence: sense strand of dsRNA

<400> 34
uacagaagcc cgcuguuuc 19

<210> 35
<211> 19
<212> RNA
<213> Artificial sequence

<220>
<223> Description of the artificial sequence: sense strand of dsRNA

<400> 35
gugacccuuu agugagcuu 19

<210> 36
<211> 19
<212> RNA
<213> Artificial sequence

<220>
<223> Description of the artificial sequence: sense strand of dsRNA

<400> 36
auagaacagg aguuccuca 19

<210> 37
<211> 19
<212> RNA
<213> Artificial sequence

<220>
<223> Description of the artificial sequence: sense strand of dsRNA

<400> 37
cuggcacuuu acaaacaaa 19

<210> 38
<211> 19
<212> RNA
<213> Artificial sequence

<220>
<223> Description of the artificial sequence: sense strand of dsRNA

<400> 38
ucuaaugaag caauacauu 19

<210> 39
<211> 19
<212> RNA
<213> Artificial sequence

<220>
<223> Description of the artificial sequence: sense strand of dsRNA

<400> 39
ucuucuuauu gacacuuac 19

<210> 40
<211> 19
<212> RNA
<213> Artificial sequence

<220>
<223> Description of the artificial sequence: sense strand of dsRNA

<400> 40
uguucggaua gaacaggag 19

<210> 41
<211> 19
<212> RNA
<213> Artificial sequence

<220>
<223> Description of the artificial sequence: sense strand of dsRNA

<400> 41
aguaccauga uaucuggca 19

<210> 42
<211> 19
<212> RNA
<213> Artificial sequence

<220>
<223> Description of the artificial sequence: sense strand of dsRNA

<400> 42
cagagaugag gguuuacac 19

<210> 43
<211> 19
<212> RNA
<213> Artificial sequence

<220>
<223> Description of the artificial sequence: sense strand of dsRNA

<400> 43
gaaacgagga cugaugccu 19

<210> 44
<211> 19
<212> RNA
<213> Artificial sequence

<220>
<223> Description of the artificial sequence: sense strand of dsRNA

<400> 44
aagagaguag gcgaguauc 19

<210> 45
<211> 19
<212> RNA
<213> Artificial sequence

<220>
<223> Description of the artificial sequence: sense strand of dsRNA

<400> 45
cauuagcuga auaauguga 19

<210> 46
<211> 19
<212> RNA
<213> Artificial sequence

<220>
<223> Description of the artificial sequence: sense strand of dsRNA

<400> 46
aguagagaac ccauuugac 19

<210> 47
<211> 19
<212> RNA

<213> Artificial sequence

<220>
<223> Description of the artificial sequence: sense strand of dsRNA

<400> 47
aggcgaguau cagaggaug 19

<210> 48
<211> 19
<212> RNA
<213> Artificial sequence

<220>
<223> Description of the artificial sequence: sense strand of dsRNA

<400> 48
uagacuaagc auguaauuu 19

<210> 49
<211> 19
<212> RNA
<213> Artificial sequence

<220>
<223> Description of the artificial sequence: sense strand of dsRNA

<400> 49
aacauuguuu guacucaca 19

<210> 50
<211> 19
<212> RNA
<213> Artificial sequence

<220>
<223> Description of the artificial sequence: sense strand of dsRNA

<400> 50
gaugggagug augucaagu 19

<210> 51
<211> 19
<212> RNA
<213> Artificial sequence

<220>
<223> Description of the artificial sequence: sense strand of dsRNA

<400> 51
cagaccauuu ccuaaucag 19

<210> 52
<211> 19
<212> RNA
<213> Artificial sequence

<220>

<223> Description of the artificial sequence: sense strand of dsRNA

<400> 52
gauuacagaa gcccgcugu 19

<210> 53
<211> 19
<212> RNA
<213> Artificial sequence

<220>
<223> Description of the artificial sequence: sense strand of dsRNA

<400> 53
cauugaaacg augccuugu 19

<210> 54
<211> 19
<212> RNA
<213> Artificial sequence

<220>
<223> Description of the artificial sequence: sense strand of dsRNA

<400> 54
acuuaugcug gaacugggu 19

<210> 55
<211> 19
<212> RNA
<213> Artificial sequence

<220>
<223> Description of the artificial sequence: sense strand of dsRNA

<400> 55
gucgacaagg agaacacgc 19

<210> 56
<211> 19
<212> RNA
<213> Artificial sequence

<220>
<223> Description of the artificial sequence: sense strand of dsRNA

<400> 56
aggaaagacu aacuucuuu 19

<210> 57
<211> 19
<212> RNA
<213> Artificial sequence

<220>
<223> Description of the artificial sequence: sense strand of dsRNA

<400> 57

caagaccgcg aggaggauc 19

<210> 58
<211> 19
<212> RNA
<213> Artificial sequence

<220>
<223> Description of the artificial sequence: sense strand of dsRNA

<400> 58
gacaauggca gucuuggcu 19

<210> 59
<211> 19
<212> RNA
<213> Artificial sequence

<220>
<223> Description of the artificial sequence: sense strand of dsRNA

<400> 59
augccuugug ucaagaaga 19

<210> 60
<211> 19
<212> RNA
<213> Artificial sequence

<220>
<223> Description of the artificial sequence: sense strand of dsRNA

<400> 60
gccucacugc uucaacgca 19

<210> 61
<211> 19
<212> RNA
<213> Artificial sequence

<220>
<223> Description of the artificial sequence: sense strand of dsRNA

<400> 61
uaccucacaa ccaguccug 19

<210> 62
<211> 19
<212> RNA
<213> Artificial sequence

<220>
<223> Description of the artificial sequence: sense strand of dsRNA

<400> 62
gagaagagag uaggcgagu 19

<210> 63

<211> 19
<212> RNA
<213> Artificial sequence

<220>
<223> Description of the artificial sequence: sense strand of dsRNA

<400> 63
agacuuaugc uggaacugg 19

<210> 64
<211> 19
<212> RNA
<213> Artificial sequence

<220>
<223> Description of the artificial sequence: sense strand of dsRNA

<400> 64
uuacagaagc ccgcuguuu 19

<210> 65
<211> 19
<212> RNA
<213> Artificial sequence

<220>
<223> Description of the artificial sequence: sense strand of dsRNA

<400> 65
uuaugcugga acuggguuu 19

<210> 66
<211> 19
<212> RNA
<213> Artificial sequence

<220>
<223> Description of the artificial sequence: sense strand of dsRNA

<400> 66
auaaacauug uuuguacuc 19

<210> 67
<211> 19
<212> RNA
<213> Artificial sequence

<220>
<223> Description of the artificial sequence: sense strand of dsRNA

<400> 67
ucaaugccau ugaaacgau 19

<210> 68
<211> 19
<212> RNA
<213> Artificial sequence

<220>
<223> Description of the artificial sequence: sense strand of dsRNA

<400> 68
auagugcugg uaguaucac 19

<210> 69
<211> 19
<212> RNA
<213> Artificial sequence

<220>
<223> Description of the artificial sequence: sense strand of dsRNA

<400> 69
cagccucacu gcuucaacg 19

<210> 70
<211> 19
<212> RNA
<213> Artificial sequence

<220>
<223> Description of the artificial sequence: sense strand of dsRNA

<400> 70
ucuuggcuuu aaagugagg 19

<210> 71
<211> 19
<212> RNA
<213> Artificial sequence

<220>
<223> Description of the artificial sequence: sense strand of dsRNA

<400> 71
ggcugugacu uaccauagc 19

<210> 72
<211> 19
<212> RNA
<213> Artificial sequence

<220>
<223> Description of the artificial sequence: sense strand of dsRNA

<400> 72
ggcuaccuau ggugaacgu 19

<210> 73
<211> 19
<212> RNA
<213> Artificial sequence

<220>
<223> Description of the artificial sequence: sense strand of dsRNA

<400> 73
cgcgaggagg aucuuccag 19

<210> 74
<211> 19
<212> RNA
<213> Artificial sequence

<220>
<223> Description of the artificial sequence: sense strand of dsRNA

<400> 74
gccauugaaa cgaugccuu 19

<210> 75
<211> 19
<212> RNA
<213> Artificial sequence

<220>
<223> Description of the artificial sequence: sense strand of dsRNA

<400> 75
agccucacug cuucaacgc 19

<210> 76
<211> 19
<212> RNA
<213> Artificial sequence

<220>
<223> Description of the artificial sequence: sense strand of dsRNA

<400> 76
ggcagacaga cuuaugcug 19

<210> 77
<211> 19
<212> RNA
<213> Artificial sequence

<220>
<223> Description of the artificial sequence: sense strand of dsRNA

<400> 77
gugacuaaag uaaguuaaa 19

<210> 78
<211> 19
<212> RNA
<213> Artificial sequence

<220>
<223> Description of the artificial sequence: sense strand of dsRNA

<400> 78
aguuauuguu accuaaagu 19

<210> 79
<211> 19
<212> RNA
<213> Artificial sequence

<220>
<223> Description of the artificial sequence: sense strand of dsRNA

<400> 79
gccuuuaugu uugggagaa 19

<210> 80
<211> 19
<212> RNA
<213> Artificial sequence

<220>
<223> Description of the artificial sequence: sense strand of dsRNA

<400> 80
uucagaguag agaacccau 19

<210> 81
<211> 19
<212> RNA
<213> Artificial sequence

<220>
<223> Description of the artificial sequence: sense strand of dsRNA

<400> 81
aaacgaggac ugaugccug 19

<210> 82
<211> 19
<212> RNA
<213> Artificial sequence

<220>
<223> Description of the artificial sequence: sense strand of dsRNA

<400> 82
guaggcgagu aucagagga 19

<210> 83
<211> 19
<212> RNA
<213> Artificial sequence

<220>
<223> Description of the artificial sequence: sense strand of dsRNA

<400> 83
aagcccgcug uuucuaugg 19

<210> 84
<211> 19
<212> RNA

<213> Artificial sequence

<220>
<223> Description of the artificial sequence: sense strand of dsRNA

<400> 84
ucagcacugg gaaucccug 19

<210> 85
<211> 19
<212> RNA
<213> Artificial sequence

<220>
<223> Description of the artificial sequence: sense strand of dsRNA

<400> 85
gaauaaugug aggauuaac 19

<210> 86
<211> 19
<212> RNA
<213> Artificial sequence

<220>
<223> Description of the artificial sequence: sense strand of dsRNA

<400> 86
uguggcagac agacuuaug 19

<210> 87
<211> 19
<212> RNA
<213> Artificial sequence

<220>
<223> Description of the artificial sequence: sense strand of dsRNA

<400> 87
agagauaaau guugaucuu 19

<210> 88
<211> 19
<212> RNA
<213> Artificial sequence

<220>
<223> Description of the artificial sequence: sense strand of dsRNA

<400> 88
uaccaugaua ucuggcaga 19

<210> 89
<211> 19
<212> RNA
<213> Artificial sequence

<220>

<223> Description of the artificial sequence: sense strand of dsRNA

<400> 89
cuuccaaauu gccauggaa 19

<210> 90
<211> 19
<212> RNA
<213> Artificial sequence

<220>
<223> Description of the artificial sequence: sense strand of dsRNA

<400> 90
accgcgagga ggaucuucc 19

<210> 91
<211> 19
<212> RNA
<213> Artificial sequence

<220>
<223> Description of the artificial sequence: sense strand of dsRNA

<400> 91
gaaauguaua gucuucuua 19

<210> 92
<211> 19
<212> RNA
<213> Artificial sequence

<220>
<223> Description of the artificial sequence: sense strand of dsRNA

<400> 92
auguucaaac accugguac 19

<210> 93
<211> 19
<212> RNA
<213> Artificial sequence

<220>
<223> Description of the artificial sequence: sense strand of dsRNA

<400> 93
agggaauuuc ucuucaaug 19

<210> 94
<211> 19
<212> RNA
<213> Artificial sequence

<220>
<223> Description of the artificial sequence: sense strand of dsRNA

<400> 94

cccuguuaag uggugaaau 19

<210> 95
<211> 19
<212> RNA
<213> Artificial sequence

<220>
<223> Description of the artificial sequence: sense strand of dsRNA

<400> 95
gaugaggguu uacacugug 19

<210> 96
<211> 19
<212> RNA
<213> Artificial sequence

<220>
<223> Description of the artificial sequence: sense strand of dsRNA

<400> 96
ugugugaguu aauucauuu 19

<210> 97
<211> 19
<212> RNA
<213> Artificial sequence

<220>
<223> Description of the artificial sequence: sense strand of dsRNA

<400> 97
uugccugaug uucaaacac 19

<210> 98
<211> 19
<212> RNA
<213> Artificial sequence

<220>
<223> Description of the artificial sequence: sense strand of dsRNA

<400> 98
aaacuugugu agacuaagc 19

<210> 99
<211> 19
<212> RNA
<213> Artificial sequence

<220>
<223> Description of the artificial sequence: sense strand of dsRNA

<400> 99
uauaucccau guucuggcu 19

<210> 100

<211> 19
<212> RNA
<213> Artificial sequence

<220>
<223> Description of the artificial sequence: sense strand of dsRNA

<400> 100
uuguguagac uaagcaugu 19

<210> 101
<211> 19
<212> RNA
<213> Artificial sequence

<220>
<223> Description of the artificial sequence: sense strand of dsRNA

<400> 101
augcuguucg gauagaaca 19

<210> 102
<211> 19
<212> RNA
<213> Artificial sequence

<220>
<223> Description of the artificial sequence: sense strand of dsRNA

<400> 102
aauuaucaau gcuguucgg 19

<210> 103
<211> 19
<212> RNA
<213> Artificial sequence

<220>
<223> Description of the artificial sequence: sense strand of dsRNA

<400> 103
gccugauguu caaacaccu 19

<210> 104
<211> 19
<212> RNA
<213> Artificial sequence

<220>
<223> Description of the artificial sequence: sense strand of dsRNA

<400> 104
cauagcagug acaauggca 19

<210> 105
<211> 19
<212> RNA
<213> Artificial sequence

<220>
<223> Description of the artificial sequence: sense strand of dsRNA

<400> 105
ugugaguuaa uucauuuau 19

<210> 106
<211> 19
<212> RNA
<213> Artificial sequence

<220>
<223> Description of the artificial sequence: sense strand of dsRNA

<400> 106
agugcuggua guaucaccu 19

<210> 107
<211> 19
<212> RNA
<213> Artificial sequence

<220>
<223> Description of the artificial sequence: sense strand of dsRNA

<400> 107
uaucaaugcu guucggaua 19

<210> 108
<211> 19
<212> RNA
<213> Artificial sequence

<220>
<223> Description of the artificial sequence: sense strand of dsRNA

<400> 108
gacuaaagua aguuaaacu 19

<210> 109
<211> 19
<212> RNA
<213> Artificial sequence

<220>
<223> Description of the artificial sequence: sense strand of dsRNA

<400> 109
aaugcuguuc ggauagaac 19

<210> 110
<211> 19
<212> RNA
<213> Artificial sequence

<220>
<223> Description of the artificial sequence: sense strand of dsRNA

<400> 110
agaauauuuc acuggaagg 19

<210> 111
<211> 19
<212> RNA
<213> Artificial sequence

<220>
<223> Description of the artificial sequence: sense strand of dsRNA

<400> 111
aucuggcaga uguauaaga 19

<210> 112
<211> 19
<212> RNA
<213> Artificial sequence

<220>
<223> Description of the artificial sequence: sense strand of dsRNA

<400> 112
uauagugcug guaguauca 19

<210> 113
<211> 19
<212> RNA
<213> Artificial sequence

<220>
<223> Description of the artificial sequence: sense strand of dsRNA

<400> 113
ggccagcaag accgcgagg 19

<210> 114
<211> 19
<212> RNA
<213> Artificial sequence

<220>
<223> Description of the artificial sequence: sense strand of dsRNA

<400> 114
ccaugauauc uggcagaug 19

<210> 115
<211> 19
<212> RNA
<213> Artificial sequence

<220>
<223> Description of the artificial sequence: sense strand of dsRNA

<400> 115
uuaaacuugu guagacuaa 19

<210> 116
<211> 19
<212> RNA
<213> Artificial sequence

<220>
<223> Description of the artificial sequence: sense strand of dsRNA

<400> 116
uucaaugcca uugaaacga 19

<210> 117
<211> 19
<212> RNA
<213> Artificial sequence

<220>
<223> Description of the artificial sequence: sense strand of dsRNA

<400> 117
agaaagcuga gacauugca 19

<210> 118
<211> 19
<212> RNA
<213> Artificial sequence

<220>
<223> Description of the artificial sequence: sense strand of dsRNA

<400> 118
cuauggcuuc caaauugcc 19

<210> 119
<211> 19
<212> RNA
<213> Artificial sequence

<220>
<223> Description of the artificial sequence: sense strand of dsRNA

<400> 119
aagugacuaa aguaaguua 19

<210> 120
<211> 19
<212> RNA
<213> Artificial sequence

<220>
<223> Description of the artificial sequence: sense strand of dsRNA

<400> 120
ugacuaaagu aaguuaaac 19

<210> 121
<211> 19
<212> RNA

<213> Artificial sequence

<220>
<223> Description of the artificial sequence: sense strand of dsRNA

<400> 121
ugcuguucgg auagaacag 19

<210> 122
<211> 19
<212> RNA
<213> Artificial sequence

<220>
<223> Description of the artificial sequence: sense strand of dsRNA

<400> 122
gcgaguauca gaggauggg 19

<210> 123
<211> 19
<212> RNA
<213> Artificial sequence

<220>
<223> Description of the artificial sequence: sense strand of dsRNA

<400> 123
gggccuugcg cuggauugg 19

<210> 124
<211> 19
<212> RNA
<213> Artificial sequence

<220>
<223> Description of the artificial sequence: sense strand of dsRNA

<400> 124
accucacaac caguccugu 19

<210> 125
<211> 19
<212> RNA
<213> Artificial sequence

<220>
<223> Description of the artificial sequence: sense strand of dsRNA

<400> 125
acuaagugac uaaaguaag 19

<210> 126
<211> 19
<212> RNA
<213> Artificial sequence

<220>

<223> Description of the artificial sequence: sense strand of dsRNA

<400> 126
auuacagaag cccgcuguu 19

<210> 127
<211> 19
<212> RNA
<213> Artificial sequence

<220>
<223> Description of the artificial sequence: sense strand of dsRNA

<400> 127
gaguaggcga guaucagag 19

<210> 128
<211> 19
<212> RNA
<213> Artificial sequence

<220>
<223> Description of the artificial sequence: sense strand of dsRNA

<400> 128
cagugacaau ggcagucuu 19

<210> 129
<211> 19
<212> RNA
<213> Artificial sequence

<220>
<223> Description of the artificial sequence: sense strand of dsRNA

<400> 129
ggccuugcgc uggauuggg 19

<210> 130
<211> 19
<212> RNA
<213> Artificial sequence

<220>
<223> Description of the artificial sequence: sense strand of dsRNA

<400> 130
uucuuauuga cacuuacau 19

<210> 131
<211> 19
<212> RNA
<213> Artificial sequence

<220>
<223> Description of the artificial sequence: sense strand of dsRNA

<400> 131

uucacuaagu gacuaaagu 19

<210> 132
<211> 19
<212> RNA
<213> Artificial sequence

<220>
<223> Description of the artificial sequence: sense strand of dsRNA

<400> 132
gugugaguua auucauuua 19

<210> 133
<211> 19
<212> RNA
<213> Artificial sequence

<220>
<223> Description of the artificial sequence: sense strand of dsRNA

<400> 133
cccgcucgcg cccaucacg 19

<210> 134
<211> 19
<212> RNA
<213> Artificial sequence

<220>
<223> Description of the artificial sequence: sense strand of dsRNA

<400> 134
guaaguuaaa cuuguguag 19

<210> 135
<211> 19
<212> RNA
<213> Artificial sequence

<220>
<223> Description of the artificial sequence: sense strand of dsRNA

<400> 135
cggaaguugg aaucagguu 19

<210> 136
<211> 19
<212> RNA
<213> Artificial sequence

<220>
<223> Description of the artificial sequence: sense strand of dsRNA

<400> 136
augugaggau uaacuucug 19

<210> 137

<211> 19
<212> RNA
<213> Artificial sequence

<220>
<223> Description of the artificial sequence: sense strand of dsRNA

<400> 137
uuaaguggug aaaucaacu 19

<210> 138
<211> 19
<212> RNA
<213> Artificial sequence

<220>
<223> Description of the artificial sequence: sense strand of dsRNA

<400> 138
uguagacuaa gcauguaau 19

<210> 139
<211> 19
<212> RNA
<213> Artificial sequence

<220>
<223> Description of the artificial sequence: sense strand of dsRNA

<400> 139
auaaugugag gauuaacuu 19

<210> 140
<211> 19
<212> RNA
<213> Artificial sequence

<220>
<223> Description of the artificial sequence: sense strand of dsRNA

<400> 140
ggcuggcugu gacuuacca 19

<210> 141
<211> 19
<212> RNA
<213> Artificial sequence

<220>
<223> Description of the artificial sequence: sense strand of dsRNA

<400> 141
aagaggcuac cuaugguga 19

<210> 142
<211> 19
<212> RNA
<213> Artificial sequence

<220>
<223> Description of the artificial sequence: sense strand of dsRNA

<400> 142
cagauuacag aagcccgcu 19

<210> 143
<211> 19
<212> RNA
<213> Artificial sequence

<220>
<223> Description of the artificial sequence: sense strand of dsRNA

<400> 143
ugaggccuug ccugugaag 19

<210> 144
<211> 19
<212> RNA
<213> Artificial sequence

<220>
<223> Description of the artificial sequence: sense strand of dsRNA

<400> 144
auaauuauca augcuguuc 19

<210> 145
<211> 19
<212> RNA
<213> Artificial sequence

<220>
<223> Description of the artificial sequence: sense strand of dsRNA

<400> 145
gugacuuacc auagcagug 19

<210> 146
<211> 19
<212> RNA
<213> Artificial sequence

<220>
<223> Description of the artificial sequence: sense strand of dsRNA

<400> 146
uagggcuacu uugaauuaa 19

<210> 147
<211> 19
<212> RNA
<213> Artificial sequence

<220>
<223> Description of the artificial sequence: sense strand of dsRNA

<400> 147
uggcagaugu auaagaagg 19

<210> 148
<211> 19
<212> RNA
<213> Artificial sequence

<220>
<223> Description of the artificial sequence: sense strand of dsRNA

<400> 148
auagcuugau uuauuuggu 19

<210> 149
<211> 19
<212> RNA
<213> Artificial sequence

<220>
<223> Description of the artificial sequence: sense strand of dsRNA

<400> 149
cagcaagacc gcgaggagg 19

<210> 150
<211> 19
<212> RNA
<213> Artificial sequence

<220>
<223> Description of the artificial sequence: sense strand of dsRNA

<400> 150
gacugaugcc uggccucac 19

<210> 151
<211> 19
<212> RNA
<213> Artificial sequence

<220>
<223> Description of the artificial sequence: sense strand of dsRNA

<400> 151
uuaccuugga ugcugacuu 19

<210> 152
<211> 19
<212> RNA
<213> Artificial sequence

<220>
<223> Description of the artificial sequence: sense strand of dsRNA

<400> 152
auucagcacu gggaauccc 19

<210> 153
<211> 19
<212> RNA
<213> Artificial sequence

<220>
<223> Description of the artificial sequence: sense strand of dsRNA

<400> 153
agcaagaccg cgaggagga 19

<210> 154
<211> 19
<212> RNA
<213> Artificial sequence

<220>
<223> Description of the artificial sequence: sense strand of dsRNA

<400> 154
agggcuacuu ugaauuaau 19

<210> 155
<211> 19
<212> RNA
<213> Artificial sequence

<220>
<223> Description of the artificial sequence: sense strand of dsRNA

<400> 155
uaaguuauug uuaccuaaa 19

<210> 156
<211> 19
<212> RNA
<213> Artificial sequence

<220>
<223> Description of the artificial sequence: sense strand of dsRNA

<400> 156
uuuauagugc ugguaguau 19

<210> 157
<211> 19
<212> RNA
<213> Artificial sequence

<220>
<223> Description of the artificial sequence: sense strand of dsRNA

<400> 157
gcaagaccgc gaggaggau 19

<210> 158
<211> 19
<212> RNA

<213> Artificial sequence

<220>
<223> Description of the artificial sequence: sense strand of dsRNA

<400> 158
ucuauggcuu ccaaauugc 19

<210> 159
<211> 19
<212> RNA
<213> Artificial sequence

<220>
<223> Description of the artificial sequence: sense strand of dsRNA

<400> 159
aaagacuaac uucuuugag 19

<210> 160
<211> 19
<212> RNA
<213> Artificial sequence

<220>
<223> Description of the artificial sequence: sense strand of dsRNA

<400> 160
accaugauau cuggcagau 19

<210> 161
<211> 19
<212> RNA
<213> Artificial sequence

<220>
<223> Description of the artificial sequence: sense strand of dsRNA

<400> 161
gaccauuucc uaaucaguu 19

<210> 162
<211> 19
<212> RNA
<213> Artificial sequence

<220>
<223> Description of the artificial sequence: sense strand of dsRNA

<400> 162
uuaccauagc agugacaau 19

<210> 163
<211> 19
<212> RNA
<213> Artificial sequence

<220>

<223> Description of the artificial sequence: sense strand of dsRNA

<400> 163
aaugugagga uuaacuucu 19

<210> 164
<211> 19
<212> RNA
<213> Artificial sequence

<220>
<223> Description of the artificial sequence: sense strand of dsRNA

<400> 164
uaguguccug ggauucucu 19

<210> 165
<211> 19
<212> RNA
<213> Artificial sequence

<220>
<223> Description of the artificial sequence: sense strand of dsRNA

<400> 165
uguuaagugg ugaaaucaa 19

<210> 166
<211> 19
<212> RNA
<213> Artificial sequence

<220>
<223> Description of the artificial sequence: sense strand of dsRNA

<400> 166
acaaauauuc uuaauaggg 19

<210> 167
<211> 19
<212> RNA
<213> Artificial sequence

<220>
<223> Description of the artificial sequence: sense strand of dsRNA

<400> 167
gcggaaguug gaaucaggu 19

<210> 168
<211> 19
<212> RNA
<213> Artificial sequence

<220>
<223> Description of the artificial sequence: sense strand of dsRNA

<400> 168

aacuugugua gacuaagca 19

<210> 169
<211> 19
<212> RNA
<213> Artificial sequence

<220>
<223> Description of the artificial sequence: sense strand of dsRNA

<400> 169
auucuuaaua gggcuacuu 19

<210> 170
<211> 19
<212> RNA
<213> Artificial sequence

<220>
<223> Description of the artificial sequence: sense strand of dsRNA

<400> 170
ccuaaaguua auccagauu 19

<210> 171
<211> 19
<212> RNA
<213> Artificial sequence

<220>
<223> Description of the artificial sequence: sense strand of dsRNA

<400> 171
uauuguuacc uaaaguuaa 19

<210> 172
<211> 19
<212> RNA
<213> Artificial sequence

<220>
<223> Description of the artificial sequence: sense strand of dsRNA

<400> 172
gugcugguag uaucaccuu 19

<210> 173
<211> 19
<212> RNA
<213> Artificial sequence

<220>
<223> Description of the artificial sequence: sense strand of dsRNA

<400> 173
cugugacuua ccauagcag 19

<210> 174

<211> 19
<212> RNA
<213> Artificial sequence

<220>
<223> Description of the artificial sequence: sense strand of dsRNA

<400> 174
gagcuucuua aguuaaauc 19

<210> 175
<211> 19
<212> RNA
<213> Artificial sequence

<220>
<223> Description of the artificial sequence: sense strand of dsRNA

<400> 175
cuguucggau agaacagga 19

<210> 176
<211> 19
<212> RNA
<213> Artificial sequence

<220>
<223> Description of the artificial sequence: sense strand of dsRNA

<400> 176
guuauuguua ccuaaaguu 19

<210> 177
<211> 19
<212> RNA
<213> Artificial sequence

<220>
<223> Description of the artificial sequence: sense strand of dsRNA

<400> 177
uaaugugagg auuaacuuc 19

<210> 178
<211> 19
<212> RNA
<213> Artificial sequence

<220>
<223> Description of the artificial sequence: sense strand of dsRNA

<400> 178
accacuaaug ggagccaau 19

<210> 179
<211> 19
<212> RNA
<213> Artificial sequence

<220>
<223> Description of the artificial sequence: sense strand of dsRNA

<400> 179
uguguagacu aagcaugua 19

<210> 180
<211> 19
<212> RNA
<213> Artificial sequence

<220>
<223> Description of the artificial sequence: sense strand of dsRNA

<400> 180
ugggccuugc gcuggauug 19

<210> 181
<211> 19
<212> RNA
<213> Artificial sequence

<220>
<223> Description of the artificial sequence: sense strand of dsRNA

<400> 181
aggagcuucu uaaguuaaa 19

<210> 182
<211> 19
<212> RNA
<213> Artificial sequence

<220>
<223> Description of the artificial sequence: sense strand of dsRNA

<400> 182
ggugacccuu uagugagcu 19

<210> 183
<211> 19
<212> RNA
<213> Artificial sequence

<220>
<223> Description of the artificial sequence: sense strand of dsRNA

<400> 183
agaguaggcg aguaucaga 19

<210> 184
<211> 19
<212> RNA
<213> Artificial sequence

<220>
<223> Description of the artificial sequence: sense strand of dsRNA

<400> 184
gcagugacaa uggcagucu 19

<210> 185
<211> 19
<212> RNA
<213> Artificial sequence

<220>
<223> Description of the artificial sequence: sense strand of dsRNA

<400> 185
aaacgaugcc uugugucaa 19

<210> 186
<211> 19
<212> RNA
<213> Artificial sequence

<220>
<223> Description of the artificial sequence: sense strand of dsRNA

<400> 186
ggacugaugc cuggccuca 19

<210> 187
<211> 19
<212> RNA
<213> Artificial sequence

<220>
<223> Description of the artificial sequence: sense strand of dsRNA

<400> 187
ugagagauaa auguugauc 19

<210> 188
<211> 19
<212> RNA
<213> Artificial sequence

<220>
<223> Description of the artificial sequence: sense strand of dsRNA

<400> 188
ugguuucuac accaaauac 19

<210> 189
<211> 19
<212> RNA
<213> Artificial sequence

<220>
<223> Description of the artificial sequence: sense strand of dsRNA

<400> 189
ucucuguaau augauacau 19

<210> 190
<211> 19
<212> RNA
<213> Artificial sequence

<220>
<223> Description of the artificial sequence: sense strand of dsRNA

<400> 190
gagagauaaa uguugaucu 19

<210> 191
<211> 19
<212> RNA
<213> Artificial sequence

<220>
<223> Description of the artificial sequence: sense strand of dsRNA

<400> 191
acucuaauga agcaauaca 19

<210> 192
<211> 19
<212> RNA
<213> Artificial sequence

<220>
<223> Description of the artificial sequence: sense strand of dsRNA

<400> 192
ugaaguguua ccaacuagc 19

<210> 193
<211> 19
<212> RNA
<213> Artificial sequence

<220>
<223> Description of the artificial sequence: sense strand of dsRNA

<400> 193
aaugaagcaa uacauugag 19

<210> 194
<211> 19
<212> RNA
<213> Artificial sequence

<220>
<223> Description of the artificial sequence: sense strand of dsRNA

<400> 194
acgaugccuu gugucaaga 19

<210> 195
<211> 19
<212> RNA

<213> Artificial sequence

<220>
<223> Description of the artificial sequence: sense strand of dsRNA

<400> 195
agaccgcgag gaggaucuu 19

<210> 196
<211> 19
<212> RNA
<213> Artificial sequence

<220>
<223> Description of the artificial sequence: sense strand of dsRNA

<400> 196
uuguuaccua aaguuaauc 19

<210> 197
<211> 19
<212> RNA
<213> Artificial sequence

<220>
<223> Description of the artificial sequence: sense strand of dsRNA

<400> 197
cagaagcccg cuguuucua 19

<210> 198
<211> 19
<212> RNA
<213> Artificial sequence

<220>
<223> Description of the artificial sequence: sense strand of dsRNA

<400> 198
uuugacuuua uggagaaua 19

<210> 199
<211> 19
<212> RNA
<213> Artificial sequence

<220>
<223> Description of the artificial sequence: sense strand of dsRNA

<400> 199
uaccuaaagu uaauccaga 19

<210> 200
<211> 19
<212> RNA
<213> Artificial sequence

<220>

<223> Description of the artificial sequence: sense strand of dsRNA

<400> 200
uucaaacacc ugguacaca 19

<210> 201
<211> 19
<212> RNA
<213> Artificial sequence

<220>
<223> Description of the artificial sequence: sense strand of dsRNA

<400> 201
uugcacucua augaagcaa 19

<210> 202
<211> 19
<212> RNA
<213> Artificial sequence

<220>
<223> Description of the artificial sequence: sense strand of dsRNA

<400> 202
uguuaccuaa aguuaaucc 19

<210> 203
<211> 19
<212> RNA
<213> Artificial sequence

<220>
<223> Description of the artificial sequence: sense strand of dsRNA

<400> 203
cacuaaguga cuaaaguaa 19

<210> 204
<211> 19
<212> RNA
<213> Artificial sequence

<220>
<223> Description of the artificial sequence: sense strand of dsRNA

<400> 204
ugccagauag aagacaggu 19

<210> 205
<211> 19
<212> RNA
<213> Artificial sequence

<220>
<223> Description of the artificial sequence: sense strand of dsRNA

<400> 205

aauguauagu cuucuuauu 19

<210> 206
<211> 19
<212> RNA
<213> Artificial sequence

<220>
<223> Description of the artificial sequence: sense strand of dsRNA

<400> 206
gaccacuaau gggagccaa 19

<210> 207
<211> 19
<212> RNA
<213> Artificial sequence

<220>
<223> Description of the artificial sequence: sense strand of dsRNA

<400> 207
guuaccuaaa guuaaucca 19

<210> 208
<211> 19
<212> RNA
<213> Artificial sequence

<220>
<223> Description of the artificial sequence: sense strand of dsRNA

<400> 208
ugaugccugg ccucacauu 19

<210> 209
<211> 19
<212> RNA
<213> Artificial sequence

<220>
<223> Description of the artificial sequence: sense strand of dsRNA

<400> 209
ccaacuuuaa agucagucc 19

<210> 210
<211> 19
<212> RNA
<213> Artificial sequence

<220>
<223> Description of the artificial sequence: sense strand of dsRNA

<400> 210
uaaacuugug uagacuaag 19

<210> 211

<211> 19
<212> RNA
<213> Artificial sequence

<220>
<223> Description of the artificial sequence: sense strand of dsRNA

<400> 211
aguagguugu gugaguuaa 19

<210> 212
<211> 19
<212> RNA
<213> Artificial sequence

<220>
<223> Description of the artificial sequence: sense strand of dsRNA

<400> 212
guuaaacuug uguagacua 19

<210> 213
<211> 19
<212> RNA
<213> Artificial sequence

<220>
<223> Description of the artificial sequence: sense strand of dsRNA

<400> 213
cugaccacua augggagcc 19

<210> 214
<211> 19
<212> RNA
<213> Artificial sequence

<220>
<223> Description of the artificial sequence: sense strand of dsRNA

<400> 214
uauucuuaau agggcuacu 19

<210> 215
<211> 19
<212> RNA
<213> Artificial sequence

<220>
<223> Description of the artificial sequence: sense strand of dsRNA

<400> 215
guaguguccu gggauucuc 19

<210> 216
<211> 19
<212> RNA
<213> Artificial sequence

<220>
<223> Description of the artificial sequence: sense strand of dsRNA

<400> 216
uaucuggcag auguauaag 19

<210> 217
<211> 19
<212> RNA
<213> Artificial sequence

<220>
<223> Description of the artificial sequence: sense strand of dsRNA

<400> 217
aggcuaccua uggugaacg 19

<210> 218
<211> 19
<212> RNA
<213> Artificial sequence

<220>
<223> Description of the artificial sequence: sense strand of dsRNA

<400> 218
ucagaccauu uccuaauca 19

<210> 219
<211> 19
<212> RNA
<213> Artificial sequence

<220>
<223> Description of the artificial sequence: sense strand of dsRNA

<400> 219
uuaccuaaag uuaauccag 19

<210> 220
<211> 19
<212> RNA
<213> Artificial sequence

<220>
<223> Description of the artificial sequence: sense strand of dsRNA

<400> 220
gguuucuaca ccaaauaca 19

<210> 221
<211> 19
<212> RNA
<213> Artificial sequence

<220>
<223> Description of the artificial sequence: sense strand of dsRNA

<400> 221
guuggugcca gauagaaga 19

<210> 222
<211> 19
<212> RNA
<213> Artificial sequence

<220>
<223> Description of the artificial sequence: sense strand of dsRNA

<400> 222
gcuaccuaug gugaacgug 19

<210> 223
<211> 19
<212> RNA
<213> Artificial sequence

<220>
<223> Description of the artificial sequence: sense strand of dsRNA

<400> 223
ucacuaagug acuaaagua 19

<210> 224
<211> 19
<212> RNA
<213> Artificial sequence

<220>
<223> Description of the artificial sequence: sense strand of dsRNA

<400> 224
uuauuguuac cuaaaguua 19

<210> 225
<211> 19
<212> RNA
<213> Artificial sequence

<220>
<223> Description of the artificial sequence: sense strand of dsRNA

<400> 225
uagcugaaua augugagga 19

<210> 226
<211> 19
<212> RNA
<213> Artificial sequence

<220>
<223> Description of the artificial sequence: sense strand of dsRNA

<400> 226
ugaccacuaa ugggagcca 19

<210> 227
<211> 19
<212> RNA
<213> Artificial sequence

<220>
<223> Description of the artificial sequence: sense strand of dsRNA

<400> 227
guagcuaccu cacaaccag 19

<210> 228
<211> 19
<212> RNA
<213> Artificial sequence

<220>
<223> Description of the artificial sequence: sense strand of dsRNA

<400> 228
ucccgcucgc gcccaucac 19

<210> 229
<211> 19
<212> RNA
<213> Artificial sequence

<220>
<223> Description of the artificial sequence: sense strand of dsRNA

<400> 229
cuuggcuuua aagugaggg 19

<210> 230
<211> 19
<212> RNA
<213> Artificial sequence

<220>
<223> Description of the artificial sequence: sense strand of dsRNA

<400> 230
agaagcccgc uguuucuau 19

<210> 231
<211> 19
<212> RNA
<213> Artificial sequence

<220>
<223> Description of the artificial sequence: sense strand of dsRNA

<400> 231
acuaaaguaa guuaaacuu 19

<210> 232
<211> 19
<212> RNA

<213> Artificial sequence

<220>
<223> Description of the artificial sequence: sense strand of dsRNA

<400> 232
aguaaguuaa acuugugua 19

<210> 233
<211> 19
<212> RNA
<213> Artificial sequence

<220>
<223> Description of the artificial sequence: sense strand of dsRNA

<400> 233
aauaauuauc aaugcuguu 19

<210> 234
<211> 19
<212> RNA
<213> Artificial sequence

<220>
<223> Description of the artificial sequence: antisense strand of dsRNA

<400> 234
auaagucugu cugccacaa 19

<210> 235
<211> 19
<212> RNA
<213> Artificial sequence

<220>
<223> Description of the artificial sequence: antisense strand of dsRNA

<400> 235
cagguguuug aacaucagg 19

<210> 236
<211> 19
<212> RNA
<213> Artificial sequence

<220>
<223> Description of the artificial sequence: antisense strand of dsRNA

<400> 236
aagaauucuc uguuggacu 19

<210> 237
<211> 19
<212> RNA
<213> Artificial sequence

<220>

<223> Description of the artificial sequence: antisense strand of dsRNA

<400> 237
auccucugau acucgccua 19

<210> 238
<211> 19
<212> RNA
<213> Artificial sequence

<220>
<223> Description of the artificial sequence: antisense strand of dsRNA

<400> 238
ccauccucug auacucgcc 19

<210> 239
<211> 19
<212> RNA
<213> Artificial sequence

<220>
<223> Description of the artificial sequence: antisense strand of dsRNA

<400> 239
uguaccaggu guuugaaca 19

<210> 240
<211> 19
<212> RNA
<213> Artificial sequence

<220>
<223> Description of the artificial sequence: antisense strand of dsRNA

<400> 240
gcucacuaaa gggucaccc 19

<210> 241
<211> 19
<212> RNA
<213> Artificial sequence

<220>
<223> Description of the artificial sequence: antisense strand of dsRNA

<400> 241
agaaguuagu cuuuccuuc 19

<210> 242
<211> 19
<212> RNA
<213> Artificial sequence

<220>
<223> Description of the artificial sequence: antisense strand of dsRNA

<400> 242

aagacuauac auuucagaa 19

<210> 243
<211> 19
<212> RNA
<213> Artificial sequence

<220>
<223> Description of the artificial sequence: antisense strand of dsRNA

<400> 243
uugugaggua gcuacacag 19

<210> 244
<211> 19
<212> RNA
<213> Artificial sequence

<220>
<223> Description of the artificial sequence: antisense strand of dsRNA

<400> 244
auugcuucau uagagugca 19

<210> 245
<211> 19
<212> RNA
<213> Artificial sequence

<220>
<223> Description of the artificial sequence: antisense strand of dsRNA

<400> 245
cuccauaaag ucaaauggg 19

<210> 246
<211> 19
<212> RNA
<213> Artificial sequence

<220>
<223> Description of the artificial sequence: antisense strand of dsRNA

<400> 246
auaagaagac uauacauuu 19

<210> 247
<211> 19
<212> RNA
<213> Artificial sequence

<220>
<223> Description of the artificial sequence: antisense strand of dsRNA

<400> 247
cugccacaaa cucaaugua 19

<210> 248

<211> 19
<212> RNA
<213> Artificial sequence

<220>
<223> Description of the artificial sequence: antisense strand of dsRNA

<400> 248
ccacaaacuc aauguauug 19

<210> 249
<211> 19
<212> RNA
<213> Artificial sequence

<220>
<223> Description of the artificial sequence: antisense strand of dsRNA

<400> 249
cagugaggaa cuccuguuc 19

<210> 250
<211> 19
<212> RNA
<213> Artificial sequence

<220>
<223> Description of the artificial sequence: antisense strand of dsRNA

<400> 250
gaaagccaga acaugggau 19

<210> 251
<211> 19
<212> RNA
<213> Artificial sequence

<220>
<223> Description of the artificial sequence: antisense strand of dsRNA

<400> 251
auuaacucac acaaccuac 19

<210> 252
<211> 19
<212> RNA
<213> Artificial sequence

<220>
<223> Description of the artificial sequence: antisense strand of dsRNA

<400> 252
gugucaauaa gaagacuau 19

<210> 253
<211> 19
<212> RNA
<213> Artificial sequence

<220>
<223> Description of the artificial sequence: antisense strand of dsRNA

<400> 253
ugcuucauua gagugcaau 19

<210> 254
<211> 19
<212> RNA
<213> Artificial sequence

<220>
<223> Description of the artificial sequence: antisense strand of dsRNA

<400> 254
auccgaacag cauugauaa 19

<210> 255
<211> 19
<212> RNA
<213> Artificial sequence

<220>
<223> Description of the artificial sequence: antisense strand of dsRNA

<400> 255
ggcaucaguc cucguuucu 19

<210> 256
<211> 19
<212> RNA
<213> Artificial sequence

<220>
<223> Description of the artificial sequence: antisense strand of dsRNA

<400> 256
gucugccaca aacucaaug 19

<210> 257
<211> 19
<212> RNA
<213> Artificial sequence

<220>
<223> Description of the artificial sequence: antisense strand of dsRNA

<400> 257
gauucccagu gcugaaugu 19

<210> 258
<211> 19
<212> RNA
<213> Artificial sequence

<220>
<223> Description of the artificial sequence: antisense strand of dsRNA

<400> 258
accagguguu ugaacauca 19

<210> 259
<211> 19
<212> RNA
<213> Artificial sequence

<220>
<223> Description of the artificial sequence: antisense strand of dsRNA

<400> 259
aggaacuccu guucuaucc 19

<210> 260
<211> 19
<212> RNA
<213> Artificial sequence

<220>
<223> Description of the artificial sequence: antisense strand of dsRNA

<400> 260
uuccuuccag ugaaauauu 19

<210> 261
<211> 19
<212> RNA
<213> Artificial sequence

<220>
<223> Description of the artificial sequence: antisense strand of dsRNA

<400> 261
aguacaaaca auguuuauu 19

<210> 262
<211> 19
<212> RNA
<213> Artificial sequence

<220>
<223> Description of the artificial sequence: antisense strand of dsRNA

<400> 262
agaaagccag aacauggga 19

<210> 263
<211> 19
<212> RNA
<213> Artificial sequence

<220>
<223> Description of the artificial sequence: antisense strand of dsRNA

<400> 263
aacuccuguu cuauccgaa 19

<210> 264
<211> 19
<212> RNA
<213> Artificial sequence

<220>
<223> Description of the artificial sequence: antisense strand of dsRNA

<400> 264
uaacucacac aaccuacuu 19

<210> 265
<211> 19
<212> RNA
<213> Artificial sequence

<220>
<223> Description of the artificial sequence: antisense strand of dsRNA

<400> 265
gauacuacca gcacuauaa 19

<210> 266
<211> 19
<212> RNA
<213> Artificial sequence

<220>
<223> Description of the artificial sequence: antisense strand of dsRNA

<400> 266
uguaaguguc aauaagaag 19

<210> 267
<211> 19
<212> RNA
<213> Artificial sequence

<220>
<223> Description of the artificial sequence: antisense strand of dsRNA

<400> 267
gaaacagcgg gcuucugua 19

<210> 268
<211> 19
<212> RNA
<213> Artificial sequence

<220>
<223> Description of the artificial sequence: antisense strand of dsRNA

<400> 268
aagcucacua aagggucac 19

<210> 269
<211> 19
<212> RNA

<213> Artificial sequence

<220>
<223> Description of the artificial sequence: antisense strand of dsRNA

<400> 269
ugaggaacuc cuguucuau 19

<210> 270
<211> 19
<212> RNA
<213> Artificial sequence

<220>
<223> Description of the artificial sequence: antisense strand of dsRNA

<400> 270
uuuguuugua aagugccag 19

<210> 271
<211> 19
<212> RNA
<213> Artificial sequence

<220>
<223> Description of the artificial sequence: antisense strand of dsRNA

<400> 271
aauguauugc uucauuaga 19

<210> 272
<211> 19
<212> RNA
<213> Artificial sequence

<220>
<223> Description of the artificial sequence: antisense strand of dsRNA

<400> 272
guaaguguca auaagaaga 19

<210> 273
<211> 19
<212> RNA
<213> Artificial sequence

<220>
<223> Description of the artificial sequence: antisense strand of dsRNA

<400> 273
cuccuguucu auccgaaca 19

<210> 274
<211> 19
<212> RNA
<213> Artificial sequence

<220>

<223> Description of the artificial sequence: antisense strand of dsRNA

<400> 274
ugccagauau caugguacu 19

<210> 275
<211> 19
<212> RNA
<213> Artificial sequence

<220>
<223> Description of the artificial sequence: antisense strand of dsRNA

<400> 275
guguaaaccc ucaucucug 19

<210> 276
<211> 19
<212> RNA
<213> Artificial sequence

<220>
<223> Description of the artificial sequence: antisense strand of dsRNA

<400> 276
aggcaucagu ccucguuuc 19

<210> 277
<211> 19
<212> RNA
<213> Artificial sequence

<220>
<223> Description of the artificial sequence: antisense strand of dsRNA

<400> 277
gauacucgcc uacucucuu 19

<210> 278
<211> 19
<212> RNA
<213> Artificial sequence

<220>
<223> Description of the artificial sequence: antisense strand of dsRNA

<400> 278
ucacauuauu cagcuaaug 19

<210> 279
<211> 19
<212> RNA
<213> Artificial sequence

<220>
<223> Description of the artificial sequence: antisense strand of dsRNA

<400> 279

gucaaauggg uucucuacu 19

<210> 280
<211> 19
<212> RNA
<213> Artificial sequence

<220>
<223> Description of the artificial sequence: antisense strand of dsRNA

<400> 280
cauccucuga uacucgccu 19

<210> 281
<211> 19
<212> RNA
<213> Artificial sequence

<220>
<223> Description of the artificial sequence: antisense strand of dsRNA

<400> 281
aaauuacaug cuuagucua 19

<210> 282
<211> 19
<212> RNA
<213> Artificial sequence

<220>
<223> Description of the artificial sequence: antisense strand of dsRNA

<400> 282
ugugaguaca aacaauguu 19

<210> 283
<211> 19
<212> RNA
<213> Artificial sequence

<220>
<223> Description of the artificial sequence: antisense strand of dsRNA

<400> 283
acuugacauc acucccauc 19

<210> 284
<211> 19
<212> RNA
<213> Artificial sequence

<220>
<223> Description of the artificial sequence: antisense strand of dsRNA

<400> 284
cugauuagga aauggucug 19

<210> 285

<211> 19
<212> RNA
<213> Artificial sequence

<220>
<223> Description of the artificial sequence: antisense strand of dsRNA

<400> 285
acagcgggcu ucuguaauc 19

<210> 286
<211> 19
<212> RNA
<213> Artificial sequence

<220>
<223> Description of the artificial sequence: antisense strand of dsRNA

<400> 286
acaaggcauc guuucaaug 19

<210> 287
<211> 19
<212> RNA
<213> Artificial sequence

<220>
<223> Description of the artificial sequence: antisense strand of dsRNA

<400> 287
acccaguucc agcauaagu 19

<210> 288
<211> 19
<212> RNA
<213> Artificial sequence

<220>
<223> Description of the artificial sequence: antisense strand of dsRNA

<400> 288
gcguguucuc cuugucgac 19

<210> 289
<211> 19
<212> RNA
<213> Artificial sequence

<220>
<223> Description of the artificial sequence: antisense strand of dsRNA

<400> 289
aaagaaguua gucuuuccu 19

<210> 290
<211> 19
<212> RNA
<213> Artificial sequence

<220>
<223> Description of the artificial sequence: antisense strand of dsRNA

<400> 290
gauccuccuc gcggucuug 19

<210> 291
<211> 19
<212> RNA
<213> Artificial sequence

<220>
<223> Description of the artificial sequence: antisense strand of dsRNA

<400> 291
agccaagacu gccauuguc 19

<210> 292
<211> 19
<212> RNA
<213> Artificial sequence

<220>
<223> Description of the artificial sequence: antisense strand of dsRNA

<400> 292
ucuucuugac acaaggcau 19

<210> 293
<211> 19
<212> RNA
<213> Artificial sequence

<220>
<223> Description of the artificial sequence: antisense strand of dsRNA

<400> 293
ugcguugaag cagugaggc 19

<210> 294
<211> 19
<212> RNA
<213> Artificial sequence

<220>
<223> Description of the artificial sequence: antisense strand of dsRNA

<400> 294
caggacuggu ugugaggua 19

<210> 295
<211> 19
<212> RNA
<213> Artificial sequence

<220>
<223> Description of the artificial sequence: antisense strand of dsRNA

<400> 295
acucgccuac ucucuucuc 19

<210> 296
<211> 19
<212> RNA
<213> Artificial sequence

<220>
<223> Description of the artificial sequence: antisense strand of dsRNA

<400> 296
ccaguuccag cauaagucu 19

<210> 297
<211> 19
<212> RNA
<213> Artificial sequence

<220>
<223> Description of the artificial sequence: antisense strand of dsRNA

<400> 297
aaacagcggg cuucuguaa 19

<210> 298
<211> 19
<212> RNA
<213> Artificial sequence

<220>
<223> Description of the artificial sequence: antisense strand of dsRNA

<400> 298
aaacccaguu ccagcauaa 19

<210> 299
<211> 19
<212> RNA
<213> Artificial sequence

<220>
<223> Description of the artificial sequence: antisense strand of dsRNA

<400> 299
gaguacaaac aauguuuau 19

<210> 300
<211> 19
<212> RNA
<213> Artificial sequence

<220>
<223> Description of the artificial sequence: antisense strand of dsRNA

<400> 300
aucguuucaa uggcauuga 19

<210> 301
<211> 19
<212> RNA
<213> Artificial sequence

<220>
<223> Description of the artificial sequence: antisense strand of dsRNA

<400> 301
gugauacuac cagcacuau 19

<210> 302
<211> 19
<212> RNA
<213> Artificial sequence

<220>
<223> Description of the artificial sequence: antisense strand of dsRNA

<400> 302
cguugaagca gugaggcug 19

<210> 303
<211> 19
<212> RNA
<213> Artificial sequence

<220>
<223> Description of the artificial sequence: antisense strand of dsRNA

<400> 303
ccucacuuua aagccaaga 19

<210> 304
<211> 19
<212> RNA
<213> Artificial sequence

<220>
<223> Description of the artificial sequence: antisense strand of dsRNA

<400> 304
gcuaugguaa gucacagcc 19

<210> 305
<211> 19
<212> RNA
<213> Artificial sequence

<220>
<223> Description of the artificial sequence: antisense strand of dsRNA

<400> 305
acguucacca uagguagcc 19

<210> 306
<211> 19
<212> RNA

<213> Artificial sequence

<220>
<223> Description of the artificial sequence: antisense strand of dsRNA

<400> 306
cuggaagauc cuccucgcg 19

<210> 307
<211> 19
<212> RNA
<213> Artificial sequence

<220>
<223> Description of the artificial sequence: antisense strand of dsRNA

<400> 307
aaggcaucgu uucaauggc 19

<210> 308
<211> 19
<212> RNA
<213> Artificial sequence

<220>
<223> Description of the artificial sequence: antisense strand of dsRNA

<400> 308
gcguugaagc agugaggcu 19

<210> 309
<211> 19
<212> RNA
<213> Artificial sequence

<220>
<223> Description of the artificial sequence: antisense strand of dsRNA

<400> 309
cagcauaagu cugucugcc 19

<210> 310
<211> 19
<212> RNA
<213> Artificial sequence

<220>
<223> Description of the artificial sequence: antisense strand of dsRNA

<400> 310
uuuaacuuac uuuagucac 19

<210> 311
<211> 19
<212> RNA
<213> Artificial sequence

<220>

<223> Description of the artificial sequence: antisense strand of dsRNA

<400> 311
acuuuaggua acaauaacu 19

<210> 312
<211> 19
<212> RNA
<213> Artificial sequence

<220>
<223> Description of the artificial sequence: antisense strand of dsRNA

<400> 312
uucucccaaa cauaaaggc 19

<210> 313
<211> 19
<212> RNA
<213> Artificial sequence

<220>
<223> Description of the artificial sequence: antisense strand of dsRNA

<400> 313
auggguucuc uacucugaa 19

<210> 314
<211> 19
<212> RNA
<213> Artificial sequence

<220>
<223> Description of the artificial sequence: antisense strand of dsRNA

<400> 314
caggcaucag uccucguuu 19

<210> 315
<211> 19
<212> RNA
<213> Artificial sequence

<220>
<223> Description of the artificial sequence: antisense strand of dsRNA

<400> 315
uccucugaua cucgccuac 19

<210> 316
<211> 19
<212> RNA
<213> Artificial sequence

<220>
<223> Description of the artificial sequence: antisense strand of dsRNA

<400> 316

ccauagaaac agcgggcuu 19

<210> 317
<211> 19
<212> RNA
<213> Artificial sequence

<220>
<223> Description of the artificial sequence: antisense strand of dsRNA

<400> 317
cagggauucc cagugcuga 19

<210> 318
<211> 19
<212> RNA
<213> Artificial sequence

<220>
<223> Description of the artificial sequence: antisense strand of dsRNA

<400> 318
guuaauccuc acauuauuc 19

<210> 319
<211> 19
<212> RNA
<213> Artificial sequence

<220>
<223> Description of the artificial sequence: antisense strand of dsRNA

<400> 319
cauaagucug ucugccaca 19

<210> 320
<211> 19
<212> RNA
<213> Artificial sequence

<220>
<223> Description of the artificial sequence: antisense strand of dsRNA

<400> 320
aagaucaaca uuuaucucu 19

<210> 321
<211> 19
<212> RNA
<213> Artificial sequence

<220>
<223> Description of the artificial sequence: antisense strand of dsRNA

<400> 321
ucugccagau aucauggua 19

<210> 322

<211> 19
<212> RNA
<213> Artificial sequence

<220>
<223> Description of the artificial sequence: antisense strand of dsRNA

<400> 322
uuccauggca auuuggaag 19

<210> 323
<211> 19
<212> RNA
<213> Artificial sequence

<220>
<223> Description of the artificial sequence: antisense strand of dsRNA

<400> 323
ggaagauccu ccucgcggu 19

<210> 324
<211> 19
<212> RNA
<213> Artificial sequence

<220>
<223> Description of the artificial sequence: antisense strand of dsRNA

<400> 324
uaagaagacu auacauuuc 19

<210> 325
<211> 19
<212> RNA
<213> Artificial sequence

<220>
<223> Description of the artificial sequence: antisense strand of dsRNA

<400> 325
guaccaggug uuugaacau 19

<210> 326
<211> 19
<212> RNA
<213> Artificial sequence

<220>
<223> Description of the artificial sequence: antisense strand of dsRNA

<400> 326
cauugaagag aaauucccu 19

<210> 327
<211> 19
<212> RNA
<213> Artificial sequence

<220>
<223> Description of the artificial sequence: antisense strand of dsRNA

<400> 327
auuucaccac uuaacaggg 19

<210> 328
<211> 19
<212> RNA
<213> Artificial sequence

<220>
<223> Description of the artificial sequence: antisense strand of dsRNA

<400> 328
cacaguguaa acccucauc 19

<210> 329
<211> 19
<212> RNA
<213> Artificial sequence

<220>
<223> Description of the artificial sequence: antisense strand of dsRNA

<400> 329
aaaugaauua acucacaca 19

<210> 330
<211> 19
<212> RNA
<213> Artificial sequence

<220>
<223> Description of the artificial sequence: antisense strand of dsRNA

<400> 330
guguuugaac aucaggcaa 19

<210> 331
<211> 19
<212> RNA
<213> Artificial sequence

<220>
<223> Description of the artificial sequence: antisense strand of dsRNA

<400> 331
gcuuagucua cacaaguuu 19

<210> 332
<211> 19
<212> RNA
<213> Artificial sequence

<220>
<223> Description of the artificial sequence: antisense strand of dsRNA

<400> 332
agccagaaca ugggauaua 19

<210> 333
<211> 19
<212> RNA
<213> Artificial sequence

<220>
<223> Description of the artificial sequence: antisense strand of dsRNA

<400> 333
acaugcuuag ucuacacaa 19

<210> 334
<211> 19
<212> RNA
<213> Artificial sequence

<220>
<223> Description of the artificial sequence: antisense strand of dsRNA

<400> 334
uguucuaucc gaacagcau 19

<210> 335
<211> 19
<212> RNA
<213> Artificial sequence

<220>
<223> Description of the artificial sequence: antisense strand of dsRNA

<400> 335
ccgaacagca uugauaauu 19

<210> 336
<211> 19
<212> RNA
<213> Artificial sequence

<220>
<223> Description of the artificial sequence: antisense strand of dsRNA

<400> 336
agguguuuga acaucaggc 19

<210> 337
<211> 19
<212> RNA
<213> Artificial sequence

<220>
<223> Description of the artificial sequence: antisense strand of dsRNA

<400> 337
ugccauuguc acugcuaug 19

<210> 338
<211> 19
<212> RNA
<213> Artificial sequence

<220>
<223> Description of the artificial sequence: antisense strand of dsRNA

<400> 338
auaaaugaau uaacucaca 19

<210> 339
<211> 19
<212> RNA
<213> Artificial sequence

<220>
<223> Description of the artificial sequence: antisense strand of dsRNA

<400> 339
aggugauacu accagcacu 19

<210> 340
<211> 19
<212> RNA
<213> Artificial sequence

<220>
<223> Description of the artificial sequence: antisense strand of dsRNA

<400> 340
uauccgaaca gcauugaua 19

<210> 341
<211> 19
<212> RNA
<213> Artificial sequence

<220>
<223> Description of the artificial sequence: antisense strand of dsRNA

<400> 341
aguuuaacuu acuuuaguc 19

<210> 342
<211> 19
<212> RNA
<213> Artificial sequence

<220>
<223> Description of the artificial sequence: antisense strand of dsRNA

<400> 342
guucuauccg aacagcauu 19

<210> 343
<211> 19
<212> RNA

<213> Artificial sequence

<220>
<223> Description of the artificial sequence: antisense strand of dsRNA

<400> 343
ccuuccagug aaauauucu 19

<210> 344
<211> 19
<212> RNA
<213> Artificial sequence

<220>
<223> Description of the artificial sequence: antisense strand of dsRNA

<400> 344
ucuuauacau cugccagau 19

<210> 345
<211> 19
<212> RNA
<213> Artificial sequence

<220>
<223> Description of the artificial sequence: antisense strand of dsRNA

<400> 345
ugauacuacc agcacuaua 19

<210> 346
<211> 19
<212> RNA
<213> Artificial sequence

<220>
<223> Description of the artificial sequence: antisense strand of dsRNA

<400> 346
ccucgcgguc uugcuggcc 19

<210> 347
<211> 19
<212> RNA
<213> Artificial sequence

<220>
<223> Description of the artificial sequence: antisense strand of dsRNA

<400> 347
caucugccag auaucaugg 19

<210> 348
<211> 19
<212> RNA
<213> Artificial sequence

<220>

<223> Description of the artificial sequence: antisense strand of dsRNA

<400> 348
uuagucuaca caaguuuaa 19

<210> 349
<211> 19
<212> RNA
<213> Artificial sequence

<220>
<223> Description of the artificial sequence: antisense strand of dsRNA

<400> 349
ucguuucaau ggcauugaa 19

<210> 350
<211> 19
<212> RNA
<213> Artificial sequence

<220>
<223> Description of the artificial sequence: antisense strand of dsRNA

<400> 350
ugcaaugucu cagcuuucu 19

<210> 351
<211> 19
<212> RNA
<213> Artificial sequence

<220>
<223> Description of the artificial sequence: antisense strand of dsRNA

<400> 351
ggcaauuugg aagccauag 19

<210> 352
<211> 19
<212> RNA
<213> Artificial sequence

<220>
<223> Description of the artificial sequence: antisense strand of dsRNA

<400> 352
uaacuuacuu uagucacuu 19

<210> 353
<211> 19
<212> RNA
<213> Artificial sequence

<220>
<223> Description of the artificial sequence: antisense strand of dsRNA

<400> 353

guuuaacuua cuuuaguca 19

<210> 354
<211> 19
<212> RNA
<213> Artificial sequence

<220>
<223> Description of the artificial sequence: antisense strand of dsRNA

<400> 354
cuguucuauc cgaacagca 19

<210> 355
<211> 19
<212> RNA
<213> Artificial sequence

<220>
<223> Description of the artificial sequence: antisense strand of dsRNA

<400> 355
cccauccucu gauacucgc 19

<210> 356
<211> 19
<212> RNA
<213> Artificial sequence

<220>
<223> Description of the artificial sequence: antisense strand of dsRNA

<400> 356
ccaauccagc gcaaggccc 19

<210> 357
<211> 19
<212> RNA
<213> Artificial sequence

<220>
<223> Description of the artificial sequence: antisense strand of dsRNA

<400> 357
acaggacugg uugugaggu 19

<210> 358
<211> 19
<212> RNA
<213> Artificial sequence

<220>
<223> Description of the artificial sequence: antisense strand of dsRNA

<400> 358
cuuacuuuag ucacuuagu 19

<210> 359

<211> 19
<212> RNA
<213> Artificial sequence

<220>
<223> Description of the artificial sequence: antisense strand of dsRNA

<400> 359
aacagcgggc uucuguaau 19

<210> 360
<211> 19
<212> RNA
<213> Artificial sequence

<220>
<223> Description of the artificial sequence: antisense strand of dsRNA

<400> 360
cucugauacu cgccuacuc 19

<210> 361
<211> 19
<212> RNA
<213> Artificial sequence

<220>
<223> Description of the artificial sequence: antisense strand of dsRNA

<400> 361
aagacugcca uugucacug 19

<210> 362
<211> 19
<212> RNA
<213> Artificial sequence

<220>
<223> Description of the artificial sequence: antisense strand of dsRNA

<400> 362
cccaauccag cgcaaggcc 19

<210> 363
<211> 19
<212> RNA
<213> Artificial sequence

<220>
<223> Description of the artificial sequence: antisense strand of dsRNA

<400> 363
auguaagugu caauaagaa 19

<210> 364
<211> 19
<212> RNA
<213> Artificial sequence

<220>
<223> Description of the artificial sequence: antisense strand of dsRNA

<400> 364
acuuuaguca cuuagugaa 19

<210> 365
<211> 19
<212> RNA
<213> Artificial sequence

<220>
<223> Description of the artificial sequence: antisense strand of dsRNA

<400> 365
uaaaugaauu aacucacac 19

<210> 366
<211> 19
<212> RNA
<213> Artificial sequence

<220>
<223> Description of the artificial sequence: antisense strand of dsRNA

<400> 366
cgugaugggc gcgagcggg 19

<210> 367
<211> 19
<212> RNA
<213> Artificial sequence

<220>
<223> Description of the artificial sequence: antisense strand of dsRNA

<400> 367
cuacacaagu uuaacuuac 19

<210> 368
<211> 19
<212> RNA
<213> Artificial sequence

<220>
<223> Description of the artificial sequence: antisense strand of dsRNA

<400> 368
aaccugauuc caacuuccg 19

<210> 369
<211> 19
<212> RNA
<213> Artificial sequence

<220>
<223> Description of the artificial sequence: antisense strand of dsRNA

<400> 369
cagaaguuaa uccucacau 19

<210> 370
<211> 19
<212> RNA
<213> Artificial sequence

<220>
<223> Description of the artificial sequence: antisense strand of dsRNA

<400> 370
aguugauuuc accacuuaa 19

<210> 371
<211> 19
<212> RNA
<213> Artificial sequence

<220>
<223> Description of the artificial sequence: antisense strand of dsRNA

<400> 371
auuacaugcu uagucuaca 19

<210> 372
<211> 19
<212> RNA
<213> Artificial sequence

<220>
<223> Description of the artificial sequence: antisense strand of dsRNA

<400> 372
aaguuaaucc ucacauuau 19

<210> 373
<211> 19
<212> RNA
<213> Artificial sequence

<220>
<223> Description of the artificial sequence: antisense strand of dsRNA

<400> 373
ugguaaguca cagccagcc 19

<210> 374
<211> 19
<212> RNA
<213> Artificial sequence

<220>
<223> Description of the artificial sequence: antisense strand of dsRNA

<400> 374
ucaccauagg uagccucuu 19

<210> 375
<211> 19
<212> RNA
<213> Artificial sequence

<220>
<223> Description of the artificial sequence: antisense strand of dsRNA

<400> 375
agcgggcuuc uguaaucug 19

<210> 376
<211> 19
<212> RNA
<213> Artificial sequence

<220>
<223> Description of the artificial sequence: antisense strand of dsRNA

<400> 376
cuucacaggc aaggccuca 19

<210> 377
<211> 19
<212> RNA
<213> Artificial sequence

<220>
<223> Description of the artificial sequence: antisense strand of dsRNA

<400> 377
gaacagcauu gauaauuau 19

<210> 378
<211> 19
<212> RNA
<213> Artificial sequence

<220>
<223> Description of the artificial sequence: antisense strand of dsRNA

<400> 378
uuaauucaaa guagcccua 19

<210> 379
<211> 19
<212> RNA
<213> Artificial sequence

<220>
<223> Description of the artificial sequence: antisense strand of dsRNA

<400> 379
ccuucuuaua caucugcca 19

<210> 380
<211> 19
<212> RNA

<213> Artificial sequence

<220>
<223> Description of the artificial sequence: antisense strand of dsRNA

<400> 380
accaaauaaa ucaagcuau 19

<210> 381
<211> 19
<212> RNA
<213> Artificial sequence

<220>
<223> Description of the artificial sequence: antisense strand of dsRNA

<400> 381
ccuccucgcg gucuugcug 19

<210> 382
<211> 19
<212> RNA
<213> Artificial sequence

<220>
<223> Description of the artificial sequence: antisense strand of dsRNA

<400> 382
gugaggccag gcaucaguc 19

<210> 383
<211> 19
<212> RNA
<213> Artificial sequence

<220>
<223> Description of the artificial sequence: antisense strand of dsRNA

<400> 383
aagucagcau ccaagguaa 19

<210> 384
<211> 19
<212> RNA
<213> Artificial sequence

<220>
<223> Description of the artificial sequence: antisense strand of dsRNA

<400> 384
gggauuccca gugcugaau 19

<210> 385
<211> 19
<212> RNA
<213> Artificial sequence

<220>

<223> Description of the artificial sequence: antisense strand of dsRNA

<400> 385
uccuccucgc ggucuugcu 19

<210> 386
<211> 19
<212> RNA
<213> Artificial sequence

<220>
<223> Description of the artificial sequence: antisense strand of dsRNA

<400> 386
auuaauucaa aguagcccu 19

<210> 387
<211> 19
<212> RNA
<213> Artificial sequence

<220>
<223> Description of the artificial sequence: antisense strand of dsRNA

<400> 387
uuuagguaac aauaacuua 19

<210> 388
<211> 19
<212> RNA
<213> Artificial sequence

<220>
<223> Description of the artificial sequence: antisense strand of dsRNA

<400> 388
auacuaccag cacuauaaa 19

<210> 389
<211> 19
<212> RNA
<213> Artificial sequence

<220>
<223> Description of the artificial sequence: antisense strand of dsRNA

<400> 389
auccuccucg cggucuugc 19

<210> 390
<211> 19
<212> RNA
<213> Artificial sequence

<220>
<223> Description of the artificial sequence: antisense strand of dsRNA

<400> 390

gcaauuugga agccauaga 19

<210> 391
<211> 19
<212> RNA
<213> Artificial sequence

<220>
<223> Description of the artificial sequence: antisense strand of dsRNA

<400> 391
cucaaagaag uuagucuuu 19

<210> 392
<211> 19
<212> RNA
<213> Artificial sequence

<220>
<223> Description of the artificial sequence: antisense strand of dsRNA

<400> 392
aucugccaga uaucauggu 19

<210> 393
<211> 19
<212> RNA
<213> Artificial sequence

<220>
<223> Description of the artificial sequence: antisense strand of dsRNA

<400> 393
aacugauuag gaaaugguc 19

<210> 394
<211> 19
<212> RNA
<213> Artificial sequence

<220>
<223> Description of the artificial sequence: antisense strand of dsRNA

<400> 394
auugucacug cuaugguaa 19

<210> 395
<211> 19
<212> RNA
<213> Artificial sequence

<220>
<223> Description of the artificial sequence: antisense strand of dsRNA

<400> 395
agaaguuaau ccucacauu 19

<210> 396

<211> 19
<212> RNA
<213> Artificial sequence

<220>
<223> Description of the artificial sequence: antisense strand of dsRNA

<400> 396
agagaauccc aggacacua 19

<210> 397
<211> 19
<212> RNA
<213> Artificial sequence

<220>
<223> Description of the artificial sequence: antisense strand of dsRNA

<400> 397
uugauuucac cacuuaaca 19

<210> 398
<211> 19
<212> RNA
<213> Artificial sequence

<220>
<223> Description of the artificial sequence: antisense strand of dsRNA

<400> 398
cccuauuaag aauauuugu 19

<210> 399
<211> 19
<212> RNA
<213> Artificial sequence

<220>
<223> Description of the artificial sequence: antisense strand of dsRNA

<400> 399
accugauucc aacuuccgc 19

<210> 400
<211> 19
<212> RNA
<213> Artificial sequence

<220>
<223> Description of the artificial sequence: antisense strand of dsRNA

<400> 400
ugcuuagucu acacaaguu 19

<210> 401
<211> 19
<212> RNA
<213> Artificial sequence

<220>
<223> Description of the artificial sequence: antisense strand of dsRNA

<400> 401
aaguagcccu auuaagaau 19

<210> 402
<211> 19
<212> RNA
<213> Artificial sequence

<220>
<223> Description of the artificial sequence: antisense strand of dsRNA

<400> 402
aaucuggauu aacuuuagg 19

<210> 403
<211> 19
<212> RNA
<213> Artificial sequence

<220>
<223> Description of the artificial sequence: antisense strand of dsRNA

<400> 403
uuaacuuuag guaacaaua 19

<210> 404
<211> 19
<212> RNA
<213> Artificial sequence

<220>
<223> Description of the artificial sequence: antisense strand of dsRNA

<400> 404
aaggugauac uaccagcac 19

<210> 405
<211> 19
<212> RNA
<213> Artificial sequence

<220>
<223> Description of the artificial sequence: antisense strand of dsRNA

<400> 405
cugcuauggu aagucacag 19

<210> 406
<211> 19
<212> RNA
<213> Artificial sequence

<220>
<223> Description of the artificial sequence: antisense strand of dsRNA

<400> 406
gauuuaacuu aagaagcuc 19

<210> 407
<211> 19
<212> RNA
<213> Artificial sequence

<220>
<223> Description of the artificial sequence: antisense strand of dsRNA

<400> 407
uccuguucua uccgaacag 19

<210> 408
<211> 19
<212> RNA
<213> Artificial sequence

<220>
<223> Description of the artificial sequence: antisense strand of dsRNA

<400> 408
aacuuuaggu aacaauaac 19

<210> 409
<211> 19
<212> RNA
<213> Artificial sequence

<220>
<223> Description of the artificial sequence: antisense strand of dsRNA

<400> 409
gaaguuaauc cucacauua 19

<210> 410
<211> 19
<212> RNA
<213> Artificial sequence

<220>
<223> Description of the artificial sequence: antisense strand of dsRNA

<400> 410
auuggcuccc auuaguggu 19

<210> 411
<211> 19
<212> RNA
<213> Artificial sequence

<220>
<223> Description of the artificial sequence: antisense strand of dsRNA

<400> 411
uacaugcuua gucuacaca 19

<210> 412
<211> 19
<212> RNA
<213> Artificial sequence

<220>
<223> Description of the artificial sequence: antisense strand of dsRNA

<400> 412
caauccagcg caaggccca 19

<210> 413
<211> 19
<212> RNA
<213> Artificial sequence

<220>
<223> Description of the artificial sequence: antisense strand of dsRNA

<400> 413
uuuaacuuaa gaagcuccu 19

<210> 414
<211> 19
<212> RNA
<213> Artificial sequence

<220>
<223> Description of the artificial sequence: antisense strand of dsRNA

<400> 414
agcucacuaa agggucacc 19

<210> 415
<211> 19
<212> RNA
<213> Artificial sequence

<220>
<223> Description of the artificial sequence: antisense strand of dsRNA

<400> 415
ucugauacuc gccuacucu 19

<210> 416
<211> 19
<212> RNA
<213> Artificial sequence

<220>
<223> Description of the artificial sequence: antisense strand of dsRNA

<400> 416
agacugccau ugucacugc 19

<210> 417
<211> 19
<212> RNA

<213> Artificial sequence

<220>
<223> Description of the artificial sequence: antisense strand of dsRNA

<400> 417
uugacacaag gcaucguuu 19

<210> 418
<211> 19
<212> RNA
<213> Artificial sequence

<220>
<223> Description of the artificial sequence: antisense strand of dsRNA

<400> 418
ugaggccagg caucagucc 19

<210> 419
<211> 19
<212> RNA
<213> Artificial sequence

<220>
<223> Description of the artificial sequence: antisense strand of dsRNA

<400> 419
gaucaacauu uaucucuca 19

<210> 420
<211> 19
<212> RNA
<213> Artificial sequence

<220>
<223> Description of the artificial sequence: antisense strand of dsRNA

<400> 420
guauuuggug uagaaacca 19

<210> 421
<211> 19
<212> RNA
<213> Artificial sequence

<220>
<223> Description of the artificial sequence: antisense strand of dsRNA

<400> 421
auguaucaua uuacagaga 19

<210> 422
<211> 19
<212> RNA
<213> Artificial sequence

<220>

<223> Description of the artificial sequence: antisense strand of dsRNA

<400> 422
agaucaacau uuaucucuc 19

<210> 423
<211> 19
<212> RNA
<213> Artificial sequence

<220>
<223> Description of the artificial sequence: antisense strand of dsRNA

<400> 423
uguauugcuu cauuagagu 19

<210> 424
<211> 19
<212> RNA
<213> Artificial sequence

<220>
<223> Description of the artificial sequence: antisense strand of dsRNA

<400> 424
gcuaguuggu aacacuuca 19

<210> 425
<211> 19
<212> RNA
<213> Artificial sequence

<220>
<223> Description of the artificial sequence: antisense strand of dsRNA

<400> 425
cucaauguau ugcuucauu 19

<210> 426
<211> 19
<212> RNA
<213> Artificial sequence

<220>
<223> Description of the artificial sequence: antisense strand of dsRNA

<400> 426
ucuugacaca aggcaucgu 19

<210> 427
<211> 19
<212> RNA
<213> Artificial sequence

<220>
<223> Description of the artificial sequence: antisense strand of dsRNA

<400> 427

aagauccucc ucgcggucu 19

<210> 428
<211> 19
<212> RNA
<213> Artificial sequence

<220>
<223> Description of the artificial sequence: antisense strand of dsRNA

<400> 428
gauuaacuuu agguaacaa 19

<210> 429
<211> 19
<212> RNA
<213> Artificial sequence

<220>
<223> Description of the artificial sequence: antisense strand of dsRNA

<400> 429
uagaaacagc gggcuucug 19

<210> 430
<211> 19
<212> RNA
<213> Artificial sequence

<220>
<223> Description of the artificial sequence: antisense strand of dsRNA

<400> 430
uauucuccau aaagucaaa 19

<210> 431
<211> 19
<212> RNA
<213> Artificial sequence

<220>
<223> Description of the artificial sequence: antisense strand of dsRNA

<400> 431
ucuggauuaa cuuuaggua 19

<210> 432
<211> 19
<212> RNA
<213> Artificial sequence

<220>
<223> Description of the artificial sequence: antisense strand of dsRNA

<400> 432
uguguaccag guguuugaa 19

<210> 433

<211> 19
<212> RNA
<213> Artificial sequence

<220>
<223> Description of the artificial sequence: antisense strand of dsRNA

<400> 433
uugcuucauu agagugcaa 19

<210> 434
<211> 19
<212> RNA
<213> Artificial sequence

<220>
<223> Description of the artificial sequence: antisense strand of dsRNA

<400> 434
ggauuaacuu uagguaaca 19

<210> 435
<211> 19
<212> RNA
<213> Artificial sequence

<220>
<223> Description of the artificial sequence: antisense strand of dsRNA

<400> 435
uuacuuuagu cacuuagug 19

<210> 436
<211> 19
<212> RNA
<213> Artificial sequence

<220>
<223> Description of the artificial sequence: antisense strand of dsRNA

<400> 436
accugucuuc uaucuggca 19

<210> 437
<211> 19
<212> RNA
<213> Artificial sequence

<220>
<223> Description of the artificial sequence: antisense strand of dsRNA

<400> 437
aauaagaaga cuauacauu 19

<210> 438
<211> 19
<212> RNA
<213> Artificial sequence

<220>
<223> Description of the artificial sequence: antisense strand of dsRNA

<400> 438
uuggcuccca uuagugguc 19

<210> 439
<211> 19
<212> RNA
<213> Artificial sequence

<220>
<223> Description of the artificial sequence: antisense strand of dsRNA

<400> 439
uggauuaacu uuagguaac 19

<210> 440
<211> 19
<212> RNA
<213> Artificial sequence

<220>
<223> Description of the artificial sequence: antisense strand of dsRNA

<400> 440
aaugugaggc caggcauca 19

<210> 441
<211> 19
<212> RNA
<213> Artificial sequence

<220>
<223> Description of the artificial sequence: antisense strand of dsRNA

<400> 441
ggacugacuu uaaaguugg 19

<210> 442
<211> 19
<212> RNA
<213> Artificial sequence

<220>
<223> Description of the artificial sequence: antisense strand of dsRNA

<400> 442
cuuagucuac acaaguuua 19

<210> 443
<211> 19
<212> RNA
<213> Artificial sequence

<220>
<223> Description of the artificial sequence: antisense strand of dsRNA

<400> 443
uuaacucaca caaccuacu 19

<210> 444
<211> 19
<212> RNA
<213> Artificial sequence

<220>
<223> Description of the artificial sequence: antisense strand of dsRNA

<400> 444
uagucuacac aaguuuaac 19

<210> 445
<211> 19
<212> RNA
<213> Artificial sequence

<220>
<223> Description of the artificial sequence: antisense strand of dsRNA

<400> 445
ggcucccauu aguggucag 19

<210> 446
<211> 19
<212> RNA
<213> Artificial sequence

<220>
<223> Description of the artificial sequence: antisense strand of dsRNA

<400> 446
aguagcccua uuaagaaua 19

<210> 447
<211> 19
<212> RNA
<213> Artificial sequence

<220>
<223> Description of the artificial sequence: antisense strand of dsRNA

<400> 447
gagaauccca ggacacuac 19

<210> 448
<211> 19
<212> RNA
<213> Artificial sequence

<220>
<223> Description of the artificial sequence: antisense strand of dsRNA

<400> 448
cuuauacauc ugccagaua 19

<210> 449
<211> 19
<212> RNA
<213> Artificial sequence

<220>
<223> Description of the artificial sequence: antisense strand of dsRNA

<400> 449
cguucaccau agguagccu 19

<210> 450
<211> 19
<212> RNA
<213> Artificial sequence

<220>
<223> Description of the artificial sequence: antisense strand of dsRNA

<400> 450
ugauuaggaa auggucuga 19

<210> 451
<211> 19
<212> RNA
<213> Artificial sequence

<220>
<223> Description of the artificial sequence: antisense strand of dsRNA

<400> 451
cuggauuaac uuuagguaa 19

<210> 452
<211> 19
<212> RNA
<213> Artificial sequence

<220>
<223> Description of the artificial sequence: antisense strand of dsRNA

<400> 452
uguauuuggu guagaaacc 19

<210> 453
<211> 19
<212> RNA
<213> Artificial sequence

<220>
<223> Description of the artificial sequence: antisense strand of dsRNA

<400> 453
ucuucuaucu ggcaccaac 19

<210> 454
<211> 19
<212> RNA

<213> Artificial sequence

<220>
<223> Description of the artificial sequence: antisense strand of dsRNA

<400> 454
cacguucacc auagguagc 19

<210> 455
<211> 19
<212> RNA
<213> Artificial sequence

<220>
<223> Description of the artificial sequence: antisense strand of dsRNA

<400> 455
uacuuuaguc acuuaguga 19

<210> 456
<211> 19
<212> RNA
<213> Artificial sequence

<220>
<223> Description of the artificial sequence: antisense strand of dsRNA

<400> 456
uaacuuuagg uaacaauaa 19

<210> 457
<211> 19
<212> RNA
<213> Artificial sequence

<220>
<223> Description of the artificial sequence: antisense strand of dsRNA

<400> 457
uccucacauu auucagcua 19

<210> 458
<211> 19
<212> RNA
<213> Artificial sequence

<220>
<223> Description of the artificial sequence: antisense strand of dsRNA

<400> 458
uggcucccau uagugguca 19

<210> 459
<211> 19
<212> RNA
<213> Artificial sequence

<220>

<223> Description of the artificial sequence: antisense strand of dsRNA

<400> 459
cugguuguga gguagcuac 19

<210> 460
<211> 19
<212> RNA
<213> Artificial sequence

<220>
<223> Description of the artificial sequence: antisense strand of dsRNA

<400> 460
gugaugggcg cgagcggga 19

<210> 461
<211> 19
<212> RNA
<213> Artificial sequence

<220>
<223> Description of the artificial sequence: antisense strand of dsRNA

<400> 461
cccucacuuu aaagccaag 19

<210> 462
<211> 19
<212> RNA
<213> Artificial sequence

<220>
<223> Description of the artificial sequence: antisense strand of dsRNA

<400> 462
auagaaacag cgggcuucu 19

<210> 463
<211> 19
<212> RNA
<213> Artificial sequence

<220>
<223> Description of the artificial sequence: antisense strand of dsRNA

<400> 463
aaguuuaacu uacuuuagu 19

<210> 464
<211> 19
<212> RNA
<213> Artificial sequence

<220>
<223> Description of the artificial sequence: antisense strand of dsRNA

<400> 464

uacacaaguu uaacuuacu 19

<210> 465
<211> 19
<212> RNA
<213> Artificial sequence

<220>
<223> Description of the artificial sequence: antisense strand of dsRNA

<400> 465
aacagcauug auaauuauu 19

<210> 466
<211> 19
<212> RNA
<213> Artificial sequence

<220>
<223> Description of the artificial sequence: antisense strand of dsRNA

<400> 466
cacugcuaug guaagucac 19

<210> 467
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 7, 11, 15, 16, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 5, 6, 8, 9, 10, 12, 13, 14, 18
<223> /mod_base = "2'-hydroxy corresponding nucleoside"

<400> 467
uuguggcaga cagacuuaut t 21

<210> 468
<211> 21
<212> DNA

<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 6, 8, 9, 10, 14, 16, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 5, 7, 11, 12, 13, 15, 19
<223> /mod_base = "2'-hydroxy corresponding nucleoside"

<400> 468
ccugauguuc aaacaccugt t 21

<210> 469
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 4, 5, 8, 15, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 6, 7, 9, 10, 11, 12, 13, 14
<223> /mod_base = "2'-hydroxy corresponding nucleoside"

<400> 469
aguccaacag agaauucuut t 21

<210> 470
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 8, 15, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 6, 7, 9, 10, 11, 12, 13, 14
<223> /mod_base = "2'-hydroxy corresponding nucleoside"

<400> 470
aguccaacag agaauucuut t 21

<210> 471
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 5, 9, 11, 12, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 6, 7, 8, 10, 13, 14, 15, 16, 17, 18
<223> /mod_base = "2'-hydroxy corresponding nucleoside"

<400> 471
uaggcgagua ucagaggaut t 21

<210> 472
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 7, 9, 10, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 5, 6, 8, 11, 12, 13, 14, 15, 16, 18, 19
<223> /mod_base = "2'-hydroxy corresponding nucleoside"

<400> 472
ggcgaguauc agaggauggt t 21

<210> 473
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 9, 11, 12, 13, 16, 18
<223> /mod_base = "2'-O-methyl corresponding nucleoside"

<220>
<221> modified_base
<222> 21
<223> /mod_base = "5'-phosphorothioate thymidine"

<220>

<221> modified_base
<222> 6, 7, 8, 10, 14, 15, 17, 19
<223> /mod_base = "2'-hydroxy corresponding nucleoside"

<400> 473
uguucaaaca ccugguacat t 21

<210> 474
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 7, 8, 9, 10, 11, 12, 15, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 5, 6, 13, 14, 16, 17, 18
<223> /mod_base = "2'-hydroxy corresponding nucleoside"

<400> 474
gggugacccu uuagugagct t 21

<210> 475
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 11, 12, 15, 16, 17, 18, 19
<223> /mod_base = "2'-O-methyl corresponding nucleoside"

<220>
<221> modified_base

<222> 21
<223> /mod_base = "5'-phosphorothioate thymidine"

<220>
<221> modified_base
<222> 1, 3, 4, 5, 6, 7, 8, 9, 10, 13, 14
<223> /mod_base = "2'-hydroxy corresponding nucleoside"

<400> 475
gaaggaaaga cuaacuucut t 21

<210> 476
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 11, 12, 15, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 5, 6, 7, 8, 9, 10, 13, 14
<223> /mod_base = "2'-hydroxy corresponding nucleoside"

<400> 476
gaaggaaaga cuaacuucut t 21

<210> 477
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 9, 11, 13, 16, 17, 18, 19

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 6, 7, 8, 10, 12, 14, 15
<223> /mod_base = "2'-hydroxy corresponding nucleoside"

<400> 477
uucugaaaug uauagucuut t 21

<210> 478
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 6, 9, 10, 12, 13, 14, 15, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 5, 7, 8, 11, 16, 18, 19
<223> /mod_base = "2'-hydroxy corresponding nucleoside"

<400> 478
cuguguagcu accucacaat t 21

<210> 479
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 6, 7, 8, 11, 16, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 9, 10, 12, 13, 14, 15, 17, 18
<223> /mod_base = "2'-hydroxy corresponding nucleoside"

<400> 479
ugcacucuaa ugaagcaaut t 21

<210> 480
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 6, 7, 10, 11, 12, 13, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 8, 9, 14, 16, 17, 18, 19
<223> /mod_base = "2'-hydroxy corresponding nucleoside"

<400> 480
cccauuugac uuuauggagt t 21

<210> 481
<211> 21
<212> DNA
<213> Artificial sequence

<220>
<223> Description of the artificial sequence: sense strand of dsRNA

<220>

<221> modified_base
<222> 1
<223> /mod_base = "nucleoside: lacks 5'-phosphate group"

<220>
<221> modified_base
<222> 2, 4, 6, 8, 11, 12, 13, 14, 15, 16, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 5, 7, 9, 10, 18
<223> /mod_base = "2'-hydroxy corresponding nucleoside"

<400> 481
aaauguauag ucuucuuaut t 21

<210> 482
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 6, 10, 11, 12, 14, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 7, 8, 9, 13, 15, 16, 18, 19
<223> /mod_base = "2'-hydroxy corresponding nucleoside"

<400> 482
uacauugagu uuguggcagt t 21

<210> 483
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 6, 8, 9, 13, 14, 15, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 5, 7, 10, 11, 12, 16, 18, 19
<223> /mod_base = "2'-hydroxy corresponding nucleoside"

<400> 483
caauacauug aguuuguggt t 21

<210> 484
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 10, 11, 12, 13, 14, 15, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 5, 6, 7, 8, 9, 16, 19
<223> /mod_base = "2'-hydroxy corresponding nucleoside"

<400> 484
gaacaggagu uccucacugt t 21

<210> 485

<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 4, 5, 7, 9, 10, 11, 12, 15, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 6, 8, 13, 14
<223> /mod_base = "2'-hydroxy corresponding nucleoside"

<400> 485
aucccauguu cuggcuuuct t 21

<210> 486
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 6, 7, 9, 11, 15, 16, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 8, 10, 12, 13, 14, 17, 18
<223> /mod_base = "2'-hydroxy corresponding nucleoside"

<400> 486
guagguugug ugaguuaaut t 21

<210> 487
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 5, 6, 7, 8, 9, 10, 11, 13, 14, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 12, 15, 16, 18
<223> /mod_base = "2'-hydroxy corresponding nucleoside"

<400> 487
auagucuucu uauugacact t 21

<210> 488
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 5, 7, 8, 9, 10, 13, 18
<223> /mod_base = "2'-O-methyl corresponding nucleoside"

<220>
<221> modified_base
<222> 21
<223> /mod_base = "5'-phosphorothioate thymidine"

<220>

<221> modified_base
<222> 1, 4, 6, 11, 12, 14, 15, 16, 17, 19
<223> /mod_base = "2'-hydroxy corresponding nucleoside"

<400> 488
auugcacucu aaugaagcat t 21

<210> 489
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 5, 8, 10, 11, 13, 14, 15, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 6, 7, 9, 12, 16, 17, 18
<223> /mod_base = "2'-hydroxy corresponding nucleoside"

<400> 489
uuaucaaugc uguucggaut t 21

<210> 490
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 6, 12, 13, 16, 18, 19
<223> /mod_base = "2'-O-methyl corresponding nucleoside"

<220>
<221> modified_base

<222> 21
<223> /mod_base = "5'-phosphorothioate thymidine"

<220>
<221> modified_base
<222> 1, 3, 4, 5, 7, 8, 9, 10, 11, 14, 15, 17
<223> /mod_base = "2'-hydroxy corresponding nucleoside"

<400> 490
agaaacgagg acugaugcct t 21

<210> 491
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 8, 9, 10, 12, 15, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 6, 7, 11, 13, 14, 16, 17, 18
<223> /mod_base = "2'-hydroxy corresponding nucleoside"

<400> 491
cauugaguuu guggcagact t 21

<210> 492
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 5, 6, 9, 11, 12, 18, 19

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 7, 8, 10, 13, 14, 15, 16, 17
<223> /mod_base = "2'-hydroxy corresponding nucleoside"

<400> 492
acauucagca cugggaauct t 21

<210> 493
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 6, 7, 8, 12, 14, 15, 16, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 5, 9, 10, 11, 13, 17, 18
<223> /mod_base = "2'-hydroxy corresponding nucleoside"

<400> 493
ugauguucaa acaccuggut t 21

<210> 494
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 9, 15, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 5, 6, 7, 8, 10, 11, 12, 13, 14
<223> /mod_base = "2'-hydroxy corresponding nucleoside"

<400> 494
ggauagaaca ggaguuccut t 21

<210> 495
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 5, 6, 7, 8, 10, 11
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 9, 12, 13, 14, 15, 16, 17, 18, 19
<223> /mod_base = "2'-hydroxy corresponding nucleoside"

<400> 495
aauauuucac uggaaggaat t 21

<210> 496
<211> 21
<212> DNA
<213> Artificial sequence

<220>
<223> Description of the artificial sequence: sense strand of dsRNA

<220>

<221> modified_base
<222> 1
<223> /mod_base = "nucleoside: lacks 5'-phosphate group"

<220>
<221> modified_base
<222> 2, 3, 7, 9, 10, 12, 13, 14, 16, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 5, 6, 8, 11, 15, 17
<223> /mod_base = "2'-hydroxy corresponding nucleoside"

<400> 496
aauaaacauu guuuguacut t 21

<210> 497
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 6, 8, 9, 10, 11, 14, 15, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 7, 12, 13
<223> /mod_base = "2'-hydroxy corresponding nucleoside"

<400> 497
ucccauguuc uggcuuucut t 21

<210> 498
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 7, 12, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 5, 6, 8, 9, 10, 11, 13, 14, 15, 16, 17
<223> /mod_base = "2'-hydroxy corresponding nucleoside"

<400> 498
uucggauaga acaggaguut t 21

<210> 499
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 8, 9, 11, 13, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 5, 6, 7, 10, 12, 14, 15, 16, 19
<223> /mod_base = "2'-hydroxy corresponding nucleoside"

<400> 499
aaguagguug ugugaguuat t 21

<210> 500

<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 7, 9, 10, 13, 16, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 5, 6, 8, 11, 12, 14, 15, 17
<223> /mod_base = "2'-hydroxy corresponding nucleoside"

<400> 500
uuauagugcu gguaguauct t 21

<210> 501
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 6, 8, 9, 12, 14, 15, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 7, 10, 11, 13, 17, 19
<223> /mod_base = "2'-hydroxy corresponding nucleoside"

<400> 501
cuucuuauug acacuuacat t 21

<210> 502
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 9, 10, 11, 13, 14, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 5, 6, 7, 8, 12, 15
<223> /mod_base = "2'-hydroxy corresponding nucleoside"

<400> 502
uacagaagcc cgcuguuuct t 21

<210> 503
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 5, 6, 7, 8, 9, 10, 13, 17, 18, 19
<223> /mod_base = "2'-O-methyl corresponding nucleoside"

<220>
<221> modified_base
<222> 21
<223> /mod_base = "5'-phosphorothioate thymidine"

<220>

<221> modified_base
<222> 1, 3, 4, 11, 12, 14, 15, 16
<223> /mod_base = "2'-hydroxy corresponding nucleoside"

<400> 503
gugacccuuu agugagcuut t 21

<210> 504
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 7, 13, 14, 15, 16, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 8, 9, 10, 11, 12, 19
<223> /mod_base = "2'-hydroxy corresponding nucleoside"

<400> 504
auagaacagg aguuccucat t 21

<210> 505
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 5, 7, 8, 9, 10, 12, 16
<223> /mod_base = "2'-O-methyl corresponding nucleoside"

<220>
<221> modified_base

<222> 21
<223> /mod_base = "5'-phosphorothioate thymidine"

<220>
<221> modified_base
<222> 3, 4, 6, 11, 13, 14, 15, 17, 18, 19
<223> /mod_base = "2'-hydroxy corresponding nucleoside"

<400> 505
cuggcacuuu acaaacaaat t 21

<210> 506
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 6, 11, 14, 16, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 5, 7, 8, 9, 10, 12, 13, 15, 17
<223> /mod_base = "2'-hydroxy corresponding nucleoside"

<400> 506
ucuaaugaag caauacauut t 21

<210> 507
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 6, 7, 9, 10, 13, 15, 16, 17, 19

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 8, 11, 12, 14, 18
<223> /mod_base = "2'-hydroxy corresponding nucleoside"

<400> 507
ucuucuuauu gacacuuact t 21

<210> 508
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 6, 9, 10, 13, 15, 16, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 7, 8, 11, 12, 14, 18
<223> /mod_base = "2'-hydroxy corresponding nucleoside"

<400> 508
ucuucuuauu gacacuuact t 21

<210> 509
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 9, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 6, 7, 8, 10, 11, 12, 13, 15, 16, 17, 18, 19
<223> /mod_base = "2'-hydroxy corresponding nucleoside"

<400> 509
uguucggaua gaacaggagt t 21

<210> 510
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 5, 6, 8, 11, 13, 14, 15, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 7, 9, 10, 12, 16, 17, 19
<223> /mod_base = "2'-hydroxy corresponding nucleoside"

<400> 510
aguaccauga uaucuggcat t 21

<210> 511
<211> 21
<212> DNA
<213> Artificial sequence

<220>
<223> Description of the artificial sequence: sense strand of dsRNA

<220>

<221> modified_base
<222> 1
<223> /mod_base = "nucleoside: lacks 5'-phosphate group"

<220>
<221> modified_base
<222> 1, 2, 7, 13, 14, 15, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 5, 6, 8, 9, 10, 11, 12, 16, 18
<223> /mod_base = "2'-hydroxy corresponding nucleoside"

<400> 511
cagagaugag gguuuacact t 21

<210> 512
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 5, 11, 12, 15, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 6, 7, 8, 9, 10, 13, 14, 16
<223> /mod_base = "2'-hydroxy corresponding nucleoside"

<400> 512
gaaacgagga cugaugccut t 21

<210> 513
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 8, 12, 16, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 7, 9, 10, 11, 13, 14, 15, 17
<223> /mod_base = "2'-hydroxy corresponding nucleoside"

<400> 513
aagagaguag gcgaguauct t    21

<210> 514
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 7, 8, 12, 15, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 6, 9, 10, 11, 13, 14, 16, 18, 19
<223> /mod_base = "2'-hydroxy corresponding nucleoside"

<400> 514
cauuagcuga auaaugugat t    21

<210> 515

<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 10, 11, 12, 14, 15, 16, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 5, 6, 7, 8, 9, 13, 17, 18
<223> /mod_base = "2'-hydroxy corresponding nucleoside"

<400> 515
aguagagaac ccauuugact t 21

<210> 516
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 8, 10, 11, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 5, 6, 7, 9, 12, 13, 14, 15, 16, 17, 19
<223> /mod_base = "2'-hydroxy corresponding nucleoside"

<400> 516
aggcgaguau cagaggaugt t 21

<210> 517
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 5, 6, 10, 12, 14, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 7, 8, 9, 11, 13, 15, 16
<223> /mod_base = "2'-hydroxy corresponding nucleoside"

<400> 517
uagacuaagc auguaauuut t 21

<210> 518
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 5, 6, 8, 9, 10, 12, 14, 15, 16, 18
<223> /mod_base = "2'-O-methyl corresponding nucleoside"

<220>
<221> modified_base
<222> 21
<223> /mod_base = "5'-phosphorothioate thymidine"

<220>

<221> modified_base
<222> 1, 4, 7, 11, 13, 17, 19
<223> /mod_base = "2'-hydroxy corresponding nucleoside"

<400> 518
aacauuguuu guacucacat t 21

<210> 519
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 9, 12, 14, 15, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 5, 6, 7, 8, 10, 11, 13, 16, 17, 18
<223> /mod_base = "2'-hydroxy corresponding nucleoside"

<400> 519
gaugggagug augucaagut t 21

<210> 520
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 5, 6, 8, 9, 10, 11, 12, 13, 16, 17
<223> /mod_base = "2'-O-methyl corresponding nucleoside"

<220>
<221> modified_base

<222> 21
<223> /mod_base = "5'-phosphorothioate thymidine"

<220>
<221> modified_base
<222> 3, 4, 7, 14, 15, 18, 19
<223> /mod_base = "2'-hydroxy corresponding nucleoside"

<400> 520
cagaccauuu ccuaaucagt t 21

<210> 521
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 4, 6, 12, 13, 14, 16, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 5, 7, 8, 9, 10, 11, 15, 18
<223> /mod_base = "2'-hydroxy corresponding nucleoside"

<400> 521
gauuacagaa gcccgcugut t 21

<210> 522
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 9, 12, 14, 15, 16, 17, 19

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 6, 7, 8, 10, 11, 13, 18
<223> /mod_base = "2'-hydroxy corresponding nucleoside"

<400> 522
cauugaaacg augccuugut t 21

<210> 523
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 4, 6, 8, 9, 14, 15, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 5, 7, 10, 11, 12, 13, 16, 17, 18
<223> /mod_base = "2'-hydroxy corresponding nucleoside"

<400> 523
acuuaugcug gaacugggut t 21

<210> 524
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 6, 15, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 5, 7, 8, 9, 10, 11, 12, 13, 14, 16, 18
<223> /mod_base = "2'-hydroxy corresponding nucleoside"

<400> 524
gucgacaagg agaacacgct t 21

<210> 525
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 9, 10, 13, 14, 15, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 7, 8, 11, 12
<223> /mod_base = "2'-hydroxy corresponding nucleoside"

<400> 525
aggaaagacu aacuucuuut t 21

<210> 526
<211> 21
<212> DNA
<213> Artificial sequence

<220>
<223> Description of the artificial sequence: sense strand of dsRNA

<220>

<221> modified_base
<222> 1
<223> /mod_base = "nucleoside: lacks 5'-phosphate group"

<220>
<221> modified_base
<222> 1, 2, 9, 10, 13, 14, 15, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 5, 6, 7, 8, 11, 12
<223> /mod_base = "2'-hydroxy corresponding nucleoside"

<400> 526
aggaaagacu aacuucuuut t 21

<210> 527
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 6, 7, 9, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 5, 8, 10, 11, 12, 13, 14, 15, 16, 17
<223> /mod_base = "2'-hydroxy corresponding nucleoside"

<400> 527
caagaccgcg aggaggauct t 21

<210> 528
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 6, 9, 12, 13, 14, 15, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 5, 7, 8, 10, 11, 16, 17
<223> /mod_base = "2'-hydroxy corresponding nucleoside"

<400> 528
gacaauggca gucuuggcut t 21

<210> 529
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 5, 6, 7, 9, 11, 12
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 8, 10, 13, 14, 15, 16, 17, 18, 19
<223> /mod_base = "2'-hydroxy corresponding nucleoside"

<400> 529
augccuugug ucaagaagat t 21

<210> 530

<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 4, 5, 7, 8, 10, 11, 12, 13, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 6, 9, 14, 15, 17, 19
<223> /mod_base = "2'-hydroxy corresponding nucleoside"

<400> 530
gccucacugc uucaacgcat t 21

<210> 531
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 6, 8, 11, 12, 15, 16, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 7, 9, 10, 13, 14, 19
<223> /mod_base = "2'-hydroxy corresponding nucleoside"

<400> 531
uaccucacaa ccaguccugt t 21

<210> 532
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 11, 15, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 7, 8, 9, 10, 12, 13, 14, 16, 17, 18
<223> /mod_base = "2'-hydroxy corresponding nucleoside"

<400> 532
gagaagagag uaggcgagut t 21

<210> 533
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 5, 6, 8, 10, 11, 16, 17
<223> /mod_base = "2'-O-methyl corresponding nucleoside"

<220>
<221> modified_base
<222> 21
<223> /mod_base = "5'-phosphorothioate thymidine"

<220>

<221> modified_base
<222> 1, 3, 7, 9, 12, 13, 14, 15, 18, 19
<223> /mod_base = "2'-hydroxy corresponding nucleoside"

<400> 533
agacuuaugc uggaacuggt t 21

<210> 534
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 10, 11, 12, 14, 15, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 5, 6, 7, 8, 9, 13, 16
<223> /mod_base = "2'-hydroxy corresponding nucleoside"

<400> 534
uuacagaagc ccgcuguuut t 21

<210> 535
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 6, 7, 12, 13, 17, 18, 19
<223> /mod_base = "2'-O-methyl corresponding nucleoside"

<220>
<221> modified_base

<222> 21
<223> /mod_base = "5'-phosphorothioate thymidine"

<220>
<221> modified_base
<222> 3, 5, 8, 9, 10, 11, 14, 15, 16
<223> /mod_base = "2'-hydroxy corresponding nucleoside"

<400> 535
uuaugcugga acuggguuut t 21

<210> 536
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 6, 8, 9, 11, 12, 13, 15, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 7, 10, 14, 16
<223> /mod_base = "2'-hydroxy corresponding nucleoside"

<400> 536
auaaacauug uuuguacuct t 21

<210> 537
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 5, 7, 8, 10, 11, 16, 19

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 6, 9, 12, 13, 14, 15, 17, 18
<223> /mod_base = "2'-hydroxy corresponding nucleoside"

<400> 537
ucaaugccau ugaaacgaut t 21

<210> 538
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 5, 7, 8, 11, 14, 16, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 6, 9, 10, 12, 13, 15, 18
<223> /mod_base = "2'-hydroxy corresponding nucleoside"

<400> 538
auagugcugg uaguaucact t 21

<210> 539
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 5, 6, 7, 9, 10, 12, 13, 14, 15, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 8, 11, 16, 17, 19
<223> /mod_base = "2'-hydroxy corresponding nucleoside"

<400> 539
cagccucacu gcuucaacgt t 21

<210> 540
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 7, 8, 9, 10, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 6, 11, 12, 13, 14, 16, 17, 18, 19
<223> /mod_base = "2'-hydroxy corresponding nucleoside"

<400> 540
ucuuggcuuu aaagugaggt t 21

<210> 541
<211> 21
<212> DNA
<213> Artificial sequence

<220>
<223> Description of the artificial sequence: sense strand of dsRNA

<220>

<221> modified_base
<222> 1
<223> /mod_base = "nucleoside: lacks 5'-phosphate group"

<220>
<221> modified_base
<222> 2, 3, 4, 6, 9, 10, 11, 13, 14, 16, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 5, 7, 8, 12, 15, 17, 18
<223> /mod_base = "2'-hydroxy corresponding nucleoside"

<400> 541
ggcugugacu uaccauagct t 21

<210> 542
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 4, 6, 7, 8, 10, 13, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 5, 9, 11, 12, 14, 15, 16, 18
<223> /mod_base = "2'-hydroxy corresponding nucleoside"

<400> 542
ggcuaccuau ggugaacgut t 21

<210> 543
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 12, 13, 14, 15, 16, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 5, 6, 7, 8, 9, 10, 11, 18, 19
<223> /mod_base = "2'-hydroxy corresponding nucleoside"

<400> 543
cgcgaggagg aucuuccagt t 21

<210> 544
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 5, 6, 11, 14, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 7, 8, 9, 10, 12, 13, 15
<223> /mod_base = "2'-hydroxy corresponding nucleoside"

<400> 544
gccauugaaa cgaugccuut t 21

<210> 545

<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 4, 5, 6, 8, 9, 11, 12, 13, 14, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 7, 10, 15, 16, 18
<223> /mod_base = "2'-hydroxy corresponding nucleoside"

<400> 545
agccucacug cuucaacgct t 21

<210> 546
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 7, 11, 12, 13, 15, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 5, 6, 8, 9, 10, 14, 16, 19
<223> /mod_base = "2'-hydroxy corresponding nucleoside"

<400> 546
ggcagacaga cuuaugcugt t 21

<210> 547
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 5, 6, 11, 15, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 7, 8, 9, 10, 12, 13, 14, 17, 18, 19
<223> /mod_base = "2'-hydroxy corresponding nucleoside"

<400> 547
gugacuaaag uaaguuaaat t 21

<210> 548
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 4, 6, 7, 9, 10, 12, 13, 14, 19
<223> /mod_base = "2'-O-methyl corresponding nucleoside"

<220>
<221> modified_base
<222> 21
<223> /mod_base = "5'-phosphorothioate thymidine"

<220>

<221> modified_base
<222> 1, 5, 8, 11, 15, 16, 17, 18
<223> /mod_base = "2'-hydroxy corresponding nucleoside"

<400> 548
aguuauuguu accuaaagut t 21

<210> 549
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 4, 5, 6, 8, 10, 11, 12
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 7, 9, 13, 14, 15, 16, 17, 18, 19
<223> /mod_base = "2'-hydroxy corresponding nucleoside"

<400> 549
gccuuuaugu uugggagaat t 21

<210> 550
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 8, 15, 16, 17, 19
<223> /mod_base = "2'-O-methyl corresponding nucleoside"

<220>
<221> modified_base

<222> 21
<223> /mod_base = "5'-phosphorothioate thymidine"

<220>
<221> modified_base
<222> 4, 5, 6, 7, 9, 10, 11, 12, 13, 14, 18
<223> /mod_base = "2'-hydroxy corresponding nucleoside"

<400> 550
uucagaguag agaacccaut t 21

<210> 551
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 10, 11, 14, 16, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 5, 6, 7, 8, 9, 12, 13, 15, 19
<223> /mod_base = "2'-hydroxy corresponding nucleoside"

<400> 551
aaacgaggac ugaugccugt t 21

<210> 552
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 6, 10, 12, 13

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 7, 8, 9, 11, 14, 15, 16, 17, 18, 19
<223> /mod_base = "2'-hydroxy corresponding nucleoside"

<400> 552
guaggcgagu aucagaggat t 21

<210> 553
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 6, 10, 12, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 5, 7, 8, 9, 11, 14, 15, 16, 17, 18, 19
<223> /mod_base = "2'-hydroxy corresponding nucleoside"

<400> 553
guaggcgagu aucagaggat t 21

<210> 554
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 5, 6, 8, 9, 11, 12, 13, 14, 15, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 7, 10, 16, 18, 19
<223> /mod_base = "2'-hydroxy corresponding nucleoside"

<400> 554
aagcccgcug uuucuauggt t 21

<210> 555
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 5, 7, 8, 14, 15, 16, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 6, 9, 10, 11, 12, 13, 19
<223> /mod_base = "2'-hydroxy corresponding nucleoside"

<400> 555
ucagcacugg gaaucccugt t 21

<210> 556
<211> 21
<212> DNA
<213> Artificial sequence

<220>
<223> Description of the artificial sequence: sense strand of dsRNA

<220>

<221> modified_base
<222> 1
<223> /mod_base = "nucleoside: lacks 5'-phosphate group"

<220>
<221> modified_base
<222> 2, 4, 7, 9, 15, 16, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 5, 6, 8, 10, 11, 12, 13, 14, 17, 18
<223> /mod_base = "2'-hydroxy corresponding nucleoside"

<400> 556
gaauaaugug aggauuaact t 21

<210> 557
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 6, 10, 14, 15, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 5, 7, 8, 9, 11, 12, 13, 17, 19
<223> /mod_base = "2'-hydroxy corresponding nucleoside"

<400> 557
uguggcagac agacuuaugt t 21

<210> 558
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 6, 10, 12, 13, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 7, 8, 9, 11, 14, 15
<223> /mod_base = "2'-hydroxy corresponding nucleoside"

<400> 558
agagauaaau guugaucuut t 21

<210> 559
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 6, 9, 11, 12, 13, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 7, 8, 10, 14, 15, 17, 18, 19
<223> /mod_base = "2'-hydroxy corresponding nucleoside"

<400> 559
uaccaugaua ucuggcagat t 21

<210> 560

<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 9, 10, 12, 13, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 6, 7, 8, 11, 14, 16, 17, 18, 19
<223> /mod_base = "2'-hydroxy corresponding nucleoside"

<400> 560
cuuccaaauu gccauggaat t     21

<210> 561
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 5, 14, 15, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 6, 7, 8, 9, 10, 11, 12, 13
<223> /mod_base = "2'-hydroxy corresponding nucleoside"

<400> 561
accgcgagga ggaucuucct t 21

<210> 562
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 5, 7, 9, 12, 13, 14, 15, 16, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 6, 8, 10, 11, 19
<223> /mod_base = "2'-hydroxy corresponding nucleoside"

<400> 562
gaaauguaua gucuucuuat t 21

<210> 563
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 5, 6, 10, 12, 13, 14, 17, 19
<223> /mod_base = "2'-O-methyl corresponding nucleoside"

<220>
<221> modified_base
<222> 21
<223> /mod_base = "5'-phosphorothioate thymidine"

<220>

<221> modified_base
<222> 1, 3, 7, 8, 9, 11, 15, 16, 18
<223> /mod_base = "2'-hydroxy corresponding nucleoside"

<400> 563
auguucaaac accugguact t 21

<210> 564
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 7, 8, 9, 10, 11, 12, 13, 14, 15, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 16, 17, 19
<223> /mod_base = "2'-hydroxy corresponding nucleoside"

<400> 564
agggaauuuc ucuucaaugt t 21

<210> 565
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 6, 7, 11, 14, 19
<223> /mod_base = "2'-O-methyl corresponding nucleoside"

<220>
<221> modified_base

<222> 21
<223> /mod_base = "5'-phosphorothioate thymidine"

<220>
<221> modified_base
<222> 5, 8, 9, 10, 12, 13, 15, 16, 17, 18
<223> /mod_base = "2'-hydroxy corresponding nucleoside"

<400> 565
cccuguuaag uggugaaaut t 21

<210> 566
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 9, 10, 11, 13, 15, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 5, 6, 7, 8, 12, 14, 17, 19
<223> /mod_base = "2'-hydroxy corresponding nucleoside"

<400> 566
gaugaggguu uacacugugt t 21

<210> 567
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 9, 10, 13, 14, 15, 17, 18, 19

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 6, 7, 8, 11, 12, 16
<223> /mod_base = "2'-hydroxy corresponding nucleoside"

<400> 567
ugugugaguu aauucauuut t 21

<210> 568
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 5, 6, 9, 11, 12, 13, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 7, 8, 10, 14, 15, 16, 18
<223> /mod_base = "2'-hydroxy corresponding nucleoside"

<400> 568
uugccugaug uucaaacact t 21

<210> 569
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 5, 6, 8, 10, 14, 15, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 7, 9, 11, 12, 13, 16, 17, 18
<223> /mod_base = "2'-hydroxy corresponding nucleoside"

<400> 569
aaacuugugu agacuaagct t 21

<210> 570
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 6, 7, 8, 10, 12, 13, 14, 15, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 9, 11, 16, 17
<223> /mod_base = "2'-hydroxy corresponding nucleoside"

<400> 570
uauaucccau guucuggcut t 21

<210> 571
<211> 21
<212> DNA
<213> Artificial sequence

<220>
<223> Description of the artificial sequence: sense strand of dsRNA

<220>

<221> modified_base
<222> 1
<223> /mod_base = "nucleoside: lacks 5'-phosphate group"

<220>
<221> modified_base
<222> 1, 2, 4, 6, 10, 11, 15, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 5, 7, 8, 9, 12, 13, 14, 16, 18
<223> /mod_base = "2'-hydroxy corresponding nucleoside"

<400> 571
uuguguagac uaagcaugut t 21

<210> 572
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 5, 7, 8, 9, 13, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 6, 10, 11, 12, 14, 15, 16, 17, 19
<223> /mod_base = "2'-hydroxy corresponding nucleoside"

<400> 572
augcuguucg gauagaacat t 21

<210> 573
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 4, 6, 7, 10, 12, 13, 15, 16, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 5, 8, 9, 11, 14, 18, 19
<223> /mod_base = "2'-hydroxy corresponding nucleoside"

<400> 573
aauuaucaau gcuguucggt t 21

<210> 574
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 4, 7, 9, 10, 11, 15, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 5, 6, 8, 12, 13, 14, 16
<223> /mod_base = "2'-hydroxy corresponding nucleoside"

<400> 574
gccugauguu caaacaccut t 21

<210> 575

<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 6, 9, 12, 15, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 5, 7, 8, 10, 11, 13, 14, 16, 17, 19
<223> /mod_base = "2'-hydroxy corresponding nucleoside"

<400> 575
cauagcagug acaauggcat t 21

<210> 576
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 7, 8, 11, 12, 13, 15, 16, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 5, 6, 9, 10, 14, 18
<223> /mod_base = "2'-hydroxy corresponding nucleoside"

<400> 576
ugugaguuaa uucauuuaut t 21

<210> 577
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 5, 6, 9, 12, 14, 15, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 7, 8, 10, 11, 13, 16
<223> /mod_base = "2'-hydroxy corresponding nucleoside"

<400> 577
agugcuggua guaucaccut t 21

<210> 578
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 7, 9, 10, 12, 13, 14, 18
<223> /mod_base = "2'-O-methyl corresponding nucleoside"

<220>
<221> modified_base
<222> 21
<223> /mod_base = "5'-phosphorothioate thymidine"

<220>

<221> modified_base
<222> 5, 6, 8, 11, 15, 16, 17, 19
<223> /mod_base = "2'-hydroxy corresponding nucleoside"

<400> 578
uaucaaugcu guucggauat t 21

<210> 579
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 4, 9, 13, 14, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 5, 6, 7, 8, 10, 11, 12, 15, 16, 17
<223> /mod_base = "2'-hydroxy corresponding nucleoside"

<400> 579
gacuaaagua aguuaaacut t 21

<210> 580
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 5, 6, 8, 9, 10, 14, 19
<223> /mod_base = "2'-O-methyl corresponding nucleoside"

<220>
<221> modified_base

<222> 21
<223> /mod_base = "5'-phosphorothioate thymidine"

<220>
<221> modified_base
<222> 1, 4, 7, 11, 12, 13, 15, 16, 17, 18
<223> /mod_base = "2'-hydroxy corresponding nucleoside"

<400> 580
aaugcuguuc ggauagaact t 21

<210> 581
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 5, 7, 8, 9, 10, 12, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 6, 11, 14, 15, 16, 17, 18, 19
<223> /mod_base = "2'-hydroxy corresponding nucleoside"

<400> 581
agaauauuuc acuggaaggt t 21

<210> 582
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 4, 7, 11, 13, 15

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 5, 6, 8, 9, 10, 12, 14, 16, 17, 18, 19
<223> /mod_base = "2'-hydroxy corresponding nucleoside"

<400> 582
aucuggcaga uguauaagat t 21

<210> 583
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 6, 8, 9, 12, 15, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 5, 7, 10, 11, 13, 14, 16, 19
<223> /mod_base = "2'-hydroxy corresponding nucleoside"

<400> 583
uauagugcug guaguaucat t 21

<210> 584
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 4, 7, 12, 13, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 5, 6, 8, 9, 10, 11, 14, 16, 17, 18, 19
<223> /mod_base = "2'-hydroxy corresponding nucleoside"

<400> 584
ggccagcaag accgcgaggt t 21

<210> 585
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 7, 9, 10, 11, 14, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 5, 6, 8, 12, 13, 15, 16, 17, 19
<223> /mod_base = "2'-hydroxy corresponding nucleoside"

<400> 585
ccaugauauc uggcagaugt t 21

<210> 586
<211> 21
<212> DNA
<213> Artificial sequence

<220>
<223> Description of the artificial sequence: sense strand of dsRNA

<220>

<221> modified_base
<222> 1
<223> /mod_base = "nucleoside: lacks 5'-phosphate group"

<220>
<221> modified_base
<222> 1, 2, 6, 7, 8, 10, 12, 16, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 5, 9, 11, 13, 14, 15, 18, 19
<223> /mod_base = "2'-hydroxy corresponding nucleoside"

<400> 586
uuaaacuugu guagacuaat t 21

<210> 587
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 6, 8, 9, 11, 12, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 5, 7, 10, 13, 14, 15, 16, 18, 19
<223> /mod_base = "2'-hydroxy corresponding nucleoside"

<400> 587
uucaaugcca uugaaacgat t 21

<210> 588
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 7, 8, 13, 15, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 9, 10, 11, 12, 14, 17, 19
<223> /mod_base = "2'-hydroxy corresponding nucleoside"

<400> 588
agaaagcuga gacauugcat t 21

<210> 589
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 7, 8, 9, 10, 11, 15, 16, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 5, 6, 12, 13, 14, 17
<223> /mod_base = "2'-hydroxy corresponding nucleoside"

<400> 589
cuauggcuuc caaauugcct t 21

<210> 590

<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 7, 8, 13, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 5, 6, 9, 10, 11, 12, 14, 15, 16, 19
<223> /mod_base = "2'-hydroxy corresponding nucleoside"

<400> 590
aagugacuaa aguaaguuat t 21

<210> 591
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 5, 10, 14, 15, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 6, 7, 8, 9, 11, 12, 13, 16, 17, 18
<223> /mod_base = "2'-hydroxy corresponding nucleoside"

<400> 591
ugacuaaagu aaguuaaact t 21

<210> 592
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 6, 7, 8, 12, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 9, 10, 11, 13, 14, 15, 16, 18, 19
<223> /mod_base = "2'-hydroxy corresponding nucleoside"

<400> 592
ugcuguucgg auagaacagt t 21

<210> 593
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 6, 8, 9, 16
<223> /mod_base = "2'-O-methyl corresponding nucleoside"

<220>
<221> modified_base
<222> 21
<223> /mod_base = "5'-phosphorothioate thymidine"

<220>

<221> modified_base
<222> 1, 3, 4, 5, 7, 10, 11, 12, 13, 14, 15, 17, 18, 19
<223> /mod_base = "2'-hydroxy corresponding nucleoside"

<400> 593
gcgaguauca gaggaugggt t 21

<210> 594
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 5, 6, 7, 9, 11, 12, 16, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 8, 10, 13, 14, 15, 18, 19
<223> /mod_base = "2'-hydroxy corresponding nucleoside"

<400> 594
gggccuugcg cuggauuggt t 21

<210> 595
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 4, 5, 7, 10, 11, 14, 15, 16, 17, 19
<223> /mod_base = "2'-O-methyl corresponding nucleoside"

<220>
<221> modified_base

<222> 21
<223> /mod_base = "5'-phosphorothioate thymidine"

<220>
<221> modified_base
<222> 1, 6, 8, 9, 12, 13, 18
<223> /mod_base = "2'-hydroxy corresponding nucleoside"

<400> 595
accucacaac caguccugut t 21

<210> 596
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 7, 10, 11, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 5, 6, 8, 9, 12, 13, 14, 15, 17, 18, 19
<223> /mod_base = "2'-hydroxy corresponding nucleoside"

<400> 596
acuaagugac uaaaguaagt t 21

<210> 597
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 5, 11, 12, 13, 15, 16, 18, 19

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 6, 7, 8, 9, 10, 14, 17
<223> /mod_base = "2'-hydroxy corresponding nucleoside"

<400> 597
auuacagaag cccgcuguut t 21

<210> 598
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 8, 12, 14, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 5, 6, 7, 9, 10, 11, 13, 16, 17, 18, 19
<223> /mod_base = "2'-hydroxy corresponding nucleoside"

<400> 598
gaguaggcga guaucagagt t 21

<210> 599
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 7, 10, 13, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 5, 6, 8, 9, 11, 12, 14, 15
<223> /mod_base = "2'-hydroxy corresponding nucleoside"

<400> 599
cagugacaau ggcagucuut t 21

<210> 600
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 4, 5, 6, 8, 10, 11, 15, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 7, 9, 12, 13, 14, 17, 18, 19
<223> /mod_base = "2'-hydroxy corresponding nucleoside"

<400> 600
ggccuugcgc uggauugggt t 21

<210> 601
<211> 21
<212> DNA
<213> Artificial sequence

<220>
<223> Description of the artificial sequence: sense strand of dsRNA

<220>

<221> modified_base
<222> 1
<223> /mod_base = "nucleoside: lacks 5'-phosphate group"

<220>
<221> modified_base
<222> 1, 2, 3, 4, 5, 7, 8, 11, 13, 14, 15, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 6, 9, 10, 12, 16, 18
<223> /mod_base = "2'-hydroxy corresponding nucleoside"

<400> 601
uucuuauuga cacuuacaut t 21

<210> 602
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 6, 10, 13, 14, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 7, 8, 9, 11, 12, 15, 16, 17, 18
<223> /mod_base = "2'-hydroxy corresponding nucleoside"

<400> 602
uucacuaagu gacuaaagut t 21

<210> 603
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 8, 9, 12, 13, 14, 16, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 5, 6, 7, 10, 11, 15, 19
<223> /mod_base = "2'-hydroxy corresponding nucleoside"

<400> 603
gugugaguua auucauuuat t 21

<210> 604
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 6, 7, 9, 11, 12, 13, 15, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 8, 10, 14, 17, 19
<223> /mod_base = "2'-hydroxy corresponding nucleoside"

<400> 604
cccgcucgcg cccaucacgt t 21

<210> 605

<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 6, 7, 11, 12, 13, 15, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 8, 9, 10, 14, 16, 18, 19
<223> /mod_base = "2'-hydroxy corresponding nucleoside"

<400> 605
guaaguuaaa cuuguguagt t 21

<210> 606
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 7, 8, 13, 14, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 5, 6, 9, 10, 11, 12, 15, 16, 17
<223> /mod_base = "2'-hydroxy corresponding nucleoside"

<400> 606
cggaaguugg aaucagguut t 21

<210> 607
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 10, 11, 14, 15, 16, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 5, 6, 7, 8, 9, 12, 13, 19
<223> /mod_base = "2'-hydroxy corresponding nucleoside"

<400> 607
augugaggau uaacuucugt t 21

<210> 608
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 6, 9, 14, 15, 18, 19
<223> /mod_base = "2'-O-methyl corresponding nucleoside"

<220>
<221> modified_base
<222> 21
<223> /mod_base = "5'-phosphorothioate thymidine"

<220>

<221> modified_base
<222> 3, 4, 5, 7, 8, 10, 11, 12, 13, 16, 17
<223> /mod_base = "2'-hydroxy corresponding nucleoside"

<400> 608
uuaaguggug aaaucaacut t 21

<210> 609
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 7, 8, 12, 14, 16, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 5, 6, 9, 10, 11, 13, 15, 17, 18
<223> /mod_base = "2'-hydroxy corresponding nucleoside"

<400> 609
uguagacuaa gcauguaaut t 21

<210> 610
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 5, 7, 13, 14, 17, 18, 19
<223> /mod_base = "2'-O-methyl corresponding nucleoside"

<220>
<221> modified_base

<222> 21
<223> /mod_base = "5'-phosphorothioate thymidine"

<220>
<221> modified_base
<222> 1, 3, 4, 6, 8, 9, 10, 11, 12, 15, 16
<223> /mod_base = "2'-hydroxy corresponding nucleoside"

<400> 610
auaaugugag gauuaacuut t 21

<210> 611
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 4, 7, 8, 10, 13, 14, 15, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 5, 6, 9, 11, 12, 16, 19
<223> /mod_base = "2'-hydroxy corresponding nucleoside"

<400> 611
ggcuggcugu gacuuaccat t 21

<210> 612
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 7, 8, 10, 11, 12, 14, 17

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 9, 13, 15, 16, 18, 19
<223> /mod_base = "2'-hydroxy corresponding nucleoside"

<400> 612
aagaggcuac cuauggugat t 21

<210> 613
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 5, 6, 8, 14, 15, 16, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 7, 9, 10, 11, 12, 13, 17
<223> /mod_base = "2'-hydroxy corresponding nucleoside"

<400> 613
cagauuacag aagcccgcut t 21

<210> 614
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 6, 7, 8, 9, 11, 12, 13, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 5, 10, 14, 16, 17, 18, 19
<223> /mod_base = "2'-hydroxy corresponding nucleoside"

<400> 614
ugaggccuug ccugugaagt t 21

<210> 615
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 5, 6, 8, 9, 12, 14, 15, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 7, 10, 11, 13, 16
<223> /mod_base = "2'-hydroxy corresponding nucleoside"

<400> 615
auaauuauca augcuguuct t 21

<210> 616
<211> 21
<212> DNA
<213> Artificial sequence

<220>
<223> Description of the artificial sequence: sense strand of dsRNA

<220>

<221> modified_base
<222> 1
<223> /mod_base = "nucleoside: lacks 5'-phosphate group"

<220>
<221> modified_base
<222> 2, 5, 6, 7, 9, 10, 12, 15, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 8, 11, 13, 14, 16, 17, 19
<223> /mod_base = "2'-hydroxy corresponding nucleoside"

<400> 616
gugacuuacc auagcagugt t 21

<210> 617
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 6, 7, 9, 10, 11, 12, 16, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 5, 8, 13, 14, 15, 18, 19
<223> /mod_base = "2'-hydroxy corresponding nucleoside"

<400> 617
uagggcuacu uugaauuaat t 21

<210> 618
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 8, 10, 12
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 5, 6, 7, 9, 11, 13, 14, 15, 16, 17, 18, 19
<223> /mod_base = "2'-hydroxy corresponding nucleoside"

<400> 618
uggcagaugu auaagaaggt t 21

<210> 619
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 5, 6, 7, 10, 11, 12, 14, 15, 16, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 8, 9, 13, 17, 18
<223> /mod_base = "2'-hydroxy corresponding nucleoside"

<400> 619
auagcuugau uuauuuggut t 21

<210> 620

<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5’-phosphate group"

<220>
<221> modified_base
<222> 1, 2, 4, 9, 10, 12
<223> /mod_base = "2’-O-methyl corresponding nucleoside"

<220>
<221> modified_base
<222> 21
<223> /mod_base = "5’-phosphorothioate thymidine"

<220>
<221> modified_base
<222> 3, 5, 6, 7, 8, 11, 13, 14, 15, 16, 17, 18, 19
<223> /mod_base = "2’-hydroxy corresponding nucleoside"

<400> 620
cagcaagacc gcgaggaggt t 21

<210> 621
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5’-phosphate group"

<220>
<221> modified_base
<222> 2, 3, 4, 7, 9, 10, 11, 14, 15, 16, 17, 19
<223> /mod_base = "2’-O-methyl corresponding nucleoside"

<220>
<221> modified_base
<222> 21
<223> /mod_base = "5’-phosphorothioate thymidine"

<220>
<221> modified_base
<222> 1, 5, 6, 8, 12, 13, 18
<223> /mod_base = "2’-hydroxy corresponding nucleoside"

<400> 621
gacugaugcc uggccucact t 21

<210> 622
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 5, 6, 7, 11, 13, 14, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 8, 9, 10, 12, 15, 16
<223> /mod_base = "2'-hydroxy corresponding nucleoside"

<400> 622
uuaccuugga ugcugacuut t 21

<210> 623
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 4, 7, 9, 10, 16, 17, 18, 19
<223> /mod_base = "2'-O-methyl corresponding nucleoside"

<220>
<221> modified_base
<222> 21
<223> /mod_base = "5'-phosphorothioate thymidine"

<220>

<221> modified_base
<222> 1, 5, 6, 8, 11, 12, 13, 14, 15
<223> /mod_base = "2'-hydroxy corresponding nucleoside"

<400> 623
auucagcacu gggaauccct t 21

<210> 624
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 8, 9, 11
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 5, 6, 7, 10, 12, 13, 14, 15, 16, 17, 18, 19
<223> /mod_base = "2'-hydroxy corresponding nucleoside"

<400> 624
agcaagaccg cgaggaggat t 21

<210> 625
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 5, 6, 8, 9, 10, 11, 15, 16, 19
<223> /mod_base = "2'-O-methyl corresponding nucleoside"

<220>
<221> modified_base

<222> 21
<223> /mod_base = "5'-phosphorothioate thymidine"

<220>
<221> modified_base
<222> 1, 3, 4, 7, 12, 13, 14, 17, 18
<223> /mod_base = "2'-hydroxy corresponding nucleoside"

<400> 625
agggcuacuu ugaauuaaut t 21

<210> 626
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 5, 6, 8, 9, 11, 12, 14, 15, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 7, 10, 13, 17, 18, 19
<223> /mod_base = "2'-hydroxy corresponding nucleoside"

<400> 626
uaaguuauug uuaccuaaat t 21

<210> 627
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 8, 10, 11, 14, 17, 19

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 6, 7, 9, 12, 13, 15, 16, 18
<223> /mod_base = "2'-hydroxy corresponding nucleoside"

<400> 627
uuuauagugc ugguaguaut t 21

<210> 628
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 7, 8, 10, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 9, 11, 12, 13, 14, 15, 16, 17, 18
<223> /mod_base = "2'-hydroxy corresponding nucleoside"

<400> 628
gcaagaccgc gaggaggaut t 21

<210> 629
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 8, 9, 10, 11, 12, 16, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 6, 7, 13, 14, 15, 18
<223> /mod_base = "2'-hydroxy corresponding nucleoside"

<400> 629
ucuauggcuu ccaaauugct t 21

<210> 630
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 6, 7, 10, 11, 12, 13, 14, 15, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 8, 9, 17, 18, 19
<223> /mod_base = "2'-hydroxy corresponding nucleoside"

<400> 630
aaagacuaac uucuuugagt t 21

<210> 631
<211> 21
<212> DNA
<213> Artificial sequence

<220>
<223> Description of the artificial sequence: sense strand of dsRNA

<220>

<221> modified_base
<222> 1
<223> /mod_base = "nucleoside: lacks 5'-phosphate group"

<220>
<221> modified_base
<222> 2, 3, 5, 8, 10, 11, 12, 15, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 6, 7, 9, 13, 14, 16, 17, 18
<223> /mod_base = "2'-hydroxy corresponding nucleoside"

<400> 631
accaugauau cuggcagaut t 21

<210> 632
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 4, 6, 7, 8, 9, 10, 11, 14, 15, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 5, 12, 13, 16, 17
<223> /mod_base = "2'-hydroxy corresponding nucleoside"

<400> 632
gaccauuucc uaaucaguut t 21

<210> 633
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 5, 7, 10, 13, 16, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 6, 8, 9, 11, 12, 14, 15, 17, 18
<223> /mod_base = "2'-hydroxy corresponding nucleoside"

<400> 633
uuaccauagc agugacaaut t 21

<210> 634
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 5, 11, 12, 15, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 6, 7, 8, 9, 10, 13, 14
<223> /mod_base = "2'-hydroxy corresponding nucleoside"

<400> 634
aaugugagga uuaacuucut t 21

<210> 635

<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 6, 7, 8, 9, 14, 15, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 5, 10, 11, 12, 13
<223> /mod_base = "2'-hydroxy corresponding nucleoside"

<400> 635
uaguguccug ggauucucut t 21

<210> 636
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 8, 11, 16, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 6, 7, 9, 10, 12, 13, 14, 15, 18, 19
<223> /mod_base = "2'-hydroxy corresponding nucleoside"

<400> 636
uguuaagugg ugaaaucaat t 21

<210> 637
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 6, 8, 9, 10, 11, 12, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 7, 13, 14, 16, 17, 18, 19
<223> /mod_base = "2'-hydroxy corresponding nucleoside"

<400> 637
acaaauauuc uuaauagggt t 21

<210> 638
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 8, 9, 14, 15, 19
<223> /mod_base = "2'-O-methyl corresponding nucleoside"

<220>
<221> modified_base
<222> 21
<223> /mod_base = "5'-phosphorothioate thymidine"

<220>

<221> modified_base
<222> 1, 3, 4, 5, 6, 7, 10, 11, 12, 13, 16, 17, 18
<223> /mod_base = "2'-hydroxy corresponding nucleoside"

<400> 638
gcggaaguug gaaucaggut t 21

<210> 639
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 4, 5, 7, 9, 13, 14, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 6, 8, 10, 11, 12, 15, 16, 17, 19
<223> /mod_base = "2'-hydroxy corresponding nucleoside"

<400> 639
aacuugugua gacuaagcat t 21

<210> 640
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 4, 5, 6, 9, 14, 15, 17, 18, 19
<223> /mod_base = "2'-O-methyl corresponding nucleoside"

<220>
<221> modified_base

<222> 21
<223> /mod_base = "5'-phosphorothioate thymidine"

<220>
<221> modified_base
<222> 1, 7, 8, 10, 11, 12, 13, 16
<223> /mod_base = "2'-hydroxy corresponding nucleoside"

<400> 640
auucuuaaua gggcuacuut t 21

<210> 641
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 8, 9, 12, 13, 14, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 5, 6, 7, 10, 11, 15, 16, 17
<223> /mod_base = "2'-hydroxy corresponding nucleoside"

<400> 641
ccuaaaguua auccagauut t 21

<210> 642
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 6, 7, 9, 10, 11, 16, 17

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 8, 12, 13, 14, 15, 18, 19
<223> /mod_base = "2'-hydroxy corresponding nucleoside"

<400> 642
uauuguuacc uaaaguuaat t 21

<210> 643
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 5, 8, 11, 13, 14, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 6, 7, 9, 10, 12, 15
<223> /mod_base = "2'-hydroxy corresponding nucleoside"

<400> 643
gugcugguag uaucaccuut t 21

<210> 644
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 7, 8, 9, 11, 12, 14, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 5, 6, 10, 13, 15, 16, 18, 19
<223> /mod_base = "2'-hydroxy corresponding nucleoside"

<400> 644
cugugacuua ccauagcagt t 21

<210> 645
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 5, 6, 7, 8, 9, 13, 14, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 10, 11, 12, 15, 16, 17
<223> /mod_base = "2'-hydroxy corresponding nucleoside"

<400> 645
gagcuucuua aguuaaauct t 21

<210> 646
<211> 21
<212> DNA
<213> Artificial sequence

<220>
<223> Description of the artificial sequence: sense strand of dsRNA

<220>

<221> modified_base
<222> 1
<223> /mod_base = "nucleoside: lacks 5'-phosphate group"

<220>
<221> modified_base
<222> 1, 2, 4, 5, 6, 10, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 7, 8, 9, 11, 12, 13, 14, 16, 17, 18, 19
<223> /mod_base = "2'-hydroxy corresponding nucleoside"

<400> 646
cuguucggau agaacaggat t 21

<210> 647
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 5, 6, 8, 9, 11, 12, 13, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 7, 10, 14, 15, 16, 17
<223> /mod_base = "2'-hydroxy corresponding nucleoside"

<400> 647
guuauuguua ccuaaaguut t 21

<210> 648
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 6, 12, 13, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 5, 7, 8, 9, 10, 11, 14, 15
<223> /mod_base = "2'-hydroxy corresponding nucleoside"

<400> 648
uaaugugagg auuaacuuct t 21

<210> 649
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 5, 6, 9, 15, 16, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 7, 8, 10, 11, 12, 13, 14, 17, 18
<223> /mod_base = "2'-hydroxy corresponding nucleoside"

<400> 649
accacuaaug ggagccaaut t 21

<210> 650

<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 9, 10, 14, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 6, 7, 8, 11, 12, 13, 15, 17, 19
<223> /mod_base = "2'-hydroxy corresponding nucleoside"

<400> 650
uguguagacu aagcauguat t 21

<210> 651
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 5, 6, 7, 8, 10, 12, 13, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 9, 11, 14, 15, 16, 19
<223> /mod_base = "2'-hydroxy corresponding nucleoside"

<400> 651
ugggccuugc gcuggauugt t 21

<210> 652
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 6, 7, 8, 9, 10, 11, 15, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 12, 13, 14, 17, 18, 19
<223> /mod_base = "2'-hydroxy corresponding nucleoside"

<400> 652
aggagcuucu uaaguuaaat t 21

<210> 653
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 6, 7, 8, 9, 10, 11, 14, 18, 19
<223> /mod_base = "2'-O-methyl corresponding nucleoside"

<220>
<221> modified_base
<222> 21
<223> /mod_base = "5'-phosphorothioate thymidine"

<220>

<221> modified_base
<222> 1, 4, 5, 12, 13, 15, 16, 17
<223> /mod_base = "2'-hydroxy corresponding nucleoside"

<400> 653
ggugacccuu uagugagcut t 21

<210> 654
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 5, 9, 13, 15, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 6, 7, 8, 10, 11, 12, 14, 17, 18, 19
<223> /mod_base = "2'-hydroxy corresponding nucleoside"

<400> 654
agaguaggcg aguaucagat t 21

<210> 655
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 5, 8, 11, 14, 17, 18, 19
<223> /mod_base = "2'-O-methyl corresponding nucleoside"

<220>
<221> modified_base

<222> 21
<223> /mod_base = "5'-phosphorothioate thymidine"

<220>
<221> modified_base
<222> 1, 3, 4, 6, 7, 9, 10, 12, 13, 15, 16
<223> /mod_base = "2'-hydroxy corresponding nucleoside"

<400> 655
gcagugacaa uggcagucut t 21

<210> 656
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 7, 9, 10, 11, 12, 14, 16, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 5, 6, 8, 13, 15, 18, 19
<223> /mod_base = "2'-hydroxy corresponding nucleoside"

<400> 656
aaacgaugcc uugugucaat t 21

<210> 657
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 5, 8, 10, 11, 12, 15, 16, 17, 18

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 6, 7, 9, 13, 14, 19
<223> /mod_base = "2'-hydroxy corresponding nucleoside"

<400> 657
ggacugaugc cuggccucat t 21

<210> 658
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 8, 12, 14, 15, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 5, 6, 7, 9, 10, 11, 13, 16, 17
<223> /mod_base = "2'-hydroxy corresponding nucleoside"

<400> 658
ugagagauaa auguugauct t 21

<210> 659
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 5, 6, 7, 8, 10, 12, 13, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 9, 11, 14, 15, 16, 18
<223> /mod_base = "2'-hydroxy corresponding nucleoside"

<400> 659
ugguuucuac accaaauact t 21

<210> 660
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 7, 10, 12, 15, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 6, 8, 9, 11, 13, 14, 16, 18
<223> /mod_base = "2'-hydroxy corresponding nucleoside"

<400> 660
ucucuguaau augauacaut t 21

<210> 661
<211> 21
<212> DNA
<213> Artificial sequence

<220>
<223> Description of the artificial sequence: sense strand of dsRNA

<220>

<221> modified_base
<222> 1
<223> /mod_base = "nucleoside: lacks 5'-phosphate group"

<220>
<221> modified_base
<222> 2, 7, 11, 13, 14, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 8, 9, 10, 12, 15, 16
<223> /mod_base = "2'-hydroxy corresponding nucleoside"

<400> 661
gagagauaaa uguugaucut t 21

<210> 662
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 4, 5, 8, 13, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 6, 7, 9, 10, 11, 12, 14, 15, 17, 19
<223> /mod_base = "2'-hydroxy corresponding nucleoside"

<400> 662
acucuaauga agcaauacat t 21

<210> 663
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 6, 8, 9, 11, 12, 15, 16, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 5, 7, 10, 13, 14, 17, 18
<223> /mod_base = "2'-hydroxy corresponding nucleoside"

<400> 663
ugaaguguua ccaacuagct t 21

<210> 664
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 8, 11, 13, 15, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 5, 6, 7, 9, 10, 12, 14, 17, 18, 19
<223> /mod_base = "2'-hydroxy corresponding nucleoside"

<400> 664
aaugaagcaa uacauugagt t 21

<210> 665

<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 5, 7, 8, 9, 10, 12, 14, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 6, 11, 13, 16, 17, 18, 19
<223> /mod_base = "2'-hydroxy corresponding nucleoside"

<400> 665
acgaugccuu gugucaagat t 21

<210> 666
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 5, 7, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 6, 8, 9, 10, 11, 12, 13, 14, 15
<223> /mod_base = "2'-hydroxy corresponding nucleoside"

<400> 666
agaccgcgag gaggaucuut t 21

<210> 667
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 5, 7, 8, 9, 14, 15, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 6, 10, 11, 12, 13, 16, 17
<223> /mod_base = "2'-hydroxy corresponding nucleoside"

<400> 667
uuguuaccua aaguuaauct t 21

<210> 668
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 7, 8, 9, 11, 12, 14, 15, 16, 17, 18
<223> /mod_base = "2'-O-methyl corresponding nucleoside"

<220>
<221> modified_base
<222> 21
<223> /mod_base = "5'-phosphorothioate thymidine"

<220>

<221> modified_base
<222> 3, 4, 5, 6, 10, 13, 19
<223> /mod_base = "2'-hydroxy corresponding nucleoside"

<400> 668
cagaagcccg cuguuucuat t 21

<210> 669
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 6, 7, 8, 9, 11, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 5, 10, 12, 13, 14, 15, 16, 17, 19
<223> /mod_base = "2'-hydroxy corresponding nucleoside"

<400> 669
uuugacuuua uggagaauat t 21

<210> 670
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 10, 11, 14, 15, 16
<223> /mod_base = "2'-O-methyl corresponding nucleoside"

<220>
<221> modified_base

<222> 21
<223> /mod_base = "5'-phosphorothioate thymidine"

<220>
<221> modified_base
<222> 6, 7, 8, 9, 12, 13, 17, 18, 19
<223> /mod_base = "2'-hydroxy corresponding nucleoside"

<400> 670
uaccuaaagu uaauccagat t 21

<210> 671
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 7, 9, 10, 11, 14, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 5, 6, 8, 12, 13, 15, 17, 19
<223> /mod_base = "2'-hydroxy corresponding nucleoside"

<400> 671
uucaaacacc ugguacacat t 21

<210> 672
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 6, 7, 8, 9, 12, 17

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 5, 10, 11, 13, 14, 15, 16, 18, 19
<223> /mod_base = "2'-hydroxy corresponding nucleoside"

<400> 672
uugcacucua augaagcaat t 21

<210> 673
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 6, 7, 8, 13, 14, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 9, 10, 11, 12, 15, 16
<223> /mod_base = "2'-hydroxy corresponding nucleoside"

<400> 673
uguuaccuaa aguuaaucct t 21

<210> 674
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 8, 11, 12, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 6, 7, 9, 10, 13, 14, 15, 16, 18, 19
<223> /mod_base = "2'-hydroxy corresponding nucleoside"

<400> 674
cacuaaguga cuaaaguaat t 21

<210> 675
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 8, 15, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 6, 7, 9, 10, 11, 12, 13, 14, 16, 17, 18
<223> /mod_base = "2'-hydroxy corresponding nucleoside"

<400> 675
ugccagauag aagacaggut t 21

<210> 676
<211> 21
<212> DNA
<213> Artificial sequence

<220>
<223> Description of the artificial sequence: sense strand of dsRNA

<220>

<221> modified_base
<222> 1
<223> /mod_base = "nucleoside: lacks 5'-phosphate group"

<220>
<221> modified_base
<222> 2, 3, 5, 7, 10, 11, 12, 13, 14, 15, 16, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 6, 8, 9, 17
<223> /mod_base = "2'-hydroxy corresponding nucleoside"

<400> 676
aauguauagu cuucuuauut t 21

<210> 677
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 4, 6, 7, 10, 16, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 5, 8, 9, 11, 12, 13, 14, 15, 18, 19
<223> /mod_base = "2'-hydroxy corresponding nucleoside"

<400> 677
gaccacuaau gggagccaat t 21

<210> 678
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 5, 6, 7, 12, 13, 16, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 8, 9, 10, 11, 14, 15, 19
<223> /mod_base = "2'-hydroxy corresponding nucleoside"

<400> 678
guuaccuaaa guuaauccat t 21

<210> 679
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 6, 7, 8, 11, 12, 13, 14, 16, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 5, 9, 10, 15, 17
<223> /mod_base = "2'-hydroxy corresponding nucleoside"

<400> 679
ugaugccugg ccucacauut t 21

<210> 680

<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 5, 6, 7, 8, 13, 14, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 9, 10, 11, 12, 15, 16
<223> /mod_base = "2'-hydroxy corresponding nucleoside"

<400> 680
ccaacuuuaa agucagucct t 21

<210> 681
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 5, 6, 7, 9, 11, 15, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 8, 10, 12, 13, 14, 17, 18, 19
<223> /mod_base = "2'-hydroxy corresponding nucleoside"

<400> 681
uaaacuugug uagacuaagt t 21

<210> 682
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 7, 8, 10, 12, 16, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 5, 6, 9, 11, 13, 14, 15, 18, 19
<223> /mod_base = "2'-hydroxy corresponding nucleoside"

<400> 682
aguagguugu gugaguuaat t 21

<210> 683
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 7, 8, 9, 11, 13, 17, 18
<223> /mod_base = "2'-O-methyl corresponding nucleoside"

<220>
<221> modified_base
<222> 21
<223> /mod_base = "5'-phosphorothioate thymidine"

<220>

<221> modified_base
<222> 1, 4, 5, 6, 10, 12, 14, 15, 16, 19
<223> /mod_base = "2'-hydroxy corresponding nucleoside"

<400> 683
guuaaacuug uguagacuat t 21

<210> 684
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 5, 6, 8, 9, 12, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 7, 10, 11, 13, 14, 15, 16, 17
<223> /mod_base = "2'-hydroxy corresponding nucleoside"

<400> 684
cugaccacua augggagcct t 21

<210> 685
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 6, 7, 10, 15, 16, 18, 19
<223> /mod_base = "2'-O-methyl corresponding nucleoside"

<220>
<221> modified_base

<222> 21
<223> /mod_base = "5'-phosphorothioate thymidine"

<220>
<221> modified_base
<222> 8, 9, 11, 12, 13, 14, 17
<223> /mod_base = "2'-hydroxy corresponding nucleoside"

<400> 685
uauucuuaau agggcuacut t 21

<210> 686
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 5, 7, 8, 9, 10, 15, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 6, 11, 12, 13, 14
<223> /mod_base = "2'-hydroxy corresponding nucleoside"

<400> 686
guaguguccu gggauucuct t 21

<210> 687
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 8, 12, 14, 16

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 6, 7, 9, 10, 11, 13, 15, 17, 18, 19
<223> /mod_base = "2'-hydroxy corresponding nucleoside"

<400> 687
uaucuggcag auguauaagt t 21

<210> 688
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 5, 7, 8, 9, 11, 14, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 6, 10, 12, 13, 15, 16, 17, 19
<223> /mod_base = "2'-hydroxy corresponding nucleoside"

<400> 688
aggcuaccua uggugaacgt t 21

<210> 689
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 6, 7, 9, 10, 11, 12, 13, 14, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 5, 8, 15, 16, 19
<223> /mod_base = "2'-hydroxy corresponding nucleoside"

<400> 689
ucagaccauu uccuaaucat t 21

<210> 690
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 5, 6, 11, 12, 15, 16, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 7, 8, 9, 10, 13, 14, 18, 19
<223> /mod_base = "2'-hydroxy corresponding nucleoside"

<400> 690
uuaccuaaag uuaauccagt t 21

<210> 691
<211> 21
<212> DNA
<213> Artificial sequence

<220>
<223> Description of the artificial sequence: sense strand of dsRNA

<220>

<221> modified_base
<222> 1
<223> /mod_base = "nucleoside: lacks 5'-phosphate group"

<220>
<221> modified_base
<222> 2, 3, 4, 5, 6, 7, 9, 11, 12, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 8, 10, 13, 14, 15, 17, 19
<223> /mod_base = "2'-hydroxy corresponding nucleoside"

<400> 691
gguuucuaca ccaaauacat t 21

<210> 692
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 6, 8, 9, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 5, 7, 10, 11, 12, 14, 15, 16, 17, 18, 19
<223> /mod_base = "2'-hydroxy corresponding nucleoside"

<400> 692
guuggugcca gauagaagat t 21

<210> 693
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 5, 6, 7, 9, 12, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 8, 10, 11, 13, 14, 15, 17, 19
<223> /mod_base = "2'-hydroxy corresponding nucleoside"

<400> 693
gcuaccuaug gugaacgugt t 21

<210> 694
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 5, 9, 12, 13, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 6, 7, 8, 10, 11, 14, 15, 16, 17, 19
<223> /mod_base = "2'-hydroxy corresponding nucleoside"

<400> 694
ucacuaagug acuaaaguat t 21

<210> 695

<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5’-phosphate group"

<220>
<221> modified_base
<222> 1, 2, 4, 5, 7, 8, 10, 11, 12, 17, 18
<223> /mod_base = "2’-O-methyl corresponding nucleoside"

<220>
<221> modified_base
<222> 21
<223> /mod_base = "5’-phosphorothioate thymidine"

<220>
<221> modified_base
<222> 3, 6, 9, 13, 14, 15, 16, 19
<223> /mod_base = "2’-hydroxy corresponding nucleoside"

<400> 695
uuauuguuac cuaaaguuat t 21

<210> 696
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5’-phosphate group"

<220>
<221> modified_base
<222> 1, 2, 4, 5, 9, 12, 14
<223> /mod_base = "2’-O-methyl corresponding nucleoside"

<220>
<221> modified_base
<222> 21
<223> /mod_base = "5’-phosphorothioate thymidine"

<220>
<221> modified_base
<222> 3, 6, 7, 8, 10, 11, 13, 15, 16, 17, 18, 19
<223> /mod_base = "2’-hydroxy corresponding nucleoside"

<400> 696
uagcugaaua augugaggat t 21

<210> 697
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 5, 7, 8, 11, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 6, 9, 10, 12, 13, 14, 15, 16, 19
<223> /mod_base = "2'-hydroxy corresponding nucleoside"

<400> 697
ugaccacuaa ugggagccat t 21

<210> 698
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 5, 6, 8, 9, 10, 11, 13, 16, 17
<223> /mod_base = "2'-O-methyl corresponding nucleoside"

<220>
<221> modified_base
<222> 21
<223> /mod_base = "5'-phosphorothioate thymidine"

<220>

<221> modified_base
<222> 1, 3, 4, 7, 12, 14, 15, 18, 19
<223> /mod_base = "2'-hydroxy corresponding nucleoside"

<400> 698
guagcuaccu cacaaccagt t 21

<210> 699
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 6, 7, 8, 10, 12, 13, 14, 16, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 9, 11, 15, 18
<223> /mod_base = "2'-hydroxy corresponding nucleoside"

<400> 699
ucccgcucgc gcccaucact t 21

<210> 700
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 6, 7, 8, 9, 14
<223> /mod_base = "2'-O-methyl corresponding nucleoside"

<220>
<221> modified_base

<222> 21
<223> /mod_base = "5'-phosphorothioate thymidine"

<220>
<221> modified_base
<222> 4, 5, 10, 11, 12, 13, 15, 16, 17, 18, 19
<223> /mod_base = "2'-hydroxy corresponding nucleoside"

<400> 700
cuuggcuuua aagugagggt t 21

<210> 701
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 6, 7, 8, 10, 11, 13, 14, 15, 16, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 9, 12, 18
<223> /mod_base = "2'-hydroxy corresponding nucleoside"

<400> 701
agaagcccgc uguuucuaut t 21

<210> 702
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 8, 12, 13, 17, 18, 19

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 5, 6, 7, 9, 10, 11, 14, 15, 16
<223> /mod_base = "2'-hydroxy corresponding nucleoside"

<400> 702
acuaaaguaa guuaaacuut t 21

<210> 703
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 7, 8, 12, 13, 14, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 5, 6, 9, 10, 11, 15, 17, 19
<223> /mod_base = "2'-hydroxy corresponding nucleoside"

<400> 703
aguaaguuaa acuuguguat t 21

<210> 704
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 3, 6, 7, 9, 10, 13, 15, 16, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 4, 5, 8, 11, 12, 14, 17
<223> /mod_base = "2'-hydroxy corresponding nucleoside"

<400> 704
aauaauuauc aaugcuguut t 21

<210> 705
<211> 21
<212> DNA
<213> Artificial sequence

<220>
<223> Description of the artificial sequence: antisense strand of dsRNA

<220>
<221> modified_base
<222> 21
<223> /mod_base = "5'-phosphorothioate thymidine"

<220>
<221> modified_base
<222> 1..19
<223> /mod_base = "2'-hydroxy corresponding nucleoside"

<400> 705
gcguguucuc cuugucgact t 21

<210> 706
<211> 21
<212> DNA
<213> Artificial sequence

<220>
<223> Description of the artificial sequence: antisense strand of dsRNA

<220>
<221> modified_base
<222> 21
<223> /mod_base = "5'-phosphorothioate thymidine"

<220>
<221> modified_base
<222> 1..19
<223> /mod_base = "2'-hydroxy corresponding nucleoside"

<400> 706

ccucgcgguc uugcuggcct t 21

<210> 707
<211> 21
<212> DNA
<213> Artificial sequence

<220>
<223> Description of the artificial sequence: antisense strand of dsRNA

<220>
<221> modified_base
<222> 21
<223> /mod_base = "5'-phosphorothioate thymidine"

<220>
<221> modified_base
<222> 1..19
<223> /mod_base = "2'-hydroxy corresponding nucleoside"

<400> 707
ccuccucgcg gucuugcugt t 21

<210> 708
<211> 21
<212> DNA
<213> Artificial sequence

<220>
<223> Description of the artificial sequence: antisense strand of dsRNA

<220>
<221> modified_base
<222> 7
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 10, 11, 12, 13, 14, 15, 16, 17, 18, 19
<223> /mod_base = "2'-hydroxy corresponding nucleoside"

<400> 708
gauucccagu gcugaaugut t 21

<210> 709
<211> 21
<212> DNA
<213> Artificial sequence

<220>
<223> Description of the artificial sequence: antisense strand of dsRNA

<220>

<221> modified_base
<222> 9
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 10, 11, 12, 13, 14, 15, 16, 17, 18, 19
<223> /mod_base = "2'-hydroxy corresponding nucleoside"

<400> 709
gggauuccca gugcugaaut t          21

<210> 710
<211> 21
<212> DNA
<213> Artificial sequence

<220>
<223> Description of the artificial sequence: antisense strand of dsRNA

<220>
<221> modified_base
<222> 7, 12
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 10, 11, 13, 14, 15, 16, 17, 18, 19
<223> /mod_base = "2'-hydroxy corresponding nucleoside"

<400> 710
gaaagccaga acaugggaut t          21

<210> 711
<211> 21
<212> DNA
<213> Artificial sequence

<220>
<223> Description of the artificial sequence: antisense strand of dsRNA

<220>
<221> modified_base
<222> 8, 13
<223> /mod_base = "2'-O-methyl corresponding nucleoside"

<220>
<221> modified_base

<222> 21
<223> /mod_base = "5'-phosphorothioate thymidine"

<220>
<221> modified_base
<222> 1, 2, 3, 4, 5, 6, 7, 9, 10, 11, 12, 14, 15, 16, 17, 18, 19
<223> /mod_base = "2'-hydroxy corresponding nucleoside"

<400> 711
agaaagccag aacaugggat t 21

<210> 712
<211> 21
<212> DNA
<213> Artificial sequence

<220>
<223> Description of the artificial sequence: antisense strand of dsRNA

<220>
<221> modified_base
<222> 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 13, 14, 16, 17, 18, 19
<223> /mod_base = "2'-hydroxy corresponding nucleoside"

<400> 712
acagcgggcu ucuguaauct t 21

<210> 713
<211> 21
<212> DNA
<213> Artificial sequence

<220>
<223> Description of the artificial sequence: antisense strand of dsRNA

<220>
<221> modified_base
<222> 1, 10, 12, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 4, 5, 6, 7, 8, 9, 11, 13, 15, 16, 17, 18, 19

<223> /mod_base = "2'-hydroxy corresponding nucleoside"

<400> 713
uaagaagacu auacauuuct t 21

<210> 714
<211> 21
<212> DNA
<213> Artificial sequence

<220>
<223> Description of the artificial sequence: antisense strand of dsRNA

<220>
<221> modified_base
<222> 11, 13, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 12, 14, 16, 17, 18, 19
<223> /mod_base = "2'-hydroxy corresponding nucleoside"

<400> 714
auaagaagac uauacauuut t 21

<210> 715
<211> 21
<212> DNA
<213> Artificial sequence

<220>
<223> Description of the artificial sequence: antisense strand of dsRNA

<220>
<221> modified_base
<222> 5, 8, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 9, 10, 11, 12, 13, 14, 15, 16, 18, 19
<223> /mod_base = "2'-hydroxy corresponding nucleoside"

<400> 715
gugucaauaa gaagacuaut t 21

<210> 716

<211> 21
<212> DNA
<213> Artificial sequence

<220>
<223> Description of the artificial sequence: antisense strand of dsRNA

<220>
<221> modified_base
<222> 1
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2..19
<223> /mod_base = "2'-hydroxy corresponding nucleoside"

<400> 716
cauugaagag aaauucccut t 21

<210> 717
<211> 21
<212> DNA
<213> Artificial sequence

<220>
<223> Description of the artificial sequence: antisense strand of dsRNA

<220>
<221> modified_base
<222> 8, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 9, 10, 11, 12, 13, 15, 16, 17, 18, 19
<223> /mod_base = "2'-hydroxy corresponding nucleoside"

<400> 717
aucguuucaa uggcauugat t 21

<210> 718
<211> 21
<212> DNA
<213> Artificial sequence

<220>
<223> Description of the artificial sequence: antisense strand of dsRNA

<220>
<221> modified_base
<222> 5, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 8, 9, 10, 11, 12, 14, 15, 16, 17, 18, 19
<223> /mod_base = "2'-hydroxy corresponding nucleoside"

<400> 718
aaggcaucgu uucaauggct t 21

<210> 719
<211> 21
<212> DNA
<213> Artificial sequence

<220>
<223> Description of the artificial sequence: antisense strand of dsRNA

<220>
<221> modified_base
<222> 5, 7, 12
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 8, 9, 10, 11, 13, 14, 15, 16, 17, 18, 19
<223> /mod_base = "2'-hydroxy corresponding nucleoside"

<400> 719
uugacacaag gcaucguuut t 21

<210> 720
<211> 21
<212> DNA
<213> Artificial sequence

<220>
<223> Description of the artificial sequence: antisense strand of dsRNA

<220>
<221> modified_base
<222> 7, 9, 14
<223> /mod_base = "2'-O-methyl corresponding nucleoside"

<220>

<221> modified_base
<222> 21
<223> /mod_base = "5'-phosphorothioate thymidine"

<220>
<221> modified_base
<222> 1, 2, 3, 4, 5, 6, 8, 10, 11, 12, 13, 15, 16, 17, 18, 19
<223> /mod_base = "2'-hydroxy corresponding nucleoside"

<400> 720
ucuugacaca aggcaucgut t 21

<210> 721
<211> 21
<212> DNA
<213> Artificial sequence

<220>
<223> Description of the artificial sequence: antisense strand of dsRNA

<220>
<221> modified_base
<222> 5, 8, 10, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 9, 11, 12, 13, 15, 16, 17, 18, 19
<223> /mod_base = "2'-hydroxy corresponding nucleoside"

<400> 721
cguucaccau agguagccut t 21

<210> 722
<211> 21
<212> DNA
<213> Artificial sequence

<220>
<223> Description of the artificial sequence: antisense strand of dsRNA

<220>
<221> modified_base
<222> 6, 9, 11, 15
<223> /mod_base = "2'-O-methyl corresponding nucleoside"

<220>
<221> modified_base
<222> 21
<223> /mod_base = "5'-phosphorothioate thymidine"

<220>
<221> modified_base

<222> 1, 2, 3, 4, 5, 7, 8, 10, 12, 13, 14, 16, 17, 18, 19
<223> /mod_base = "2'-hydroxy corresponding nucleoside"

<400> 722
acguucacca uagguagcct t 21

<210> 723
<211> 21
<212> DNA
<213> Artificial sequence

<220>
<223> Description of the artificial sequence: antisense strand of dsRNA

<220>
<221> modified_base
<222> 1, 7, 10, 12, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 4, 5, 6, 8, 9, 11, 13, 14, 15, 17, 18, 19
<223> /mod_base = "2'-hydroxy corresponding nucleoside"

<400> 723
cacguucacc auagguagct t 21

<210> 724
<211> 21
<212> DNA
<213> Artificial sequence

<220>
<223> Description of the artificial sequence: antisense strand of dsRNA

<220>
<221> modified_base
<222> 1, 5, 8
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 4, 6, 7, 9, 10, 11, 12, 13, 14, 15, 16, 17, 18, 19
<223> /mod_base = "2'-hydroxy corresponding nucleoside"

<400> 724
caggcaucag uccucguuut t 21

<210> 725
<211> 21
<212> DNA
<213> Artificial sequence

<220>
<223> Description of the artificial sequence: antisense strand of dsRNA

<220>
<221> modified_base
<222> 3, 6, 12, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 7, 8, 9, 10, 11, 13, 14, 15, 17, 18, 19
<223> /mod_base = "2'-hydroxy corresponding nucleoside"

<400> 725
aacagcauug auaauuauut t 21

<210> 726
<211> 21
<212> DNA
<213> Artificial sequence

<220>
<223> Description of the artificial sequence: antisense strand of dsRNA

<220>
<221> modified_base
<222> 4, 7, 13, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 8, 9, 10, 11, 12, 14, 15, 16, 18, 19
<223> /mod_base = "2'-hydroxy corresponding nucleoside"

<400> 726
gaacagcauu gauaauuaut t 21

<210> 727
<211> 21
<212> DNA
<213> Artificial sequence

<220>

<223> Description of the artificial sequence: antisense strand of dsRNA

<220>
<221> modified_base
<222> 6, 9, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 7, 8, 10, 11, 12, 13, 14, 16, 17, 18, 19
<223> /mod_base = "2'-hydroxy corresponding nucleoside"

<400> 727
ccgaacagca uugauaauut t 21

<210> 728
<211> 21
<212> DNA
<213> Artificial sequence

<220>
<223> Description of the artificial sequence: antisense strand of dsRNA

<220>
<221> modified_base
<222> 8, 11, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 9, 10, 12, 13, 14, 15, 16, 18, 19
<223> /mod_base = "2'-hydroxy corresponding nucleoside"

<400> 728
auccgaacag cauugauaat t 21

<210> 729
<211> 21
<212> DNA
<213> Artificial sequence

<220>
<223> Description of the artificial sequence: antisense strand of dsRNA

<220>
<221> modified_base
<222> 5, 13, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 8, 9, 10, 11, 12, 14, 15, 17, 18, 19
<223> /mod_base = "2'-hydroxy corresponding nucleoside"

<400> 729
guucuauccg aacagcauut t 21

<210> 730
<211> 21
<212> DNA
<213> Artificial sequence

<220>
<223> Description of the artificial sequence: antisense strand of dsRNA

<220>
<221> modified_base
<222> 6, 14, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 7, 8, 9, 10, 11, 12, 13, 15, 16, 18, 19
<223> /mod_base = "2'-hydroxy corresponding nucleoside"

<400> 730
uguucuaucc gaacagcaut t 21

<210> 731
<211> 21
<212> DNA
<213> Artificial sequence

<220>
<223> Description of the artificial sequence: antisense strand of dsRNA

<220>
<221> modified_base
<222> 7, 15, 18
<223> /mod_base = "2'-O-methyl corresponding nucleoside"

<220>
<221> modified_base
<222> 21
<223> /mod_base = "5'-phosphorothioate thymidine"

<220>

<221> modified_base
<222> 1, 2, 3, 4, 5, 6, 8, 9, 10, 11, 12, 13, 14, 16, 17, 19
<223> /mod_base = "2'-hydroxy corresponding nucleoside"

<400> 731
cuguucuauc cgaacagcat t 21

<210> 732
<211> 21
<212> DNA
<213> Artificial sequence

<220>
<223> Description of the artificial sequence: antisense strand of dsRNA

<220>
<221> modified_base
<222> 9, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 10, 11, 12, 13, 14, 15, 16, 18, 19
<223> /mod_base = "2'-hydroxy corresponding nucleoside"

<400> 732
uccuguucua uccgaacagt t 21

<210> 733
<211> 21
<212> DNA
<213> Artificial sequence

<220>
<223> Description of the artificial sequence: antisense strand of dsRNA

<220>
<221> modified_base
<222> 10, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 11, 12, 13, 14, 15, 16, 17, 19
<223> /mod_base = "2'-hydroxy corresponding nucleoside"

<400> 733
cuccuguucu auccgaacat t 21

<210> 734
<211> 21
<212> DNA
<213> Artificial sequence

<220>
<223> Description of the artificial sequence: antisense strand of dsRNA

<220>
<221> modified_base
<222> 12
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 13, 14, 15, 16, 17, 18, 19
<223> /mod_base = "2'-hydroxy corresponding nucleoside"

<400> 734
aacuccuguu cuauccgaat t 21

<210> 735
<211> 21
<212> DNA
<213> Artificial sequence

<220>
<223> Description of the artificial sequence: antisense strand of dsRNA

<220>
<221> modified_base
<222> 6, 9, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 7, 8, 10, 11, 12, 13, 14, 15, 17, 18, 19
<223> /mod_base = "2'-hydroxy corresponding nucleoside"

<400> 735
ugcuucauua gagugcaaut t 21

<210> 736
<211> 21
<212> DNA
<213> Artificial sequence

<220>

<223> Description of the artificial sequence: antisense strand of dsRNA

<220>
<221> modified_base
<222> 3, 11, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 8, 9, 10, 12, 13, 15, 16, 17, 18, 19
<223> /mod_base = "2'-hydroxy corresponding nucleoside"

<400> 736
uguauugcuu cauuagagut t 21

<210> 737
<211> 21
<212> DNA
<213> Artificial sequence

<220>
<223> Description of the artificial sequence: antisense strand of dsRNA

<220>
<221> modified_base
<222> 15, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 13, 14, 16, 18, 19
<223> /mod_base = "2'-hydroxy corresponding nucleoside"

<400> 737
auaagucugu cugccacaat t 21

<210> 738
<211> 21
<212> DNA
<213> Artificial sequence

<220>
<223> Description of the artificial sequence: antisense strand of dsRNA

<220>
<221> modified_base
<222> 3, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 8, 9, 10, 11, 12, 13, 14, 15, 17, 18, 19
<223> /mod_base = "2'-hydroxy corresponding nucleoside"

<400> 738
gucaaauggg uucucuacut t 21

<210> 739
<211> 21
<212> DNA
<213> Artificial sequence

<220>
<223> Description of the artificial sequence: antisense strand of dsRNA

<220>
<221> modified_base
<222> 4, 6, 12
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 7, 8, 9, 10, 11, 13, 14, 15, 16, 17, 18, 19
<223> /mod_base = "2'-hydroxy corresponding nucleoside"

<400> 739
cuccauaaag ucaaaugggt t 21

<210> 740
<211> 21
<212> DNA
<213> Artificial sequence

<220>
<223> Description of the artificial sequence: antisense strand of dsRNA

<220>
<221> modified_base
<222> 6, 14
<223> /mod_base = "2'-O-methyl corresponding nucleoside"

<220>
<221> modified_base
<222> 21
<223> /mod_base = "5'-phosphorothioate thymidine"

<220>

<221> modified_base
<222> 1, 2, 3, 4, 5, 7, 8, 9, 10, 11, 12, 13, 15, 16, 17, 18, 19
<223> /mod_base = "2'-hydroxy corresponding nucleoside"

<400> 740
ccuuccagug aaauauucut t 21

<210> 741
<211> 21
<212> DNA
<213> Artificial sequence

<220>
<223> Description of the artificial sequence: antisense strand of dsRNA

<220>
<221> modified_base
<222> 8, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 9, 10, 11, 12, 13, 14, 15, 17, 18, 19
<223> /mod_base = "2'-hydroxy corresponding nucleoside"

<400> 741
uuccuuccag ugaaauauut t 21

<210> 742
<211> 21
<212> DNA
<213> Artificial sequence

<220>
<223> Description of the artificial sequence: antisense strand of dsRNA

<220>
<221> modified_base
<222> 21
<223> /mod_base = "5'-phosphorothioate thymidine"

<220>
<221> modified_base
<222> 1..19
<223> /mod_base = "2'-hydroxy corresponding nucleoside"

<400> 742
aagaauucuc uguuggacut t 21

<210> 743
<211> 21
<212> DNA
<213> Artificial sequence

<220>
<223> Description of the artificial sequence: antisense strand of dsRNA

<220>
<221> modified_base
<222> 3, 10, 13, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 8, 9, 11, 12, 14, 15, 16, 17, 19
<223> /mod_base = "2'-hydroxy corresponding nucleoside"

<400> 743
gcuaguuggu aacacuucat t 21

<210> 744
<211> 21
<212> DNA
<213> Artificial sequence

<220>
<223> Description of the artificial sequence: antisense strand of dsRNA

<220>
<221> modified_base
<222> 9, 13, 15, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 10, 11, 12, 14, 16, 18, 19
<223> /mod_base = "2'-hydroxy corresponding nucleoside"

<400> 744
uugugaggua gcuacacagt t 21

<210> 745
<211> 21
<212> DNA
<213> Artificial sequence

<220>
<223> Description of the artificial sequence: antisense strand of dsRNA

<220>
<221> modified_base
<222> 1, 18

<223> /mod_base = "2’-O-methyl corresponding nucleoside"

<220>
<221> modified_base
<222> 21
<223> /mod_base = "5’-phosphorothioate thymidine"

<220>
<221> modified_base
<222> 2, 3, 4, 5, 6, 7, 8, 9, 10, 11, 12, 13, 14, 15, 16, 17, 19
<223> /mod_base = "2’-hydroxy corresponding nucleoside"

<400> 745
caggacuggu ugugagguat t 21

<210> 746
<211> 21
<212> DNA
<213> Artificial sequence

<220>
<223> Description of the artificial sequence: antisense strand of dsRNA

<220>
<221> modified_base
<222> 3, 6, 9, 12, 15, 17
<223> /mod_base = "2’-O-methyl corresponding nucleoside"

<220>
<221> modified_base
<222> 21
<223> /mod_base = "5’-phosphorothioate thymidine"

<220>
<221> modified_base
<222> 1, 2, 4, 5, 7, 8, 10, 11, 13, 14, 16, 18, 19
<223> /mod_base = "2’-hydroxy corresponding nucleoside"

<400> 746
gauacuacca gcacuauaat t 21

<210> 747
<211> 21
<212> DNA
<213> Artificial sequence

<220>
<223> Description of the artificial sequence: antisense strand of dsRNA

<220>
<221> modified_base
<222> 4, 7, 10, 13, 16, 18
<223> /mod_base = "2’-O-methyl corresponding nucleoside"

<220>
<221> modified_base
<222> 21
<223> /mod_base = "5’-phosphorothioate thymidine"

<220>
<221> modified_base
<222> 1, 2, 3, 5, 6, 8, 9, 11, 12, 14, 15, 17, 19
<223> /mod_base = "2'-hydroxy corresponding nucleoside"

<400> 747
ugauacuacc agcacuauat t 21

<210> 748
<211> 21
<212> DNA
<213> Artificial sequence

<220>
<223> Description of the artificial sequence: antisense strand of dsRNA

<220>
<221> modified_base
<222> 5, 8, 11, 14, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 9, 10, 12, 13, 15, 16, 18, 19
<223> /mod_base = "2'-hydroxy corresponding nucleoside"

<400> 748
gugauacuac cagcacuaut t 21

<210> 749
<211> 21
<212> DNA
<213> Artificial sequence

<220>
<223> Description of the artificial sequence: antisense strand of dsRNA

<220>
<221> modified_base
<222> 7, 10, 13, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 11, 12, 14, 15, 17, 18, 19
<223> /mod_base = "2'-hydroxy corresponding nucleoside"

<400> 749

aggugauacu accagcacut t 21

<210> 750
<211> 21
<212> DNA
<213> Artificial sequence

<220>
<223> Description of the artificial sequence: antisense strand of dsRNA

<220>
<221> modified_base
<222> 8, 11, 14, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 9, 10, 12, 13, 15, 16, 18, 19
<223> /mod_base = "2'-hydroxy corresponding nucleoside"

<400> 750
aaggugauac uaccagcact t 21

<210> 751
<211> 21
<212> DNA
<213> Artificial sequence

<220>
<223> Description of the artificial sequence: antisense strand of dsRNA

<220>
<221> modified_base
<222> 3, 8, 13, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 9, 10, 11, 12, 14, 16, 17, 18, 19
<223> /mod_base = "2'-hydroxy corresponding nucleoside"

<400> 751
gcuaugguaa gucacagcct t 21

<210> 752
<211> 21
<212> DNA
<213> Artificial sequence

<220>
<223> Description of the artificial sequence: antisense strand of dsRNA

<220>
<221> modified_base
<222> 5, 10, 15, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 8, 9, 11, 12, 13, 14, 16, 18, 19
<223> /mod_base = "2'-hydroxy corresponding nucleoside"

<400> 752
cugcuauggu aagucacagt t 21

<210> 753
<211> 21
<212> DNA
<213> Artificial sequence

<220>
<223> Description of the artificial sequence: antisense strand of dsRNA

<220>
<221> modified_base
<222> 1, 7, 12, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 4, 5, 6, 8, 9, 10, 11, 13, 14, 15, 16, 18, 19
<223> /mod_base = "2'-hydroxy corresponding nucleoside"

<400> 753
cacugcuaug guaagucact t 21

<210> 754
<211> 21
<212> DNA
<213> Artificial sequence

<220>
<223> Description of the artificial sequence: antisense strand of dsRNA

<220>
<221> modified_base
<222> 6, 12, 17

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 7, 8, 9, 10, 11, 13, 14, 15, 16, 18, 19
<223> /mod_base = "2'-hydroxy corresponding nucleoside"

<400> 754
auugucacug cuaugguaat t 21

<210> 755
<211> 21
<212> DNA
<213> Artificial sequence

<220>
<223> Description of the artificial sequence: antisense strand of dsRNA

<220>
<221> modified_base
<222> 4, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 7, 8, 9, 10, 11, 12, 14, 15, 16, 17, 18, 19
<223> /mod_base = "2'-hydroxy corresponding nucleoside"

<400> 755
agccaagacu gccauuguct t 21

<210> 756
<211> 21
<212> DNA
<213> Artificial sequence

<220>
<223> Description of the artificial sequence: antisense strand of dsRNA

<220>
<221> modified_base
<222> 4, 7, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 8, 9, 10, 11, 12, 13, 14, 16, 17, 18, 19
<223> /mod_base = "2'-hydroxy corresponding nucleoside"

<400> 756
gcucacuaaa gggucaccct t 21

<210> 757
<211> 21
<212> DNA
<213> Artificial sequence

<220>
<223> Description of the artificial sequence: antisense strand of dsRNA

<220>
<221> modified_base
<222> 5, 8, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 9, 10, 11, 12, 13, 14, 15, 17, 18, 19
<223> /mod_base = "2'-hydroxy corresponding nucleoside"

<400> 757
agcucacuaa agggucacct t 21

<210> 758
<211> 21
<212> DNA
<213> Artificial sequence

<220>
<223> Description of the artificial sequence: antisense strand of dsRNA

<220>
<221> modified_base
<222> 10
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 11, 12, 13, 14, 15, 16, 17, 18, 19
<223> /mod_base = "2'-hydroxy corresponding nucleoside"

<400> 758

gcguugaagc agugaggcut t 21

<210> 759
<211> 21
<212> DNA
<213> Artificial sequence

<220>
<223> Description of the artificial sequence: antisense strand of dsRNA

<220>
<221> modified_base
<222> 3, 7, 12, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 8, 9, 10, 11, 13, 14, 15, 16, 18, 19
<223> /mod_base = "2'-hydroxy corresponding nucleoside"

<400> 759
accaaauaaa ucaagcuaut t 21

<210> 760
<211> 21
<212> DNA
<213> Artificial sequence

<220>
<223> Description of the artificial sequence: antisense strand of dsRNA

<220>
<221> modified_base
<222> 11, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 12, 13, 14, 15, 16, 17, 19
<223> /mod_base = "2'-hydroxy corresponding nucleoside"

<400> 760
guauuuggug uagaaaccat t 21

<210> 761
<211> 21
<212> DNA
<213> Artificial sequence

<220>
<223> Description of the artificial sequence: antisense strand of dsRNA

<220>
<221> modified_base
<222> 3, 12
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 8, 9, 10, 11, 13, 14, 15, 16, 17, 18, 19
<223> /mod_base = "2'-hydroxy corresponding nucleoside"

<400> 761
uguauuuggu guagaaacct t 21

<210> 762
<211> 21
<212> DNA
<213> Artificial sequence

<220>
<223> Description of the artificial sequence: antisense strand of dsRNA

<220>
<221> modified_base
<222> 7, 10, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 11, 12, 13, 14, 15, 16, 18, 19
<223> /mod_base = "2'-hydroxy corresponding nucleoside"

<400> 762
ggcucccauu aguggucagt t 21

<210> 763
<211> 21
<212> DNA
<213> Artificial sequence

<220>
<223> Description of the artificial sequence: antisense strand of dsRNA

<220>
<221> modified_base
<222> 8, 11, 18

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 9, 10, 12, 13, 14, 15, 16, 17, 19
<223> /mod_base = "2'-hydroxy corresponding nucleoside"

<400> 763
uggcucccau uaguggucat t 21

<210> 764
<211> 21
<212> DNA
<213> Artificial sequence

<220>
<223> Description of the artificial sequence: antisense strand of dsRNA

<220>
<221> modified_base
<222> 10, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 11, 12, 14, 15, 16, 17, 18, 19
<223> /mod_base = "2'-hydroxy corresponding nucleoside"

<400> 764
auuggcuccc auuaguggut t 21

<210> 765
<211> 21
<212> DNA
<213> Artificial sequence

<220>
<223> Description of the artificial sequence: antisense strand of dsRNA

<220>
<221> modified_base
<222> 5, 9, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 8, 10, 11, 12, 14, 15, 16, 17, 18, 19
<223> /mod_base = "2'-hydroxy corresponding nucleoside"

<400> 765
acuuuaguca cuuagugaat t 21

<210> 766
<211> 21
<212> DNA
<213> Artificial sequence

<220>
<223> Description of the artificial sequence: antisense strand of dsRNA

<220>
<221> modified_base
<222> 1, 6, 10, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 4, 5, 7, 8, 9, 11, 12, 13, 15, 16, 17, 18, 19
<223> /mod_base = "2'-hydroxy corresponding nucleoside"

<400> 766
uacuuuaguc acuuagugat t 21

<210> 767
<211> 21
<212> DNA
<213> Artificial sequence

<220>
<223> Description of the artificial sequence: antisense strand of dsRNA

<220>
<221> modified_base
<222> 7, 11, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 10, 12, 13, 14, 16, 17, 18, 19
<223> /mod_base = "2'-hydroxy corresponding nucleoside"

<400> 767

uuacuuuagu cacuuagugt t 21

<210> 768
<211> 21
<212> DNA
<213> Artificial sequence

<220>
<223> Description of the artificial sequence: antisense strand of dsRNA

<220>
<221> modified_base
<222> 3, 8, 12, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 9, 10, 11, 13, 14, 15, 17, 18, 19
<223> /mod_base = "2'-hydroxy corresponding nucleoside"

<400> 768
cuuacuuuag ucacuuagut t 21

<210> 769
<211> 21
<212> DNA
<213> Artificial sequence

<220>
<223> Description of the artificial sequence: antisense strand of dsRNA

<220>
<221> modified_base
<222> 1, 6, 11, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 4, 5, 7, 8, 9, 10, 12, 13, 14, 16, 17, 18, 19
<223> /mod_base = "2'-hydroxy corresponding nucleoside"

<400> 769
uaacuuacuu uagucacuut t 21

<210> 770
<211> 21
<212> DNA
<213> Artificial sequence

<220>
<223> Description of the artificial sequence: antisense strand of dsRNA

<220>
<221> modified_base
<222> 3, 8, 13, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 9, 10, 11, 12, 14, 15, 16, 18, 19
<223> /mod_base = "2'-hydroxy corresponding nucleoside"

<400> 770
uuuaacuuac uuuagucact t 21

<210> 771
<211> 21
<212> DNA
<213> Artificial sequence

<220>
<223> Description of the artificial sequence: antisense strand of dsRNA

<220>
<221> modified_base
<222> 4, 9, 14, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 7, 8, 10, 11, 12, 13, 15, 16, 17, 19
<223> /mod_base = "2'-hydroxy corresponding nucleoside"

<400> 771
guuuaacuua cuuuagucat t 21

<210> 772
<211> 21
<212> DNA
<213> Artificial sequence

<220>
<223> Description of the artificial sequence: antisense strand of dsRNA

<220>
<221> modified_base
<222> 5, 10, 15

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 8, 9, 11, 12, 13, 14, 16, 17, 18, 19
<223> /mod_base = "2'-hydroxy corresponding nucleoside"

<400> 772
aguuuaacuu acuuuaguct t 21

<210> 773
<211> 21
<212> DNA
<213> Artificial sequence

<220>
<223> Description of the artificial sequence: antisense strand of dsRNA

<220>
<221> modified_base
<222> 6, 11, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 7, 8, 9, 10, 12, 13, 14, 15, 17, 18, 19
<223> /mod_base = "2'-hydroxy corresponding nucleoside"

<400> 773
aaguuuaacu uacuuuagut t 21

<210> 774
<211> 21
<212> DNA
<213> Artificial sequence

<220>
<223> Description of the artificial sequence: antisense strand of dsRNA

<220>
<221> modified_base
<222> 1, 3, 5, 11, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 4, 6, 7, 8, 9, 10, 12, 13, 14, 15, 17, 18, 19
<223> /mod_base = "2'-hydroxy corresponding nucleoside"

<400> 774
uacacaaguu uaacuuacut t 21

<210> 775
<211> 21
<212> DNA
<213> Artificial sequence

<220>
<223> Description of the artificial sequence: antisense strand of dsRNA

<220>
<221> modified_base
<222> 4, 6, 12, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 7, 8, 9, 10, 11, 13, 14, 15, 16, 18, 19
<223> /mod_base = "2'-hydroxy corresponding nucleoside"

<400> 775
cuacacaagu uuaacuuact t 21

<210> 776
<211> 21
<212> DNA
<213> Artificial sequence

<220>
<223> Description of the artificial sequence: antisense strand of dsRNA

<220>
<221> modified_base
<222> 1, 6, 8, 10, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 4, 5, 7, 9, 11, 12, 13, 14, 15, 17, 18, 19
<223> /mod_base = "2'-hydroxy corresponding nucleoside"

<400> 776

uagucuacac aaguuuaact t 21

<210> 777
<211> 21
<212> DNA
<213> Artificial sequence

<220>
<223> Description of the artificial sequence: antisense strand of dsRNA

<220>
<221> modified_base
<222> 7, 9, 11, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 10, 12, 13, 14, 15, 16, 18, 19
<223> /mod_base = "2'-hydroxy corresponding nucleoside"

<400> 777
uuagucuaca caaguuuaat t 21

<210> 778
<211> 21
<212> DNA
<213> Artificial sequence

<220>
<223> Description of the artificial sequence: antisense strand of dsRNA

<220>
<221> modified_base
<222> 3, 8, 10, 12, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 9, 11, 13, 14, 15, 16, 17, 19
<223> /mod_base = "2'-hydroxy corresponding nucleoside"

<400> 778
cuuagucuac acaaguuuat t 21

<210> 779
<211> 21
<212> DNA
<213> Artificial sequence

<220>
<223> Description of the artificial sequence: antisense strand of dsRNA

<220>
<221> modified_base
<222> 4, 9, 11, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 7, 8, 10, 12, 14, 15, 16, 17, 18, 19
<223> /mod_base = "2'-hydroxy corresponding nucleoside"

<400> 779
gcuuagucua cacaaguuut t 21

<210> 780
<211> 21
<212> DNA
<213> Artificial sequence

<220>
<223> Description of the artificial sequence: antisense strand of dsRNA

<220>
<221> modified_base
<222> 5, 10, 12, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 8, 9, 11, 13, 15, 16, 17, 18, 19
<223> /mod_base = "2'-hydroxy corresponding nucleoside"

<400> 780
ugcuuagucu acacaaguut t 21

<210> 781
<211> 21
<212> DNA
<213> Artificial sequence

<220>
<223> Description of the artificial sequence: antisense strand of dsRNA

<220>
<221> modified_base
<222> 8, 13, 15, 17

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 9, 10, 11, 12, 14, 16, 18, 19
<223> /mod_base = "2'-hydroxy corresponding nucleoside"

<400> 781
acaugcuuag ucuacacaat t 21

<210> 782
<211> 21
<212> DNA
<213> Artificial sequence

<220>
<223> Description of the artificial sequence: antisense strand of dsRNA

<220>
<221> modified_base
<222> 1, 3, 9, 14, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 4, 5, 6, 7, 8, 10, 11, 12, 13, 15, 17, 19
<223> /mod_base = "2'-hydroxy corresponding nucleoside"

<400> 782
uacaugcuua gucuacacat t 21

<210> 783
<211> 21
<212> DNA
<213> Artificial sequence

<220>
<223> Description of the artificial sequence: antisense strand of dsRNA

<220>
<221> modified_base
<222> 3, 5, 11, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 6, 7, 8, 9, 10, 12, 13, 14, 15, 17, 19
<223> /mod_base = "2'-hydroxy corresponding nucleoside"

<400> 783
auuacaugcu uagucuacat t 21

<210> 784
<211> 21
<212> DNA
<213> Artificial sequence

<220>
<223> Description of the artificial sequence: antisense strand of dsRNA

<220>
<221> modified_base
<222> 5, 7, 13, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 8, 9, 10, 11, 12, 14, 15, 16, 17, 19
<223> /mod_base = "2'-hydroxy corresponding nucleoside"

<400> 784
aaauuacaug cuuagucuat t 21

<210> 785
<211> 21
<212> DNA
<213> Artificial sequence

<220>
<223> Description of the artificial sequence: antisense strand of dsRNA

<220>
<221> modified_base
<222> 6, 13, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 7, 8, 9, 10, 11, 12, 14, 15, 17, 18, 19
<223> /mod_base = "2'-hydroxy corresponding nucleoside"

<400> 785

ucuucuaucu ggcaccaact t 21

<210> 786
<211> 21
<212> DNA
<213> Artificial sequence

<220>
<223> Description of the artificial sequence: antisense strand of dsRNA

<220>
<221> modified_base
<222> 11, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 12, 13, 14, 15, 16, 17, 19
<223> /mod_base = "2'-hydroxy corresponding nucleoside"

<400> 786
accugucuuc uaucuggcat t 21

<210> 787
<211> 21
<212> DNA
<213> Artificial sequence

<220>
<223> Description of the artificial sequence: antisense strand of dsRNA

<220>
<221> modified_base
<222> 9, 14, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 10, 11, 12, 13, 15, 16, 18, 19
<223> /mod_base = "2'-hydroxy corresponding nucleoside"

<400> 787
gagaauccca ggacacuact t 21

<210> 788
<211> 21
<212> DNA
<213> Artificial sequence

<220>
<223> Description of the artificial sequence: antisense strand of dsRNA

<220>
<221> modified_base
<222> 3, 8
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 9, 10, 11, 12, 13, 14, 15, 16, 17, 18, 19
<223> /mod_base = "2'-hydroxy corresponding nucleoside"

<400> 788
uuuaacuuaa gaagcuccut t 21

<210> 789
<211> 21
<212> DNA
<213> Artificial sequence

<220>
<223> Description of the artificial sequence: antisense strand of dsRNA

<220>
<221> modified_base
<222> 5, 8, 12, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 9, 10, 11, 13, 14, 16, 17, 18, 19
<223> /mod_base = "2'-hydroxy corresponding nucleoside"

<400> 789
auuucaccac uuaacagggt t 21

<210> 790
<211> 21
<212> DNA
<213> Artificial sequence

<220>
<223> Description of the artificial sequence: antisense strand of dsRNA

<220>
<221> modified_base
<222> 1, 6, 8, 10, 15

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 4, 5, 7, 9, 11, 12, 13, 14, 16, 17, 18, 19
<223> /mod_base = "2'-hydroxy corresponding nucleoside"

<400> 790
uaacucacac aaccuacuut t 21

<210> 791
<211> 21
<212> DNA
<213> Artificial sequence

<220>
<223> Description of the artificial sequence: antisense strand of dsRNA

<220>
<221> modified_base
<222> 1, 10, 15, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 4, 5, 6, 7, 8, 9, 11, 12, 13, 14, 16, 18, 19
<223> /mod_base = "2'-hydroxy corresponding nucleoside"

<400> 791
uaaaugaauu aacucacact t 21

<210> 792
<211> 21
<212> DNA
<213> Artificial sequence

<220>
<223> Description of the artificial sequence: antisense strand of dsRNA

<220>
<221> modified_base
<222> 4, 7, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 8, 9, 10, 11, 12, 14, 15, 16, 17, 18, 19
<223> /mod_base = "2'-hydroxy corresponding nucleoside"

<400> 792
cccuauuaag aauauuugut t 21

<210> 793
<211> 21
<212> DNA
<213> Artificial sequence

<220>
<223> Description of the artificial sequence: antisense strand of dsRNA

<220>
<221> modified_base
<222> 3, 9, 12, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 8, 10, 11, 13, 14, 15, 16, 17, 19
<223> /mod_base = "2'-hydroxy corresponding nucleoside"

<400> 793
aguagcccua uuaagaauat t 21

<210> 794
<211> 21
<212> DNA
<213> Artificial sequence

<220>
<223> Description of the artificial sequence: antisense strand of dsRNA

<220>
<221> modified_base
<222> 4, 10, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 7, 8, 9, 11, 12, 14, 15, 16, 17, 18, 19
<223> /mod_base = "2'-hydroxy corresponding nucleoside"

<400> 794

aaguagcccu auuaagaaut t 21

<210> 795
<211> 21
<212> DNA
<213> Artificial sequence

<220>
<223> Description of the artificial sequence: antisense strand of dsRNA

<220>
<221> modified_base
<222> 7, 12, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 10, 11, 13, 14, 15, 16, 17, 19
<223> /mod_base = "2'-hydroxy corresponding nucleoside"

<400> 795
uuaauucaaa guagcccuat t 21

<210> 796
<211> 21
<212> DNA
<213> Artificial sequence

<220>
<223> Description of the artificial sequence: antisense strand of dsRNA

<220>
<221> modified_base
<222> 3, 8, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 9, 10, 11, 12, 14, 15, 16, 17, 18, 19
<223> /mod_base = "2'-hydroxy corresponding nucleoside"

<400> 796
auuaauucaa aguagcccut t 21

<210> 797
<211> 21
<212> DNA
<213> Artificial sequence

<220>
<223> Description of the artificial sequence: antisense strand of dsRNA

<220>
<221> modified_base
<222> 4, 7, 11, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 8, 9, 10, 12, 13, 14, 15, 16, 17, 19
<223> /mod_base = "2'-hydroxy corresponding nucleoside"

<400> 797
gaucaacauu uaucucucat t 21

<210> 798
<211> 21
<212> DNA
<213> Artificial sequence

<220>
<223> Description of the artificial sequence: antisense strand of dsRNA

<220>
<221> modified_base
<222> 6, 9, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 7, 8, 10, 11, 12, 14, 15, 16, 17, 18, 19
<223> /mod_base = "2'-hydroxy corresponding nucleoside"

<400> 798
aagaucaaca uuuaucucut t 21

<210> 799
<211> 21
<212> DNA
<213> Artificial sequence

<220>
<223> Description of the artificial sequence: antisense strand of dsRNA

<220>
<221> modified_base
<222> 3, 7, 10, 13, 18

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 8, 9, 11, 12, 14, 15, 16, 17, 19
<223> /mod_base = "2'-hydroxy corresponding nucleoside"

<400> 799
uuuagguaac aauaacuuat t 21

<210> 800
<211> 21
<212> DNA
<213> Artificial sequence

<220>
<223> Description of the artificial sequence: antisense strand of dsRNA

<220>
<221> modified_base
<222> 5, 9, 12, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 8, 10, 11, 13, 14, 16, 17, 18, 19
<223> /mod_base = "2'-hydroxy corresponding nucleoside"

<400> 800
acuuuaggua acaauaacut t 21

<210> 801
<211> 21
<212> DNA
<213> Artificial sequence

<220>
<223> Description of the artificial sequence: antisense strand of dsRNA

<220>
<221> modified_base
<222> 6, 10, 13, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 7, 8, 9, 11, 12, 14, 15, 17, 18, 19
<223> /mod_base = "2'-hydroxy corresponding nucleoside"

<400> 801
aacuuuaggu aacaauaact t 21

<210> 802
<211> 21
<212> DNA
<213> Artificial sequence

<220>
<223> Description of the artificial sequence: antisense strand of dsRNA

<220>
<221> modified_base
<222> 1, 7, 11, 14, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 4, 5, 6, 8, 9, 10, 12, 13, 15, 16, 18, 19
<223> /mod_base = "2'-hydroxy corresponding nucleoside"

<400> 802
uaacuuuagg uaacaauaat t 21

<210> 803
<211> 21
<212> DNA
<213> Artificial sequence

<220>
<223> Description of the artificial sequence: antisense strand of dsRNA

<220>
<221> modified_base
<222> 8, 12, 15, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 9, 10, 11, 13, 14, 16, 17, 19
<223> /mod_base = "2'-hydroxy corresponding nucleoside"

<400> 803

uuaacuuuag guaacaauat t 21

<210> 804
<211> 21
<212> DNA
<213> Artificial sequence

<220>
<223> Description of the artificial sequence: antisense strand of dsRNA

<220>
<221> modified_base
<222> 4, 10, 14, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 7, 8, 9, 11, 12, 13, 15, 16, 18, 19
<223> /mod_base = "2'-hydroxy corresponding nucleoside"

<400> 804
gauuaacuuu agguaacaat t 21

<210> 805
<211> 21
<212> DNA
<213> Artificial sequence

<220>
<223> Description of the artificial sequence: antisense strand of dsRNA

<220>
<221> modified_base
<222> 5, 11, 15, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 8, 9, 10, 12, 13, 14, 16, 17, 19
<223> /mod_base = "2'-hydroxy corresponding nucleoside"

<400> 805
ggauuaacuu uagguaacat t 21

<210> 806
<211> 21
<212> DNA
<213> Artificial sequence

<220>
<223> Description of the artificial sequence: antisense strand of dsRNA

<220>
<221> modified_base
<222> 7, 13, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 10, 11, 12, 14, 15, 16, 18, 19
<223> /mod_base = "2'-hydroxy corresponding nucleoside"

<400> 806
cuggauuaac uuuagguaat t 21

<210> 807
<211> 21
<212> DNA
<213> Artificial sequence

<220>
<223> Description of the artificial sequence: antisense strand of dsRNA

<220>
<221> modified_base
<222> 8, 14, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 9, 10, 11, 12, 13, 15, 16, 17, 19
<223> /mod_base = "2'-hydroxy corresponding nucleoside"

<400> 807
ucuggauuaa cuuuagguat t 21

<210> 808
<211> 21
<212> DNA
<213> Artificial sequence

<220>
<223> Description of the artificial sequence: antisense strand of dsRNA

<220>
<221> modified_base
<222> 5, 7, 10, 14, 18

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 8, 9, 11, 12, 13, 15, 16, 17, 19
<223> /mod_base = "2'-hydroxy corresponding nucleoside"

<400> 808
uccucacauu auucagcuat t 21

<210> 809
<211> 21
<212> DNA
<213> Artificial sequence

<220>
<223> Description of the artificial sequence: antisense strand of dsRNA

<220>
<221> modified_base
<222> 7, 14, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 10, 11, 12, 13, 15, 17, 18, 19
<223> /mod_base = "2'-hydroxy corresponding nucleoside"

<400> 809
agaaguuaau ccucacauut t 21

<210> 810
<211> 21
<212> DNA
<213> Artificial sequence

<220>
<223> Description of the artificial sequence: antisense strand of dsRNA

<220>
<221> modified_base
<222> 1, 8, 15, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 4, 5, 6, 7, 9, 10, 11, 12, 13, 14, 16, 18, 19
<223> /mod_base = "2'-hydroxy corresponding nucleoside"

<400> 810
cagaaguuaa uccucacaut t 21

<210> 811
<211> 21
<212> DNA
<213> Artificial sequence

<220>
<223> Description of the artificial sequence: antisense strand of dsRNA

<220>
<221> modified_base
<222> 8
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 9, 10, 11, 12, 13, 14, 15, 16, 17, 18, 19
<223> /mod_base = "2'-hydroxy corresponding nucleoside"

<400> 811
aacugauuag gaaaugguct t 21

<210> 812
<211> 21
<212> DNA
<213> Artificial sequence

<220>
<223> Description of the artificial sequence: antisense strand of dsRNA

<220>
<221> modified_base
<222> 21
<223> /mod_base = "5'-phosphorothioate thymidine"

<220>
<221> modified_base
<222> 1..19
<223> /mod_base = "2'-hydroxy corresponding nucleoside"

<400> 812
gugaugggcg cgagcgggat t 21

<210> 813
<211> 21
<212> DNA

<213> Artificial sequence

<220>
<223> Description of the artificial sequence: antisense strand of dsRNA

<220>
<221> modified_base
<222> 21
<223> /mod_base = "5'-phosphorothioate thymidine"

<220>
<221> modified_base
<222> 1..19
<223> /mod_base = "2'-hydroxy corresponding nucleoside"

<400> 813
cgugaugggc gcgagcgggt t 21

<210> 814
<211> 21
<212> DNA
<213> Artificial sequence

<220>
<223> Description of the artificial sequence: antisense strand of dsRNA

<220>
<221> modified_base
<222> 21
<223> /mod_base = "5'-phosphorothioate thymidine"

<220>
<221> modified_base
<222> 1..19
<223> /mod_base = "2'-hydroxy corresponding nucleoside"

<400> 814
uccuccucgc ggucuugcut t 21

<210> 815
<211> 21
<212> DNA
<213> Artificial sequence

<220>
<223> Description of the artificial sequence: antisense strand of dsRNA

<220>
<221> modified_base
<222> 21
<223> /mod_base = "5'-phosphorothioate thymidine"

<220>
<221> modified_base
<222> 1..19
<223> /mod_base = "2'-hydroxy corresponding nucleoside"

<400> 815

auccuccucg cggucuugct t 21

<210> 816
<211> 21
<212> DNA
<213> Artificial sequence

<220>
<223> Description of the artificial sequence: antisense strand of dsRNA

<220>
<221> modified_base
<222> 21
<223> /mod_base = "5'-phosphorothioate thymidine"

<220>
<221> modified_base
<222> 1..19
<223> /mod_base = "2'-hydroxy corresponding nucleoside"

<400> 816
gauccuccuc gcggucuugt t 21

<210> 817
<211> 21
<212> DNA
<213> Artificial sequence

<220>
<223> Description of the artificial sequence: antisense strand of dsRNA

<220>
<221> modified_base
<222> 21
<223> /mod_base = "5'-phosphorothioate thymidine"

<220>
<221> modified_base
<222> 1..19
<223> /mod_base = "2'-hydroxy corresponding nucleoside"

<400> 817
aagauccucc ucgcggucut t 21

<210> 818
<211> 21
<212> DNA
<213> Artificial sequence

<220>
<223> Description of the artificial sequence: antisense strand of dsRNA

<220>
<221> modified_base
<222> 21
<223> /mod_base = "5'-phosphorothioate thymidine"

<220>

<221> modified_base
<222> 1..19
<223> /mod_base = "2'-hydroxy corresponding nucleoside"

<400> 818
ggaagauccu ccucgcggut t 21

<210> 819
<211> 21
<212> DNA
<213> Artificial sequence

<220>
<223> Description of the artificial sequence: antisense strand of dsRNA

<220>
<221> modified_base
<222> 21
<223> /mod_base = "5'-phosphorothioate thymidine"

<220>
<221> modified_base
<222> 1..19
<223> /mod_base = "2'-hydroxy corresponding nucleoside"

<400> 819
cuggaagauc cuccucgcgt t 21

<210> 820
<211> 21
<212> DNA
<213> Artificial sequence

<220>
<223> Description of the artificial sequence: antisense strand of dsRNA

<220>
<221> modified_base
<222> 4, 8, 11, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 7, 9, 10, 12, 13, 14, 15, 17, 18, 19
<223> /mod_base = "2'-hydroxy corresponding nucleoside"

<400> 820
ugccagauau caugguacut t 21

<210> 821
<211> 21
<212> DNA
<213> Artificial sequence

<220>
<223> Description of the artificial sequence: antisense strand of dsRNA

<220>
<221> modified_base
<222> 6, 10, 13, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 7, 8, 9, 11, 12, 14, 15, 16, 17, 19
<223> /mod_base = "2'-hydroxy corresponding nucleoside"

<400> 821
ucugccagau aucaugguat t 21

<210> 822
<211> 21
<212> DNA
<213> Artificial sequence

<220>
<223> Description of the artificial sequence: antisense strand of dsRNA

<220>
<221> modified_base
<222> 7, 11, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 10, 12, 13, 15, 16, 17, 18, 19
<223> /mod_base = "2'-hydroxy corresponding nucleoside"

<400> 822
aucugccaga uaucauggut t 21

<210> 823
<211> 21
<212> DNA
<213> Artificial sequence

<220>
<223> Description of the artificial sequence: antisense strand of dsRNA

<220>
<221> modified_base
<222> 1, 8, 12, 15

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 4, 5, 6, 7, 9, 10, 11, 13, 14, 16, 17, 18, 19
<223> /mod_base = "2'-hydroxy corresponding nucleoside"

<400> 823
caucugccag auaucauggt t 21

<210> 824
<211> 21
<212> DNA
<213> Artificial sequence

<220>
<223> Description of the artificial sequence: antisense strand of dsRNA

<220>
<221> modified_base
<222> 3, 5, 7, 14, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 6, 8, 9, 10, 11, 12, 13, 15, 16, 17, 19
<223> /mod_base = "2'-hydroxy corresponding nucleoside"

<400> 824
cuuauacauc ugccagauat t 21

<210> 825
<211> 21
<212> DNA
<213> Artificial sequence

<220>
<223> Description of the artificial sequence: antisense strand of dsRNA

<220>
<221> modified_base
<222> 4, 6, 8, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 7, 9, 10, 11, 12, 13, 14, 16, 17, 18, 19
<223> /mod_base = "2'-hydroxy corresponding nucleoside"

<400> 825
ucuuauacau cugccagaut t 21

<210> 826
<211> 21
<212> DNA
<213> Artificial sequence

<220>
<223> Description of the artificial sequence: antisense strand of dsRNA

<220>
<221> modified_base
<222> 7, 9, 11, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 10, 12, 13, 14, 15, 16, 17, 19
<223> /mod_base = "2'-hydroxy corresponding nucleoside"

<400> 826
ccuucuuaua caucugccat t 21

<210> 827
<211> 21
<212> DNA
<213> Artificial sequence

<220>
<223> Description of the artificial sequence: antisense strand of dsRNA

<220>
<221> modified_base
<222> 1, 11
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 4, 5, 6, 7, 8, 9, 10, 12, 13, 14, 15, 16, 17, 18, 19
<223> /mod_base = "2'-hydroxy corresponding nucleoside"

<400> 827

cagggauucc cagugcugat t 21

<210> 828
<211> 21
<212> DNA
<213> Artificial sequence

<220>
<223> Description of the artificial sequence: antisense strand of dsRNA

<220>
<221> modified_base
<222> 4, 9, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 7, 8, 10, 11, 12, 13, 14, 15, 17, 19
<223> /mod_base = "2'-hydroxy corresponding nucleoside"

<400> 828
agccagaaca ugggauauat t 21

<210> 829
<211> 21
<212> DNA
<213> Artificial sequence

<220>
<223> Description of the artificial sequence: antisense strand of dsRNA

<220>
<221> modified_base
<222> 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 14, 15, 16, 17, 18, 19
<223> /mod_base = "2'-hydroxy corresponding nucleoside"

<400> 829
agcgggcuuc uguaaucugt t 21

<210> 830
<211> 21
<212> DNA
<213> Artificial sequence

<220>
<223> Description of the artificial sequence: antisense strand of dsRNA

<220>
<221> modified_base
<222> 3, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 8, 9, 10, 11, 12, 13, 14, 15, 17, 18, 19
<223> /mod_base = "2'-hydroxy corresponding nucleoside"

<400> 830
aacagcgggc uucuguaaut t 21

<210> 831
<211> 21
<212> DNA
<213> Artificial sequence

<220>
<223> Description of the artificial sequence: antisense strand of dsRNA

<220>
<221> modified_base
<222> 4, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 7, 8, 9, 10, 11, 12, 13, 14, 15, 16, 18, 19
<223> /mod_base = "2'-hydroxy corresponding nucleoside"

<400> 831
aaacagcggg cuucuguaat t 21

<210> 832
<211> 21
<212> DNA
<213> Artificial sequence

<220>
<223> Description of the artificial sequence: antisense strand of dsRNA

<220>
<221> modified_base
<222> 5, 18

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 8, 9, 10, 11, 12, 13, 14, 15, 16, 17, 19
<223> /mod_base = "2'-hydroxy corresponding nucleoside"

<400> 832
gaaacagcgg gcuucuguat t 21

<210> 833
<211> 21
<212> DNA
<213> Artificial sequence

<220>
<223> Description of the artificial sequence: antisense strand of dsRNA

<220>
<221> modified_base
<222> 1, 7
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 4, 5, 6, 8, 9, 10, 11, 12, 13, 14, 15, 16, 17, 18, 19
<223> /mod_base = "2'-hydroxy corresponding nucleoside"

<400> 833
uagaaacagc gggcuucugt t 21

<210> 834
<211> 21
<212> DNA
<213> Artificial sequence

<220>
<223> Description of the artificial sequence: antisense strand of dsRNA

<220>
<221> modified_base
<222> 8
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 9, 10, 11, 12, 13, 14, 15, 16, 17, 18, 19
<223> /mod_base = "2'-hydroxy corresponding nucleoside"

<400> 834
auagaaacag cgggcuucut t 21

<210> 835
<211> 21
<212> DNA
<213> Artificial sequence

<220>
<223> Description of the artificial sequence: antisense strand of dsRNA

<220>
<221> modified_base
<222> 4, 10
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 7, 8, 9, 11, 12, 13, 14, 15, 16, 17, 18, 19
<223> /mod_base = "2'-hydroxy corresponding nucleoside"

<400> 835
ccauagaaac agcgggcuut t 21

<210> 836
<211> 21
<212> DNA
<213> Artificial sequence

<220>
<223> Description of the artificial sequence: antisense strand of dsRNA

<220>
<221> modified_base
<222> 14, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 13, 15, 17, 18, 19
<223> /mod_base = "2'-hydroxy corresponding nucleoside"

<400> 836

gcaauuugga agccauagat t 21

<210> 837
<211> 21
<212> DNA
<213> Artificial sequence

<220>
<223> Description of the artificial sequence: antisense strand of dsRNA

<220>
<221> modified_base
<222> 3, 15, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 8, 9, 10, 11, 12, 13, 14, 16, 18, 19
<223> /mod_base = "2'-hydroxy corresponding nucleoside"

<400> 837
ggcaauuugg aagccauagt t 21

<210> 838
<211> 21
<212> DNA
<213> Artificial sequence

<220>
<223> Description of the artificial sequence: antisense strand of dsRNA

<220>
<221> modified_base
<222> 4, 9
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 7, 8, 10, 11, 12, 13, 14, 15, 16, 17, 18, 19
<223> /mod_base = "2'-hydroxy corresponding nucleoside"

<400> 838
uuccauggca auuuggaagt t 21

<210> 839
<211> 21
<212> DNA
<213> Artificial sequence

<220>
<223> Description of the artificial sequence: antisense strand of dsRNA

<220>
<221> modified_base
<222> 6, 8, 10, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 7, 9, 11, 12, 13, 14, 16, 17, 18, 19
<223> /mod_base = "2'-hydroxy corresponding nucleoside"

<400> 839
aagacuauac auuucagaat t 21

<210> 840
<211> 21
<212> DNA
<213> Artificial sequence

<220>
<223> Description of the artificial sequence: antisense strand of dsRNA

<220>
<221> modified_base
<222> 3, 12, 14, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 8, 9, 10, 11, 13, 15, 17, 18, 19
<223> /mod_base = "2'-hydroxy corresponding nucleoside"

<400> 840
aauaagaaga cuauacauut t 21

<210> 841
<211> 21
<212> DNA
<213> Artificial sequence

<220>
<223> Description of the artificial sequence: antisense strand of dsRNA

<220>
<221> modified_base
<222> 9, 12

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 10, 11, 13, 14, 15, 16, 17, 18, 19
<223> /mod_base = "2'-hydroxy corresponding nucleoside"

<400> 841
guaaguguca auaagaagat t 21

<210> 842
<211> 21
<212> DNA
<213> Artificial sequence

<220>
<223> Description of the artificial sequence: antisense strand of dsRNA

<220>
<221> modified_base
<222> 3, 10, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 8, 9, 11, 12, 14, 15, 16, 17, 18, 19
<223> /mod_base = "2'-hydroxy corresponding nucleoside"

<400> 842
uguaaguguc aauaagaagt t 21

<210> 843
<211> 21
<212> DNA
<213> Artificial sequence

<220>
<223> Description of the artificial sequence: antisense strand of dsRNA

<220>
<221> modified_base
<222> 4, 11, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 7, 8, 9, 10, 12, 13, 15, 16, 17, 18, 19
<223> /mod_base = "2'-hydroxy corresponding nucleoside"

<400> 843
auguaagugu caauaagaat t 21

<210> 844
<211> 21
<212> DNA
<213> Artificial sequence

<220>
<223> Description of the artificial sequence: antisense strand of dsRNA

<220>
<221> modified_base
<222> 7, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 10, 11, 12, 14, 15, 16, 17, 18, 19
<223> /mod_base = "2'-hydroxy corresponding nucleoside"

<400> 844
ucguuucaau ggcauugaat t 21

<210> 845
<211> 21
<212> DNA
<213> Artificial sequence

<220>
<223> Description of the artificial sequence: antisense strand of dsRNA

<220>
<221> modified_base
<222> 7, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 10, 11, 12, 13, 14, 16, 17, 18, 19
<223> /mod_base = "2'-hydroxy corresponding nucleoside"

<400> 845

acaaggcauc guuucaaugt t 21

<210> 846
<211> 21
<212> DNA
<213> Artificial sequence

<220>
<223> Description of the artificial sequence: antisense strand of dsRNA

<220>
<221> modified_base
<222> 10, 12, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 11, 13, 14, 15, 16, 18, 19
<223> /mod_base = "2'-hydroxy corresponding nucleoside"

<400> 846
ucuucuugac acaaggcaut t 21

<210> 847
<211> 21
<212> DNA
<213> Artificial sequence

<220>
<223> Description of the artificial sequence: antisense strand of dsRNA

<220>
<221> modified_base
<222> 1, 6, 11, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 4, 5, 7, 8, 9, 10, 12, 13, 14, 15, 16, 17, 19
<223> /mod_base = "2'-hydroxy corresponding nucleoside"

<400> 847
caauccagcg caaggcccat t 21

<210> 848
<211> 21
<212> DNA
<213> Artificial sequence

<220>
<223> Description of the artificial sequence: antisense strand of dsRNA

<220>
<221> modified_base
<222> 7, 12
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 10, 11, 13, 14, 15, 16, 17, 18, 19
<223> /mod_base = "2'-hydroxy corresponding nucleoside"

<400> 848
ccaauccagc gcaaggccct t 21

<210> 849
<211> 21
<212> DNA
<213> Artificial sequence

<220>
<223> Description of the artificial sequence: antisense strand of dsRNA

<220>
<221> modified_base
<222> 3, 8, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 9, 10, 11, 12, 14, 15, 16, 17, 18, 19
<223> /mod_base = "2'-hydroxy corresponding nucleoside"

<400> 849
cccaauccag cgcaaggcct t 21

<210> 850
<211> 21
<212> DNA
<213> Artificial sequence

<220>
<223> Description of the artificial sequence: antisense strand of dsRNA

<220>
<221> modified_base
<222> 5, 7, 11

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 8, 9, 10, 12, 13, 14, 15, 16, 17, 18, 19
<223> /mod_base = "2'-hydroxy corresponding nucleoside"

<400> 850
ucaccauagg uagccucuut t 21

<210> 851
<211> 21
<212> DNA
<213> Artificial sequence

<220>
<223> Description of the artificial sequence: antisense strand of dsRNA

<220>
<221> modified_base
<222> 3, 6
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 7, 8, 9, 10, 11, 12, 13, 14, 15, 16, 17, 18, 19
<223> /mod_base = "2'-hydroxy corresponding nucleoside"

<400> 851
ggcaucaguc cucguuucut t 21

<210> 852
<211> 21
<212> DNA
<213> Artificial sequence

<220>
<223> Description of the artificial sequence: antisense strand of dsRNA

<220>
<221> modified_base
<222> 4, 7
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 8, 9, 10, 11, 12, 13, 14, 15, 16, 17, 18, 19
<223> /mod_base = "2'-hydroxy corresponding nucleoside"

<400> 852
aggcaucagu ccucguuuct t 21

<210> 853
<211> 21
<212> DNA
<213> Artificial sequence

<220>
<223> Description of the artificial sequence: antisense strand of dsRNA

<220>
<221> modified_base
<222> 7, 11, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 10, 12, 13, 15, 16, 17, 18, 19
<223> /mod_base = "2'-hydroxy corresponding nucleoside"

<400> 853
ugaggccagg caucagucct t 21

<210> 854
<211> 21
<212> DNA
<213> Artificial sequence

<220>
<223> Description of the artificial sequence: antisense strand of dsRNA

<220>
<221> modified_base
<222> 8, 12, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 9, 10, 11, 13, 14, 16, 17, 18, 19
<223> /mod_base = "2'-hydroxy corresponding nucleoside"

<400> 854

gugaggccag gcaucaguct t 21

<210> 855
<211> 21
<212> DNA
<213> Artificial sequence

<220>
<223> Description of the artificial sequence: antisense strand of dsRNA

<220>
<221> modified_base
<222> 11, 15, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 12, 13, 14, 16, 17, 19
<223> /mod_base = "2'-hydroxy corresponding nucleoside"

<400> 855
aaugugaggc caggcaucat t 21

<210> 856
<211> 21
<212> DNA
<213> Artificial sequence

<220>
<223> Description of the artificial sequence: antisense strand of dsRNA

<220>
<221> modified_base
<222> 4, 12
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 7, 8, 9, 10, 11, 13, 14, 15, 16, 17, 18, 19
<223> /mod_base = "2'-hydroxy corresponding nucleoside"

<400> 856
guguaaaccc ucaucucugt t 21

<210> 857
<211> 21
<212> DNA
<213> Artificial sequence

<220>
<223> Description of the artificial sequence: antisense strand of dsRNA

<220>
<221> modified_base
<222> 1, 3, 8, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 4, 5, 6, 7, 9, 10, 11, 12, 13, 14, 15, 17, 18, 19
<223> /mod_base = "2'-hydroxy corresponding nucleoside"

<400> 857
cacaguguaa acccucauct t 21

<210> 858
<211> 21
<212> DNA
<213> Artificial sequence

<220>
<223> Description of the artificial sequence: antisense strand of dsRNA

<220>
<221> modified_base
<222> 10, 13, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 11, 12, 14, 15, 16, 18, 19
<223> /mod_base = "2'-hydroxy corresponding nucleoside"

<400> 858
guguuugaac aucaggcaat t 21

<210> 859
<211> 21
<212> DNA
<213> Artificial sequence

<220>
<223> Description of the artificial sequence: antisense strand of dsRNA

<220>
<221> modified_base
<222> 12, 15

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 13, 14, 16, 17, 18, 19
<223> /mod_base = "2'-hydroxy corresponding nucleoside"

<400> 859
agguguuuga acaucaggct t 21

<210> 860
<211> 21
<212> DNA
<213> Artificial sequence

<220>
<223> Description of the artificial sequence: antisense strand of dsRNA

<220>
<221> modified_base
<222> 1, 13, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 4, 5, 6, 7, 8, 9, 10, 11, 12, 14, 15, 17, 18, 19
<223> /mod_base = "2'-hydroxy corresponding nucleoside"

<400> 860
cagguguuug aacaucaggt t 21

<210> 861
<211> 21
<212> DNA
<213> Artificial sequence

<220>
<223> Description of the artificial sequence: antisense strand of dsRNA

<220>
<221> modified_base
<222> 3, 15, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 8, 9, 10, 11, 12, 13, 14, 16, 17, 19
<223> /mod_base = "2'-hydroxy corresponding nucleoside"

<400> 861
accagguguu ugaacaucat t 21

<210> 862
<211> 21
<212> DNA
<213> Artificial sequence

<220>
<223> Description of the artificial sequence: antisense strand of dsRNA

<220>
<221> modified_base
<222> 5, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 8, 9, 10, 11, 12, 13, 14, 15, 16, 18, 19
<223> /mod_base = "2'-hydroxy corresponding nucleoside"

<400> 862
guaccaggug uuugaacaut t 21

<210> 863
<211> 21
<212> DNA
<213> Artificial sequence

<220>
<223> Description of the artificial sequence: antisense strand of dsRNA

<220>
<221> modified_base
<222> 3, 6, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 7, 8, 9, 10, 11, 12, 13, 14, 15, 16, 17, 19
<223> /mod_base = "2'-hydroxy corresponding nucleoside"

<400> 863

uguaccaggu guuugaacat t 21

<210> 864
<211> 21
<212> DNA
<213> Artificial sequence

<220>
<223> Description of the artificial sequence: antisense strand of dsRNA

<220>
<221> modified_base
<222> 5, 8
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 9, 10, 11, 12, 13, 14, 15, 16, 17, 18, 19
<223> /mod_base = "2'-hydroxy corresponding nucleoside"

<400> 864
uguguaccag guguuugaat t 21

<210> 865
<211> 21
<212> DNA
<213> Artificial sequence

<220>
<223> Description of the artificial sequence: antisense strand of dsRNA

<220>
<221> modified_base
<222> 1, 9, 12, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 4, 5, 6, 7, 8, 10, 11, 13, 14, 15, 16, 17, 19
<223> /mod_base = "2'-hydroxy corresponding nucleoside"

<400> 865
uauccgaaca gcauugauat t 21

<210> 866
<211> 21
<212> DNA
<213> Artificial sequence

<220>
<223> Description of the artificial sequence: antisense strand of dsRNA

<220>
<221> modified_base
<222> 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 13, 14, 16, 17, 18, 19
<223> /mod_base = "2'-hydroxy corresponding nucleoside"

<400> 866
aggaacuccu guucuaucct t 21

<210> 867
<211> 21
<212> DNA
<213> Artificial sequence

<220>
<223> Description of the artificial sequence: antisense strand of dsRNA

<220>
<221> modified_base
<222> 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 13, 14, 15, 16, 18, 19
<223> /mod_base = "2'-hydroxy corresponding nucleoside"

<400> 867
ugaggaacuc cuguucuaut t 21

<210> 868
<211> 21
<212> DNA
<213> Artificial sequence

<220>
<223> Description of the artificial sequence: antisense strand of dsRNA

<220>
<221> modified_base
<222> 1

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2..19
<223> /mod_base = "2'-hydroxy corresponding nucleoside"

<400> 868
cagugaggaa cuccuguuct t 21

<210> 869
<211> 21
<212> DNA
<213> Artificial sequence

<220>
<223> Description of the artificial sequence: antisense strand of dsRNA

<220>
<221> modified_base
<222> 4, 6, 10, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 7, 8, 9, 11, 12, 13, 14, 15, 16, 17, 19
<223> /mod_base = "2'-hydroxy corresponding nucleoside"

<400> 869
cuucacaggc aaggccucat t 21

<210> 870
<211> 21
<212> DNA
<213> Artificial sequence

<220>
<223> Description of the artificial sequence: antisense strand of dsRNA

<220>
<221> modified_base
<222> 7, 10, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 11, 12, 13, 14, 15, 16, 18, 19
<223> /mod_base = "2'-hydroxy corresponding nucleoside"

<400> 870
uugcuucauu agagugcaat t 21

<210> 871
<211> 21
<212> DNA
<213> Artificial sequence

<220>
<223> Description of the artificial sequence: antisense strand of dsRNA

<220>
<221> modified_base
<222> 8, 11, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 9, 10, 12, 13, 14, 15, 16, 17, 19
<223> /mod_base = "2'-hydroxy corresponding nucleoside"

<400> 871
auugcuucau uagagugcat t 21

<210> 872
<211> 21
<212> DNA
<213> Artificial sequence

<220>
<223> Description of the artificial sequence: antisense strand of dsRNA

<220>
<221> modified_base
<222> 5, 13, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 8, 9, 10, 11, 12, 14, 15, 17, 18, 19
<223> /mod_base = "2'-hydroxy corresponding nucleoside"

<400> 872

aauguauugc uucauuagat t 21

<210> 873
<211> 21
<212> DNA
<213> Artificial sequence

<220>
<223> Description of the artificial sequence: antisense strand of dsRNA

<220>
<221> modified_base
<222> 3, 8, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 9, 10, 11, 12, 13, 14, 15, 17, 18, 19
<223> /mod_base = "2'-hydroxy corresponding nucleoside"

<400> 873
cucaauguau ugcuucauut t 21

<210> 874
<211> 21
<212> DNA
<213> Artificial sequence

<220>
<223> Description of the artificial sequence: antisense strand of dsRNA

<220>
<221> modified_base
<222> 4, 10, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 7, 8, 9, 11, 12, 13, 14, 16, 17, 18, 19
<223> /mod_base = "2'-hydroxy corresponding nucleoside"

<400> 874
ccacaaacuc aauguauugt t 21

<210> 875
<211> 21
<212> DNA
<213> Artificial sequence

<220>
<223> Description of the artificial sequence: antisense strand of dsRNA

<220>
<221> modified_base
<222> 5, 7, 13, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 8, 9, 10, 11, 12, 14, 15, 16, 17, 19
<223> /mod_base = "2'-hydroxy corresponding nucleoside"

<400> 875
cugccacaaa cucaauguat t 21

<210> 876
<211> 21
<212> DNA
<213> Artificial sequence

<220>
<223> Description of the artificial sequence: antisense strand of dsRNA

<220>
<221> modified_base
<222> 7, 9, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 10, 11, 12, 13, 14, 16, 17, 18, 19
<223> /mod_base = "2'-hydroxy corresponding nucleoside"

<400> 876
gucugccaca aacucaaugt t 21

<210> 877
<211> 21
<212> DNA
<213> Artificial sequence

<220>
<223> Description of the artificial sequence: antisense strand of dsRNA

<220>
<221> modified_base
<222> 1, 3, 16, 18

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 4, 5, 6, 7, 8, 9, 10, 11, 12, 13, 14, 15, 17, 19
<223> /mod_base = "2'-hydroxy corresponding nucleoside"

<400> 877
cauaagucug ucugccacat t 21

<210> 878
<211> 21
<212> DNA
<213> Artificial sequence

<220>
<223> Description of the artificial sequence: antisense strand of dsRNA

<220>
<221> modified_base
<222> 1, 4, 6
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 5, 7, 8, 9, 10, 11, 12, 13, 14, 15, 16, 17, 18, 19
<223> /mod_base = "2'-hydroxy corresponding nucleoside"

<400> 878
cagcauaagu cugucugcct t 21

<210> 879
<211> 21
<212> DNA
<213> Artificial sequence

<220>
<223> Description of the artificial sequence: antisense strand of dsRNA

<220>
<221> modified_base
<222> 8, 11, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 9, 10, 12, 14, 15, 16, 17, 18, 19
<223> /mod_base = "2'-hydroxy corresponding nucleoside"

<400> 879
ccaguuccag cauaagucut t 21

<210> 880
<211> 21
<212> DNA
<213> Artificial sequence

<220>
<223> Description of the artificial sequence: antisense strand of dsRNA

<220>
<221> modified_base
<222> 4, 10, 13, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 7, 8, 9, 11, 12, 14, 16, 17, 18, 19
<223> /mod_base = "2'-hydroxy corresponding nucleoside"

<400> 880
acccaguucc agcauaagut t 21

<210> 881
<211> 21
<212> DNA
<213> Artificial sequence

<220>
<223> Description of the artificial sequence: antisense strand of dsRNA

<220>
<221> modified_base
<222> 6, 12, 15, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 7, 8, 9, 10, 11, 13, 14, 16, 18, 19
<223> /mod_base = "2'-hydroxy corresponding nucleoside"

<400> 881

aaacccaguu ccagcauaat t 21

<210> 882
<211> 21
<212> DNA
<213> Artificial sequence

<220>
<223> Description of the artificial sequence: antisense strand of dsRNA

<220>
<221> modified_base
<222> 11
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 12, 13, 14, 15, 16, 17, 18, 19
<223> /mod_base = "2'-hydroxy corresponding nucleoside"

<400> 882
auggguucuc uacucugaat t 21

<210> 883
<211> 21
<212> DNA
<213> Artificial sequence

<220>
<223> Description of the artificial sequence: antisense strand of dsRNA

<220>
<221> modified_base
<222> 1, 8, 10, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 4, 5, 6, 7, 9, 11, 12, 13, 14, 15, 17, 18, 19
<223> /mod_base = "2'-hydroxy corresponding nucleoside"

<400> 883
uauucuccau aaagucaaat t 21

<210> 884
<211> 21
<212> DNA
<213> Artificial sequence

<220>
<223> Description of the artificial sequence: antisense strand of dsRNA

<220>
<221> modified_base
<222> 7
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 10, 11, 12, 13, 14, 15, 16, 17, 18, 19
<223> /mod_base = "2'-hydroxy corresponding nucleoside"

<400> 884
agaaguuagu cuuuccuuct t 21

<210> 885
<211> 21
<212> DNA
<213> Artificial sequence

<220>
<223> Description of the artificial sequence: antisense strand of dsRNA

<220>
<221> modified_base
<222> 9
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 10, 11, 12, 13, 14, 15, 16, 17, 18, 19
<223> /mod_base = "2'-hydroxy corresponding nucleoside"

<400> 885
aaagaaguua gucuuuccut t 21

<210> 886
<211> 21
<212> DNA
<213> Artificial sequence

<220>
<223> Description of the artificial sequence: antisense strand of dsRNA

<220>
<221> modified_base
<222> 3, 12

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 8, 9, 10, 11, 13, 14, 15, 16, 17, 18, 19
<223> /mod_base = "2'-hydroxy corresponding nucleoside"

<400> 886
cucaaagaag uuagucuuut t 21

<210> 887
<211> 21
<212> DNA
<213> Artificial sequence

<220>
<223> Description of the artificial sequence: antisense strand of dsRNA

<220>
<221> modified_base
<222> 8
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 9, 10, 11, 12, 13, 14, 15, 16, 17, 18, 19
<223> /mod_base = "2'-hydroxy corresponding nucleoside"

<400> 887
acucgccuac ucucuucuct t 21

<210> 888
<211> 21
<212> DNA
<213> Artificial sequence

<220>
<223> Description of the artificial sequence: antisense strand of dsRNA

<220>
<221> modified_base
<222> 3, 11
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 8, 9, 10, 12, 13, 14, 15, 16, 17, 18, 19
<223> /mod_base = "2'-hydroxy corresponding nucleoside"

<400> 888
gauacucgcc uacucucuut t 21

<210> 889
<211> 21
<212> DNA
<213> Artificial sequence

<220>
<223> Description of the artificial sequence: antisense strand of dsRNA

<220>
<221> modified_base
<222> 6, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 7, 8, 9, 10, 11, 12, 13, 15, 16, 17, 18, 19
<223> /mod_base = "2'-hydroxy corresponding nucleoside"

<400> 889
ucugauacuc gccuacucut t 21

<210> 890
<211> 21
<212> DNA
<213> Artificial sequence

<220>
<223> Description of the artificial sequence: antisense strand of dsRNA

<220>
<221> modified_base
<222> 7, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 10, 11, 12, 13, 14, 16, 17, 18, 19
<223> /mod_base = "2'-hydroxy corresponding nucleoside"

<400> 890

cucugauacu cgccuacuct t 21

<210> 891
<211> 21
<212> DNA
<213> Artificial sequence

<220>
<223> Description of the artificial sequence: antisense strand of dsRNA

<220>
<221> modified_base
<222> 9, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 10, 11, 12, 13, 14, 15, 16, 18, 19
<223> /mod_base = "2'-hydroxy corresponding nucleoside"

<400> 891
uccucugaua cucgccuact t 21

<210> 892
<211> 21
<212> DNA
<213> Artificial sequence

<220>
<223> Description of the artificial sequence: antisense strand of dsRNA

<220>
<221> modified_base
<222> 10, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 11, 12, 13, 14, 15, 16, 17, 19
<223> /mod_base = "2'-hydroxy corresponding nucleoside"

<400> 892
auccucugau acucgccuat t 21

<210> 893
<211> 21
<212> DNA
<213> Artificial sequence

<220>
<223> Description of the artificial sequence: antisense strand of dsRNA

<220>
<221> modified_base
<222> 1, 11
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 4, 5, 6, 7, 8, 9, 10, 12, 13, 14, 15, 16, 17, 18, 19
<223> /mod_base = "2'-hydroxy corresponding nucleoside"

<400> 893
cauccucuga uacucgccut t 21

<210> 894
<211> 21
<212> DNA
<213> Artificial sequence

<220>
<223> Description of the artificial sequence: antisense strand of dsRNA

<220>
<221> modified_base
<222> 12
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 13, 14, 15, 16, 17, 18, 19
<223> /mod_base = "2'-hydroxy corresponding nucleoside"

<400> 894
ccauccucug auacucgcct t 21

<210> 895
<211> 21
<212> DNA
<213> Artificial sequence

<220>
<223> Description of the artificial sequence: antisense strand of dsRNA

<220>
<221> modified_base
<222> 3, 13

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 8, 9, 10, 11, 12, 14, 15, 16, 17, 18, 19
<223> /mod_base = "2'-hydroxy corresponding nucleoside"

<400> 895
cccauccucu gauacucgct t 21

<210> 896
<211> 21
<212> DNA
<213> Artificial sequence

<220>
<223> Description of the artificial sequence: antisense strand of dsRNA

<220>
<221> modified_base
<222> 7, 10, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 11, 12, 13, 14, 15, 17, 18, 19
<223> /mod_base = "2'-hydroxy corresponding nucleoside"

<400> 896
acuugacauc acucccauct t 21

<210> 897
<211> 21
<212> DNA
<213> Artificial sequence

<220>
<223> Description of the artificial sequence: antisense strand of dsRNA

<220>
<221> modified_base
<222> 5, 8, 12, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 9, 10, 11, 13, 14, 15, 16, 18, 19
<223> /mod_base = "2'-hydroxy corresponding nucleoside"

<400> 897
aagucagcau ccaagguaat t 21

<210> 898
<211> 21
<212> DNA
<213> Artificial sequence

<220>
<223> Description of the artificial sequence: antisense strand of dsRNA

<220>
<221> modified_base
<222> 13, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 14, 15, 16, 18, 19
<223> /mod_base = "2'-hydroxy corresponding nucleoside"

<400> 898
cugguuguga gguagcuact t 21

<210> 899
<211> 21
<212> DNA
<213> Artificial sequence

<220>
<223> Description of the artificial sequence: antisense strand of dsRNA

<220>
<221> modified_base
<222> 21
<223> /mod_base = "5'-phosphorothioate thymidine"

<220>
<221> modified_base
<222> 1..19
<223> /mod_base = "2'-hydroxy corresponding nucleoside"

<400> 899
acaggacugg uugugaggut t 21

<210> 900
<211> 21
<212> DNA

<213> Artificial sequence

<220>
<223> Description of the artificial sequence: antisense strand of dsRNA

<220>
<221> modified_base
<222> 5, 8, 11, 14, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 9, 10, 12, 13, 15, 17, 18, 19
<223> /mod_base = "2'-hydroxy corresponding nucleoside"

<400> 900
auacuaccag cacuauaaat t 21

<210> 901
<211> 21
<212> DNA
<213> Artificial sequence

<220>
<223> Description of the artificial sequence: antisense strand of dsRNA

<220>
<221> modified_base
<222> 4, 9, 11, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 7, 8, 10, 12, 13, 14, 16, 17, 18, 19
<223> /mod_base = "2'-hydroxy corresponding nucleoside"

<400> 901
ugguaaguca cagccagcct t 21

<210> 902
<211> 21
<212> DNA
<213> Artificial sequence

<220>
<223> Description of the artificial sequence: antisense strand of dsRNA

<220>

<221> modified_base
<222> 4, 10, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 7, 8, 9, 11, 12, 13, 14, 15, 17, 18, 19
<223> /mod_base = "2'-hydroxy corresponding nucleoside"

<400> 902
ugccauuguc acugcuaugt t 21

<210> 903
<211> 21
<212> DNA
<213> Artificial sequence

<220>
<223> Description of the artificial sequence: antisense strand of dsRNA

<220>
<221> modified_base
<222> 8, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 9, 10, 11, 12, 13, 15, 16, 17, 18, 19
<223> /mod_base = "2'-hydroxy corresponding nucleoside"

<400> 903
agacugccau ugucacugct t 21

<210> 904
<211> 21
<212> DNA
<213> Artificial sequence

<220>
<223> Description of the artificial sequence: antisense strand of dsRNA

<220>
<221> modified_base
<222> 9, 15
<223> /mod_base = "2'-O-methyl corresponding nucleoside"

<220>
<221> modified_base

<222> 21
<223> /mod_base = "5'-phosphorothioate thymidine"

<220>
<221> modified_base
<222> 1, 2, 3, 4, 5, 6, 7, 8, 10, 11, 12, 13, 14, 16, 17, 18, 19
<223> /mod_base = "2'-hydroxy corresponding nucleoside"

<400> 904
aagacugcca uugucacugt t 21

<210> 905
<211> 21
<212> DNA
<213> Artificial sequence

<220>
<223> Description of the artificial sequence: antisense strand of dsRNA

<220>
<221> modified_base
<222> 4, 9, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 7, 8, 10, 11, 12, 13, 14, 16, 17, 18, 19
<223> /mod_base = "2'-hydroxy corresponding nucleoside"

<400> 905
ccucacuuua aagccaagat t 21

<210> 906
<211> 21
<212> DNA
<213> Artificial sequence

<220>
<223> Description of the artificial sequence: antisense strand of dsRNA

<220>
<221> modified_base
<222> 5, 10, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 8, 9, 11, 12, 13, 14, 15, 17, 18, 19

<223> /mod_base = "2'-hydroxy corresponding nucleoside"

<400> 906
cccucacuuu aaagccaagt t 21

<210> 907
<211> 21
<212> DNA
<213> Artificial sequence

<220>
<223> Description of the artificial sequence: antisense strand of dsRNA

<220>
<221> modified_base
<222> 6, 9, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 7, 8, 10, 11, 12, 13, 14, 15, 16, 18, 19
<223> /mod_base = "2'-hydroxy corresponding nucleoside"

<400> 907
aagcucacua aagggucact t 21

<210> 908
<211> 21
<212> DNA
<213> Artificial sequence

<220>
<223> Description of the artificial sequence: antisense strand of dsRNA

<220>
<221> modified_base
<222> 9
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 10, 11, 12, 13, 14, 15, 16, 17, 18, 19
<223> /mod_base = "2'-hydroxy corresponding nucleoside"

<400> 908
cguugaagca gugaggcugt t 21

<210> 909

<211> 21
<212> DNA
<213> Artificial sequence

<220>
<223> Description of the artificial sequence: antisense strand of dsRNA

<220>
<221> modified_base
<222> 11
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 12, 13, 14, 15, 16, 17, 18, 19
<223> /mod_base = "2'-hydroxy corresponding nucleoside"

<400> 909
ugcguugaag cagugaggct t 21

<210> 910
<211> 21
<212> DNA
<213> Artificial sequence

<220>
<223> Description of the artificial sequence: antisense strand of dsRNA

<220>
<221> modified_base
<222> 9, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 10, 11, 12, 13, 14, 15, 16, 18, 19
<223> /mod_base = "2'-hydroxy corresponding nucleoside"

<400> 910
uuuguuugua aagugccagt t 21

<210> 911
<211> 21
<212> DNA
<213> Artificial sequence

<220>
<223> Description of the artificial sequence: antisense strand of dsRNA

<220>
<221> modified_base
<222> 3, 5, 9, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 6, 7, 8, 10, 11, 12, 13, 14, 15, 17, 18, 19
<223> /mod_base = "2'-hydroxy corresponding nucleoside"

<400> 911
aguacaaaca auguuuauut t 21

<210> 912
<211> 21
<212> DNA
<213> Artificial sequence

<220>
<223> Description of the artificial sequence: antisense strand of dsRNA

<220>
<221> modified_base
<222> 4, 6, 10, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 7, 8, 9, 11, 12, 13, 14, 15, 16, 18, 19
<223> /mod_base = "2'-hydroxy corresponding nucleoside"

<400> 912
gaguacaaac aauguuuaut t 21

<210> 913
<211> 21
<212> DNA
<213> Artificial sequence

<220>
<223> Description of the artificial sequence: antisense strand of dsRNA

<220>
<221> modified_base
<222> 7, 9, 13
<223> /mod_base = "2'-O-methyl corresponding nucleoside"

<220>

<221> modified_base
<222> 21
<223> /mod_base = "5'-phosphorothioate thymidine"

<220>
<221> modified_base
<222> 1, 2, 3, 4, 5, 6, 8, 10, 11, 12, 14, 15, 16, 17, 18, 19
<223> /mod_base = "2'-hydroxy corresponding nucleoside"

<400> 913
ugugaguaca aacaauguut t 21

<210> 914
<211> 21
<212> DNA
<213> Artificial sequence

<220>
<223> Description of the artificial sequence: antisense strand of dsRNA

<220>
<221> modified_base
<222> 9, 12
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 10, 11, 13, 14, 15, 16, 17, 18, 19
<223> /mod_base = "2'-hydroxy corresponding nucleoside"

<400> 914
uuggcuccca uuagugguct t 21

<210> 915
<211> 21
<212> DNA
<213> Artificial sequence

<220>
<223> Description of the artificial sequence: antisense strand of dsRNA

<220>
<221> modified_base
<222> 11
<223> /mod_base = "2'-O-methyl corresponding nucleoside"

<220>
<221> modified_base
<222> 21
<223> /mod_base = "5'-phosphorothioate thymidine"

<220>
<221> modified_base

<222> 1, 2, 3, 4, 5, 6, 7, 8, 9, 10, 12, 13, 14, 15, 16, 17, 18, 19
<223> /mod_base = "2'-hydroxy corresponding nucleoside"

<400> 915
ggacugacuu uaaaguuggt t 21

<210> 916
<211> 21
<212> DNA
<213> Artificial sequence

<220>
<223> Description of the artificial sequence: antisense strand of dsRNA

<220>
<221> modified_base
<222> 10, 15, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 11, 12, 13, 14, 16, 17, 19
<223> /mod_base = "2'-hydroxy corresponding nucleoside"

<400> 916
agagaauccc aggacacuat t 21

<210> 917
<211> 21
<212> DNA
<213> Artificial sequence

<220>
<223> Description of the artificial sequence: antisense strand of dsRNA

<220>
<221> modified_base
<222> 5, 10
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 8, 9, 11, 12, 13, 14, 15, 16, 17, 18, 19
<223> /mod_base = "2'-hydroxy corresponding nucleoside"

<400> 917
gauuuaacuu aagaagcuct t 21

<210> 918
<211> 21
<212> DNA
<213> Artificial sequence

<220>
<223> Description of the artificial sequence: antisense strand of dsRNA

<220>
<221> modified_base
<222> 8, 11, 15, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 9, 10, 12, 13, 14, 16, 17, 19
<223> /mod_base = "2'-hydroxy corresponding nucleoside"

<400> 918
uugauuucac cacuuaacat t 21

<210> 919
<211> 21
<212> DNA
<213> Artificial sequence

<220>
<223> Description of the artificial sequence: antisense strand of dsRNA

<220>
<221> modified_base
<222> 10, 13, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 11, 12, 14, 15, 16, 18, 19
<223> /mod_base = "2'-hydroxy corresponding nucleoside"

<400> 919
aguugauuuc accacuuaat t 21

<210> 920
<211> 21
<212> DNA
<213> Artificial sequence

<220>

<223> Description of the artificial sequence: antisense strand of dsRNA

<220>
<221> modified_base
<222> 7, 9, 11, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 10, 12, 13, 14, 15, 17, 18, 19
<223> /mod_base = "2'-hydroxy corresponding nucleoside"

<400> 920
uuaacucaca caaccuacut t 21

<210> 921
<211> 21
<212> DNA
<213> Artificial sequence

<220>
<223> Description of the artificial sequence: antisense strand of dsRNA

<220>
<221> modified_base
<222> 3, 8, 10, 12, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 9, 11, 13, 14, 15, 16, 18, 19
<223> /mod_base = "2'-hydroxy corresponding nucleoside"

<400> 921
auuaacucac acaaccuact t 21

<210> 922
<211> 21
<212> DNA
<213> Artificial sequence

<220>
<223> Description of the artificial sequence: antisense strand of dsRNA

<220>
<221> modified_base
<222> 9, 14, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 10, 11, 12, 13, 15, 17, 19
<223> /mod_base = "2'-hydroxy corresponding nucleoside"

<400> 922
aaaugaauua acucacacat t 21

<210> 923
<211> 21
<212> DNA
<213> Artificial sequence

<220>
<223> Description of the artificial sequence: antisense strand of dsRNA

<220>
<221> modified_base
<222> 11, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 12, 13, 14, 15, 17, 19
<223> /mod_base = "2'-hydroxy corresponding nucleoside"

<400> 923
auaaaugaau uaacucacat t 21

<210> 924
<211> 21
<212> DNA
<213> Artificial sequence

<220>
<223> Description of the artificial sequence: antisense strand of dsRNA

<220>
<221> modified_base
<222> 7, 11, 13
<223> /mod_base = "2'-O-methyl corresponding nucleoside"

<220>
<221> modified_base
<222> 21
<223> /mod_base = "5'-phosphorothioate thymidine"

<220>

<221> modified_base
<222> 1, 2, 3, 4, 5, 6, 8, 9, 10, 12, 14, 15, 16, 17, 18, 19
<223> /mod_base = "2'-hydroxy corresponding nucleoside"

<400> 924
uucucccaaa cauaaaggct t 21

<210> 925
<211> 21
<212> DNA
<213> Artificial sequence

<220>
<223> Description of the artificial sequence: antisense strand of dsRNA

<220>
<221> modified_base
<222> 3, 11
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 8, 9, 10, 12, 13, 14, 15, 16, 17, 18, 19
<223> /mod_base = "2'-hydroxy corresponding nucleoside"

<400> 925
ugcaaugucu cagcuuucut t 21

<210> 926
<211> 21
<212> DNA
<213> Artificial sequence

<220>
<223> Description of the artificial sequence: antisense strand of dsRNA

<220>
<221> modified_base
<222> 5, 8, 12
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 9, 10, 11, 13, 14, 15, 16, 17, 18, 19
<223> /mod_base = "2'-hydroxy corresponding nucleoside"

<400> 926
agaucaacau uuaucucuct t 21

<210> 927
<211> 21
<212> DNA
<213> Artificial sequence

<220>
<223> Description of the artificial sequence: antisense strand of dsRNA

<220>
<221> modified_base
<222> 4, 7, 9, 12, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 8, 10, 11, 13, 15, 16, 17, 18, 19
<223> /mod_base = "2'-hydroxy corresponding nucleoside"

<400> 927
auguaucaua uuacagagat t 21

<210> 928
<211> 21
<212> DNA
<213> Artificial sequence

<220>
<223> Description of the artificial sequence: antisense strand of dsRNA

<220>
<221> modified_base
<222> 6, 12, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 7, 8, 9, 10, 11, 13, 14, 15, 17, 18, 19
<223> /mod_base = "2'-hydroxy corresponding nucleoside"

<400> 928
uggauuaacu uuagguaact t 21

<210> 929
<211> 21
<212> DNA
<213> Artificial sequence

<220>

<223> Description of the artificial sequence: antisense strand of dsRNA

<220>
<221> modified_base
<222> 10, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 11, 12, 13, 14, 15, 17, 18, 19
<223> /mod_base = "2'-hydroxy corresponding nucleoside"

<400> 929
aaucuggauu aacuuuaggt t 21

<210> 930
<211> 21
<212> DNA
<213> Artificial sequence

<220>
<223> Description of the artificial sequence: antisense strand of dsRNA

<220>
<221> modified_base
<222> 10
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 11, 12, 13, 14, 15, 16, 17, 18, 19
<223> /mod_base = "2'-hydroxy corresponding nucleoside"

<400> 930
accugauucc aacuuccgct t 21

<210> 931
<211> 21
<212> DNA
<213> Artificial sequence

<220>
<223> Description of the artificial sequence: antisense strand of dsRNA

<220>
<221> modified_base
<222> 11
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 12, 13, 14, 15, 16, 17, 18, 19
<223> /mod_base = "2'-hydroxy corresponding nucleoside"

<400> 931
aaccugauuc caacuuccgt t 21

<210> 932
<211> 21
<212> DNA
<213> Artificial sequence

<220>
<223> Description of the artificial sequence: antisense strand of dsRNA

<220>
<221> modified_base
<222> 4, 7, 11, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 8, 9, 10, 12, 13, 14, 16, 17, 18, 19
<223> /mod_base = "2'-hydroxy corresponding nucleoside"

<400> 932
ucacauuauu cagcuaaugt t 21

<210> 933
<211> 21
<212> DNA
<213> Artificial sequence

<220>
<223> Description of the artificial sequence: antisense strand of dsRNA

<220>
<221> modified_base
<222> 3, 10, 12, 15
<223> /mod_base = "2'-O-methyl corresponding nucleoside"

<220>
<221> modified_base
<222> 21
<223> /mod_base = "5'-phosphorothioate thymidine"

<220>

<221> modified_base
<222> 1, 2, 4, 5, 6, 7, 8, 9, 11, 13, 14, 16, 17, 18, 19
<223> /mod_base = "2'-hydroxy corresponding nucleoside"

<400> 933
guuaauccuc acauuauuct t 21

<210> 934
<211> 21
<212> DNA
<213> Artificial sequence

<220>
<223> Description of the artificial sequence: antisense strand of dsRNA

<220>
<221> modified_base
<222> 5, 12, 14, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 8, 9, 10, 11, 13, 15, 16, 18, 19
<223> /mod_base = "2'-hydroxy corresponding nucleoside"

<400> 934
aaguuaaucc ucacauuaut t 21

<210> 935
<211> 21
<212> DNA
<213> Artificial sequence

<220>
<223> Description of the artificial sequence: antisense strand of dsRNA

<220>
<221> modified_base
<222> 6, 13, 15, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 7, 8, 9, 10, 11, 12, 14, 16, 17, 19
<223> /mod_base = "2'-hydroxy corresponding nucleoside"

<400> 935
gaaguuaauc cucacauuat t 21

<210> 936
<211> 21
<212> DNA
<213> Artificial sequence

<220>
<223> Description of the artificial sequence: antisense strand of dsRNA

<220>
<221> modified_base
<222> 5
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 8, 9, 10, 11, 12, 13, 14, 15, 16, 17, 18, 19
<223> /mod_base = "2'-hydroxy corresponding nucleoside"

<400> 936
ugauuaggaa auggucugat t 21

<210> 937
<211> 21
<212> DNA
<213> Artificial sequence

<220>
<223> Description of the artificial sequence: antisense strand of dsRNA

<220>
<221> modified_base
<222> 6
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 7, 8, 9, 10, 11, 12, 13, 14, 15, 16, 17, 18, 19
<223> /mod_base = "2'-hydroxy corresponding nucleoside"

<400> 937
cugauuagga aauggucugt t 21

<210> 938
<211> 21
<212> DNA
<213> Artificial sequence

<220>

<223> Description of the artificial sequence: antisense strand of dsRNA

<220>
<221> modified_base
<222> 2, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 7, 8, 9, 10, 11, 12, 13, 14, 16, 17, 18, 19
<223> /mod_base = "2'-hydroxy corresponding nucleoside"

<400> 938
acagcgggcu ucuguaauct t 21

<210> 939
<211> 21
<212> DNA
<213> Artificial sequence

<220>
<223> Description of the artificial sequence: antisense strand of dsRNA

<220>
<221> modified_base
<222> 2, 11, 13, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 7, 8, 9, 10, 12, 14, 16, 17, 18, 19
<223> /mod_base = "2'-hydroxy corresponding nucleoside"

<400> 939
auaagaagac uauacauuut t 21

<210> 940
<211> 21
<212> DNA
<213> Artificial sequence

<220>
<223> Description of the artificial sequence: antisense strand of dsRNA

<220>
<221> modified_base
<222> 2, 15, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 7, 8, 9, 10, 11, 12, 13, 14, 16, 18, 19
<223> /mod_base = "2'-hydroxy corresponding nucleoside"

<400> 940
auaagucugu cugccacaat t 21

<210> 941
<211> 21
<212> DNA
<213> Artificial sequence

<220>
<223> Description of the artificial sequence: antisense strand of dsRNA

<220>
<221> modified_base
<222> 2, 11, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 7, 8, 9, 10, 12, 13, 14, 15, 16, 17, 19
<223> /mod_base = "2'-hydroxy corresponding nucleoside"

<400> 941
guauuuggug uagaaaccat t 21

<210> 942
<211> 21
<212> DNA
<213> Artificial sequence

<220>
<223> Description of the artificial sequence: antisense strand of dsRNA

<220>
<221> modified_base
<222> 2, 7, 11, 15
<223> /mod_base = "2'-O-methyl corresponding nucleoside"

<220>
<221> modified_base
<222> 21
<223> /mod_base = "5'-phosphorothioate thymidine"

<220>

<221> modified_base
<222> 1, 3, 4, 5, 6, 8, 9, 10, 12, 13, 14, 16, 17, 18, 19
<223> /mod_base = "2'-hydroxy corresponding nucleoside"

<400> 942
uuacuuuagu cacuuagugt t 21

<210> 943
<211> 21
<212> DNA
<213> Artificial sequence

<220>
<223> Description of the artificial sequence: antisense strand of dsRNA

<220>
<221> modified_base
<222> 2, 4, 6, 12, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 5, 7, 8, 9, 10, 11, 13, 14, 15, 16, 18, 19
<223> /mod_base = "2'-hydroxy corresponding nucleoside"

<400> 943
cuacacaagu uuaacuuact t 21

<210> 944
<211> 21
<212> DNA
<213> Artificial sequence

<220>
<223> Description of the artificial sequence: antisense strand of dsRNA

<220>
<221> modified_base
<222> 2, 7, 9, 11, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 8, 10, 12, 13, 14, 15, 16, 18, 19
<223> /mod_base = "2'-hydroxy corresponding nucleoside"

<400> 944
uuagucuaca caaguuuaat t 21

<210> 945
<211> 21
<212> DNA
<213> Artificial sequence

<220>
<223> Description of the artificial sequence: antisense strand of dsRNA

<220>
<221> modified_base
<222> 2, 8, 13, 15, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 7, 9, 10, 11, 12, 14, 16, 18, 19
<223> /mod_base = "2'-hydroxy corresponding nucleoside"

<400> 945
acaugcuuag ucuacacaat t 21

<210> 946
<211> 21
<212> DNA
<213> Artificial sequence

<220>
<223> Description of the artificial sequence: antisense strand of dsRNA

<220>
<221> modified_base
<222> 2, 7, 12, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 8, 9, 10, 11, 13, 14, 15, 16, 17, 19
<223> /mod_base = "2'-hydroxy corresponding nucleoside"

<400> 946
uuaauucaaa guagcccuat t 21

<210> 947
<211> 21
<212> DNA
<213> Artificial sequence

<220>

<223> Description of the artificial sequence: antisense strand of dsRNA

<220>
<221> modified_base
<222> 2, 8, 12, 15, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 7, 9, 10, 11, 13, 14, 16, 17, 19
<223> /mod_base = "2'-hydroxy corresponding nucleoside"

<400> 947
uuaacuuuag guaacaauat t 21

<210> 948
<211> 21
<212> DNA
<213> Artificial sequence

<220>
<223> Description of the artificial sequence: antisense strand of dsRNA

<220>
<221> modified_base
<222> 2, 8
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 7, 9, 10, 11, 12, 13, 14, 15, 16, 17, 18, 19
<223> /mod_base = "2'-hydroxy corresponding nucleoside"

<400> 948
auagaaacag cgggcuucut t 21

<210> 949
<211> 21
<212> DNA
<213> Artificial sequence

<220>
<223> Description of the artificial sequence: antisense strand of dsRNA

<220>
<221> modified_base
<222> 2, 9, 12
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 7, 8, 10, 11, 13, 14, 15, 16, 17, 18, 19
<223> /mod_base = "2'-hydroxy corresponding nucleoside"

<400> 949
guaaguguca auaagaagat t 21

<210> 950
<211> 21
<212> DNA
<213> Artificial sequence

<220>
<223> Description of the artificial sequence: antisense strand of dsRNA

<220>
<221> modified_base
<222> 2, 7, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 8, 9, 10, 11, 12, 13, 14, 16, 17, 18, 19
<223> /mod_base = "2'-hydroxy corresponding nucleoside"

<400> 950
acaaggcauc guuucaaugt t 21

<210> 951
<211> 21
<212> DNA
<213> Artificial sequence

<220>
<223> Description of the artificial sequence: antisense strand of dsRNA

<220>
<221> modified_base
<222> 2, 5, 7, 11
<223> /mod_base = "2'-O-methyl corresponding nucleoside"

<220>
<221> modified_base
<222> 21
<223> /mod_base = "5'-phosphorothioate thymidine"

<220>

<221> modified_base
<222> 1, 3, 4, 6, 8, 9, 10, 12, 13, 14, 15, 16, 17, 18, 19
<223> /mod_base = "2'-hydroxy corresponding nucleoside"

<400> 951
ucaccauagg uagccucuut t 21

<210> 952
<211> 21
<212> DNA
<213> Artificial sequence

<220>
<223> Description of the artificial sequence: antisense strand of dsRNA

<220>
<221> modified_base
<222> 2, 5, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 6, 7, 8, 9, 10, 11, 12, 13, 14, 15, 16, 18, 19
<223> /mod_base = "2'-hydroxy corresponding nucleoside"

<400> 952
guaccaggug uuugaacaut t 21

<210> 953
<211> 21
<212> DNA
<213> Artificial sequence

<220>
<223> Description of the artificial sequence: antisense strand of dsRNA

<220>
<221> modified_base
<222> 2, 4, 10, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 5, 6, 7, 8, 9, 11, 12, 13, 14, 16, 17, 18, 19
<223> /mod_base = "2'-hydroxy corresponding nucleoside"

<400> 953
ccacaaacuc aauguauugt t 21

<210> 954
<211> 21
<212> DNA
<213> Artificial sequence

<220>
<223> Description of the artificial sequence: antisense strand of dsRNA

<220>
<221> modified_base
<222> 2, 8, 11, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 7, 9, 10, 12, 14, 15, 16, 17, 18, 19
<223> /mod_base = "2'-hydroxy corresponding nucleoside"

<400> 954
ccaguuccag cauaagucut t 21

<210> 955
<211> 21
<212> DNA
<213> Artificial sequence

<220>
<223> Description of the artificial sequence: antisense strand of dsRNA

<220>
<221> modified_base
<222> 2, 12
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 7, 8, 9, 10, 11, 13, 14, 15, 16, 17, 18, 19
<223> /mod_base = "2'-hydroxy corresponding nucleoside"

<400> 955
ccauccucug auacucgcct t 21

<210> 956
<211> 21
<212> DNA
<213> Artificial sequence

<220>

<223> Description of the artificial sequence: antisense strand of dsRNA

<220>
<221> modified_base
<222> 2
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 7, 8, 9, 10, 11, 12, 13, 14, 15, 16, 17, 18, 19
<223> /mod_base = "2'-hydroxy corresponding nucleoside"

<400> 956
acaggacugg uugugaggut t 21

<210> 957
<211> 21
<212> DNA
<213> Artificial sequence

<220>
<223> Description of the artificial sequence: antisense strand of dsRNA

<220>
<221> modified_base
<222> 2, 5, 8, 11, 14, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 6, 7, 9, 10, 12, 13, 15, 17, 18, 19
<223> /mod_base = "2'-hydroxy corresponding nucleoside"

<400> 957
auacuaccag cacuauaaat t 21

<210> 958
<211> 21
<212> DNA
<213> Artificial sequence

<220>
<223> Description of the artificial sequence: antisense strand of dsRNA

<220>
<221> modified_base
<222> 2, 7, 9, 11, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 8, 10, 12, 13, 14, 15, 17, 18, 19
<223> /mod_base = "2'-hydroxy corresponding nucleoside"

<400> 958
uuaacucaca caaccuacut t 21

<210> 959
<211> 21
<212> DNA
<213> Artificial sequence

<220>
<223> Description of the artificial sequence: antisense strand of dsRNA

<220>
<221> modified_base
<222> 2, 11, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 7, 8, 9, 10, 12, 13, 14, 15, 17, 19
<223> /mod_base = "2'-hydroxy corresponding nucleoside"

<400> 959
auaaaugaau uaacucacat t 21

<210> 960
<211> 21
<212> DNA
<213> Artificial sequence

<220>
<223> Description of the artificial sequence: antisense strand of dsRNA

<220>
<221> modified_base
<222> 2, 4, 7, 11, 15
<223> /mod_base = "2'-O-methyl corresponding nucleoside"

<220>
<221> modified_base
<222> 21
<223> /mod_base = "5'-phosphorothioate thymidine"

<220>

<221> modified_base
<222> 1, 3, 5, 6, 8, 9, 10, 12, 13, 14, 16, 17, 18, 19
<223> /mod_base = "2'-hydroxy corresponding nucleoside"

<400> 960
ucacauuauu cagcuaaugt t 21

<210> 961
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 8, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 9, 10, 11, 12, 14, 15, 16, 17, 18, 19
<223> /mod_base = "2'-hydroxy corresponding nucleoside"

<400> 961
agaaagccag aacaugggat t 21

<210> 962
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 21
<223> /mod_base = "5'-phosphorothioate thymidine"

<220>
<221> modified_base

<222> 1..19
<223> /mod_base = "2'-hydroxy corresponding nucleoside"

<400> 962
aagaauucuc uguuggacut t 21

<210> 963
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 6, 8, 10, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 7, 9, 11, 12, 13, 14, 16, 17, 18, 19
<223> /mod_base = "2'-hydroxy corresponding nucleoside"

<400> 963
aagacuauac auuucagaat t 21

<210> 964
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 9, 12
<223> /mod_base = "2'-O-methyl corresponding nucleoside"

<220>
<221> modified_base
<222> 21

<223> /mod_base = "5'-phosphorothioate thymidine"

<220>
<221> modified_base
<222> 1, 2, 3, 4, 5, 6, 7, 8, 10, 11, 13, 14, 15, 16, 17, 18, 19
<223> /mod_base = "2'-hydroxy corresponding nucleoside"

<400> 964
guaaguguca auaagaagat t 21

<210> 965
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 3, 10, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 8, 9, 11, 12, 14, 15, 16, 17, 18, 19
<223> /mod_base = "2'-hydroxy corresponding nucleoside"

<400> 965
uguaaguguc aauaagaagt t 21

<210> 966
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 7
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 10, 11, 12, 13, 14, 15, 16, 17, 18, 19
<223> /mod_base = "2'-hydroxy corresponding nucleoside"

<400> 966
agaaguuagu cuuuccuuct t 21

<210> 967
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 9
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 10, 11, 12, 13, 14, 15, 16, 17, 18, 19
<223> /mod_base = "2'-hydroxy corresponding nucleoside"

<400> 967
aaagaaguua gucuuuccut t 21

<210> 968
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>

<221> modified_base
<222> 9, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 10, 11, 12, 13, 14, 15, 16, 18, 19
<223> /mod_base = "2'-hydroxy corresponding nucleoside"

<400> 968
uccucugaua cucgccuact t 21

<210> 969
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 8, 13, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 9, 10, 11, 12, 15, 16, 17, 18, 19
<223> /mod_base = "2'-hydroxy corresponding nucleoside"

<400> 969
agaaagccag aacaugggat t 21

<210> 970
<211> 21
<212> DNA
<213> Artificial sequence

<220>
<223> Description of the artificial sequence: antisense strand of dsRNA

<220>
<221> modified_base

<222> 1
<223> /mod_base = "nucleoside: lacks 5'-phosphate group"

<220>
<221> modified_base
<222> 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 13, 15, 16, 17, 18, 19
<223> /mod_base = "2'-hydroxy corresponding nucleoside"

<400> 970
aagaauucuc uguuggacut t 21

<210> 971
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 6, 8, 10, 14, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 7, 9, 11, 12, 13, 16, 17, 18, 19
<223> /mod_base = "2'-hydroxy corresponding nucleoside"

<400> 971
aagacuauac auuucagaat t 21

<210> 972
<211> 21
<212> DNA
<213> Artificial sequence

<220>

<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 9, 12, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 10, 11, 13, 15, 16, 17, 18, 19
<223> /mod_base = "2'-hydroxy corresponding nucleoside"

<400> 972
guaaguguca auaagaagat t 21

<210> 973
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 3, 10, 13, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 8, 9, 11, 12, 15, 16, 17, 18, 19
<223> /mod_base = "2'-hydroxy corresponding nucleoside"

<400> 973
uguaaguguc aauaagaagt t 21

<210> 974
<211> 21

<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 7, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 10, 11, 12, 13, 15, 16, 17, 18, 19
<223> /mod_base = "2'-hydroxy corresponding nucleoside"

<400> 974
agaaguuagu cuuuccuuct t 21

<210> 975
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 9, 14
<223> /mod_base = "2'-O-methyl corresponding nucleoside"

<220>
<221> modified_base
<222> 21
<223> /mod_base = "5'-phosphorothioate thymidine"

<220>
<221> modified-base
<222> 1, 2, 3, 4, 5, 6, 7, 8, 10, 11, 12, 13, 15, 16, 17, 18, 19
<223> /mod_base = "2'-hydroxy corresponding nucleoside"

<400> 975

aaagaaguua gucuuuccut t 21

<210> 976
<211> 21
<212> DNA
<213> Artificial sequence

<220>
<223> Description of the artificial sequence: antisense strand of dsRNA

<220>
<221> modified-base
<222> 1
<223> /mod_base = "nucleoside: lacks 5'-phosphate group"

<220>
<221> modified-base
<222> 9, 14, 17
<223> /mod_base = "2'-O-methyl corresponding nucleoside"

<220>
<221> modified-base
<222> 21
<223> /mod_base = "5'-phosphorothioate thymidine"

<220>
<221> modified-base
<222> 1, 2, 3, 4, 5, 6, 7, 8, 10, 11, 12, 13, 15, 16, 18, 19
<223> /mod_base = "2'-hydroxy corresponding nucleoside"

<400> 976
uccucugaua cucgccuact t 21

<210> 977
<211> 21
<212> DNA
<213> Artificial sequence

<220>
<223> Description of the artificial sequence: antisense strand of dsRNA

<220>
<221> modified-base
<222> 1
<223> /mod_base = "nucleoside: lacks 5'-phosphate group"

<220>
<221> modified-base
<222> 2, 8, 13, 14
<223> /mod_base = "2'-O-methyl corresponding nucleoside"

<220>
<221> modified-base
<222> 21
<223> /mod_base = "5'-phosphorothioate thymidine"

<220>
<221> modified-base

<222> 1, 3, 4, 5, 6, 7, 9, 10, 11, 12, 15, 16, 17, 18, 19
<223> /mod_base = "2'-hydroxy corresponding nucleoside"

<400> 977
agaaagccag aacaugggat t 21

<210> 978
<211> 21
<212> DNA
<213> Artificial sequence

<220>
<223> Description of the artificial sequence: antisense strand of dsRNA

<220>
<221> modified-base
<222> 1
<223> /mod_base = "nucleoside: lacks 5'-phosphate group"

<220>
<221> modified-base
<222> 2, 14
<223> /mod_base = "2'-O-methyl corresponding nucleoside"

<220>
<221> modified-base
<222> 21
<223> /mod_base = "5'-phosphorothioate thymidine"

<220>
<221> modified-base
<222> 1, 3, 4, 5, 6, 7, 8, 9, 10, 11, 12, 13, 15, 16, 17, 18, 19
<223> /mod_base = "2'-hydroxy corresponding nucleoside"

<400> 978
aagaauucuc uguuggacut t 21

<210> 979
<211> 21
<212> DNA
<213> Artificial sequence

<220>
<223> Description of the artificial sequence: antisense strand of dsRNA

<220>
<221> modified-base
<222> 1
<223> /mod_base = "nucleoside: lacks 5'-phosphate group"

<220>
<221> modified-base
<222> 2, 6, 8, 10, 14, 15
<223> /mod_base = "2'-O-methyl corresponding nucleoside"

<220>
<221> modified-base
<222> 21

<223> /mod_base = "5'-phosphorothioate thymidine"

<220>
<221> modified-base
<222> 1, 3, 4, 5, 7, 9, 11, 12, 13, 16, 17, 18, 19
<223> /mod_base = "2'-hydroxy corresponding nucleoside"

<400> 979
aagacuauac auuucagaat t 21

<210> 980
<211> 21
<212> DNA
<213> Artificial sequence

<220>
<223> Description of the artificial sequence: antisense strand of dsRNA

<220>
<221> modified-base
<222> 1
<223> /mod_base = "nucleoside: lacks 5'-phosphate group"

<220>
<221> modified-base
<222> 2, 9, 12, 14
<223> /mod_base = "2'-O-methyl corresponding nucleoside"

<220>
<221> modified-base
<222> 21
<223> /mod_base = "5'-phosphorothioate thymidine"

<220>
<221> modified-base
<222> 1, 3, 4, 5, 6, 7, 8, 10, 11, 13, 15, 16, 17, 18, 19
<223> /mod_base = "2'-hydroxy corresponding nucleoside"

<400> 980
guaaguguca auaagaagat t 21

<210> 981
<211> 21
<212> DNA
<213> Artificial sequence

<220>
<223> Description of the artificial sequence: antisense strand of dsRNA

<220>
<221> modified-base
<222> 1
<223> /mod_base = "nucleoside: lacks 5'-phosphate group"

<220>
<221> modified-base
<222> 2, 3, 10, 13, 14
<223> /mod_base = "2'-O-methyl corresponding nucleoside"

<220>
<221> modified-base
<222> 21
<223> /mod_base = "5'-phosphorothioate thymidine"

<220>
<221> modified-base
<222> 1, 4, 5, 6, 7, 8, 9, 11, 12, 15, 16, 17, 18, 19
<223> /mod_base = "2'-hydroxy corresponding nucleoside"

<400> 981
uguaaguguc aauaagaagt t 21

<210> 982
<211> 21
<212> DNA
<213> Artificial sequence

<220>
<223> Description of the artificial sequence: antisense strand of dsRNA

<220>
<221> modified-base
<222> 1
<223> /mod_base = "nucleoside: lacks 5'-phosphate group"

<220>
<221> modified-base
<222> 2, 7, 14
<223> /mod_base = "2'-O-methyl corresponding nucleoside"

<220>
<221> modified-base
<222> 21
<223> /mod_base = "5'-phosphorothioate thymidine"

<220>
<221> modified-base
<222> 1, 3, 4, 5, 6, 8, 9, 10, 11, 12, 13, 15, 16, 17, 18, 19
<223> /mod_base = "2'-hydroxy corresponding nucleoside"

<400> 982
agaaguuagu cuuuccuuct t 21

<210> 983
<211> 21
<212> DNA
<213> Artificial sequence

<220>
<223> Description of the artificial sequence: antisense strand of dsRNA

<220>
<221> modified-base
<222> 1
<223> /mod_base = "nucleoside: lacks 5'-phosphate group"

<220>

<221> modified-base
<222> 2, 9, 14
<223> /mod_base = "2'-O-methyl corresponding nucleoside"

<220>
<221> modified-base
<222> 21
<223> /mod_base = "5'-phosphorothioate thymidine"

<220>
<221> modified-base
<222> 1, 3, 4, 5, 6, 7, 8, 10, 11, 12, 13, 15, 16, 17, 18, 19
<223> /mod_base = "2'-hydroxy corresponding nucleoside"

<400> 983
aaagaaguua gucuuuccut t 21

<210> 984
<211> 21
<212> DNA
<213> Artificial sequence

<220>
<223> Description of the artificial sequence: antisense strand of dsRNA

<220>
<221> modified-base
<222> 1
<223> /mod_base = "nucleoside: lacks 5'-phosphate group"

<220>
<221> modified-base
<222> 2, 9, 14, 17
<223> /mod_base = "2'-O-methyl corresponding nucleoside"

<220>
<221> modified-base
<222> 21
<223> /mod_base = "5'-phosphorothioate thymidine"

<220>
<221> modified-base
<222> 1, 3, 4, 5, 6, 7, 8, 10, 11, 12, 13, 15, 16, 18, 19
<223> /mod_base = "2'-hydroxy corresponding nucleoside"

<400> 984
uccucugaua cucgccuact t 21

<210> 985
<211> 21
<212> DNA
<213> Artificial sequence

<220>
<223> Description of the artificial sequence: antisense strand of dsRNA

<220>
<221> modified-base

<222> 1
<223> /mod_base = "nucleoside: lacks 5'-phosphate group"

<220>
<221> modified-base
<222> 2, 9, 12
<223> /mod_base = "2'-O-methyl corresponding nucleoside"

<220>
<221> modified-base
<222> 21
<223> /mod_base = "5'-phosphorothioate thymidine"

<220>
<221> modified-base
<222> 1, 3, 4, 5, 6, 7, 8, 10, 11, 13, 14, 15, 16, 17, 18, 19
<223> /mod_base = "2'-hydroxy corresponding nucleoside"

<400> 985
guaaguguca auaagaagat t 21

<210> 986
<211> 41
<212> DNA
<213> Artificial sequence

<220>
<223> Description of the artificial sequence: bDNA probes for human GAPDH

<400> 986
gaatttgcca tgggtggaat tttttctctt ggaaagaaag t 41

<210> 987
<211> 41
<212> DNA
<213> Artificial sequence

<220>
<223> Description of the artificial sequence: bDNA probes for human GAPDH

<400> 987
ggagggatct cgctcctgga tttttctctt ggaaagaaag t 41

<210> 988
<211> 40
<212> DNA
<213> Artificial sequence

<220>
<223> Description of the artificial sequence: bDNA probes for human GAPDH

<400> 988
ccccagcctt ctccatggtt ttttctcttg gaaagaaagt 40

<210> 989
<211> 40
<212> DNA
<213> Artificial sequence

<220>
<223> Description of the artificial sequence: bDNA probes for human GAPDH

<400> 989
gctcccccct gcaaatgagt ttttctcttg gaaagaaagt 40

<210> 990
<211> 42
<212> DNA
<213> Artificial sequence

<220>
<223> Description of the artificial sequence: bDNA probes for human GAPDH

<400> 990
agccttgacg gtgccatgtt tttaggcata ggacccgtgt ct 42

<210> 991
<211> 45
<212> DNA
<213> Artificial sequence

<220>
<223> Description of the artificial sequence: bDNA probes for human GAPDH

<400> 991
gatgacaagc ttcccgttct ctttttaggc ataggacccg tgtct 45

<210> 992
<211> 46
<212> DNA
<213> Artificial sequence

<220>
<223> Description of the artificial sequence: bDNA probes for human GAPDH

<400> 992
agatggtgat gggatttcca tttttttagg cataggaccc gtgtct 46

<210> 993
<211> 44
<212> DNA
<213> Artificial sequence

<220>
<223> Description of the artificial sequence: bDNA probes for human GAPDH

<400> 993
gcatcgcccc acttgatttt tttttaggca taggacccgt gtct 44

<210> 994
<211> 43
<212> DNA
<213> Artificial sequence

<220>
<223> Description of the artificial sequence: bDNA probes for human GAPDH

<400> 994
cacgacgtac tcagcgccat ttttaggcat aggacccgtg tct 43

<210> 995
<211> 46
<212> DNA
<213> Artificial sequence

<220>
<223> Description of the artificial sequence: bDNA probes for human GAPDH

<400> 995
ggcagagatg atgacccttt tgtttttagg cataggaccc gtgtct 46

<210> 996
<211> 21
<212> DNA
<213> Artificial sequence

<220>
<223> Description of the artificial sequence: bDNA probes for human GAPDH

<400> 996
ggtgaagacg ccagtggact c 21

<210> 997
<211> 41
<212> DNA
<213> Artificial sequence

<220>
<223> Description of the artificial sequence: bDNA probes for human RRM2

<400> 997
cgggtttcag ggattcccag tttttctctt ggaaagaaag t 41

<210> 998
<211> 41
<212> DNA
<213> Artificial sequence

<220>
<223> Description of the artificial sequence: bDNA probes for human RRM2

<400> 998
gcttgctgca aagaaagcca tttttctctt ggaaagaaag t 41

<210> 999
<211> 42
<212> DNA
<213> Artificial sequence

<220>
<223> Description of the artificial sequence: bDNA probes for human RRM2

<400> 999
cttcttggct aaatcgctcc atttttctct tggaaagaaa gt 42

<210> 1000
<211> 42
<212> DNA
<213> Artificial sequence

<220>
<223> Description of the artificial sequence: bDNA probes for human RRM2

<400> 1000
agcgggcttc tgtaatctga atttttctct tggaaagaaa gt 42

<210> 1001
<211> 43
<212> DNA
<213> Artificial sequence

<220>
<223> Description of the artificial sequence: bDNA probes for human RRM2

<400> 1001
gagaaattcc ctttctttgg gatttttctc ttggaaagaa agt 43

<210> 1002
<211> 42
<212> DNA
<213> Artificial sequence

<220>
<223> Description of the artificial sequence: bDNA probes for human RRM2

<400> 1002
ggtagcctct ttgtccccaa ttttttctct tggaaagaaa gt 42

<210> 1003
<211> 51
<212> DNA
<213> Artificial sequence

<220>
<223> Description of the artificial sequence: bDNA probes for human RRM2

<400> 1003
gaacatggga tataaaatat ctctcctttt ttaggcatag gacccgtgtc t 51

<210> 1004
<211> 49
<212> DNA
<213> Artificial sequence

<220>
<223> Description of the artificial sequence: bDNA probes for human RRM2

<400> 1004
ccaagttttc atttactatg ccatcttttt aggcatagga cccgtgtct 49

<210> 1005
<211> 49
<212> DNA

<213> Artificial sequence

<220>
<223> Description of the artificial sequence: bDNA probes for human RRM2

<400> 1005
catttcagaa tgtatgtttt ccatgttttt aggcatagga cccgtgtct 49

<210> 1006
<211> 45
<212> DNA
<213> Artificial sequence

<220>
<223> Description of the artificial sequence: bDNA probes for human RRM2

<400> 1006
catcgtttca atggcattga atttttaggc ataggacccg tgtct 45

<210> 1007
<211> 41
<212> DNA
<213> Artificial sequence

<220>
<223> Description of the artificial sequence: bDNA probes for human RRM2

<400> 1007
ccagcgcaag gcccagtttt ttaggcatag gacccgtgtc t 41

<210> 1008
<211> 47
<212> DNA
<213> Artificial sequence

<220>
<223> Description of the artificial sequence: bDNA probes for human RRM2

<400> 1008
aaggctacaa cacgttcacc atatttttag gcataggacc cgtgtct 47

<210> 1009
<211> 43
<212> DNA
<213> Artificial sequence

<220>
<223> Description of the artificial sequence: bDNA probes for human RRM2

<400> 1009
aatgccttcc actgcagcat ttttaggcat aggacccgtg tct 43

<210> 1010
<211> 22
<212> DNA
<213> Artificial sequence

<220>

<223> Description of the artificial sequence: bDNA probes for human RRM2

<400> 1010
gcaatttgga agccatagaa ac 22

<210> 1011
<211> 32
<212> DNA
<213> Artificial sequence

<220>
<223> Description of the artificial sequence: bDNA probes for human RRM2

<400> 1011
tcttttatgt aagtgtcaat aagaagacta ta 32

<210> 1012
<211> 22
<212> DNA
<213> Artificial sequence

<220>
<223> Description of the artificial sequence: bDNA probes for human RRM2

<400> 1012
ctgccttctt cttgacacaa gg 22

<210> 1013
<211> 3284
<212> DNA
<213> Homo sapiens

<220>
<223> cDNA of human RRM2

<300>
<308> NM_001034.3
<309> 2010-09-26

<400> 1013

```
cccgtgcaccctgtcccagccgtcctgtcctggctgctcgctctgcttcgctgcgcctcc 60
actatgctctccctccgtgtcccgctcgcgcccatcacggacccgcagcagctgcagctc 120
tcgccgctgaaggggctcagcttggtcgacaaggagaacacgccgccggccctgagcggg 180
acccgcgtcctggccagcaagaccgcgaggaggatcttccaggagcccacggagccgaaa 240
actaaagcagctgcccccggcgtggaggatgagccgctgctgagagaaaacccccgccgc 300
tttgtcatcttccccatcgagtaccatgatatctggcagatgtataagaaggcagaggct 360
tccttttggaccgccgaggaggtggacctctccaaggacattcagcactgggaatccctg 420
aaacccgaggagagatattttatatcccatgttctggctttctttgcagcaagcgatggc 480
atagtaaatgaaaacttggtggagcgatttagccaagaagttcagattacagaagcccgc 540
tgtttctatggcttccaaattgccatggaaaacatacattctgaaatgtatagtcttctt 600
attgacacttacataaaagatcccaaagaaagggaatttctcttcaatgccattgaaacg 660
atgccttgtgtcaagaagaaggcagactgggccttgcgctggattggggacaaagaggct 720
acctatggtgaacgtgttgtagcctttgctgcagtggaaggcattttcttttccggttct 780
tttgcgtcgatattctggctcaagaaacgaggactgatgcctggcctcacattttctaat 840
gaacttattagcagagatgagggtttacactgtgattttgcttgcctgatgttcaaacac 900
ctggtacacaaaccatcggaggagagagtaagagaaataattatcaatgctgttcggata 960
gaacaggagttcctcactgaggccttgcctgtgaagctcattgggatgaattgcactcta 1020
atgaagcaatacattgagtttgtggcagacagacttatgctggaactgggttttagcaag 1080
gttttcagagtagagaacccatttgactttatggagaatatttcactggaaggaaagact 1140
aacttctttgagaagagagtaggcgagtatcagaggatgggagtgatgtcaagtccaaca 1200
gagaattcttttaccttggatgctgacttctaaatgaactgaagatgtgcccttacttgg 1260
ctgatttttttttccatctcataagaaaaatcagctgaagtgttaccaactagccacac 1320
catgaattgtccgtaatgttcattaacagcatctttaaaactgtgtagctacctcacaac 1380
cagtcctgtctgtttatagtgctggtagtatcacctttgccagaaggcctggctggctg 1440
tgacttaccatagcagtgacaatggcagtcttggctttaaagtgaggggtgaccctttag 1500
tgagcttagcacagcgggattaaacagtcctttaaccagcacagccagttaaaagatgca 1560

gcctcactgcttcaacgcagattttaatgtttacttaaatataaacctggcactttacaa 1620
acaaataaacattgtttgtactcacaaggcgataatagcttgatttatttggtttctaca 1680
ccaaatacattctcctgaccactaatgggagccaattcacaattcactaagtgactaaag 1740
taagttaaacttgtgtagactaagcatgtaattttaagttttattttaatgaattaaaa 1800
tatttgttaaccaactttaaagtcagtcctgtgtatacctagatattagtcagttggtgc 1860
cagatagaagacaggttgtgtttttatcctgtggcttgtgtagtgtcctgggattctctg 1920
ccccctctgagtagagtgttgtgggataaaggaatctctcagggcaaggagcttcttaag 1980
ttaaatcactagaaatttaggggtgatctgggccttcatatgtgtgagaagccgtttcat 2040
tttatttctcactgtattttcctcaacgtctggttgatgagaaaaaattcttgaagagtt 2100
ttcatatgtgggagctaaggtagtattgtaaaatttcaagtcatccttaaacaaaatgat 2160
ccacctaagatcttgcccctgttaagtggtgaaatcaactagaggtggttcctacaagtt 2220
gttcattctagttttgtttggtgtaagtaggttgtgtgagttaattcatttatatttact 2280
atgtctgttaaatcagaaatttttattatctatgttcttctagattttacctgtagttc 2340
atacttcagtcacccagtgtcttattctggcattgtctaaatctgagcattgtctagggg 2400
gatcttaaactttagtaggaaaccatgagctgttaatacagtttccattcaaatattaat 2460
ttcagaatgaaacataatttttttttttttttgagatggagtctcgctctgttgccca 2520
ggctggagtgcagtggcgcgattttggctcactgtaacctccatctcctgggttcaagca 2580
attctcctgtctcagcctccctagtagctgggactgcaggtatgtgctaccacacctggc 2640
taatttttgtatttttagtagagatggagtttcaccatattggtcaggctggtcttgaac 2700
tcctgacctcaggtgatccacccacctcggcctcccaaagtgctgggattgcaggcgtga 2760
taaacaaatattcttaatagggctactttgaattaatctgcctttatgtttgggagaaga 2820
aagctgagacattgcatgaaagatgatgagagataaatgttgatcttttggccccatttg 2880
ttaattgtattcagtatttgaacgtcgtcctgtttattgttagttttcttcatcatttat 2940
tgtatagacaattttaaatctctgtaatatgatacatttcctatcttttaagttattg 3000
ttacctaaagttaatccagattatatggtccttatatgtgtacaacattaaaatgaaagg 3060
ctttgtcttgcattgtgaggtacaggcggaagttggaatcaggttttaggattctgtctc 3120
tcattagctgaataatgtgaggattaacttctgccagctcagaccatttcctaatcagtt 3180
gaaagggaaacaagtatttcagtctcaaaattgaataatgcacaagtcttaagtgattaa 3240
aataaaactgttcttatgtcagtttcaaaaaaaaaaaaaaaaa                 3284
```

<210> 1014
<211> 3452
<212> DNA

<213> Homo sapiens

<220>
<223> cDNA of human RRM2

<300>
<308> NM_001165931.1
<309> 2009-09-26

<400> 1014

```
aaaatcgcgcgcggccccgcggccagcctgggtaggggcaaggcgcagccaatgggaagg 60
gtcggaggcatggcacagccaatgggaagggccggggcaccaaagccaatgggaagggcc 120
gggagcgcgcggcgcgggagatttaaaggctgctggagtgaggggtcgcccgtgcaccct 180
gtcccagccgtcctgtcctggctgctcgctctgcttcgctgcgcctccactatgctctcc 240
ctccgtgtcccgctcgcgcccatcacggacccgcagcagctgcagctctcgccgctgaag 300
gggctcagcttggtcgacaaggagaacacgccgccggccctgagcgggacccgcgtcctg 360
gccagcaagaccgcgaggaggatcttccaggagcccacggagccgaaaactaaagcagct 420
gcccccggcgtggaggatgagccgctgctgagagaaaacccccgccgctttgtcatcttc 480
cccatcgagtaccatgatatctggcagatgtataagaaggcagaggcttccttttggacc 540
gccgaggaggtggacctctccaaggacattcagcactgggaatccctgaaacccgaggag 600
agatattttatatcccatgttctggctttctttgcagcaagcgatggcatagtaaatgaa 660
aacttggtggagcgatttagccaagaagttcagattacagaagcccgctgtttctatggc 720
ttccaaattgccatggaaaacatacattctgaaatgtatagtcttcttattgacacttac 780
ataaaagatcccaaagaaagggaatttctcttcaatgccattgaaacgatgccttgtgtc 840
aagaagaaggcagactgggccttgcgctggattggggacaaagaggctacctatggtgaa 900
cgtgttgtagcctttgctgcagtggaaggcattttcttttccggttcttttgcgtcgata 960
ttctggctcaagaaacgaggactgatgcctggcctcacattttctaatgaacttattagc 1020
agagatgagggtttacactgtgattttgcttgcctgatgttcaaacacctggtacacaaa 1080
ccatcggaggagagagtaagagaaataattatcaatgctgttcggatagaacaggagttc 1140
```

```
ctcactgaggccttgcctgtgaagctcattgggatgaattgcactctaatgaagcaatac 1200
attgagtttgtggcagacagacttatgctggaactgggttttagcaaggttttcagagta 1260
gagaacccatttgactttatggagaatatttcactggaaggaaagactaacttctttgag 1320
aagagagtaggcgagtatcagaggatgggagtgatgtcaagtccaacagagaattctttt 1380
accttggatgctgacttctaaatgaactgaagatgtgcccttacttggctgatttttttt 1440
ttccatctcataagaaaaatcagctgaagtgttaccaactagccacaccatgaattgtcc 1500
gtaatgttcattaacagcatctttaaaactgtgtagctacctcacaaccagtcctgtctg 1560
tttatagtgctggtagtatcacctttgccagaaggcctggctggctgtgacttaccata 1620
gcagtgacaatggcagtcttggctttaaagtgaggggtgaccctttagtgagcttagcac 1680
agcgggattaaacagtcctttaaccagcacagccagttaaaagatgcagcctcactgctt 1740
caacgcagatttaatgtttacttaaatataaacctggcactttacaaacaaataaacat 1800
tgtttgtactcacaaggcgataatagcttgatttatttggtttctacaccaaatacattc 1860
tcctgaccactaatgggagccaattcacaattcactaagtgactaaagtaagttaaactt 1920
gtgtagactaagcatgtaatttttaagttttattttaatgaattaaaatatttgttaacc 1980
aactttaaagtcagtcctgtgtatacctagatattagtcagttggtgccagatagaagac 2040
aggttgtgtttttatcctgtggcttgtgtagtgtcctgggattctctgccccctctgagt 2100
agagtgttgtgggataaaggaatctctcagggcaaggagcttcttaagttaaatcactag 2160
aaatttaggggtgatctgggccttcatatgtgtgagaagccgtttcattttatttctcac 2220
tgtattttcctcaacgtctggttgatgagaaaaaattcttgaagagttttcatatgtggg 2280
agctaaggtagtattgtaaaatttcaagtcatccttaaacaaaatgatccacctaagatc 2340
ttgcccctgttaagtggtgaaatcaactagaggtggttcctacaagttgttcattctagt 2400
tttgtttggtgtaagtaggttgtgtgagttaattcatttatatttactatgtctgttaaa 2460
tcagaaatttttattatctatgttcttctagattttacctgtagttcatacttcagtca 2520
cccagtgtcttattctggcattgtctaaatctgagcattgtctagggggatcttaaactt 2580
tagtaggaaaccatgagctgttaatacagtttccattcaaatattaatttcagaatgaaa 2640
cataattttttttttttttttgagatggagtctcgctctgttgcccaggctggagtgca 2700
gtggcgcgattttggctcactgtaacctccatctcctgggttcaagcaattctcctgtct 2760
cagcctccctagtagctgggactgcaggtatgtgctaccacacctggctaattttttgtat 2820
ttttagtagagatggagtttcaccatattggtcaggctggtcttgaactcctgacctcag 2880
gtgatccacccacctcggcctcccaaagtgctgggattgcaggcgtgataaacaaatatt 2940
cttaatagggctactttgaattaatctgcctttatgtttgggagaagaaagctgagacat 3000
tgcatgaaagatgatgagagataaatgttgatcttttggccccatttgttaattgtattc 3060
agtatttgaacgtcgtcctgtttattgttagtttcttcatcatttattgtatagacaat 3120
ttttaaatctctgtaatatgatacattttcctatcttttaagttattgttacctaaagtt 3180
aatccagattatatggtccttatatgtgtacaacattaaaatgaaaggctttgtcttgca 3240
ttgtgaggtacaggcggaagttggaatcaggttttaggattctgtctctcattagctgaa 3300
taatgtgaggattaacttctgccagctcagaccatttcctaatcagttgaaagggaaaca 3360
agtatttcagtctcaaaattgaataatgcacaagtcttaagtgattaaaataaaactgtt 3420
cttatgtcagtttcaaaaaaaaaaaaaaaaaa                                  3452
```

<210> 1015
<211> 3262
<212> DNA
<213> Macaca fascicularis

<220>
<223> cDNA of cynomolgous monkey RRM2

<220>
<221> modified-base
<222> 2354, 2355, 2356, 2357, 2358, 2359, 2360, 2361, 2362, 2363, 2364, 2365, 2366, 2367, 2368, 2369, 2370, 2371
<223> /mod_base = "unknown nucleotide"

<400> 1015

```
aaaatcgcgcgctgtcccgcggccagcctgggtggggtcaaggtgcagccaatggaaggg 60
tcgggggcacggcacagccaatgggaagggccggggcgccaaagcgaatgggaagggccg 120
gcacgggagatttaaaggctgctggaccgaggggtcgcccgtgcttcgcgtcccagccat 180
cctgttctggcctgtcgctgtacttcgctgcgccgccactatgctctccgtccgcatccc 240
gctcgcgcccatcacgaacccgcagcagctgcagctctcgccgctgaaggggctaagcct 300
ggtcgacaaggagaacacgccgccagccctgagcggggcccgcgtcctggccagcaagac 360
cgcgaggaggatcttccaggagcccgcggagccgaaaactaaagcagctgccccccggcgt 420

ggaggatgaaccgctgctgagagaaaccccccgccgctttgtcatcttccccatcgagta 480
ccatgatatctggcagatgtataagaaggcggaggcttccttctggactgccgaggaggt 540
ggacctgtccaaggacattcagcactgggaatccctgaagcccgaggagagatattttat 600
atcccatgttctggctttctttgcagcaagtgatggcatagtaaatgaaaacttggtgga 660
gcgatttagccaagaagttcagattacagaagcccgctgtttctatggcttccaaattgc 720
catggaaaacatacattctgaaatgtatagtcttcttattgacacttacataaaagatcc 780
caaagaaagggaatttctcttcaatgccattgaaacgatgccttgtgtcaagaagaaggc 840
agattgggccttgcgctggattggggacaaagaggctacctatggtgaacgtgtcgtagc 900
ctttgccgcagtggaaggcatcttcttttccggttcttttgcatcgatattctggctcaa 960
gaaacgaggactgatgcctggcctcacattttccaatgaacttatcagcagagatgaggg 1020
tttacactgtgattttgcttgcctgatgttcaaacacctggtacacaaaccatcagagga 1080
gagagtaagagaaataattatcaatgctgttcggatagaacaggagttcctcactgaggc 1140
cttgcctgtgaagctaattgggatgaattgcactctaatgaagcaatacattgagtttgt 1200
ggcagacagacttatgctggaactgggttttagcaaggttttcagagtagagaacccatt 1260
tgactttatggagaatatttcactggaaggaaagactaacttctttgagaagagagtagg 1320
cgagtatcagaggatgggagtgatgtcaagtccaacagagaattcttttaccttggatgc 1380
tgacttctaaatgaactgaagatatgcccttattttgctgattttttttcccatgtcata 1440
aagaaaatcagctgaagtgttaccaactagcaacaccgtgaattgtccataatgttcatt 1500
aacagcatctttaaaactgtgtagctacctcacaaccagtcctgtttatttatagtgctg 1560
gtagtatcaccttttgccagcaggcctggctggctgtgacttaccatagcagtgacaatg 1620
gcagtcttggctttaaagtgaggggtgaccctttagtgagcttggcacagcaggattaaa 1680
cagtcttttaaccagcacagccaattgaaagaagcagcctcactgcttcaacgcacgttt 1740
taatgtttacttaaatataaaactggcactttacaaacaaataaacattgtttgtactca 1800
caaggtaataatagcttgatttatttggtttctacaccaaatacaaagcattctgaccac 1860
taatgggagccaattcacagttcactaagtgactaaagtaagttaaacttgtgtagacta 1920
agcatgtaatttctaagttttattttaatggattgaaatactcattaaccaactttaaag 1980
tcagtcccgtgtatagctagatattagtctgttggtgccagatagaagacaggttgtgtt 2040
tttatcctgtggcttgggtagtgtcctgggattctctgcgccatctgagtagtgttgtgg 2100
gttaaaggaatctctcaggacaaggagcttcttaagttaaatcattagaaatttaggcat 2160
gatctgggccttcatatgtgtaagaagccatttcatcttatttctcactgtattttcctc 2220
aacttctagttgataaaaaattcttgaagagttttcatatgtgggatctaaggtagtact 2280
gtaaaatttcaagtcatccttaaacaaagtgacccacctaagatcttgcccctgttaagt 2340
ggtgaaatcaactnnnnnnnnnnnnnnnnnnnnttgttgtttattctagttttgtttgtaag 2400
taggttgtgtgagttaattcatttatatttactatgtcttttttttttttttttttttt 2460
tgagacggagtctcgctctgttgcccaggctagagtgcagtagtgcgatttcggctcact 2520
gcaacctccgcctcctgggctcaagcaattctcctgtctcagcctcctgagtagctgaaa 2580
ctgcaggtatgtgccaccacacctggctaatttttgtatttttagtagagacggagtttc 2640
actatattggtcaggctggtcttgaactcctgacctcaggtgatccgcccacctgggcct 2700
cccaaagtgctgggattacaggcatgataaacaaatattcttaatagggctactttgaat 2760
taatttgcctttatgtttgggagaagaaagctgagacattgcaagaaagatgatgagaga 2820
taaatgttgatcttttggccccatttgttcattgtattcgctatttgaacattgtcctgt 2880
tctattgttagtttcttcttcatttattgtatagtcaattttaaatctctgtaatatg 2940
atacattttcctatcttaagttattgttacctaaagttaatccagattacattgtcctta 3000
tacttgtacaacattaaaatgaaaggctttgctttgcattgtgaggttcaggcggaagtt 3060
ggaatcaggttttagggttctgtgtctcattagctgaataatgtgaggattaacttctgc 3120
caactcagaccatttcctaatcagttgaaagggaaacaagtatttcaatctcaaaattga 3180
ataatgcacaagtgttaagtgattaaaataaaactgttcttatgtcagtttcttgattgg 3240
taaaatttgcattttaattcag                                        3262
```